# EUROPÄISCHE PATENTANMELDUNG

(11) **EP 2 366 799 A2**
(43) Veröffentlichungstag der Anmeldung: **21.09.2011**
(21) Anmeldenummer: 10195110.1
(22) Anmeldetag: 31.03.2004
(51) Int. Cl.: C12Q 1/68

(54) **Verfahren zur in vitro Erkennung schwerer Sepsis**

(30) Priorität: 02.04.2003 DE 10315031; 08.08.2003 DE 10336511; 02.09.2003 DE 10340395
(62) Teilanmeldung aus: 04724591.5
(71) Anmelder: SIRS-Lab GmbH, 07745 Jena (DE)
(72) Erfinder: Russwurm, Stefan, 07743 Jena (DE); Deigner, Hans-Peter, 68623 Lampertheim (DE); Reinhart, Konrad, 07443 Jena (DE)
(74) Vertreter: Winter, Brandl, Fürniss, Hübner, Röss, Kaiser, Polte - Partnerschaft

(57) **Zusammenfassung**

Die vorliegende Erfindung betrifft ein Verfahren zur in vitro Erkennung von schwerer Sepsis mittels eines Genexpressionsprofils aus bis zu 130 für schwere Sepsis spezifischen DNA-Sequenzen und Fragmenten davon.

SEQUENCE LISTING

<110>  SIRS-Lab GmbH

<120>  Verfahren zur Erkennung schwerer Sepsis
<130>  SL06029

<140>  Teilanmeldung aus EP04724591.5 (SL0518-  SIRS P5-EP)
<141>  2010-12-13

<160>  10540

<170>  PatentIn version 3.1

<210> 1
<211>  494
<212>  DNA
<213>  Homo sapiens


<220>
<223>  II.1 (XM_086400)

<400>  1
gagcagcctt cctgagagag gagagagaaa gctcagggag gtctggagca aagatactcc      60

tggaggtggg gagtgaggca gggataagga aggagagtat cctccagcac cttccagtgg     120

gtggggcaag tccgtgggca tcatgttgac cgagctggag aaagccttga actctatcat     180

cgacgtctac cacaagtact ccctgataaa ggggaatttc catgccgtct acagggatga     240

cctgaagaaa ttgctagaga ccgagtgtcc tcagtatatc aggaaaaagg gtgcagacgt     300

ctggttcaaa gagttggata tcaacactga tggtgcagtt aacttccagg agttcctcat     360

tctggtgata aagatgggcg tggcagccca caaaaaaagc catgaagaaa gccacaaaga     420

gtagctgagt tactgggccc agaggctggg cccctggaca tgtacctgca gaataataaa     480

gtcatcaata cctc                                                       494


<210>  2
<211>  466
<212>  DNA
<213>  Homo sapiens


<220>
<223>  II.2 (XM_001682)

<400>  2
accactgctg gctttttgct gtagctccac attcctgtgc attgaggggt taacattagg      60

ctgggaagat gacaaaactt gaagagcatc tggagggaat tgtcaatatc ttccaccaat     120

actcagttcg gaaggggcat tttgacaccc tctctaaggg tgagctgaag cagctgctta     180

caaaggagct tgcaaacacc atcaagaata tcaaagataa agctgtcatt gatgaaatat     240

tccaaggcct ggatgctaat caagatgaac aggtcgactt tcaagaattc atatccctgg     300

tagccattgc gctgaaggct gcccattacc acacccacaa agagtaggta gctctctgaa     360

ggctttttac ccagcaatgt cctcaatgag ggtcttttct ttccctcacc aaaacccagc     420

cttgcccgtg gggagtaaga gttaataaac acactcacga aaagtt                    466

<210>  3
<211>  439
<212>  DNA
<213>  Homo sapiens


<220>
<223>  II.3 (NM_002619)

<400>  3
ccgcagcatg agctccgcag ccgggttctg cgcctcacgc cccgggctgc tgttcctggg      60

gttgctgctc ctgccacttg tggtcgcctt cgccagcgct gaagctgaag aagatgggga     120

cctgcagtgc ctgtgtgtga agaccacctc ccaggtccgt cccaggcaca tcaccagcct     180

ggaggtgatc aaggccggac cccactgccc cactgcccaa ctgatagcca cgctgaagaa     240

tggaaggaaa atttgcttgg acctgcaagc cccgctgtac aagaaaataa ttaagaaact     300

tttggagagt tagctactag ctgcctacgt gtgtgcattt gctatatagc atacttcttt     360

tttccagttt caatctaact gtgaaagaaa cttctgatat ttgtgttatc cttatgattt     420

taaataaaca aaataaatc                                                  439


<210>  4
<211>  673
<212>  DNA
<213>  Homo sapiens


<220>
<223>  II.4 (NM_002704)

<400>  4
gggcaactca ccctcactca gaggtcttct ggttctggaa acaactctag ctcagccttc      60

tccaccatga gcctcagact tgataccacc ccttcctgta acagtgcgag accacttcat     120

gccttgcagg tgctgctgct tctgtcattg ctgctgactg ctctggcttc ctccaccaaa     180

ggacaaacta agagaaactt ggcgaaaggc aaagaggaaa gtctagacag tgacttgtat     240

gctgaactcc gctgcatgtg tataaagaca acctctggaa ttcatcccaa aaacatccaa     300

agtttggaag tgatcgggaa aggaacccat tgcaaccaag tcgaagtgat agccacactg     360

aaggatggga ggaaaatctg cctggaccca gatgctccca gaatcaagaa aattgtacag     420

aaaaaattgg caggtgatga atctgctgat taatttgttc tgtttctgcc aaacttcttt     480

aactcccagg aagggtagaa ttttgaaacc ttgattttct agagttctca tttattcagg     540

atacctattc ttactgtatt aaaatttgga tatgtgtttc attctgtctc aaaaatcaca     600

ttttattctg agaaggttgg ttaaaagatg gcagaaagaa gatgaaaata aataagcctg     660

gtttcaaccc tct                                                        673


<210>  5
<211>  1793
<212>  DNA
<213>  Homo sapiens

<220>
<223>  II.5 (NM_001101)

<400>  5
   cgcgtccgcc ccgcgagcac agagcctcgc ctttgccgat ccgccgcccg tccacacccg      60
   ccgccagctc accatggatg atgatatcgc cgcgctcgtc gtcgacaacg gctccggcat     120
   gtgcaaggcc ggcttcgcgg gcgacgatgc cccccgggcc gtcttcccct ccatcgtggg     180
   gcgccccagg caccagggcg tgatggtggg catgggtcag aaggattcct atgtgggcga     240
   cgaggcccag agcaagagag gcatcctcac cctgaagtac cccatcgagc acggcatcgt     300
   caccaactgg gacgacatgg agaaaatctg gcaccacacc ttctacaatg agctgcgtgt     360
   ggctcccgag gagcaccccg tgctgctgac cgaggccccc ctgaacccca aggccaaccg     420
   cgagaagatg acccagatca tgtttgagac cttcaacacc ccagccatgt acgttgctat     480
   ccaggctgtg ctatccctgt acgcctctgg ccgtaccact ggcatcgtga tggactccgg     540
   tgacggggtc acccacactg tgcccatcta cgaggggtat gccctccccc atgccatcct     600
   gcgtctggac ctggctggcc gggacctgac tgactacctc atgaagatcc tcaccgagcg     660
   cggctacagc ttcaccacca cggccgagcg ggaaatcgtg cgtgacatta aggagaagct     720
   gtgctacgtc gccctggact tcgagcaaga gatggccacg gctgcttcca gctcctccct     780
   ggagaagagc tacgagctgc ctgacggcca ggtcatcacc attggcaatg agcggttccg     840
   ctgccctgag gcactcttcc agccttcctt cctgggcatg gagtcctgtg gcatccacga     900
   aactaccttc aactccatca tgaagtgtga cgtggacatc cgcaaagacc tgtacgccaa     960
   cacagtgctg tctggcggca ccaccatgta ccctggcatt gccgacagga tgcagaagga    1020
   gatcactgcc ctggcaccca gcacaatgaa gatcaagatc attgctcctc ctgagcgcaa    1080
   gtactccgtg tggatcggcg gctccatcct ggcctcgctg tccaccttcc agcagatgtg    1140
   gatcagcaag caggagtatg acgagtccgg cccctccatc gtccaccgca aatgcttcta    1200
   ggcggactat gacttagttg cgttacaccc tttcttgaca aaacctaact tgcgcagaaa    1260
   acaagatgag attggcatgg ctttatttgt tttttttgtt ttgttttggt tttttttttt    1320
   tttttggctt gactcaggat ttaaaaactg gaacggtgaa ggtgacagca gtcggttgga    1380
   gcgagcatcc cccaaagttc acaatgtggc cgaggacttt gattgcacat tgttgttttt    1440
   ttaatagtca ttccaaatat gagatgcatt gttacaggaa gtcccttgcc atcctaaaag    1500
   ccaccccact tctctctaag gagaatggcc cagtcctctc ccaagtccac acaggggagg    1560
   tgatagcatt gctttcgtgt aaattatgta atgcaaaatt tttttaatct tcgccttaat    1620
   acttttttat tttgttttat tttgaatgat gagccttcgt gccccccctt cccccttttt    1680
   gtcccccaac ttgagatgta tgaaggcttt tggtctccct gggagtgggt ggaggcagcc    1740
   agggcttacc tgtacactga cttgagacca gttgaataaa agtgcacacc tta          1793

<210>  6

<211>  691
<212>  DNA
<213>  Homo sapiens


<220>
<223>  II.6 (NM_001013)

<400>  6
cccgcgcagg cgcagacggg gaagcggagc caacatgcca gtggcccgga gctgggtttg      60

tcgcaaaact tatgtgaccc cgcggagacc cttcgagaaa tctcgtctcg accaagagct     120

gaagctgatc ggcgagtatg ggctccggaa caaacgtgag gtctggaggg tcaaatttac     180

cctggccaag atccgcaagg ccgcccggga actgctgacg cttgatgaga aggacccacg     240

gcgtctgttc gaaggcaacg ccctgctgcg gcggctggtc cgcattgggg tgctggatga     300

gggcaagatg aagctggatt acatcctggg cctgaagata gaggatttct tagagagacg     360

cctgcagacc caggtcttca agctgggctt ggccaagtcc atccaccacg ctcgcgtgct     420

gatccgccag cgccatatca gggtccgcaa gcaggtggtg aacatcccgt ccttcattgt     480

ccgcctggat tcccagaagc acatcgactt ctctctgcgc tctccctacg ggggtggccg      540

cccgggccgc gtgaagagga agaatgccaa gaagggccag ggtggggctg gggctggaga      600

cgacgaggag gaggattaag tccacctgtc cctcctgggc tgctggattg tctcgttttc     660

ctgccaaata aacaggatca gcgctttaaa a                                    691


<210>  7
<211>  3144
<212>  DNA
<213>  Homo sapiens


<220>
<223>  II.7 (XM_057445)

<400>  7
tgggcagaag gcagaaaacc agcagagtca cagaggagat ggccaactgc caaatagcca      60

tcttgtacca gagattccag agagtggtct ttggaatttc ccaactcctt tgcttcagtg     120

ccctgatctc tggaactaac aaaccagaaa gaagtggcag catggactta tcattacagc     180

acaaaagcat actcatggaa tatttcccgt aaatactgcc agaatcgcta cacagactta     240

gtggccatcc agaataaaaa tgaaattgat tacctcaata aggtcctacc ctactacagc     300

tcctactact ggattgggat ccgaaagaac aataagacat ggacatgggt gggaaccaaa     360

aaggctctca ccaacgaggc tgagaactgg gctgataatg aacctaacaa caaaaggaac     420

aacgaggact gcgtggagat atacatcaag agtccgtcag cccctggcaa gtggaatgat     480

gagcactgct tgaagaaaaa gcacgcattg tgttacacag cctcctgcca ggacatgtcc     540

tgcagcaaac aaggagagtg cctcgagacc atcgggaact acacctgctc ctgttaccct     600

ggattctatg ggccagaatg tgaatacggt gagagagtgt ggagaacttg agctccctca     660

acacgtgctc atgaactgca gccaccctct gggaaacttc tcttttaact cgcagtgcag     720

cttccactgc actgacgggt accaagtaaa tgggcccagc aagctggaat gcttggcttc 780
tggaatctgg acaaataagc ctccacagtg tttagctgcc cagtgcccac ccctgaagat 840
tcctgaacga ggaaacatga cctgccttca ttctgcaaaa gcattccagc atcagtctag 900
ctgcagcttc agttgtgaag agggatttgc attagttgga ccggaagtgg tgcaatgcac 960
agcctcgggg gtatggacag ccccagcccc agtgtgtaaa gctgtgcagt gtcagcacct 1020
ggaagccccc agtgaaggaa ccatggactg tgttcatccg ctcactgctt ttgcctatgg 1080
ctccagctgt aaatttgagt gccagcccgg ctacagagtg aggggcttgg acatgctccg 1140
ctgcattgac tctggacact ggtctgcacc cttgccaacc tgtgaggcta tttcgtgtga 1200
gccgctggag agtcctgtcc acggaagcat ggattgctct ccatccttga gagcgtttca 1260
gtatgacacc aactgtagct tccgctgtgc tgaaggtttc atgctgagag gagccgatat 1320
agttcggtgt gataacttgg gacagtggac agcaccagcc ccagtctgtc aagctttgca 1380
gtgccaggat ctcccagttc caaatgaggc ccgggtgaac tgctcccacc ccttcggtgc 1440
ctttaggtac cagtcagtct gcagcttcac ctgcaatgaa ggcttgctcc tggtgggagc 1500
aagtgtgcta cagtgcttgg ctactggaaa ctggaattct gttcctccag aatgccaagc 1560
cattccctgc acacctttgc taagccctca gaatggaaca atgacctgtg ttcaacctct 1620
tggaagttcc agttataaat ccacatgtca attcatctgt gacgagggat attctttgtc 1680
tggaccagaa agattggatt gtactcgatc gggacgctgg acagactccc caccaatgtg 1740
tgaagccatc aagtgcccag aactctttgc cccagagcag ggcagcctgg attgttctga 1800
cactcgtgga gaattcaatg ttggctccac ctgccatttc tcttgtgaca acggctttaa 1860
gctggagggg cccaataatg tggaatgcac aacttctgga agatggtcag ctactccacc 1920
aacctgcaaa ggcatagcat cacttcctac tccaggggtg caatgtccag ccctcaccac 1980
tcctgggcag ggaaccatgt actgtaggca tcatccggga acctttggtt ttaataccac 2040
ttgttacttt ggctgcaacg ctggattcac actcatagga gacagcactc tcagctgcag 2100
accttcagga caatggacag cagtaactcc agcatgcaga gctgtgaaat gctcagaact 2160
acatgttaat aagccaatag cgatgaactg ctccaacctc tggggaaact tcagttatgg 2220
atcaatctgc tctttccatt gtctagaggg ccagttactt aatggctctg cacaaacagc 2280
atgccaagag aatggccact ggtcaactac cgtgccaacc tgccaagcag gaccattgac 2340
tatccaggaa gccctgactt actttggtgg agcggtggct tctacgatag gtctgataat 2400
gggtgggacg ctcctggctt tgctaagaaa gcgtttcaga caaaaagatg atgggaaatg 2460
ccccttgaat cctcacagcc acctaggaac atatggagtt tttacaaacg ctgcatttga 2520
cccgagtcct taaggtttcc ataaacaccc atgaatcaaa gacatggaat taccttagat 2580
tagctctgga ccagcctgtt ggacccgctc tggaccaacc ctgtttcctg agtttgggat 2640
tgtggtacaa tctcaaattc tcaacctacc accccttcct gtcccacctc ttctcttcct 2700
gtaacacaag ccacagaagc caggagcaaa tgtttctgca gtagtctctg tgctttgact 2760

cacctgttac ttgaaatacc agtgaaccaa agagactgga gcatctgact cacaagaaga 2820

ccagactgtg gagaaataaa aatacctctt tattttttga ttgaaggaag gttttctcca 2880

ctttgttgga aagcaggtgg catctctaat tggaagaaat tcctgtagca tcttctggag 2940

tctccagtgg ttgctgttga tgaggcctct tggacctctg ctctgaggct tccagagagt 3000

cctctggatg gcaccagagg ctgcagaagg ccaagaatca agctagaagg ccacatgtca 3060

ccgtggacct tcctgccacc agtcactgtc cctcaaatga cccaaagacc aatattcaaa 3120

tgcgtaatta aaagaatttt cccc 3144

<210> 8
<211> 944
<212> DNA
<213> Homo sapiens

<220>
<223> II.8 (BC018761)

<400> 8
gcaagatggt gttgcagacc caggtcttca tttctctgtt gctctggatc tctggtgtct 60

acggggacat cgtgatgacc cagtctccag actccctggc tgtgtctctg ggcgagaggg 120

ccaccatcaa ctgcaagtcc agccagactg ttttatacag ctccaacaat aagaactact 180

tagcttggta ccagcagaaa ccaggacagc ctcctaagct gctcatttac tgggcatcta 240

cccgggaatc cggggtccct gaccgattca gtggcagcgg gtctgggaca gatttcactc 300

tcaccatcag cagcctgcag gctgaagatg tggcagttta ttactgtcag caatattata 360

ctactcctat cactttcggc cctgggaccc aagtggatat caaacgaact gtggctgcac 420

catctgtctt catcttcccg ccatctgatg agcagttgaa atctggaact gcctctgttg 480

tgtgcctgct gaataacttc tatcccagag aggccaaagt acagtggaag gtggataacg 540

ccctccaatc gggtaactcc caggagagtg tcacagagca ggacagcaag gacagcacct 600

acagcctcag cagcaccctg acgctgagca aagcagacta cgagaaacac aaagtctacg 660

cctgcgaagt cacccatcag ggcctgagct cgcccgtcac aaagagcttc aacaggggag 720

agtgttagag ggagaagtgc ccccacctgc tcctcagttc cagcctgacc ccctcccatc 780

ctttggcctc tgaccctttt tccacagggg acctacccct attgcggtcc tccagctcat 840

ctttcacctc acccccctcc tcctccttgg ctttaattat gctaatgttg gaggagaatg 900

aataaataaa gtgaatcttt gcaaaaaaaa aaaaaaaaaa aaaa 944

<210> 9
<211> 1762
<212> DNA
<213> Homo sapiens

<220>
<223> II.9 (XM_030906)

<400> 9
   acacggcgtc cctcaggcgc ccccattccg gaccagccct cgggagtcgc cgacccggcc 60
   tcccgcaaag acttttcccc agacctcggg cgcaccccct gcacgccgcc ttcatccccg 120
   gcctgtctcc tgagcccccg cgcatcctag accctttctc ctccaggaga cggatctctc 180
   tccgacctgc cacagatccc ctattcaaga ccacccacct tctggtacca gatcgcgccc 240
   atctaggtta tttccgtggg atactgagac acccccggtc caagcctccc ctccaccact 300
   gcgcccttct ccctgaggac ctcagctttc cctcgaggcc ctcctacctt ttgccgggag 360
   acccccagcc cctgcagggg cggggcctcc ccaccacacc agccctgttc gcgctctcgg 420
   cagtgccggg gggcgccgcc tcccccatgc cgccctccgg gctgcggctg ctgccgctgc 480
   tgctaccgct gctgtggcta ctggtgctga cgcctggccg gccggccgcg ggactatcca 540
   cctgcaagac tatcgacatg gagctggtga agcggaagcg catcgaggcc atccgcggcc 600
   agatcctgtc caagctgcgg ctcgccagcc ccccgagcca gggggaggtg ccgcccggcc 660
   cgctgcccga ggccgtgctc gccctgtaca acagcacccg cgaccgggtg gccggggaga 720
   gtgcagaacc ggagcccgag cctgaggccg actactacgc caaggaggtc acccgcgtgc 780
   taatggtgga aacccacaac gaaatctatg acaagttcaa gcagagtaca cacagcatat 840
   atatgttctt caacacatca gagctccgag aagcggtacc tgaacccgtg ttgctctccc 900
   gggcagagct gcgtctgctg aggctcaagt taaaagtgga gcagcacgtg gagctgtacc 960
   agaaatacag caacaattcc tggcgatacc tcagcaaccg gctgctggca cccagcgact 1020
   cgccagagtg gttatctttt gatgtcaccg gagttgtgcg gcagtggttg agccgtggag 1080
   gggaaattga gggctttcgc cttagcgccc actgctcctg tgacagcagg gataacacac 1140
   tgcaagtgga catcaacggg ttcactaccg gccgccgagg tgacctggcc accattcatg 1200
   gcatgaaccg gcctttcctg cttctcatgg ccaccccgct ggagagggcc cagcatctgc 1260
   aaagctcccg gcaccgccga gccctggaca ccaactattg cttcagctcc acggagaaga 1320
   actgctgcgt gcggcagctg tacattgact tccgcaagga cctcggctgg aagtggatcc 1380
   acgagcccaa gggctaccat gccaacttct gcctcgggcc ctgcccctac atttggagcc 1440
   tggacacgca gtacagcaag gtcctggccc tgtacaacca gcataacccg ggcgcctcgg 1500
   cggcgccgtg ctgcgtgccg caggcgctgg agccgctgcc catcgtgtac tacgtgggcc 1560
   gcaagcccaa ggtggagcag ctgtccaaca tgatcgtgcg ctcctgcaag tgcagctgag 1620
   gtcccgcccc gccccgcccc gccccggcag gcccggcccc accccgcccc gcccccgctg 1680
   ccttgcccat gggggctgta tttaaggaca cccgtgcccc aagcccacct ggggccccat 1740
   taaagatgga gagaggactg cg 1762

<210> 10
<211> 2095
<212> DNA
<213> Homo sapiens

<220>

<223>  II.10 (NM_001760)

<400>  10
gcgcctgtca gggaagcggc gcgcgcgcgc gggcggcggg cgggctgggg atccgccgcg      60

cagtgccagc gccagcgcca gacccgcgcc ccgcgctctc cggcccgtcg cctgccttgg     120

gactcgcgag cccgcactcc cgccctgcct gttcgctgcc cgagtatgga gctgctgtgt     180

tgcgaaggca cccggcacgc gccccgggcc gggccggacc cgcggctgct gggggaccag     240

cgtgtcctgc agagcctgct ccgcctggag gagcgctacg taccccgcgc ctcctacttc     300

cagtgcgtgc agcgggagat caagccgcac atgcggaaga tgctggctta ctggatgctg     360

gaggtatgtg aggagcagcg ctgtgaggag gaagtcttcc ccctggccat gaactacctg     420

gatcgctacc tgtcttgcgt ccccacccga aaggcgcagt tgcagctcct gggtgcggtc     480

tgcatgctgc tggcctccaa gctgcgcgag accacgcccc tgaccatcga aaaactgtgc     540

atctacaccg accacgctgt ctctccccgc cagttgcggg actgggaggt gctggtccta     600

gggaagctca agtgggacct ggctgctgtg attgcacatg atttcctggc cttcattctg     660

caccggctct ctctgccccg tgaccgacag gccttggtca aaaagcatgc ccagaccttt     720

ttggccctct gtgctacaga ttataccttt gccatgtacc cgccatccat gatcgccacg     780

ggcagcattg gggctgcagt gcaaggcctg ggtgcctgct ccatgtccgg ggatgagctc     840

acagagctgc tggcagggat cactggcact gaagtggact gcctgcgggc ctgtcaggag     900

cagatcgaag ctgcactcag ggagagcctc agggaagcct ctcagaccag ctccagccca     960

gcgcccaaag ccccccgggg ctccagcagc caagggccca gccagaccag cactcctaca    1020

gatgtcacag ccatacacct gtagccctgg agaggccctc tggagtggcc actaagcaga    1080

ggagggggcg ctgccaccca cctccctgcc tccaggaacc acaccacatc taagcctgaa    1140

ggggcgtctg ttcccccttc acaaagccca agggatctgg tcctacccat ccccgcagtg    1200

tgcactaagg ggcccggcca gccatgtctg catttcggtg gctagtcaag ctcctcctcc    1260

ctgcatctga ccagcagcgc ctttcccaac tctagctggg ggtgggccag gctgatggga    1320

cagaattgga tacatacacc agcattcctt ttgaacgccc ccccccaccc ctgggggctc    1380

tcatgttttc aactgccaaa atgctctagt gccttctaaa ggtgttgtcc cttctagggt    1440

tattgcattt ggattggggt ccctctaaaa tttaatgcat gatagacaca tatgaggggg    1500

aatagtctag atggctcctc tcagtacttt ggaggcccct atgtagtccg tgctgacagc    1560

tgctcctaga gggaggggcc taggcctcag ccagagaagc tataaattcc tctttgcttt    1620

gctttctgct cagcttctcc tgtgtgattg acagctttgc tgctgaaggc tcattttaat    1680

ttattaattg ctttgagcac aactttaaga ggacataatg ggggcctggc catcccacaa    1740

gtggtggtaa ccctggtggt tgctgttttc ctcccttctg ctactggcaa aaggatcttt    1800

gtggccaagg agctgctata gcctggggtg gggtcatgcc ctcctctccc attgtccctc    1860

tgccccatcc tccagcaggg aaaatgcagc agggatgccc tggaggtggc tgagcccctg    1920

tctagagagg gaggcaagcc ctgttgacac aggtctttcc taaggctgca aggtttaggc    1980

tggtggccca ggaccatcat cctactgtaa taaagatgat tgtgaaataa aactggcttt 2040

ggcttcttgg aaaaaaaaaa aaaaaaaaaa aaaaaaaaaa aaaaaaaaaa aaaaa 2095

<210> 11
<211> 1185
<212> DNA
<213> Homo sapiens

<220>
<223> II.11 (XM_035922)

<400> 11
gctcagggca catgcctccc ctccccaggc cgcggcccag ctgaccctcg gggctccccc 60

ggcagcggac agggaagggt taaaggcccc cggctccctg ccccctgccc tggggaaccc 120

ctggccctgt ggggacatga actgtgtttg ccgcctggtc ctggtcgtgc tgagcctgtg 180

gccagataca gctgtcgccc ctgggccacc acctggcccc cctcgagttt ccccagaccc 240

tcgggccgag ctggacagca ccgtgctcct gacccgctct ctcctggcgg acacgcggca 300

gctggctgca cagctgaggg acaaattccc agctgacggg gaccacaacc tggattccct 360

gcccaccctg gccatgagtg cgggggcact gggagctcta cagctcccag gtgtgctgac 420

aaggctgcga gcggacctac tgtcctacct gcggcacgtg cagtggctgc gccgggcagg 480

tggctcttcc ctgaagaccc tggagcccga gctgggcacc ctgcaggccc gactggaccg 540

gctgctgcgc cggctgcagc tcctgatgtc ccgcctggcc ctgccccagc cacccccgga 600

cccgccggcg cccccgctgg cgcccccctc ctcagcctgg gggggcatca gggccgccca 660

cgccatcctg ggggggctgc acctgacact tgactgggcc gtgaggggac tgctgctgct 720

gaagactcgg ctgtgacccg gggcccaaag ccaccaccgt ccttccaaag ccagatctta 780

tttatttatt tatttcagta ctgggggcga aacagccagg tgatcccccc gccattatct 840

ccccctagtt agagacagtc cttccgtgag gcctgggggg catctgtgcc ttatttatac 900

ttatttattt caggagcagg ggtgggaggc aggtggactc ctgggtcccc gaggaggagg 960

ggactggggt cccggattct tgggtctcca agaagtctgt ccacagactt ctgccctggc 1020

tcttccccat ctaggcctgg gcaggaacat atattattta tttaagcaat tacttttcat 1080

gttggggtgg ggacggaggg gaaagggaag cctgggtttt tgtacaaaaa tgtgagaaac 1140

ctttgtgaga cagagaacag ggaattaaat gtgtcataca tatcc 1185

<210> 12
<211> 2617
<212> DNA
<213> Homo sapiens

<220>
<223> II.12 (XM_039625)

<400> 12
ggattcttat tggcccaacc cgccgtcggt cggcggcgag tccgccccct cagaacctta 60

catttacagt gtagcaaaag agaaagtaac tatgttgctg ctggagatca atgaagccga 120
gtgaatgggg gctgaatgtg cgagtccata gctgaagagg agcgccagat ggtggaggaa 180
tacacttatt tatgaaactg tcttgagttc ttcttgaatt gccagttttc agcctcctca 240
tgcctccgtc tcctttagac gacagggtag tagtggcact atctaggccc gtccgacctc 300
aggatctcaa cctttgttta gactctagtt accttggctc tgccaaccca ggcagtaaca 360
gccaccctcc tgtcatcgcc accaccgttg tgtccctcaa ggctgcgaat ctgacgtata 420
tgccctcatc cagcggctct gcccgctcgc tgaattgtgg atgcagcagt gccagctgct 480
gcactgtggc aacctacgac aaggacaatc aggcccaaac ccaagccatt gccgctggca 540
ccaccaccac tgccatcgga acctctacca cctgccctgc taaccagatg gtcaacaata 600
atgagaatac aggctctcta agtccatcaa gtggggtggg cagccctgtg tcagggaccc 660
ccaagcagct agccagcatc aaaataatct accccaatga cttggcaaag aagatgacca 720
aatgcagcaa gagtcacctg ccgagtcagg gccctgtcat cattgactgc aggcccttca 780
tggagtacaa caagagtcac atccaaggag ctgtccacat taactgtgcc gataagatca 840
gccggcggag actgcagcag ggcaagatca ctgtcctaga cttgatttcc tgtagggaag 900
gcaaggactc tttcaagagg atcttttcca aagaaattat agtttatgat gagaatacca 960
atgaaccaag ccgagtgatg ccctcccagc cacttcacat agtcctcgag tccctgaaga 1020
gagaaggcaa agaacctctg gtgttgaaag gtggacttag tagttttaag cagaaccatg 1080
aaaacctctg tgacaactcc ctccagctcc aagagtgccg ggaggtgggg ggcggcgcat 1140
ccgcggcctc gagcttgcta cctcagccca tccccaccac ccctgacatc gagaacgctg 1200
agctcacccc catcttgccc ttcctgttcc ttggcaatga gcaggatgct caggacctgg 1260
acaccatgca gcggctgaac atcggctacg tcatcaacgt caccactcat cttcccctct 1320
accactatga gaaaggcctg ttcaactaca agcggctgcc agccactgac agcaacaagc 1380
agaacctgcg gcagtacttt gaagaggctt ttgagttcat tgaggaagct caccagtgtg 1440
ggaaggggct tctcatccac tgccaggctg gggtgtcccg ctccgccacc atcgtcatcg 1500
cttacttgat gaagcacact cggatgacca tgactgatgc ttataaattt gtcaaaggca 1560
aacgaccaat tatctcccca aaccttaact tcatggggca gttgctagag ttcgaggaag 1620
acctaaacaa cggtgtgaca ccgagaatcc ttacaccaaa gctgatgggc gtggagacgg 1680
ttgtgtgaca atggtctgga tggaaaggat tgctgctctc cattaggaga caatgaggaa 1740
ggaggatgga ttctggtttt ttttctttct tttttttttt gtagttggga gtaagtttgt 1800
gaatggaaac aaacttgttt aaacacttta tttttaacaa gtgtaagaag actataactt 1860
ttgatgccat tgagattcac ctcccacaaa ctgacaaatt aaggaggtta aagaagtaat 1920
ttttttaagc caacaataaa aatataatac aacttgtttc tccccctttt ccttttaagc 1980
tatttgtaga gtttatgact aaatagtctg tgcaggttca tagaccgaag atactacaca 2040
ctttaaacca attaaaaaga accaaaagta aatagaaaag acattgaatc accaaggcct 2100

gggatcaacc tgggctgtcc acacagaaaa caaaaaccca accaaaccaa gccctgttgt 2160

gctcactggt gcaaagagaa gatcagggca gcttaagtgg tctaagaatc cttcaggcat 2220

tctttaagga gaaaaaggat acctttgatt ttgtgtgttt catgctctgg attttttttt 2280

tttttccttc tctgggttta agagattttt tttgaaatag tgaggaactg accattatat 2340

gccttcactg gcttcttgtg caataatatg atgttttaag tgtgcaaaca agttagagct 2400

ggcagctgaa tgatagacaa atagtgcaaa tttgccagct tggagataga aaggaattca 2460

acaatatatc aaatactttc cttcccacct tttccttttt tttttttttt ttctgatttg 2520

attctggtta cagtgccata aaccttgtta catatgtata tcagaatgta agaaaaaaaa 2580

atttatttaa aaatattttt cgcaaaaaaa aaaaaaa 2617

<210> 13
<211> 1195
<212> DNA
<213> Homo sapiens

<220>
<223> II.13 (XM_002762)

<400> 13
cactgctctg agaatttgtg agcagcccct aacaggctgt tacttcacta caactgacga 60

tatgatcatc ttaatttact tatttctctt gctatgggaa gacactcaag gatggggatt 120

caaggatgga atttttcata actccatatg gcttgaacga gcagccggtg tgtaccacag 180

agaagcacgg tctggcaaat acaagctcac ctacgcagaa gctaaggcgg tgtgtgaatt 240

tgaaggcggc catctcgcaa cttacaagca gctagaggca gccagaaaaa ttggatttca 300

tgtctgtgct gctggatgga tggctaaggg cagagttgga taccccattg tgaagccagg 360

gcccaactgt ggatttggaa aaactggcat tattgattat ggaatccgtc tcaataggag 420

tgaaagatgg gatgcctatt gctacaaccc acacgcaaag gagtgtggtg gcgtctttac 480

agatccaaag caaattttta aatctccagg cttcccaaat gagtacgaag ataaccaaat 540

ctgctactgg cacattagac tcaagtatgg tcagcgtatt cacctgagtt ttttagattt 600

tgaccttgaa gatgacccag gttgcttggc tgattatgtt gaaatatatg acagttacga 660

tgatgtccat ggctttgtgg gaagatactg tggagatgag cttccagatg acatcatcag 720

tacaggaaat gtcatgacct tgaagtttct aagtgatgct tcagtgacag ctggaggttt 780

ccaaatcaaa tatgttgcaa tggatcctgt atccaaatcc agtcaaggaa aaaatacaag 840

gagaaaaaaa cacagttgtt actcggtaat gagggatgtt gggactcaga cgatacccta 900

aagtatgaca ctttgggata ctgagtactt tgagctaaag gagactggaa ggcctcagaa 960

gcaagatctc tgtgaccttc tccttccatc ttccctcttg ccccaagtct tcctcttcca 1020

aagcaagtca taaaaaccag aattcaccgg gcgcagtggc tcactcctgt aatcccatca 1080

ctttggaagg ccgaggcaga ggatctcttg agctcaggag ttcaaaacca ggctgggcaa 1140

catagcaaga cctcatctct atgaaaaaaa ttttaaatta aaaaattaaa aatta 1195

<210> 14
<211> 873
<212> DNA
<213> Homo sapiens

<220>
<223> II.14 (XM_015396)

<400> 14
acttcccctt cctgtacagg gcaggttgtg cagctggagg cagagcagtc ctctctgggg 60

agcctgaagc aaacatggat caagaaactg taggcaatgt tgtcctgttg gccatcgtca 120

ccctcatcag cgtggtccag aatggattct ttgcccataa agtggagcac gaaagcagga 180

cccagaatgg gaggagcttc cagaggaccg gaacacttgc ctttgagcgg gtctacactg 240

ccaaccagaa ctgtgtagat gcgtacccca ctttcctcgc tgtgctctgg tctgcggggc 300

tactttgcag ccaagttcct gctgcgtttg ctggactgat gtacttgttt gtgaggcaaa 360

agtactttgt cggttaccta ggagagagaa cgcagagcac ccctggctac atatttggga 420

aacgcatcat actcttcctg ttcctcatgt ccgttgctgg catattcaac tattacctca 480

tcttcttttt cggaagtgac tttgaaaact acataaagac gatctccacc accatctccc 540

ctctacttct cattccctaa ctctctgctg aatatggggt tggtgttctc atctaatcaa 600

tacctacaag tcatcataat tcagctcttg agagcattct gctcttcttt agatggctgt 660

aaatctattg gccatctggg cttcacagct tgagttaacc ttgcttttcc gggaacaaaa 720

tgatgtcatg tcagctccgc cccttgaaca tgaccgtggc cccaaatttg ctattcccat 780

gcattttgtt tgtttcttca cttatcctgt tctctgaaga tgttttgtga ccaggtttgt 840

gttttcttaa aataaaatgc agagacatgt ttt 873

<210> 15
<211> 1124
<212> DNA
<213> Homo sapiens

<220>
<223> II.15 (NM_003823)

<400> 15
tccgcaggcg gaccgggggc aaaggaggtg gcatgtcggt caggcacagc agggtcctgt 60

gtccgcgctg agccgcgctc tccctgctcc agcaaggacc atgagggcgc tggaggggcc 120

aggcctgtcg ctgctgtgcc tggtgttggc gctgcctgcc ctgctgccgg tgccggctgt 180

acgcggagtg gcagaaacac ccacctaccc ctggcgggac gcagagacag gggagcggct 240

ggtgtgcgcc cagtgccccc caggcacctt tgtgcagcgg ccgtgccgcc gagacagccc 300

cacgacgtgt ggcccgtgtc caccgcgcca ctacacgcag ttctggaact acctggagcg 360

ctgccgctac tgcaacgtcc tctgcgggga gcgtgaggag gaggcacggg cttgccacgc 420

cacccacaac cgtgcctgcc gctgccgcac cggcttcttc gcgcacgctg gtttctgctt 480

ggagcacgca tcgtgtccac ctggtgccgg cgtgattgcc ccgggcaccc ccagccagaa 540

cacgcagtgc cagccgtgcc ccccaggcac cttctcagcc agcagctcca gctcagagca 600

gtgccagccc caccgcaact gcacggccct gggcctggcc ctcaatgtgc caggctcttc 660

ctcccatgac accctgtgca ccagctgcac tggcttcccc ctcagcacca gggtaccagg 720

agctgaggag tgtgagcgtg ccgtcatcga ctttgtggct ttccaggaca tctccatcaa 780

gaggctgcag cggctgctgc aggccctcga ggccccggag ggctggggtc cgacaccaag 840

ggcgggccgc gcggccttgc agctgaagct gcgtcggcgg ctcacggagc tcctgggggc 900

gcaggacggg gcgctgctgg tgcggctgct gcaggcgctg cgcgtggcca ggatgcccgg 960

gctggagcgg agcgtccgtg agcgcttcct ccctgtgcac tgatcctggc cccctcttat 1020

ttattctaca tccttggcac cccacttgca ctgaaagagg ctttttttta aatagaagaa 1080

atgaggtttc ttaaagctta tttttataaa gctttttcat aaaa 1124

<210> 16
<211> 3410
<212> DNA
<213> Homo sapiens

<220>
<223> II.16 (XM_029300)

<400> 16
cgcgcgtctc cgccgcccgc ggtgacttct gcctgcgctc cttctctgaa cgctcacttc 60

cgaggagacg ccgacgatga agacaccgtg gaaggttctt ctgggactgc tgggtgctgc 120

tgcgcttgtc accatcatca ccgtgcccgt ggttctgctg aacaaaggca cagatgatgc 180

tacagctgac agtcgcaaaa cttacactct aactgattac ttaaaaaata cttatagact 240

gaagttatac tccttaagat ggatttcaga tcatgaatat ctctacaaac aagaaaataa 300

tatcttggta ttcaatgctg aatatggaaa cagctcagtt ttcttggaga acagtacatt 360

tgatgagttt ggacattcta tcaatgatta ttcaatatct cctgatgggc agtttattct 420

cttagaatac aactacgtga agcaatggag gcattcctac acagcttcat atgacattta 480

tgatttaaat aaaaggcagc tgattacaga agagaggatt ccaaacaaca cacagtgggt 540

cacatggtca ccagtgggtc ataaattggc atatgtttgg aacaatgaca tttatgttaa 600

aattgaacca aatttaccaa gttacagaat cacatggacg gggaaagaag atataatata 660

taatggaata actgactggg tttatgaaga ggaagtcttc agtgcctact ctgctctgtg 720

gtggtctcca aacggcactt ttttagcata tgcccaattt aacgacacag aagtcccact 780

tattgaatac tccttctact ctgatgagtc actgcagtac ccaaagactg tacgggttcc 840

atatccaaag gcaggagctg tgaatccaac tgtaaagttc tttgttgtaa atacagactc 900

tctcagctca gtcaccaatg caacttccat acaaatcact gctcctgctt ctatgttgat 960

aggggatcac tacttgtgtg atgtgacatg ggcaacacaa gaaagaattt ctttgcagtg 1020

gctcaggagg attcagaact attcggtcat ggatatttgt gactatgatg aatccagtgg 1080
aagatggaac tgcttagtgg cacggcaaca cattgaaatg agtactactg gctgggttgg 1140
aagatttagg ccttcagaac ctcattttac ccttgatggt aatagcttct acaagatcat 1200
cagcaatgaa gaaggttaca gacacatttg ctatttccaa atagataaaa aagactgcac 1260
atttattaca aaaggcacct gggaagtcat cgggatagaa gctctaacca gtgattatct 1320
atactacatt agtaatgaat ataaaggaat gccaggagga aggaatcttt ataaaatcca 1380
acttagtgac tatacaaaag tgacatgcct cagttgtgag ctgaatccgg aaaggtgtca 1440
gtactattct gtgtcattca gtaaagaggc gaagtattat cagctgagat gttccggtcc 1500
tggtctgccc ctctatactc tacacagcag cgtgaatgat aaagggctga gagtcctgga 1560
agacaattca gctttggata aaatgctgca gaatgtccag atgccctcca aaaaactgga 1620
cttcattatt ttgaatgaaa caaaattttg gtatcagatg atcttgcctc ctcattttga 1680
taaatccaag aaatatcctc tactattaga tgtgtatgca ggcccatgta gtcaaaaagc 1740
agacactgtc ttcagactga actgggccac ttaccttgca agcacagaaa acattatagt 1800
agctagcttt gatggcagag gaagtggtta ccaaggagat aagatcatgc atgcaatcaa 1860
cagaagactg ggaacatttg aagttgaaga tcaaattgaa gcagccagac aattttcaaa 1920
aatgggattt gtggacaaca aacgaattgc aatttggggc tggtcatatg gagggtacgt 1980
aacctcaatg gtcctgggat cgggaagtgg cgtgttcaag tgtggaatag ccgtggcgcc 2040
tgtatcccgg tgggagtact atgactcagt gtacacagaa cgttacatgg gtctcccaac 2100
tccagaagac aaccttgacc attacagaat tcaacagtca tgagcagagc tgaaaatttt 2160
aaacaagttg agtacctcct tattcatgga acagcagatg ataacgttca ctttcagcag 2220
tcagctcaga tctccaaagc cctggtcgat gttggagtgg atttccaggc aatgtggtat 2280
actgatgaag accatggaat agctagcagc acagcacacc aacatatata tacccacatg 2340
agccacttca taaaacaatg tttctcttta ccttagcacc tcaaaatacc atgccattta 2400
aagcttatta aaactcattt ttgttttcat tatctcaaaa ctgcactgtc aagatgatga 2460
tgatctttaa aatacacact caaatcaaga aacttaaggt tacctttgtt cccaaatttc 2520
atacctatca tcttaagtag ggacttctgt cttcacaaca gattattacc ttacagaagt 2580
ttgaattatc cggtcgggtt ttattgttta aaatcatttc tgcatcagct gctgaaacaa 2640
caaataggaa ttgtttttat ggaggctttg catagattcc ctgagcagga ttttaatctt 2700
tttctaactg gactggttca aatgttgttc tcttctttaa agggatggca agatgtgggc 2760
agtgatgtca ctagggcagg gacaggataa gagggattag ggagagaaga tagcagggca 2820
tggctgggaa cccaagtcca agcataccaa cacgagcagg ctactgtcag ctcccctcgg 2880
agaagagctg ttcacagcca gactggcaca gttttctgag aaagactatt caaacagtct 2940
caggaaatca aatatgcaaa gcactgactt ctaagtaaaa ccacagcagt tgaaaagact 3000
ccaaagaaat gtaagggaaa ctgccagcaa cgcaggcccc caggtgccag ttatggctat 3060

aggtgctaca aaaacacagc aagggtgatg ggaaagcatt gtaaatgtgc ttttaaaaaa 3120

aaatactgat gttcctagtg aaagaggcag cttgaaactg agatgtgaac acatcagctt 3180

gccctgttaa aagatgaaaa tatttgtatc acaaatctta acttgaagga gtccttgcat 3240

caatttttct tatttcattt ctttgagtgt cttaattaaa agaatatttt aacttccttg 3300

gactcatttt aaaaaatgga acataaaata caatgttatg tattattatt cccattctac 3360

atactatgga atttctccca gtcatttaat aaatgtgcct tcattttttc 3410

<210> 17
<211> 1145
<212> DNA
<213> Homo sapiens

<220>
<223> II.17 (NM_001562)

<400> 17
attctctccc cagcttgctg agccctttgc tcccctggcg actgcctgga cagtcagcaa 60

ggaattgtct cccagtgcat tttgccctcc tggctgccaa ctctggctgc taaagcggct 120

gccacctgct gcagtctaca cagcttcggg aagaggaaag gaacctcaga ccttccagat 180

cgcttcctct cgcaacaaac tatttgtcgc aggaataaag atggctgctg aaccagtaga 240

agacaattgc atcaactttg tggcaatgaa atttattgac aatacgcttt actttatagc 300

tgaagatgat gaaaacctgg aatcagatta ctttggcaag cttgaatcta aattatcagt 360

cataagaaat ttgaatgacc aagttctctt cattgaccaa ggaaatcggc ctctatttga 420

agatatgact gattctgact gtagagataa tgcaccccgg accatattta ttataagtat 480

gtataaagat agccagccta gaggtatggc tgtaactatc tctgtgaagt gtgagaaaat 540

ttcaactctc tcctgtgaga acaaaattat ttcctttaag gaaatgaatc ctcctgataa 600

catcaaggat acaaaaagtg acatcatatt ctttcagaga agtgtcccag gacatgataa 660

taagatgcaa tttgaatctt catcatacga aggatacttt ctagcttgtg aaaaagagag 720

agaccttttt aaactcattt tgaaaaaaga ggatgaattg ggggatagat ctataatgtt 780

cactgttcaa aacgaagact agctattaaa atttcatgcc gggcgcagtg gctcacgcct 840

gtaatcccag ccctttggga ggctgaggcg ggcagatcac cagaggtcag gtgttcaaga 900

ccagcctgac caacatggtg aaacctcatc tctactaaaa atacaaaaaa ttagctgagt 960

gtagtgacgc atgccctcaa tcccagctac tcaagaggct gaggcaggag aatcacttgc 1020

actccggagg tagaggttgt ggtgagccga gattgcacca ttgcgctcta gcctgggcaa 1080

caacagcaaa actccatctc aaaaaataaa ataaataaat aaacaaataa aaaattcata 1140

atgtg 1145

<210> 18
<211> 1735
<212> DNA

<213>  Homo sapiens

<220>
<223>  II.18 (NM_005037)

<400>  18
cactcggagc ccgagcccga gccgcagccg ccgcctgggg cgcttgggtc ggcctcgagg      60
acaccggaga ggggcgccac gccgccgtgg ccgcagaaat gaccatggtt gacacagaga     120
tgccattctg gcccaccaac tttgggatca gctccgtgga tctctccgta atggaagacc     180
actcccactc ctttgatatc aagcccttca ctactgttga cttctccagc atttctactc     240
cacattacga agacattcca ttcacaagaa cagatccagt ggttgcagat tacaagtatg     300
acctgaaact tcaagagtac caaagtgcaa tcaaagtgga gcctgcatct ccaccttatt     360
attctgagaa gactcagctc tacaataagc ctcatgaaga gccttccaac tccctcatgg     420
caattgaatg tcgtgtctgt ggagataaag cttctggatt tcactatgga gttcatgctt     480
gtgaaggatg caagggtttc ttccggagaa caatcagatt gaagcttatc tatgacagat     540
gtgatcttaa ctgtcggatc cacaaaaaaa gtagaaataa atgtcagtac tgtcggtttc     600
agaaatgcct tgcagtgggg atgtctcata atgccatcag gtttgggcgg atgccacagg     660
ccgagaagga gaagctgttg gcggagatct ccagtgatat cgaccagctg aatccagagt     720
ccgctgacct ccgggccctg gcaaaacatt tgtatgactc atacataaag tccttcccgc     780
tgaccaaagc aaaggcgagg gcgatcttga caggaaagac aacagacaaa tcaccattcg     840
ttatctatga catgaattcc ttaatgatgg gagaagataa aatcaagttc aaacacatca     900
cccccctgca ggagcagagc aaagaggtgg ccatccgcat ctttcagggc tgccagtttc     960
gctccgtgga ggctgtgcag gagatcacag agtatgccaa aagcattcct ggttttgtaa    1020
atcttgactt gaacgaccaa gtaactctcc tcaaatatgg agtccacgag atcatttaca    1080
caatgctggc ctccttgatg aataaagatg gggttctcat atccgagggc caaggcttca    1140
tgacaaggga gtttctaaag agcctgcgaa agccttttgg tgactttatg gagcccaagt    1200
ttgagtttgc tgtgaagttc aatgcactgg aattagatga cagcgacttg gcaatattta    1260
ttgctgtcat tattctcagt ggagaccgcc caggtttgct gaatgtgaag cccattgaag    1320
acattcaaga caacctgcta caagccctgg agctccagct gaagctgaac caccctgagt    1380
cctcacagct gtttgccaag ctgctccaga aaatgacaga cctcagacag attgtcacgg    1440
aacacgtgca gctactgcag gtgatcaaga agacggagac agacatgagt cttcacccgc    1500
tcctgcagga gatctacaag gacttgtact agcagagagt cctgagccac tgccaacatt    1560
tcccttcttc cagttgcact attctgaggg aaaatctgac acctaagaaa tttactgtga    1620
aaaagcattt taaaaagaaa aggttttaga atatgatcta ttttatgcat attgtttata    1680
aagacacatt tacaatttac ttttaatatt aaaaattacc atattatgaa attgc         1735

<210>  19
<211>  1406

<212>  DNA
<213>  Homo sapiens


<220>
<223>  II.19 (M90746)

<400>  19
tttttcatct ctccacttct cctaataggt ttggcagtgt caaccatctc atcattctct      60

ccacctgggt accaagtctc tttctgcttg gtgatggtac tcctttttgc agtggacaca     120

ggactatatt tctctgtgaa gacaaacatt tgaagctcaa caagagactg gaaggaccat     180

aaacttaaat ggagaaagga ccctcaagac aaatgacccc catcccatgg gagtaataag     240

agcagtggca gcagcatctc tgaacatttc tctggatttg caaccccatc atcctcaggc     300

ctctctacaa gcagcaggaa acatagaact cagagccaga tcctttatcc aactctcgat     360

ttttccttgg tctccagtgg aagggaaaag cccatgatct tcaagcaggg aagccccagt     420

gagtagctgc attcctagaa attgaagttt cagagctaca caaacacttt ttctgtccca     480

accattccct cacagtaaaa caacaataca ggctagggat ggtaatcctt taaacataca     540

aaaattgctc gtattataaa ttacccagtt tagaccggaa aaaagaaaat aattattcct     600

aaacaaatgg ataagtagaa ttaatgattg aggcaggacc ctacagagtg tgggaactgc     660

tggggatcta gagaattcag tgggaccaat gaaagcatgg ctgagaaata gcagggtagt     720

ccaggagagt ctaagggagg tgttcccatc tgagcccaga gataagggtg tcttcctaga     780

acattagccg tagtggaatt aacaggaaat catgagggtg acgtagaatt gagtcttcca     840

ggggactcta tcagaactgg accatttcca agtatataac gatgagccct ctaatgctag     900

gagtagcaaa tggtcctagg aaggggactg aggattgggg tgggggtggg gtggaaaaga     960

aagtacagaa caaaccctgt gtcactgtcc caagttaagc taagtgaaca gaactatctc    1020

agcatcagaa tgagaaagcc tgagaagaaa gaaccaacca caagcacaca ggaaggaaag    1080

cgcaggaggt gaaaatgctt tcttggccag ggtagtaaga attagaggtt aatgcaggga    1140

ctgtaaaacc accttttctg cttcaatgtc tagttcctgt atagctttgt tcattgcatt    1200

tattaaacaa atgttgtata accaatacta aatgtactac tgagcttcac tgagttacgc    1260

tgtgaaactt tcaaatcctt cttcatgtca gttccaatga ggtggggatg gagaagacaa    1320

ttgttgctta tgaaaaaaag ctttagctgt ctctgttttg taagctttca gtgcaacatt    1380

tcttggttcc aataaagcat tttaca                                         1406


<210>  20
<211>  3757
<212>  DNA
<213>  Homo sapiens


<220>
<223>  II.20 (NM_001315)

<400>  20
ggaaccgcga ccactggagc cttagcgggc gcagcagctg gaacgggagt actgcgacgc      60

agcccggagt cggccttgta ggggcgaagg tgcagggaga tcgcggcggg cgcagtcttg 120
agcgccggag cgcgtccctg cccttagcgg ggcttgcccc agtcgcaggg gcacatccag 180
ccgctgcggc tgacagcagc cgcgcgcgcg ggagtctgcg gggtcgcggc agccgcacct 240
gcgcgggcga ccagcgcaag gtccccgccc ggctgggcgg gcagcaaggg ccggggagag 300
ggtgcgggtg caggcggggg ccccacaggg ccaccttctt gcccggcggc tgccgctgga 360
aaatgtctca ggagaggccc acgttctacc ggcaggagct gaacaagaca atctgggagg 420
tgcccgagcg ttaccagaac ctgtctccag tgggctctgg cgcctatggc tctgtgtgtg 480
ctgcttttga cacaaaaacg gggttacgtg tggcagtgaa gaagctctcc agaccatttc 540
agtccatcat tcatgcgaaa agaacctaca gagaactgcg gttacttaaa catatgaaac 600
atgaaaatgt gattggtctg ttggacgttt ttacacctgc aaggtctctg gaggaattca 660
atgatgtgta tctggtgacc catctcatgg gggcagatct gaacaacatt gtgaaatgtc 720
agaagcttac agatgaccat gttcagttcc ttatctacca aattctccga ggtctaaagt 780
atatacattc agctgacata attcacaggg acctaaaacc tagtaatcta gctgtgaatg 840
aagactgtga gctgaagatt ctggattttg gactggctcg gcacacagat gatgaaatga 900
caggctacgt ggccactagg tggtacaggg ctcctgagat catgctgaac tggatgcatt 960
acaaccagac agttgatatt tggtcagtgg gatgcataat ggccgagctg ttgactggaa 1020
gaacattgtt tcctggtaca gaccatatta accagcttca gcagattatg cgtctgacag 1080
gaacaccccc cgcttatctc attaacagga tgccaagcca tgaggcaaga aactatattc 1140
agtctttgac tcagatgccg aagatgaact ttgcgaatgt atttattggt gccaatcccc 1200
tggctgtcga cttgctggag aagatgcttg tattggactc agataagaga attacagcgg 1260
cccaagccct tgcacatgcc tactttgctc agtaccacga tcctgatgat gaaccagtgg 1320
ccgatcctta tgatcagtcc tttgaaagca gggacctcct tatagatgag tggaaaagcc 1380
tgacctatga tgaagtcatc agctttgtgc caccacccct tgaccaagaa gagatggagt 1440
cctgagcacc tggtttctgt tctgttgatc ccacttcact gtgaggggaa ggccttttca 1500
cgggaactct ccaaatatta ttcaagtgcc tcttgttgca gagatttcct ccatggtgga 1560
agggggtgtg cgtgcgtgtg cgtgcgtgtt agtgtgtgtg catgtgtgtg tctgtctttg 1620
tgggagggta agacaatatg aacaaactat gatcacagtg actttacagg aggttgtgga 1680
tgctccaggg cagcctccac cttgctcttc tttctgagag ttggctcagg cagacaagag 1740
ctgctgtcct tttaggaata tgttcaatgc aaagtaaaaa aatatgaatt gtccccaatc 1800
ccggtcatgc ttttgccact ttggcttctc ctgtgacccc accttgacgg tggggcgtag 1860
acttgacaac atcccacagt ggcacggaga gaaggcccat accttctggt tgcttcagac 1920
ctgacaccgt ccctcagtga tacgtacagc caaaaaggac caactggctt ctgtgcacta 1980
gcctgtgatt aacttgctta gtatggttct cagatcttga cagtatattt gaaactgtaa 2040
atatgtttgt gccttaaaag gagagaagaa agtgtagata gttaaaagac tgcagctgct 2100

gaagttctga gccgggcaag tcgagagggc tgttggacag ctgcttgtgg gcccggagta 2160

atcaggcagc cttcataggc ggtcatgtgt gcatgtgagc acatgcgtat atgtgcgtct 2220

ctctttctcc ctcaccccca ggtgttgcca tttctctgct tacccttcac ctttggtgca 2280

gaggtttctt gaatatctgc cccagtagtc agaagcaggt tcttgatgtc atgtacttcc 2340

tgtgtactct ttatttctag cagagtgagg atgtgttttg cacgtcttgc tatttgagca 2400

tgcacagctg cttgtcctgc tctcttcagg aggccctggt gtcaggcagg tttgccagtg 2460

aagacttctt gggtagttta gatcccatgt cacctcagct gatattatgg caagtgatat 2520

cacctctctt cagcccctag tgctattctg tgttgaacac aattgatact tcaggtgctt 2580

ttgatgtgaa aatcatgaaa agaggaacag gtggatgtat agcattttta ttcatgccat 2640

ctgttttcaa ccaactattt ttgaggaatt atcatgggaa aagaccaggg cttttcccag 2700

gaatatccca aacttcggaa acaagttatt ctcttcactc ccaataacta atgctaagaa 2760

atgctgaaaa tcaaagtaaa aaattaaagc ccataaggcc agaaactcct tttgctgtct 2820

ttctctaaat atgattactt taaaataaaa aagtaacaag gtgtcttttc cactcctatg 2880

gaaaagggtc ttcttggcag cttaacattg acttcttggt ttggggagaa ataaattttg 2940

tttcagaatt ttgtatattg taggaatccc tttgagaatg tgattccttt tgatggggag 3000

aaagggcaaa ttattttaat attttgtatt ttcaacttta taaagataaa atatcctcag 3060

gggtggagaa gtgtcgtttt cataacttgc tgaatttcag gcattttgtt ctacatgagg 3120

actcatatat ttaagccttt tgtgtaataa gaaagtataa agtcacttcc agtgttggct 3180

gtgtgacaga atcttgtatt tgggccaagg tgtttccatt tctcaatcag tgcagtgata 3240

catgtactcc agagggacag ggtggacccc ctgagtcaac tggagcaaga aggaaggagg 3300

cagactgatg gcgattccct ctcacccggg actctccccc tttcaaggaa agtgaacctt 3360

taaagtaaag gcctcatctc ctttattgca gttcaaatcc tcaccatcca cagcaagatg 3420

aattttatca gccatgtttg gttgtaaatg ctcgtgtgat ttcctacaga aatactgctc 3480

tgaatatttt gtaataaagg tctttgcaca tgtgaccaca tacgtgttag gaggctgcat 3540

gctctggaag cctggactct aagctggagc tcttggaaga gctcttcggt ttctgagcat 3600

aatgctccca tctcctgatt tctctgaaca gaaaacaaaa gagagaatga gggaaattgc 3660

tattttattt gtattcatga acttggctgt aatcagttat gccgtatagg atgtcagaca 3720

ataccactgg ttaaaataaa gcctattttt caaattt 3757

<210> 21
<211> 1946
<212> DNA
<213> Homo sapiens

<220>
<223> II.21 (BC001506)

<400> 21

ggcacgagga gaggatcctg ggcgccgcca ggttctgtgg acaatcacaa tgggaatcca 60
aggagggtct gtcctgttcg ggctgctgct cgtcctggct gtcttctgcc attcaggtca 120
tagcctgcag tgctacaact gtcctaaccc aactgctgac tgcaaaacag ccgtcaattg 180
ttcatctgat tttgatgcgt gtctcattac caaagctggg ttacaagtgt ataacaagtg 240
ttggaagttt gagcattgca atttcaacga cgtcacaacc cgcttgaggg aaaatgagct 300
aacgtactac tgctgcaaga aggacctgtg taactttaac gaacagcttg aaaatggtgg 360
gacatcctta tcagagaaaa cagttcttct gctggtgact ccatttctgg cagcagcctg 420
gagccttcat ccctaagtca acaccaggag agcttctccc aaactccccg ttcctgcgta 480
gtccgctttc tcttgctgcc acattctaaa ggcttgatat tttccaaatg gatcctgttg 540
ggaaagaata aaattagctt gagcaacctg gctaagatag aggggctctg ggagactttg 600
aagaccagtc ctgtttgcag ggaagcccca cttgaaggaa gaagtctaag agtgaagtag 660
gtgtgacttg aactagattg catgcttcct cctttgctct tgggaagacc agctttgcag 720
tgacagcttg agtgggttct ctgcagccct cagattattt ttcctctggc tccttggatg 780
tagtcagtta gcatcattag tacatctttg gagggtgggg caggagtata tgagcatcct 840
ctctcacatg gaacgctttc ataaacttca gggatcccgt gttgccatgg aggcatgcca 900
aatgttccat atgtgggtgt cagtcaggga caacaagatc cttaatgcag agctagagga 960
cttctggcag ggaagtgggg aagtgttcca gatagcaggg catgaaaact tagagaggta 1020
caagtggctg aaaatcgagt ttttcctctg tctttaaatt ttatatgggc tttgttatct 1080
tccactggaa aagtgtaata gcatacatca atggtgtgtt aaagctattt ccttgccttt 1140
tttttattgg aatggtagga tatcttggct ttgccacaca cagttacaga gtgaacactc 1200
tactacatgt gactggcagt attaagtgtg cttattttaa atgttactgg tagaaaggca 1260
gttcaggtat gtgtgtatat agtatgaatg cagtggggac accctttgtg gttacagttt 1320
gagacttcca aaggtcatcc ttaataacaa cagatctgca ggggtatgtt ttaccatctg 1380
catccagcct cctgctaact cctagctgac tcagcataga ttgtataaaa tacctttgta 1440
acggctctta gcacactcac agatgtttga ggctttcaga agctcttcta aaaaatgata 1500
cacacctttc acaagggcaa actttttcct tttccctgtg tattctagtg aatgaatctc 1560
aagattcagt agacctaatg acatttgtat tttatgatct tggctgtatt taatggcata 1620
ggctgacttt tgcagatgga ggaatttctt gattaatgtt gaaaaaaaac ccttgattat 1680
actctgttgg acaaaccgag tgcaatgaat gatgcttttc tgaaaatgaa atataacaag 1740
tgggtgaatg tggttatggc cgaaaaggat atgcagtatg cttaatggta gcaactgaaa 1800
gaagacatcc tgagcagtgc cagctttctt ctgttgatgc cgttccctga acataggaaa 1860
atagaaactt gcttatcaaa acttaaaaaa aaaaaaaaaa aaaaaaaaaa aaaaaaaaaa 1920
aaaaaaaaaa aaaaaaaaaa aaaaaa 1946

<210> 22

<211>  1615
<212>  DNA
<213>  Homo sapiens


<220>
<223>  II.22 (XM_042018)

<400>  22
gccgcgggcg gcggcggcag cggttggagg ttgtaggacc ggcgaggaat aggaatcatg      60

gcggctgcgc tgttcgtgct gctgggattc gcgctgctgg gcacccacgg agcctccggg     120

gctgccggca cagtcttcac taccgtagaa gaccttggct ccaagatact cctcacctgc     180

tccttgaatg acagcgccac agaggtcaca gggcaccgct ggctgaaggg gggcgtggtg     240

ctgaaggagg acgcgctgcc cggccagaaa acggagttca aggtggactc cgacgaccag     300

tggggagagt actcctgcgt cttcctcccc gagcccatgg gcacggccaa catccagctc     360

cacgggcctc ccagagtgaa ggctgtgaag tcgtcagaac acatcaacga gggggagacg     420

gccatgctgg tctgcaagtc agagtccgtg ccacctgtca ctgactgggc ctggtacaag     480

atcactgact ctgaggacaa ggccctcatg aacggctccg agagcaggtt cttcgtgagt     540

tcctcgcagg gccggtcaga gctacacatt gagaacctga acatggaggc cgaccccggc     600

cagtaccggt gcaacggcac cagctccaag ggctccgacc aggccatcat cacgctccgc     660

gtgcgcagcc acctggccgc cctctggccc ttcctgggca tcgtggctga ggtgctggtg     720

ctggtcacca tcatcttcat ctacgagaag cgccggaagc ccgaggacgt cctggatgat     780

gacgacgccg gctctgcacc cctgaagagc agcgggcagc accagaatga caaaggcaag     840

aacgtccgcc agaggaactc ttcctgaggc aggtggcccg aggacgctcc ctgctccacg     900

tctgcgccgc cgccggagtc cactcccagt gcttgcaaga ttccaagttc tcacctctta     960

aagaaaaccc accccgtaga ttcccatcat acacttcctt cttttttaaa aaagttgggt    1020

tttctccatt caggattctg ttccttaggt ttttttcctt ctgaagtgtt tcacgagagc    1080

ccgggagctg ctgccctgcg gccccgtctg tggctttcag cctctgggtc tgagtcatgg    1140

ccgggtgggc ggcacagcct tctccactgg ccggagtcag tgccaggtcc ttgccctttg    1200

tggaaagtca caggtcacac gaggggcccc gtgtcctgcc tgtctgaagc caatgctgtc    1260

tggttgcgcc atttttgtgc ttttatgttt aattttatga gggccacggg tctgtgttcg    1320

actcagcctc agggacgact ctgacctctt ggccacagag gactcacttg cccacaccga    1380

gggcgacccc gtcacagcct caagtcactc ccaagccccc tccttgtctg tgcatccggg    1440

ggcagctctg gagggggttt gctggggaac tggcgccatc gccgggactc cagaaccgca    1500

gaagcctccc cagctcaccc ctggaggacg gccggctctc tatagcacca gggctcacgt    1560

gggaaccccc ctcccaccca ccgccacaat aaagatcgcc cccacctcca ccctc         1615


<210>  23
<211>  2285
<212>  DNA
<213>  Homo sapiens

<220>
<223>  II.23 (XM_010177)

<400>  23
cgcttcccgc cgcccgagct tcggaaactt cccggccgcg acgcagggaa ccggcgcgga      60

gaaccgagca gagcggagcg cccgtggtcc agcgtgtagg gagccgatcg cccatggagg     120

gtctgggccg ctcgtgcctg tggctgcgtc gggagctgtc gcccccgcgg ccgcggctcc     180

tgctcctgga ctgccgcagc cgcgagctgt acgagtcggc gcgcatcggt ggggcgctga     240

gcgtggccct gccggcgctc ctgctgcgcc gcctgcggag gggcagcctg tcggtgcgcg     300

cgctcctgcc tgggccgccg ctgcagccgc ccccgcctgc ccccgtgctc ctgtacgacc     360

agggcggggg ccggcgccgg cgcggggagg ccgaggccga ggccgaggag tgggaggccg     420

agtcggtgct gggcaccctg ctgcagaagc tgcgagagga aggctacctg gcctactacc     480

tccagggagg cttcagcaga ttccaggccg agtgccctca cctgtgtgag accagccttg     540

ctggccgtgc cggctccagc atggcgccgg tgcccggtcc agtgcccgtg gtggggttgg     600

gcagcctgtg cctgggctcc gactgctctg atgcggaatc cgaggctgac cgcgactcca     660

tgagctgtgg cctggattcg gagggtgcca cacccccacc agtggggctg cgggcatcct     720

tccctgtcca gatcctgccc aacctctatc tgggcagtgc ccgggattcc gccaatttgg     780

agagcctggc caaactgggc atccgctaca tcctcaatgt cacccccaac ctcccaaact     840

tcttcgagaa gaatggtgac tttcactaca agcagatccc catctccgac cactggagcc     900

agaacctgtc gcggttcttt ccggaggcca ttgagttcat tgatgaggcc ttgtcccaga     960

actgcggggt gctcgtccac tgcttggcgg gggtcagccg ttctgtcacc gtcactgtgg    1020

cctacctcat gcagaagctc cacctctctc tcaacgatgc ctatgacctg gtcaagagga    1080

agaagtctaa catctccccc aacttcaact tcatggggca gttgctggac tttgagcgca    1140

gcttgcggct ggaggagcgc cactcgcagg agcagggcag tggggggcag gcatctgcgg    1200

cctccaaccc gccctccttc ttcaccaccc ccaccagtga tggcgccttc gagctggccc    1260

ccacctaggg ccccgtggcc ggcaggccgg cccctgcccc acccccaccc acgggtgtcc    1320

ctgcccactc gtgtggcaag ggaggggagg gcaggagggc tcggcctgag cagggtgctg    1380

gggggagagc gcaatacctc acgcgggctg ccgtcctaat caacgtgcct atggcgggac    1440

cacgctcgga gcctgcctct tctgcgactg ttactttttc tttgcgggat gggggtgggg    1500

gttccctctc caggtggttg tccaggccca tgtcccggcc ctgggtgctc agccagctcg    1560

gctaggccct gcgcctccct gcgcttcccc cttcaggaag ggtgtgtgcc acctcgttgc    1620

actggatccc agtggctgct tgggggagag gcgtttgcca tcactggtgt tgtcacctcc    1680

ctgtttctcc accaagggct tgggcctctc ggggctgggg cctcccaggg gatggggacc    1740

cagaggtgca gtggccgccc acatccatgg cctaggagct actgggcagg ttcccggcca    1800

cacatctggt gggctgtttt gttttttttt tttcctcttc ccccagatgt cttgacggga    1860

tcactggggc tctttgtgag tgagggtggc caaactaccg ccggaggaga tggggtctca 1920

gagcgagagc tgcggagggg gaggggaaga agaaggcctc acttttgctg ctgcggggcc 1980

cacacagccg ctgctacttt ggggggtggg gaaggggcca agctgcagac acacacagtc 2040

attcatttct gtccacaccc ctgtgggtgg cgggtgtgcg tgtgtgtgct tgtgtgtgcg 2100

cacgtgtcgg cgctcacaca cacatgctag cccactgatg cacccagccc agggctggca 2160

gtctttgcag cgtggggccg tctcaccctg gagcctggag aggatctatg cttgtttgtt 2220

tttgtaatcc atatcatagt tgctttcttt aattgttcct tctgaataaa cagtttattt 2280

aagat 2285

<210> 24
<211> 1813
<212> DNA
<213> Homo sapiens

<220>
<223> II.24 (BC013992)

<400> 24
gaggagtgga gatggcggcg gcggcggctc aggggggcgg gggcggggag ccccgtagaa 60

ccgagggggt cggcccgggg gtcccggggg aggtggagat ggtgaagggg cagccgttcg 120

acgtgggccc gcgctacacg cagttgcagt acatcggcga gggcgcgtac ggcatggtca 180

gctcggccta tgaccacgtg cgcaagactc gcgtggccat caagaagatc agccccttcg 240

aacatcagac ctactgccag cgcacgctcc gggagatcca gatcctgctg cgcttccgcc 300

atgagaatgt catcggcatc cgagacattc tgcgggcgtc caccctggaa gccatgagag 360

atgtctacat tgtgcaggac ctgatggaga ctgacctgta caagttgctg aaaagccagc 420

agctgagcaa tgaccatatc tgctacttcc tctaccagat cctgcggggc ctcaagtaca 480

tccactccgc caacgtgctc caccgagatc taaagccctc caacctgctc atcaacacca 540

cctgcgacct taagatttgt gatttcggcc tggcccggat tgccgatcct gagcatgacc 600

acaccggctt cctgacggag tatgtggcta cgcgctggta ccgggcccca gagatcatgc 660

tgaactccaa gggctatacc aagtccatcg acatctggtc tgtgggctgc attctggctg 720

agatgctctc taaccggccc atcttccctg gcaagcacta cctggatcag ctcaaccaca 780

ttctgggcat cctgggctcc ccatcccagg aggacctgaa ttgtatcatc aacatgaagg 840

cccgaaacta cctacagtct ctgccctcca agaccaaggt ggcttgggcc aagcttttcc 900

ccaagtcaga ctccaaagcc cttgacctgc tggaccggat gttaaccttt aaccccaata 960

aacggatcac agtggaggaa gcgctggctc acccctacct ggagcagtac tatgacccga 1020

cggatgagcc agtggccgag gagcccttca ccttcgccat ggagctggat gacctaccta 1080

aggagcggct gaaggagctc atcttccagg agacagcacg cttccagccc ggagtgctgg 1140

aggcccccta gcccagacag acatctctgc accctggggc ctggacctgc ctcctgcctg 1200

cccctctccc gccagactgt tagaaaatgg acactgtgcc cagcccggac cttggcagcc 1260

caggccgggg tggagcatgg gcctggccac ctctctcctt tgctgaggcc tccagcttca 1320

ggcaggccaa ggccttctcc tccccacccg ccctccccac ggggcctcgg gagctcaggt 1380

ggccccagtt caatctcccg ctgctgctgc tgcgccctta ccttccccag cgtcccagtc 1440

tctggcagtt ctggaatgga agggttctgg ctgccccaac ctgctgaagg gcagaggtgg 1500

agggtggggg gcgctgagta gggactcagg gccatgcctg cccccctcat ctcattcaaa 1560

ccccacccta gtttccctga aggaacattc cttagtctca agggctagca tccctgagga 1620

gccaggccgg gccgaatccc ctccctgtca aagctgtcac ttcgcgtgcc ctcgctgctt 1680

ctgtgtgtgg tgagcagaag tggagctggg gggcgtggag agcccggcgc ccctgccacc 1740

tccctgaccc gtctaatata taaatataga gatgtgtcta tggctgaaaa aaaaaaaaaa 1800

aaaaaaaaaa aaa 1813

<210> 25
<211> 4006
<212> DNA
<213> Homo sapiens

<220>
<223> II.25 (NM_001560)

<400> 25
tgccaaggct ccagcccggc cgggctccga ggcgagaggc tgcatggagt ggccggcgcg 60

gctctgcggg ctgtgggcgc tgctgctctg cgccggcggc gggggcgggg gcgggggcgc 120

cgcgcctacg gaaactcagc cacctgtgac aaatttgagt gtctctgttg aaaacctctg 180

cacagtaata tggacatgga atccacccga gggagccagc tcaaattgta gtctatggta 240

ttttagtcat tttggcgaca aacaagataa gaaaatagct ccggaaactc gtcgttcaat 300

agaagtaccc ctgaatgaga ggatttgtct gcaagtgggg tcccagtgta gcaccaatga 360

gagtgagaag cctagcattt tggttgaaaa atgcatctca cccccagaag gtgatcctga 420

gtctgctgtg actgagcttc aatgcatttg gcacaacctg agctacatga agtgttcttg 480

gctccctgga aggaatacca gtcccgacac taactatact ctctactatt ggcacagaag 540

cctggaaaaa attcatcaat gtgaaaacat ctttagagaa ggccaatact ttggttgttc 600

ctttgatctg accaaagtga aggattccag ttttgaacaa cacagtgtcc aaataatggt 660

caaggataat gcaggaaaaa ttaaaccatc cttcaatata gtgcctttaa cttcccgtgt 720

gaaacctgat cctccacata ttaaaaacct ctccttccac aatgatgacc tatatgtgca 780

atgggagaat ccacagaatt ttattagcag atgcctattt tatgaagtag aagtcaataa 840

cagccaaact gagacacata atgttttcta cgtccaagag gctaaatgtg agaatccaga 900

atttgagaga aatgtggaga atacatcttg tttcatggtc cctggtgttc ttcctgatac 960

tttgaacaca gtcagaataa gagtcaaaac aaataagtta tgctatgagg atgacaaact 1020

ctggagtaat tggagccaag aaatgagtat aggtaagaag cgcaattcca cactctacat 1080

aaccatgtta ctcattgttc cagtcatcgt cgcaggtgca atcatagtac tcctgcttta 1140
cctaaaaagg ctcaagatta ttatattccc tccaattcct gatcctggca agatttttaa 1200
agaaatgttt ggagaccaga atgatgatac tctgcactgg aagaagtacg acatctatga 1260
gaagcaaacc aaggaggaaa ccgactctgt agtgctgata gaaaacctga agaaagcctc 1320
tcagtgatgg agataattta tttttacctt cactgtgacc ttgagaagat tcttcccatt 1380
ctccatttgt tatctgggaa cttattaaat ggaaactgaa actactgcac catttaaaaa 1440
caggcagctc ataagagcca caggtcttta tgttgagtcg cgcaccgaaa aactaaaaat 1500
aatgggcgct ttggagaaga gtgtggagtc attctcattg aattataaaa gccagcaggc 1560
ttcaaactag gggacaaagc aaaaagtgat gatagtggtg gagttaatct tatcaagagt 1620
tgtgacaact tcctgaggga tctatacttg ctttgtgttc tttgtgtcaa catgaacaaa 1680
ttttatttgt aggggaactc atttggggtg caaatgctaa tgtcaaactt gagtcacaaa 1740
gaacatgtag aaaacaaaat ggataaaatc tgatatgtat tgtttgggat cctattgaac 1800
catgtttgtg gctattaaaa ctcttttaac agtctgggct gggtccggtg gctcacgcct 1860
gtaatcccag caatttggga gtccgaggcg ggcggatcac tcgaggtcag gagttccaga 1920
ccagcctgac caaaatggtg aaacctcctc tctactaaaa ctacaaaaat taactgggtg 1980
tggtggcgcg tgcctgtaat cccagctact cgggaagctg aggcaggtga attgtttgaa 2040
cctgggaggt ggaggttgca gtgagcagag atcacaccac tgcactctag cctgggtgac 2100
agagcaagac tctgtctaaa aaacaaaaca aaacaaaaca aaacaaaaaa acctcttaat 2160
attctggagt catcattccc ttcgacagca ttttcctctg ctttgaaagc cccagaaatc 2220
agtgttggcc atgatgacaa ctacagaaaa accagaggca gcttctttgc caagaccttt 2280
caaagccatt ttaggctgtt aggggcagtg gaggtagaat gactccttgg gtattagagt 2340
ttcaaccatg aagtctctaa caatgtattt tcttcacctc tgctactcaa gtagcattta 2400
ctgtgtcttt ggtttgtgct aggcccccgg gtgtgaagca cagacccctt ccaggggttt 2460
acagtctatt tgagactcct cagttcttgc cacttttttt tttaatctcc accagtcatt 2520
tttcagacct tttaactcct caattccaac actgatttcc ccttttgcat tctccctcct 2580
tcccttcctt gtagcctttt gactttcatt ggaaattagg atgtaaatct gctcaggaga 2640
cctggaggag cagaggataa ttagcatctc aggttaagtg tgagtaatct gagaaacaat 2700
gactaattct tgcatatttt gtaacttcca tgtgagggtt ttcagcattg atatttgtgc 2760
attttctaaa cagagatgag gtggtatctt cacgtagaac attggtattc gcttgagaaa 2820
aaaagaatag ttgaacctat ttctctttct ttacaagatg ggtccaggat tcctcttttc 2880
tctgccataa atgattaatt aaatagcttt tgtgtcttac attggtagcc agccagccaa 2940
ggctctgttt atgcttttgg ggggcatata ttgggttcca ttctcaccta tccacacaac 3000
atatccgtat atatcccctc tactcttact tcccccaaat ttaaagaagt atgggaaatg 3060
agaggcattt cccccacccc atttctctcc tcacacacag actcatatta ctggtaggaa 3120

cttgagaact ttatttccaa gttgttcaaa catttaccaa tcatattaat acaatgatgc 3180

tatttgcaat tcctgctcct aggggagggg agataagaaa ccctcactct ctacaggttt 3240

gggtacaagt ggcaacctgc ttccatggcc gtgtagaagc atggtgccct ggcttctctg 3300

aggaagctgg ggttcatgac aatggcagat gtaaagttat tcttgaagtc agattgaggc 3360

tgggagacag ccgtagtaga tgttctactt tgttctgctg ttctctagaa agaatatttg 3420

gttttcctgt ataggaatga gattaattcc tttccaggta ttttataatt ctgggaagca 3480

aaacccatgc ctccccctag ccatttttac tgttatccta tttagatggc catgaagagg 3540

atgctgtgaa attcccaaca aacattgatg ctgacagtca tgcagtctgg gagtggggaa 3600

gtgatctttt gttcccatcc tcttctttta gcagtaaaat agctgaggga aaagggaggg 3660

aaaaggaagt tatgggaata cctgtggtgg ttgtgatccc taggtcttgg gagctcttgg 3720

aggtgtctgt atcagtggat ttcccatccc ctgtgggaaa ttagtaggct catttactgt 3780

tttaggtcta gcctatgtgg attttttcct aacataccta agcaaaccca gtgtcaggat 3840

ggtaattctt attctttcgt tcagttaagt ttttcccttc atctgggcac tgaagggata 3900

tgtgaaacaa tgttaacatt tttggtagtc ttcaaccagg gattgtttct gtttaacttc 3960

ttataggaaa gcttgagtaa aataaatatt gtctttttgt atgtca 4006

<210> 26
<211> 4005
<212> DNA
<213> Homo sapiens

<220>
<223> II.26 (NM_004555)

<400> 26
cggctgcggt tcctggtgct gctcggcgcg cggccagctt tcggaacgga acgctcggcg 60

tcgcgggccc cgcccggaaa gtttgccgtg gagtcgcgac ctcttggccc gcgcggcccg 120

gcatgaagcg gcgttgagga gctgctgccg ccgcttgccg ctgccgccgc cgccgcctga 180

ggaggagctg cagcaccctg ggccacgccg atgactactg caaactgtgg cgcccacgac 240

gagctcgact tcaaactcgt ctttggcgag gacggggcgc cggcgccgcc gcccccgggc 300

tcgcggcctg cagatcttga gccagatgat tgtgcatcca tttacatctt taatgtagat 360

ccacctccat ctactttaac cacaccactt tgcttaccac atcatggatt accgtctcac 420

tcttctgttt tgtcaccatc gtttcagctc caaagtcaca aaaactatga aggaacttgt 480

gagattcctg aatctaaata tagcccatta ggtggtccca aaccctttga gtgcccaagt 540

attcaaatta catctatctc tcctaactgt catcaagaat tagatgcaca tgaagatgac 600

ctacagataa atgacccaga acgggaattt ttggaaaggc cttctagaga tcatctctat 660

cttcctcttg agccatccta ccgggagtct tctcttagtc ctagtcctgc cagcagcatc 720

tcttctagga gttggttctc tgatgcatct tcttgtgaat cgctttcaca tatttatgat 780

gatgtggact cagagttgaa tgaagctgca gcccgattta cccttggatc ccctctgact 840

tctcctggtg gctctccagg gggctgccct ggagaagaaa cttggcatca acagtatgga 900
cttggacact cattatcacc caggcaatct ccttgccact ctcctagatc cagtgtcact 960
gatgagaatt ggctgagccc caggccagcc tcaggaccct catcaaggcc cacatccccc 1020
tgtgggaaac ggaggcactc cagtgctgaa gtttgttatg ctgggtccct ttcaccccat 1080
cactcacctg ttccttcacc tggtcactcc cccaggggaa gtgtgacaga agatacgtgg 1140
ctcaatgctt ctgtccatgg tgggtcaggc cttggccctg cagtttttcc atttcagtac 1200
tgtgtagaga ctgacatccc tctcaaaaca aggaaaactt ctgaagatca agctgccata 1260
ctaccaggaa aattagagct gtgttcagat gaccaaggga gtttatcacc agcccgggag 1320
acttcaatag atgatggcct tggatctcag tatcctttaa agaaagattc atgtggtgat 1380
cagtttcttt cagttccttc accctttacc tggagcaaac caaagcctgg ccacacccct 1440
atatttcgca catcttcatt acctccacta gactggcctt taccagctca ttttggacaa 1500
tgtgaactga aaatagaagt gcaacctaaa actcatcatc gagcccatta tgaaactgaa 1560
ggtagccgag gggcagtaaa agcatctact gggggacatc ctgttgtgaa gctcctgggc 1620
tataacgaaa agccaataaa tctacaaatg tttattggga cagcagatga tcgatattta 1680
cgacctcatg cattttacca ggtgcatcga atcactggga agacagtcgc tactgcaagc 1740
caagagataa taattgccag tacaaaagtt ctggaaattc cacttcttcc tgaaaataat 1800
atgtcagcca gtattgattg tgcaggtatt ttgaaactcc gcaattcaga tatagaactt 1860
cgaaaaggag aaactgatat tggcagaaag aatactagag tacgacttgt gtttcgtgta 1920
cacatcccac agcccagtgg aaaagtcctt tctctgcaga tagcctctat acccgttgag 1980
tgctcccagc ggtctgctca agaacttcct catattgaga agtacagtat caacagttgt 2040
tctgtaaatg gaggtcatga aatggttgtg actggatcta attttcttcc agaatccaaa 2100
atcatttttc ttgaaaaagg acaagatgga cgacctcagt gggaggtaga agggaagata 2160
atcagggaaa aatgtcaagg ggctcacatt gtccttgaag ttcctccata tcataaccca 2220
gcagttacag ctgcagtgca ggtgcacttt tatctttgca atggcaagag gaaaaaaagc 2280
cagtctcaac gttttactta tacaccagtt ttgatgaagc aagaacacag agaagagatt 2340
gatttgtctt cagttccatc tttgcctgtg cctcatcctg ctcagaccca gaggccttcc 2400
tctgattcag ggtgttcaca tgacagtgta ctgtcaggac agagaagttt gatttgctcc 2460
atcccacaaa catatgcatc catggtgacc tcatcccatc tgccacagtt gcagtgtaga 2520
gatgagagtg ttagtaaaga acagcatatg attccttctc caattgtaca ccagcctttt 2580
caagtcacac caacacctcc tgtggggtct tcctatcagc ctatgcaaac taatgttgtg 2640
tacaatggac caacttgtct tcctattaat gctgcctcta gtcaagaatt tgattcagtt 2700
ttgtttcagc aggatgcaac tctttctggt ttagtgaatc ttggctgtca accactgtca 2760
tccataccat ttcattcttc aaattcaggc tcaacaggac atctcttagc ccatacacct 2820
cattctgtgc ataccctgcc tcatctgcaa tcaatgggat atcattgttc aaatacagga 2880

caaagatctc tttcttctcc agtggctgac cagattacag gtcagccttc gtctcagtta 2940

caacctatta catatggtcc ttcacattca gggtctgcta caacagcttc cccagcagct 3000

tctcatccct tggctagttc accgctttct gggccaccat ctcctcagct tcagcctatg 3060

ccttaccaat ctcctagctc aggaactgcc tcatcaccgt ctccagccac cagaatgcat 3120

tctggacagc actcaactca agcacaaagt acgggccagg ggggtctttc tgcaccttca 3180

tccttaatat gtcacagttt gtgtgatcca gcgtcatttc cacctgatgg ggcaactgtg 3240

agcattaaac ctgaaccaga agatcgagag cctaactttg caaccattgg tctgcaggac 3300

atcactttag atgatgacca atttatatct gacttggaac accagccatc aggttcagca 3360

gagaaatggc ctaaccacag tgtgctctca tgtccagctc ctttctggag aatctagagg 3420

tgaacgagat aattgggaga gacatgtccc agatttctgt ttcccaagga gcaggggtga 3480

gcaggcaggc tcccctcccg agtcctgagt ccctggattt aggaagatct gatgggctct 3540

aacagtgctt actgcagcct tgtgtccacc accaacttct cagcatgttt ctctccttgg 3600

accttgggtt tccaactctg cagccttcag gtctggggcc aggagtggga cccaccattt 3660

gtggggaaag tagcattcct ccacctcagg ccttgggtag atttggcaaa agaacaggag 3720

cagcataggc tgtttgagct ttggggaaat gaactttgct ttttatattt aactaggata 3780

cttttatatg atgggtgctt tgagtgtgaa tgcagcaggc tctcttgttt ccgaggtgct 3840

gcttttgcag gtgacctggt tacttagcta ggattggtga tttgtactgc tttatggtca 3900

tttgaagggc cctttagttt ttatgataat ttttaaaata ggaacttttg ataagacctt 3960

ctagaagcaa aaaaaaaaaa aaaaaaaaaa aaaagaaaaa aaaag 4005

<210> 27
<211> 1630
<212> DNA
<213> Homo sapiens

<220>
<223> II.27 (NM_001154)

<400> 27
agggccgggg tggggcgctg gcgtttccgt tgcttggatc agtctaggtg cagctgcgga 60

tccttcagcg tctgcatctc ggcgtcgccc cgcgtaccgt cgcccggctc tccgccgctc 120

tcccgggggt tcggggcact tgggtcccac agtctggtcc tgcttcacct tcccctgacc 180

tgagtagtcg ccatggcaca ggttctcaga ggcactgtga ctgacttccc tggatttgat 240

gagcgggctg atgcagaaac tcttcggaag gctatgaaag gcttgggcac agatgaggag 300

agcatcctga ctctgttgac atcccgaagt aatgctcagc gccaggaaat ctctgcagct 360

tttaagactc tgtttggcag ggatcttctg gatgacctga aatcagaact aactggaaaa 420

tttgaaaaat taattgtggc tctgatgaaa ccctctcggc tttatgatgc ttatgaactg 480

aaacatgcct tgaagggagc tggaacaaat gaaaaagtac tgacagaaat tattgcttca 540

aggacacctg aagaactgag agccatcaaa caagtttatg aagaagaata tggctcaagc 600

ctggaagatg acgtggtggg ggacacttca gggtactacc agcggatgtt ggtggttctc 660

cttcaggcta acagagaccc tgatgctgga attgatgaag ctcaagttga acaagatgct 720

caggctttat ttcaggctgg agaacttaaa tgggggacag atgaagaaaa gtttatcacc 780

atctttggaa cacgaagtgt gtctcatttg agaaaggtgt ttgacaagta catgactata 840

tcaggatttc aaattgagga aaccattgac cgcgagactt ctggcaattt agagcaacta 900

ctccttgctg ttgtgaaatc tattcgaagt atacctgcct accttgcaga gaccctctat 960

tatgctatga agggagctgg gacagatgat cataccctca tcagagtcat ggtttccagg 1020

agtgagattg atctgtttaa catcaggaag gagtttagga agaattttgc cacctctctt 1080

tattccatga ttaagggaga tacatctggg gactataaga aagctcttct gctgctctgt 1140

ggagaagatg actaacgtgt cacggggaag agctccctgc tgtgtgcctg caccacccca 1200

ctgccttcct tcagcacctt tagctgcatt tgtatgccag tgcttaacac attgccttat 1260

tcatactagc atgctcatga ccaacacata cacgtcatag aagaaaatag tggtgcttct 1320

ttctgatctc tagtggagat ctctttgact gctgtagtac taaagtgtac ttaatgttac 1380

taagtttaat gcctggccat tttccattta tatatatttt ttaagaggct agagtgcttt 1440

tagccttttt taaaaactcc atttatatta catttgtaac catgatactt taattagaag 1500

cttagccttg aaattgtgaa ctcttggaaa tgttattagt gaagttcgca actaaactaa 1560

acctgtaaaa ttatgatgat tgtattcaaa agattaatga aaaataaaca tttctgtccc 1620

cctgaattat 1630

<210> 28
<211> 3748
<212> DNA
<213> Homo sapiens

<220>
<223> II.28 (NM_001310)

<400> 28
   cgtctgtaaa cacccagaga ctgtcatgga gggggaggag gaggcggcgg cggcgaaggg 60
   aggcgtttgg ggccgcctcc agggtccgct ctgccattcc tgaactggtc cctcgtcccc 120
   gtgactctgg catcagggaa gcgaactgtt aggcgagagg aggaggcagc cagaaccata 180
   tccccttctt cctcggggcg gggggccggc caggccggct gagccggggg agggctccgg 240
   gagggagtgc ctggccaggc cggcctgtct gccgcgatgg atgacagtaa ggtggttgga 300
   ggcaaagtaa agaagcccgg taaacgtggt cggaagccag ccaaaattga cttgaaagca 360
   aaacttgaga ggagccggca gagtgcaaga gaatgccgag cccgaaaaaa gctgagatat 420
   cagtatttgg aagagttggt atccagtcga gaaagagcta tatgtgccct cagagaggaa 480
   ctggaaatgt acaagcagtg gtgcatggca atggaccaag gaaaaatccc ttctgaaata 540
   aaggccctac tcactggaga agagcagaac aaatctcagc agaactcaag caggcatacc 600
   aaggctggga agacagatgc taatagcaat tcctggtgaa gattatataa agatgagtca 660
   gtgattgaag ccaatattct gattcccatg gaagatggat gggcaagagt gtacttcttg 720
   gctccattta ctacctactg ctcagtagtc atctctgtaa atctgcaatt tctaccaaaa 780
   tgtgtgatcg tagatctcaa aggatcttgc tttaactttc aacacttaga aaatctacaa 840
   acattcagac ctgtctgggt tggtattgcc acccatgaca tttaacatgt tgtgatgctt 900
   gaaaacacag gagtagagaa aatcgatgaa gattgtattt ttgcacctta actccacatt 960
   gctttattgg ttaatttata ttctttccat gtaattcatg taattgtatg tctgtgtgtg 1020
   ttttatgtgt caccaccttt catgtttttg attgccctac aaagagaaac caaatgagct 1080
   gattactgac tataagttct cagcctttat ggacctaatc ttatttttat ttacttgagt 1140
   aatgtttatt ctctgcatga accatgattt ctcctgtgag ccattccagc ataagctgtg 1200
   aatatgtatt aacaaatata tacatttcta tttttataat ccataaggat atgcctgttt 1260
   taaataacat acatattaac aatatctatc aggaaaaccc tcaagacagc ttctagttaa 1320
   aacctttgtt gctgtcctct caaactatat ttataaaaat ttgctagggc caaatccata 1380
   cttgcagaat aattcatcaa attttatttt taagtgaaaa gtaacctttc aggcatttca 1440
   gcagcataca ttgacaatct agggtatata tgtatgtatg tttcttattg tatgtctata 1500
   tatgtatgtg gggaggacag gagtgaatgt tcacacactt ttcttgcgta ctcaactaaa 1560
   ttggagaatg tttctgaaga aaattggatg aaattagctg ctgagattga gtttctgcct 1620
   taaaatctga aacaaaaaaa gggacaaatt gctggtagat ctactgactg tagccatcac 1680
   cagaacactt agtttcttcc cagacatgaa tttcctgaca ggctctgagc cagaaacaca 1740
   ctgtgggcgt gcatctggtt cagccctgga tatgcctcca ctgtggaggg aagcattgga 1800
   actggagttg tgcacagcac cgcagctagt gggcctggct gggggtgcag aagggcaggg 1860
   tagttctttc catttcccct tgcagctaca cttactttct ccctgactac caaatggagt 1920
   gacggttgaa gaatcaagct cataatatga tacacccaga cagacccaga aatcttttga 1980
   tttccccagc gagatactta ccactctctc ttctctttcc catggttaaa acaggagttg 2040
   tgttctctgt cgctgctaaa ttattcttca cttgagtgaa gcaaatattt agcttccaaa 2100
   taaagtatat tttgttctga actggcagtt taagaggaga taccgtagtc tagattgtct 2160
   ttgaggtaga atatatgatg gaccacatag tttaagatta caaggtactt tccctcttct 2220
   ctcaggccat gcatttgaat aaagtgcctc ccttagcctg agctcctaaa gacctcttgt 2280
   acttggttgc aaactcagat ctctagtcta gtctagtcta gtctagtcta gtcatgtgtg 2340
   actgtgtact gagataccac tagcccagtt tgctgggtct ctatgttttg gaagttgggg 2400
   gtttaataat atttaatgtc tttttaggat cataaacata aataggtttg ttgatattca 2460
   ggccttcaga atttaagggt tttaaatata gcattcagat tgtaattggt atgtttttgc 2520
   catctggtcc actcaattgt atatttttat tttgcaaaac aacccaaaac ttactttatg 2580
   ttgctttgtt cagtaccttt tgaattcccc cagaagagtt gttttcaaaa caaacattcc 2640
   agaatgaatg tcgctcttca atggaacgtc tttattgtgc ttgaagtatt tcttctctgg 2700
   ttgtaattct aaattacaga gtcatttgag ctttctcccc tcccgctagt atctttacag 2760
   gagcagggaa gagcagtaga ggatgtataa ttttgggcga agttaaatta caatttattt 2820
   gaggttattc ctaaacctat ttatttggtg ttttggagga gatcacacac taagagaacg 2880
   ttgattgcct cggctattgt gctggctgga cactttggtc acttttgaag catgttaata 2940
   aatgtcactg attaaaacag ctggagcatt tccttccctt tccagtgaag gagcagcata 3000
   gtgaaatcct ggcacccctc acctttttgt gatgacaaga tggctgacag tcacggagtc 3060
   caggagaagg tgctgctctc cttgctttct gagtaaacag agtaatgttt tttttcttat 3120
   tttcccaaag aaagaaaagg gggaaaatat atttgtatgt ttagaccagc caaattctgt 3180
   cattatttta gtatcaacct gctgtatatt agagcaactg aatataatat ggaataagtt 3240
   tgaccaggtc aagttttagg atatagttga atttctttaa aaagttttct attttatcta 3300
   aaatctttct attatatcta aattagaatg atcatcctgc gcaaattcag attactgttt 3360
   gctggctaat tgacagtatt tctcagcatc actatgtagc attctgtttt agcatctttg 3420
   aattatgatc atttgatttg gctaattaca aaaagtacag gtttaaactg gacttacctt 3480
   gagttaaagg caaagaaaat ctaaaaacat aaatgttata tttcacaaga aatcagttgc 3540
   atattatcca ttacatattt agttttaaaa ggaggagtca ccttgaatta ttttctgagt 3600
   caccttgagt tatttgatct ctcagcctgc tttctcatct gtaaaaaagg ggggaggata 3660
   actttcacag ggtaattggt aggctaaata ggagttacag aaaactgtta aatagggaag 3720
   aactacatta aatttaaata ttcacatt 3748

<210> 29
<211> 2844
<212> DNA
<213> Homo sapiens

<220>
<223> II.29 (XM_036107)

<400> 29
tgtcaggact ttacgacccg cgcctccagc tgaggtttct agacgtgacc cagggcagac 60

tggtagcaaa gcccccacgc ccagccagga gcaccgccga ggactccagc acaccgaggg 120

acatgctggg cctgcgcccc ccactgctcg ccctggtggg gctgctctcc ctcgggtgcg 180

tcctctctca ggagtgcacg aagttcaagg tcagcagctg ccgggaatgc atcgagtcgg 240

ggcccggctg cacctggtgc cagaagctga acttcacagg gccgggggat cctgactcca 300

ttcgctgcga cacccggcca cagctgctca tgaggggctg tgcggctgac gacatcatgg 360

accccacaag cctcgctgaa acccaggaag accacaatgg gggccagaag cagctgtccc 420

cacaaaaagt gacgctttac ctgcgaccag gccaggcagc agcgttcaac gtgaccttcc 480

ggcgggccaa gggctacccc atcgacctgt actatctgat ggacctctcc tactccatgc 540

ttgatgacct caggaatgtc aagaagctag gtggcgacct gctccgggcc ctcaacgaga 600
tcaccgagtc cggccgcatt ggcttcgggt ccttcgtgga caagaccgtg ctgccgttcg 660
tgaacacgca ccctgataag ctgcgaaacc catgccccaa caaggagaaa gagtgccagc 720
ccccgtttgc cttcaggcac gtgctgaagc tgaccaacaa ctccaaccag tttcagaccg 780
aggtcgggaa gcagctgatt tccggaaacc tggatgcacc cgagggtggg ctggacgcca 840
tgatgcaggt cgccgcctgc ccggaggaaa tcggctggcg caacgtcacg cggctgctgg 900
tgtttgccac tgatgacggc ttccatttcg cgggcgacgg gaagctgggc gccatcctga 960
cccccaacga cggccgctgt cacctggagg acaacttgta caagaggagc aacgaattcg 1020
actacccatc ggtgggccag ctggcgcaca agctggctga aaacaacatc cagcccatct 1080
tcgcggtgac cagtaggatg gtgaagacct acgagaaact caccgagatc atccccaagt 1140
cagccgtggg ggagctgtct gaggactcca gcaatgtggt ccaactcatt aagaatgctt 1200
acaataaact ctcctccagg gtcttcctgg atcacaacgc cctccccgac accctgaaag 1260
tcacctacga ctccttctgc agcaatggag tgacgcacag gaaccagccc agaggtgact 1320
gtgatggcgt gcagatcaat gtcccgatca ccttccaggt gaaggtcacg gccacagagt 1380
gcatccagga gcagtcgttt gtcatccggg cgctgggctt cacggacata gtgaccgtgc 1440
aggttcttcc ccagtgtgag tgccggtgcc gggaccagag cagagaccgc agcctctgcc 1500
atggcaaggg cttcttggag tgcggcatct gcaggtgtga cactggctac attgggaaaa 1560
actgtgagtg ccagacacag ggccggagca gccaggagct ggaaggaagc tgccggaagg 1620
acaacaactc catcatctgc tcagggctgg gggactgtgt ctgcgggcag tgcctgtgcc 1680
acaccagcga cgtccccggc aagctgatat acgggcagta ctgcgagtgt gacaccatca 1740
actgtgagcg ctacaacggc caggtctgcg gcggcccggg gagggggctc tgcttctgcg 1800
ggaagtgccg ctgccacccg ggctttgagg gctcagcgtg ccagtgcgag aggaccactg 1860
agggctgcct gaacccgcgg cgtgttgagt gtagtggtcg tggccggtgc cgctgcaacg 1920
tatgcgagtg ccattcaggc taccagctgc ctctgtgcca ggagtgcccc ggctgcccct 1980
caccctgtgg caagtacatc tcctgcgccg agtgcctgaa gttcgaaaag ggcccctttg 2040
ggaagaactg cagcgcggcg tgtccgggcc tgcagctgtc gaacaacccc gtgaagggca 2100
ggacctgcaa ggagagggac tcagagggct gctgggtggc ctacacgctg gagcagcagg 2160
acgggatgga ccgctacctc atctatgtgg atgagagccg agagtgtgtg gcaggcccca 2220
acatcgccgc catcgtcggg ggcaccgtgg caggcatcgt gctgatcggc attctcctgc 2280
tggtcatctg gaaggctctg atccacctga gcgacctccg ggagtacagg cgctttgaga 2340
aggagaagct caagtcccag tggaacaatg ataatcccct tttcaagagc gccaccacga 2400
cggtcatgaa ccccaagttt gctgagagtt aggagcactt ggtgaagaca aggccgtcag 2460
gacccaccat gtctgcccca tcacgcggcc gagacatggc ttgccacagc tcttgaggat 2520
gtcaccaatt aaccagaaat ccagttattt tccgccctca aaatgacagc catggccggc 2580

cgggtgcttc tgggggctcg tcggggggac agctccactc tgactggcac agtctttgca 2640

tggagacttg aggagggagg gcttgaggtt ggtgaggtta ggtgcgtgtt tcctgtgcaa 2700

gtcaggacat cagtctgatt aaaggtggtg ccaatttatt tacatttaaa cttgtcaggg 2760

tataaaatga catcccatta attatattgt taatcaatca cgtgtataga aaaaaaataa 2820

aacttcaata caggctgtcc atgg 2844

<210> 30
<211> 1797
<212> DNA
<213> Homo sapiens

<220>
<223> II.30 (XM_009064)

<400> 30
ccagcaggga gctgggagct gggggaaacg acgccaggaa agctatcgcg ccagagaggg 60

cgacgggggc tcgggaagcc tgacagggct tttgcgcaca gctgccggct ggctgctacc 120

cgcccgcgcc agcccccgag aacgcgcgac caggcaccca gtccggtcac cgcagcggag 180

agctcgccgc tcgctgcagc gaggcccgga gcggccccgc agggaccctc cccagaccgc 240

ctgggccgcc cggatgtgca ctaaaatgga acagcccttc taccacgacg actcatacac 300

agctacggga tacggccggg cccctggtgg cctctctcta cacgactaca aactcctgaa 360

accgagcctg gcggtcaacc tggccgaccc ctaccggagt ctcaaagcgc ctggggctcg 420

cggacccggc ccagagggcg gcggtggcgg cagctacttt tctggtcagg gctcggacac 480

cggcgcgtct ctcaagctcg cctcttcgga gctggaacgc ctgattgtcc ccaacagcaa 540

cggcgtgatc acgacgacgc ctacaccccc gggacagtac ttttaccccc gcgggggtgg 600

cagcggtgga ggtgcagggg gcgcaggggg cggcgtcacc gaggagcagg agggcttcgc 660

cgacggcttt gtcaaagccc tggacgatct gcacaagatg aaccacgtga caccccccaa 720

cgtgtccctg ggcgctaccg gggggccccc ggctgggccc gggggcgtct acgccggccc 780

ggagccacct cccgtttaca ccaacctcag cagctactcc ccagcctctg cgtcctcggg 840

aggcgccggg gctgccgtcg ggaccgggag ctcgtacccg acgaccacca tcagctacct 900

cccacacgcg ccgcccttcg ccggtggcca cccggcgcag ctgggcttgg gccgcggcgc 960

ctccaccttc aaggaggaac cgcagaccgt gccggaggcg cgcagccggg acgccacgcc 1020

gccggtgtcc cccatcaaca tggaagacca agagcgcatc aaagtggagc gcaagcggct 1080

gcggaaccgg ctggcggcca ccaagtgccg gaagcggaag ctggagcgca tcgcgcgcct 1140

ggaggacaag gtgaagacgc tcaaggccga gaacgcgggg ctgtcgagta ccgccggcct 1200

cctccgggag caggtggccc agctcaaaca gaaggtcatg acccacgtca gcaacggctg 1260

tcagctgctg cttggggtca agggacacgc cttctgaacg tcccctgccc ctttacggac 1320

accccctcgc ttggacggct gggcacacgc ctcccactgg ggtccaggga gcaggcggtg 1380

ggcacccacc ctgggaccta ggggcgccgc aaaccacact ggactccggc cctcctaccc 1440

tgcgcccagt ccttccacct cgacgtttac aagccccccc ttccactttt ttttgtatgt 1500

tttttttctg ctggaaacag actcgattca tattgaatat aatatatttg tgtatttaac 1560

agggagggga agagggggcg atcgcggcgg agctggcccc gccgcctggt actcaagccc 1620

gcggggacat tgggaagggg acccccgccc cctgccctcc cctctctgca ccgtactgtg 1680

gaaaagaaac acgcacttag tctctaaaga gtttatttta agacgtgttt gtgtttgtgt 1740

gtgtttgttc tttttattga atctatttaa gtaaaaaaaa aattggttct ttattaa 1797

<210> 31
<211> 1125
<212> DNA
<213> Homo sapiens

<220>
<223> II.31 (NM_001774)

<400> 31
gtctccccca ctgtcagcac ctcttctgtg tggtgagtgg accgcttacc ccactaggtg 60

aagatgtcag cccaggagag ctgcctcagc ctcatcaagt acttcctctt cgttttcaac 120

ctcttcttct tcgtcctcgg cagcctgatc ttctgcttcg gcatctggat cctcatcgac 180

aagaccagct tcgtgtcctt tgtgggcttg gccttcgtgc ctctgcagat ctggtccaaa 240

gtcctggcca tctcaggaat cttcaccatg ggcatcgccc tcctgggttg tgtgggggcc 300

ctcaaggagc tccgctgcct cctgggcctg tattttggga tgctgctgct cctgtttgcc 360

acacagatca ccctgggaat cctcatctcc actcagcggg cccagctgga gcgaagcttg 420

cgggacgtcg tagagaaaac catccaaaag tacggcacca accccgagga gaccgcggcc 480

gaggagagct gggactatgt gcagttccag ctgcgctgct gcggctggca ctacccgcag 540

gactggttcc aagtcctcat cctgagaggt aacgggtcgg aggcgcaccg cgtgccctgc 600

tcctgctaca acttgtcggc gaccaacgac tccacaatcc tagataaggt gatcttgccc 660

cagctcagca ggcttggaca cctggcgcgg tccagacaca gtgcagacat ctgcgctgtc 720

cctgcagaga gccacatcta ccgcgagggc tgcgcgcagg gcctccagaa gtggctgcac 780

aacaacctta tttccatagt gggcatttgc ctgggcgtcg gcctactcga gctcgggttc 840

atgacgctct cgatattcct gtgcagaaac ctggaccacg tctacaaccg gctcgctcga 900

taccgttagg ccccgccctc cccaaagtcc cgccccgccc ccgtcacgtg cgctgggcac 960

ttccctgctg cctgtaaata tttgtttaat ccccagttcg cctggagccc tccgccttca 1020

cattcccctg gggacccacg tggctgcgtg cccctgctgc tgtcacctct cccacgggac 1080

ctggggcttt cgtccacagc ttcctgtccc catctgtcgg cctac 1125

<210> 32
<211> 1894
<212> DNA
<213> Homo sapiens

<220>
<223>  II.32 (XM_049849)

<400>  32
   tccggaggtc tccctaccac caggctctgt ggggcaggga agcccagtgg ggtgcaggga      60
   gccaggaagc tggggtgggg tcagggcagg gtccacaggg agaccgggcg aggctggcag     120
   ccttcccagt ccgcgcagcg tctctgcagg gggagcaaga gctgcccttc cacccctccc     180
   aggggacggg taggggcact ctgggctttt cccaccccct cacgcaggga cacaggcctg     240
   gtgggtctat gactgaaatt ggccagaccg cattctggtg gttttattcg gaagggaagt     300
   ttaccctgtt cagcagaagc tgagatggga acaggaaacc cacagggccc ctttattcgg     360
   caaaaatgtc agtcagcgcc ccggggagca gccgagggtc cctgagtgtc cgcagcaatg     420
   gcgctgagtt cctctgctgg agttcatcct gctagctggg ttcccgagct gccggtctga     480
   gcctgaggca tggagcctcc tggagactgg gggcctcctc cctggagatc cacccccaaa     540
   accgacgtct tgaggctggt gctgtatctc accttcctgg gagccccctg ctacgcccca     600
   gctctgccgt cctgcaagga ggacgagtac ccagtgggct ccgagtgctg ccccaagtgc     660
   agtccaggtt atcgtgtgaa ggaggcctgc ggggagctga cgggcacagt gtgtgaaccc     720
   tgccctccag gcacctacat tgcccacctc aatggcctaa gcaagtgtct gcagtgccaa     780
   atgtgtgacc cagccatggg cctgcgcgcg agccggaact gctccaggac agagaacgcc     840
   gtgtgtggct gcagcccagg ccacttctgc atcgtccagg acggggacca ctgcgccgcg     900
   tgccgcgctt acgccacctc cagcccgggc cagagggtgc agaagggagg caccgagagt     960
   caggacaccc tgtgtcagaa ctgccccccg gggaccttct ctcccaatgg gaccctggag    1020
   gaatgtcagc accagaccaa gtgcagctgg ctggtgacga aggccggagc tgggaccagc    1080
   agctcccact gggtatggtg gtttctctca gggagcctcg tcatcgtcat tgtttgctcc    1140
   acagttggcc taatcatatg tgtgaaaaga agaaagccaa ggggtgatgt agtcaaggtg    1200
   atcgtctccg tccagcggaa aagacaggag gcagaaggtg aggccacagt cattgaggcc    1260
   ctgcaggccc ctccggacgt caccacggtg gccgtggagg agacaatacc ctcattcacg    1320
   gggaggagcc caaaccactg acccacagac tctgcacccc gacgccagag atacctggag    1380
   cgacggctgc tgaaagaggc tgtccacctg gcggaaccac cggagcccgg aggcttgggg    1440
   gctccgccct gggctggctt ccgtctcctc cagtggaggg agaggtgggg cccctgctgg    1500
   ggtagagctg ggacgccacg gtgccattcc catgggccag tgagggcctg gggcctctgt    1560
   tctgctgtgg cctgagctcc ccagagtcct gaggaggagc gccagttgcc cctcgctcac    1620
   agaccacaca cccagccctc ctgggccagc ccagagggcc cttcagaccc cagctgtctg    1680
   cgcgtctgac tcttgtggcc tcagcaggac aggccccggg cactgcctca cagccaaggc    1740
   tggactgggt tggctgcagt gtggtgttta gtggatacca catcggaagt gattttctaa     1800
   attggatttg aattcggctc ctgttttcta tttgtcatga aacagtgtat ttggggagat    1860
   gctgtgggag gatgtaaata tcttgtttct cctc                                1894

<210>  33
<211>  1084
<212>  DNA
<213>  Homo sapiens

<220>
<223>  II.33 (NM_003327)

<400>  33
cgaggatgtg cgtgggggct cggcggctgg gccgcgggcc gtgtgcggct ctgctcctcc      60

tgggcctggg gctgagcacc gtgacggggc tccactgtgt cggggacacc taccccagca     120

acgaccggtg ctgccacgag tgcaggccag gcaacgggat ggtgagccgc tgcagccgct     180

cccagaacac ggtgtgccgt ccgtgcgggc cgggcttcta caacgacgtg gtcagctcca     240

agccgtgcaa gccctgcacg tggtgtaacc tcagaagtgg gagtgagcgg aagcagctgt     300

gcacggccac acaggacaca gtctgccgct gccgggcggg cacccagccc ctggacagct     360

acaagcctgg agttgactgt gccccctgcc ctccagggca cttctcccca ggcgacaacc     420

aggcctgcaa gccctggacc aactgcacct tggctgggaa gcacaccctg cagccggcca     480

gcaatagctc ggacgcaatc tgtgaggaca gggacccccc agccacgcag ccccaggaga     540

cccagggccc cccggccagg cccatcactg tccagcccac tgaagcctgg cccagaacct     600

cacagggacc ctccacccgg cccgtggagg tccccggggg ccgtgcggtt gccgccatcc     660

tgggcctggg cctggtgctg gggctgctgg gccccctggc catcctgctg gccctgtacc     720

tgctccggag ggaccagagg ctgccccccg atgcccacaa gccccctggg ggaggcagtt     780

tccggacccc catccaagag gagcaggccg acgcccactc caccctggcc aagatctgac     840

ctgggcccac caaggtggac gctgggcccc gccaggctgg agcccggagg gtctgctggg     900

cgagcagggc aggtgcaggc cgcctgcccc gccacgctcc tgggccaact ctgcaccgtt     960

ctaggtgccg atggctgcct ccggctctct gcttacgtat gccatgcata cctcctgccc    1020

cgcgggacca caataaaaac cttggcagac gggagtctcc gaccggcaaa aaaaaaaaaa    1080

aaaa                                                                 1084

<210>  34
<211>  1522
<212>  DNA
<213>  Homo sapiens

<220>
<223>  II.34 (BC001374)

<400>  34
ggcacgaggc ggacgcgtgg tagcctagag tcctggggag cttctgtcca cctgtcctgc      60

agaggagtcg tttccagccc ggctgcccca ggatgggtga gttcaacgag aagaagacaa     120

catgtggcac cgtttgcctc aagtacctgc tgtttaccta caattgctgc ttctggctgg     180

ctggcctggc tgtcatggca gtgggcatct ggacgctggc cctcaagagt gactacatca     240

gcctgctggc ctcaggcacc tacctggcca cagcctacat cctggtggtg gcgggcactg 300

tcgtcatggt gactggggtc ttgggctgct gcgccacctt caaggagcgt cggaacctgc 360

tgcgcctgta cttcatcctg ctcctcatca tctttctgct ggagatcatc gctggtatcc 420

tcgcctacgc ctactaccag cagctgaaca cggagctcaa ggagaacctg aaggacacca 480

tgaccaagcg ctaccaccag ccgggccatg aggctgtgac cagcgctgtg gaccagctgc 540

agcaggagtt ccactgctgt ggcagcaaca actcacagga ctggcgagac agtgagtgga 600

tccgctcaca ggaggccggt ggccgtgtgg tcccagacag ctgctgcaag acggtggtgg 660

ctctttgtgg acagcgagac catgcctcca acatctacaa ggtggagggc ggctgcatca 720

ccaagttgga gaccttcatc caggagcacc tgagggtcat tggggctgtg gggatcggca 780

ttgcctgtgt gcaggtcttt ggcatgatct tcacgtgctg cctgtacagg agtctcaagc 840

tggagcacta ctgaccctgc cttgggcctt gctgctgctg cacccaacta ctgagctgag 900

accactgagt accaggggct gggctccctg atgacaccca ccctgtgcca tcaccataac 960

ctctggggac cccaacctca gaggcagctt caagtgcctt ttgctgcgca ccaatgccca 1020

gcaggggagg tgaggggggc tggcggggcg aagtttgggg ggtgttttgt ggggctcccc 1080

ggacatactc tctgcctggt ggtcagatgc aggttggaag gggccttgct gagtggcgca 1140

aggccgagcg ttcccagcag ggggagaaac ccttcacacc ccaggccctt caggaactgg 1200

ggctttgcct tgcagccaca tggccccatc ccagttgggg aagccaggtg agctctgacc 1260

cttgggcctg ggcctctgcc cctcccaacc cagccgtcgt ctccctcgac agcgcccctg 1320

ctgtcttccc caccgcagtc accaccaccc gaaatgccac gtggtcactg tgcactgccc 1380

tgttcatgtg cctctgcggg gcagggcctt cctggttttg tacactgctg tacccagatg 1440

cctacaacca tccctgccac atacaggtgc tcaataaaca cttgtagagc agatggaaaa 1500

aaaaaaaaaa aaaaaaaaaa aa 1522

<210> 35
<211> 2481
<212> DNA
<213> Homo sapiens

<220>
<223> II.35 (XM_051958)

<400> 35
cccggcgctc gctgctcccg cggcccgcgc catgccctcc tacacggtca ccgggaccgt 60

acttcacccc gtggtgaaga cactgacgac tacatctacc tcagcctcgt gggctcggcg 120

ggctgcagcg agaagcacct gctggacaag cccttctaca acgacttcga gcgtggcgcg 180

gtggattcat acgacgtgac tgtggacgag gaactgggcg agatccagct ggtcagaatc 240

gagaagcgca agtactggct gaatgacgac tggtacctga agtacatcac gctgaagacg 300

ccccacgggg actacatcga gttcccctgc taccgctgga tcaccggcga tgtcgaggtt 360

gtcctgaggg atggacgcgc aaagttggcc cgagatgacc aaattcacat tctcaagcaa 420
caccgacgta aagaactgga aacacggcaa aaacaatatc gatggatgga gtggaaccct 480
ggcttcccct tgagcatcga tgccaaatgc cacaaggatt taccccgtga tatccagttt 540
gatagtgaaa aaggagtgga ctttgttctg aattactcca aagcgatgga gaacctgttc 600
atcaaccgct tcatgcacat gttccagtct tcttggaatg acttcgccga ctttgagaaa 660
atctttgtca agatcagcaa cactatttct gagcgggtca tgaatcactg gcaggaagac 720
ctgatgtttg gctaccagtt cctgaatggc tgcaaccctg tgttgatccg gcgctgcaca 780
gagctgcccg agaagctccc ggtgaccacg gagatggtag agtgcagcct ggagcggcag 840
ctcagcttgg agcaggaggt ccagcaaggg aacattttca tcgtggactt tgagctgctg 900
gatggcatcg atgccaacaa aacagacccc tgcacactcc agttcctggc cgctcccatc 960
tgcttgctgt ataagaacct ggccaacaag attgtcccca ttgccatcca gctcaaccaa 1020
atcccgggag atgagaaccc tattttcctc ccttcggatg caaaatacga ctggcttttg 1080
gccaaaatct gggtgcgttc cagtgacttc cacgtccacc agaccatcac ccaccttctg 1140
cgaacacatc tggtgtctga ggtttttggc attgcaatgt accgccagct gcctgctgtg 1200
caccccattt tcaagctgct ggtggcacac gtgagattca ccattgcaat caacaccaag 1260
gcccgtgagc agctcatctg cgagtgtggc ctctttgaca aggccaacgc cacagggggc 1320
ggtgggcacg tgcagatggt gcagagggcc atgaaggacc tgacctatgc ctccctgtgc 1380
tttcccgagg ccatcaaggc ccggggcatg gagagcaaag aagacatccc ctactacttc 1440
taccgggacg acgggctcct ggtgtgggaa gccatcagga cgttcacggc cgaggtggta 1500
gacatctact acgagggcga ccaggtggtg gaggaggacc cggagctgca ggacttcgtg 1560
aacgatgtct acgtgtacgg catgcggggc cgcaagtcct caggcttccc caagtcggtc 1620
aagagccggg agcagctgtc ggagtacctg accgtggtga tcttcaccgc ctccgcccag 1680
cacgccgcgg tcaacttcgg ccagtacgac tggtgctcct ggatccccaa tgcgccccca 1740
accatgcgag ccccgccacc gactgccaag ggcgtggtga ccattgagca gatcgtggac 1800
acgctgcccg accgcggccg ctcctgctgg catctgggtg cagtgtgggc gctgagccag 1860
ttccaggaaa acgagctgtt cctgggcatg tacccagaag agcattttat cgagaagcct 1920
gtgaaggaag ccatggcccg attccgcaag aacctcgagg ccattgtcag cgtgattgct 1980
gagcgcaaca agaagaagca gctgccatat tactacttgt ccccagaccg gattccgaac 2040
agtgtggcca tctgagcaca ctgccagtct cactgtggga aggccagctg ccccagccag 2100
atggactcca gcctgcctgg caggctgtct ggccaggcct cttggcagtc acatctcttc 2160
ctccgaggcc agtacctttc catttattct ttgatcttca gggaactgca tagattgatc 2220
aaagtgtaaa caccataggg acccattcta cacagagcag gactgcacag cgtcctgtcc 2280
acacccagct cagcatttcc acaccaagca gcaacagcaa atcacgacca ctgatagatg 2340
tctattcttg ttggagacat gggatgatta ttttctgttc tatttgtgct tagtccaatt 2400

ccttgcacat agtaggtacc caattcaatt actattgaat gaattaagaa ttggttgcca 2460

taaaaataaa tcagttcatt t 2481

<210> 36
<211> 1695
<212> DNA
<213> Homo sapiens

<220>
<223> II.36 (NM_021805)

<400> 36
gttgccgctg cgcacctggc tcaggtgagc tgccccgccc ccgcccggcg cgagccccag 60

gtcctggcag cagcccctga cctgtccagg tgccctgtcc agctgactgc aaggacagag 120

aggagtcctg cccagctctt ggatcagtct gctggccgag gagcccggtg gagccagggg 180

tgaccctgga gcccagcctg ccccgaggag gccccggctc agagccatgc caggtgtctg 240

tgatagggcc cctgacttcc tctccccgtc tgaagaccag gtgctgaggc ctgccttggg 300

cagctcagtg gctctgaact gcacggcttg ggtagtctct gggccccact gctccctgcc 360

ttcagtccag tggctgaaag acgggcttcc attgggaatt gggggccact acagcctcca 420

cgagtactcc tgggtcaagg ccaacctgtc agaggtgctt gtgtccagtg tcctgggggt 480

caacgtgacc agcactgaag tctatggggc cttcacctgc tccatccaga acatcagctt 540

ctcctccttc actcttcaga gagctggccc tacaagccac gtggctgcgg tgctggcctc 600

cctcctggtc ctgctggccc tgctgctggc cgccctgctc tatgtcaagt gccgtctcaa 660

cgtgctgctc tggtaccagg acgcgtatgg ggaggtggag ataaacgacg ggaagctcta 720

cgacgcctac gtctcctaca gcgactgccc cgaggaccgc aagttcgtga acttcatcct 780

aaagccgcag ctggagcggc gtcggggcta caagctcttc ctggacgacc gcgacctcct 840

gccgcgcgct gagccctccg ccgacctctt ggtgaacctg agccgctgcc gacgcctcat 900

cgtggtgctt tcggacgcct tcctgagccg ggcctggtgc agccacagct tccgggaggg 960

cctgtgccgg ctgctggagc tcacccgcag acccatcttc atcaccttcg agggccagag 1020

gcgcgacccc gcgcacccgg cgctccgcct gctgcgccag caccgccacc tggtgacctt 1080

gctgctctgg aggcccggct ccgtgactcc ttcctccgat ttttggaaag aagtgcagct 1140

ggcgctgccg cggaaggtgc ggtacaggcc ggtggaagga gacccccaga cgcagctgca 1200

ggacgacaag gaccccatgc tgattcttcg aggccgagtc cctgagggcc gggccctgga 1260

ctcagaggtg gacccggacc ctgagggcga cctgggtgtc cgggggcctg tttttggaga 1320

gccatcagct ccaccgcaca ccagtggggt ctcgctggga gagagccgga gcagcgaagt 1380

ggacgtctcg gatctcggct cgcgaaacta cagtgcccgc acagacttct actgcctggt 1440

gtccaaggat gatatgtagc tcccacccca gagtgcagga tcatagggac agcgggggcc 1500

agggcagcgg cgtcgctcct ctgctcaaca ggaccacaac ccctgccagc agccctggga 1560

ccctgccagc agccctggga aaaggctgtg gcctcagggc gcctcccagt gccagaaaat 1620

aaagtccttt tggattctga aaaaaaaaaa aaaaaaaaaa aaaaaaaaaa aaaaaaaaaa 1680

aaaaaaaaaa aaaaa 1695

<210> 37
<211> 1114
<212> DNA
<213> Homo sapiens

<220>
<223> II.37 (NM_017526)

<400> 37
gtctggcttg ggcaggctgc ccgggccgtg gcaggaagcc ggaagcagcc gcggccccag 60

ttcgggagac atggcgggcg ttaaagctct cgtggcatta tccttcagtg gggctattgg 120

actgactttt cttatgctgg gatgtgcctt agaggattat ggcgtttact ggcccttatt 180

cgtcctgatt ttccacgcca tctcccccat cccccatttc attgccaaaa gagtcaccta 240

tgactcagat gcaaccagta gtgcctgtcg ggaactggca tatttcttca ctactggaat 300

tgttgtttct gcctttggat ttcctgttat tcttgctcgt gtggctgtga tcaaatgggg 360

agcctgcggc cttgtgttgg caggcaatgc agtcattttc cttacaattc aagggttttt 420

ccttatattt ggaagaggag atgattttag ctgggagcag tggtagcact ttattctgat 480

tacagtgcat tgaatttctt agaactcata ctatctgtat acatgtgcac atgcggcatt 540

ttactatgaa atttaatatg ctgggttttt taataccttt atatatcatg ttcactttaa 600

gaaagacttc ataagtagga gatgagtttt attctcagca aatagacctg tcaaatttag 660

attatgttac tcaaattatg ttacttgttt ggctgttcat gtagtcacgg tgctctcaga 720

aaatatatta acgcagtctt gtaggcagct gccaccttat gcagtgcatc gaaacctttt 780

gcttggggat gtgcttggag aggcagataa cgctgaagca ggcctctcat gacccaggaa 840

ggccggggtg gatccctctt tgtgttgtag tccatgctat taaaagtgtg gcccacagac 900

caagagcctc aacatttcct agagccttat tagaaatgca gaatctgaag ccccactctg 960

gacccaggac attttgatga gatccaaagg agttgtatgc acatgaaagt ttgagaagca 1020

tcatcataga gaagtaaaca tcacacccaa cttccttatc tttccagtgg ctaaaccact 1080

taacctctct gggtgttacc tgctcatttg ttta 1114

<210> 38
<211> 5893
<212> DNA
<213> Homo sapiens

<220>
<223> II.38 (XM_011780)

<400> 38
   ggaggacagc cggaccgagc caacgccggg gactttgttc cctccgcgga ggggactcgg 60
   caactcgcag cggcagggtc tggggccggc gcctgggagg gatctgcgcc ccccactcac 120
   tccctagctg tgttcccgcc gccgccccgg ctagtctccg gcgctggcgc ctatggtcgg 180
   cctccgacag cgctccggag ggaccggggg agctcccagg cgcccgggac tggagactga 240
   tgcatgaggg ggctacggag gcgcaggagc ggtggtgatg gtctgggaag cggagctgaa 300
   gtgccctggg ctttggtgag gcgtgacagt ttatcatgac cgtgttcagg caggaaaacg 360
   tggatgatta ctacgacacc ggcgaggaac ttggcagtgg acagtttgcg gttgtgaaga 420
   aatgccgtga gaaaagcacc ggcctccagt atgccgccaa attcatcaag aaaaggagga 480
   ctaagtccag ccggcggggt gtgagccgcg aggacatcga gcgggaggtc agcatcctga 540
   aggagatcca gcaccccaat gtcatcaccc tgcacgaggt ctatgagaac aagacggacg 600
   tcatcctgat cttggaactc gttgcaggtg gcgagctgtt tgacttctta gctgaaaagg 660
   aatctttaac tgaagaggaa gcaactgaat ttctcaaaca aattcttaat ggtgtttact 720
   acctgcactc ccttcaaatc gcccactttg atcttaagcc tgagaacata atgcttttgg 780
   atagaaatgt ccccaaacct cggatcaaga tcattgactt tgggttggcc cataaaattg 840
   actttggaaa tgaatttaaa aacatatttg ggactccaga gtttgtcgct cctgagatag 900
   tcaactatga acctcttggt cttgaggcag atatgtggag tatcggggta ataacctata 960
   tcctcctaag tggggcctcc ccatttcttg gagacactaa gcaagaaacg ttagcaaatg 1020
   tatccgctgt caactacgaa tttgaggatg aatacttcag taataccagt gccctagcca 1080
   aagatttcat aagaagactt ctggtcaagg atccaaagaa gagaatgaca attcaagata 1140
   gtttgcagca tccctggatc aagcctaaag atacacaaca ggcacttagt agaaaagcat 1200
   cagcagtaaa catggagaaa ttcaagaagt ttgcagcccg gaaaaaatgg aaacaatccg 1260
   ttcgcttgat atcactgtgc caaagattat ccaggtcatt cctgtccaga agtaacatga 1320
   gtgttgccag aagcgatgat actctggatg aggaagactc ctttgtgatg aaagccatca 1380
   tccatgccat caacgatgac aatgtcccag gcctgcagca ccttctgggc tcattatcca 1440
   actatgatgt taaccaaccc aacaagcacg ggacacctcc attactcatt gctgctggct 1500
   gtgggaatat tcaaatacta cagttgctca ttaaaagagg ctcgagaatc gatgtccagg 1560
   ataagggcgg gtccaatgcc gtctactggg ctgctcggca tggccacgtc gataccttga 1620
   aatttctcag tgagaacaaa tgccctttgg atgtgaaaga caagtctgga gagatggccc 1680
   tccacgtggc agctcgctat ggccatgctg acgtggctca gttactgtgc agcttcggct 1740
   caaatcccaa tatccaggac aaaggaagaa gaaacccccc tgcactgtgc tgcttggcac 1800
   ggctattact ctgtggccaa agccctttgt gaagccggct gtaacgtgaa catcaagaac 1860
   cgagaaggag agacgcccct cctgacagcc tctgccaggg gctaccacga catcgtggag 1920
   tgtctggccg aacatggagc cgaccttaat gcttgcgaca aggacggaca cattgccctt 1980
   catctggctg taagacggtg tcagatggag gtaatcaaga ctctcctcag ccaagggtgt 2040
   ttcgtcgatt atcaagacag gcacggcaat actcccctcc atgtggcatg taaagatggc 2100
   aacatgccta tcgtggtggc cctctgtgaa gcaaactgca atttggacat ctccaacaag 2160
   tatgggcgaa cgcctctgca ccttgcggcc aacaacggaa tcctagacgt ggtccggtat 2220
   ctctgtctga tgggagccag cgttgaggcg ctgaccacgg acggaaagac ggcagaagat 2280
   cttgctagat cggaacagca cgagcacgta gcaggtctcc ttgcaagact tcgaaaggat 2340
   acgcaccgag gactcttcat ccagcagctc cgacccacac agaacctgca gccaagaatt 2400
   aagctcaagc tgtttggcca ctcgggatcc gggaaaacca cccttgtaga atctctcaag 2460
   tgtgggctgc tgaggagctt tttcagaagg cgtcggccca gactgtcttc caccaactcc 2520
   agcaggttcc caccttcacc cctggcttct aagcccacag tctcagtgag catcaacaac 2580
   ctgtacccag gctgcgagaa cgtgagtgtg aggagccgca gcatgatgtt cgagccgggt 2640
   cttaccaaag ggatgctgga ggtgtttgtg gccccgaccc accacccgca ctgctcggcc 2700
   gatgaccagt ccaccaaggc catcgacatc cagaacgctt atttgaatgg agttggcgat 2760
   ttcagcgtgt gggagttctc tggaaatcct gtgtatttct gctgttatga ctattttgct 2820
   gcaaatgatc ccacgtcaat ccatgttgtt gtctttagtc tagaagagcc ctatgagatc 2880
   cagctgaacc aagtgatttt ctggctcagt ttcctgaagt cccttgtccc agttgaagaa 2940
   cccatagcct tcggtggcaa gctgaagaac ccactccaag ttgtcctggt ggccacccac 3000
   gctgacatca tgaatgttcc tcgaccggct ggaggcgagt ttggatatga caaagacaca 3060
   tcgttgctga aagagattag gaacaggttt ggaaatgatc ttcacatttc aaataagctg 3120
   tttgttctgg atgctggggc ttctgggtca aaggacatga aggtacttcg aaatcatctg 3180
   caagaaatac gaagccagat tgtttcggtc tgtcctccca tgactcacct gtgtgagaaa 3240
   atcatctcca cgctgccttc ctggaggaag ctcaatggac ccaaccagct gatgtcgctg 3300
   cagcagtttg tgtacgacgt gcaggaccag ctgaaccccc tggccagcga ggaggacctc 3360
   aggcgcattg ctcagcagct ccacagcaca ggcgagatca acatcatgca aagtgaaaca 3420
   gttcaggacg tgctgctcct ggacccccgc tggctctgca caaacgtcct ggggaagttg 3480
   ctgtccgtgg agaccccacg ggcgctgcac cactaccggg gccgctacac cgtggaggac 3540
   atccagcgcc tggtgcccga cagcgacgtg gaggagctgc tgcagatcct cgatgccatg 3600
   gacatctgcg cccgggacct gagcagcggg accatggtgg acgtcccagc cctgatcaag 3660
   acagacaacc tgcaccgctc ctgggctgat gaggaggacg aggtgatggt gtatggtggc 3720
   gtgcgcatcg tgcccgtgga acacctcacc cccttcccat gtggcatctt tcacaaggtc 3780
   caggtgaacc tgtgccggtg gatccaccag caaagcacag agggcgacgc ggacatccgc 3840
   ctgtgggtga atggctgcaa gctggccaac cgtggggccg agctgctggt gctgctggtc 3900
   aaccacggcc agggcattga ggtccaggtc cgcggcctgg agacggagaa gatcaagtgc 3960
   tgcctgctgc tggactcggt gtgcagcacc attgagaacg tcatggccac cacgctgcca 4020
   gggctcctga ccgtgaagca ttacctgagc ccccagcagc tgcgggagca ccatgagccc 4080
   gtcatgatct accagccacg ggacttcttc cgggcacaga ctctgaagga aacctcactg 4140
   accaacacca tgggggggta caaggaaagc ttcagcagca tcatgtgctt cgggtgtcac 4200
   gacgtctact cacaggccag cctcggcatg gacatccatg catcagacct gaacctcctc 4260
   actcggagga aactgagtcg cctgctggac ccgcccgacc ccctggggaa ggactggtgc 4320
   cttctcgcca tgaacttagg cctccctgac ctcgtggcaa agtacaacac cagtaacggg 4380
   gctcccaagg atttcctccc cagccccctc cacgccctgc tgcgggaatg gaccacctac 4440
   cctgagagca cagtgggcac cctcatgtcc aaactgaggg agctgggtcg ccgggatgcc 4500
   gcagactttt tgctgaaggc atcctctgtg ttcaaaatca acctggatgg caatggccag 4560
   gaggcctatg cctcgagctg caacagcggc acctcttaca attccattag ctctgttgta 4620
   tcccggtgag ggcagcctct ggcttgggca gggtctgttt ggactgcaga agcaaggggg 4680
   tgatgtagcc catccttccc tttggagatg ctgagggtgt ttcttcctgc acccacagcc 4740
   agggggatgc cactcctccc tccggcttga cctgtttctc tgccgctacc tccctccccg 4800
   tctcattccg ttgtctgtgg atggtcattg cagtttaaga gcagaacaga tcttttactt 4860
   tggccgcttg aaaagctagt gtacctcctc tcagtgtttt ggactccatc tctcatcctc 4920
   cagtaccttg cttcttactg ataattttgc tggaattcct aacttttcaa tgacattttt 4980
   tttaactact atattgattg tcctttaaaa aagaaaagtg catatttatc caaaatgtgt 5040
   atttcttata cgcttttctt tgttatacca tttcctcagc ttatctcttt tatatttgta 5100
   ggagaaactc ccatgtatgg aatcccactg tatgatttat aaacagacaa tatgtgagtg 5160
   ccttttgcag aagagggtgt gtttgaaatc atcggagtca gccaggagct gtcaccaagg 5220
   aaacgctacc tctctgtccc ttgctgtatg ctgatcatcg ccagaggtgc ttcaccctga 5280
   gttttgtttt gtattgtttt ctgacagttt ttctgttttg tttggcaagg aaaggggaga 5340
   agggaatcct cctccagggt gattttatga tcagtgttgt tgctctagga agacattttt 5400
   ccgtttgctt ttgttccaat gtcaatgtga acgtccacat gaaacctaca cactgtcatg 5460
   cttcatcatt ccctctcatc tcaggtagaa ggttgacaca gttgtagggt tacagagacc 5520
   tatgtaagaa ttcagaagac ccctgactca tcatttgtgg cagtccctta taattggtgc 5580
   atagcagatg gtttccacat ttagatcctg gtttcataac ttcctgtact tgaagtctaa 5640
   aagcagaaaa taaaggaagc aagtttcttt ccatgatttt aaattgtgat cgagttttaa 5700
   attgatagga gggaacatgt cctaattctt ctgtcctgag aagcatgtaa tgttaatgtt 5760
   atatcatatg tatatatata tatgcactat gtatatacat atatattaat actggtattt 5820
   ttacttaatc tataaaatgt cgttaaaaag ttgtttgttt ttttcttttt ttataaataa 5880
   actgttgctc gtt 5893

<210> 39
<211> 3794
<212> DNA
<213> Homo sapiens

<220>
<223> II.39 (NM_006017)

<400> 39
ccaagttcta cctcatgttt ggaggatctt gctagctatg gccctcgtac tcggctccct 60
gttgctgctg gggctgtgcg ggaactcctt ttcaggaggg cagccttcat ccacagatgc 120
tcctaaggct tggaattatg aattgcctgc aacaaattat gagacccaag actcccataa 180
agctggaccc attggcattc tctttgaact agtgcatatc tttctctatg tggtacagcc 240
gcgtgatttc ccagaagata ctttgagaaa attcttacag aaggcatatg aatccaaaat 300
tgattatgac aagccagaaa ctgtaatctt aggtctaaag attgtctact atgaagcagg 360
gattattcta tgctgtgtcc tggggctgct gtttattatt ctgatgcctc tggtggggta 420
tttcttttgt atgtgtcgtt gctgtaacaa atgtggtgga gaaatgcacc agcgacagaa 480
ggaaaatggg cccttcctga ggaaatgctt tgcaatctcc ctgttggtga tttgtataat 540
aataagcatt ggcatcttct atggttttgt ggcaaatcac caggtaagaa cccggatcaa 600
aaggagtcgg aaactggcag atagcaattt caaggacttg cgaactctct tgaatgaaac 660
tccagagcaa atcaaatata tattggccca gtacaacact accaaggaca aggcgttcac 720
agatctgaac agtatcaatt cagtgctagg aggcggaatt cttgaccgac tgagacccaa 780
catcatccct gttcttgatg agattaagtc catggcaaca gcgatcaagg agaccaaaga 840
ggcgttggag aacatgaaca gcaccttgaa gagcttgcac caacaaagta cacagcttag 900
cagcagtctg accagcgtga aaactagcct gcggtcatct ctcaatgacc ctctgtgctt 960
ggtgcatcca tcaagtgaaa cctgcaacag catcagattg tctctaagcc agctgaatag 1020
caaccctgaa ctgaggcagc ttccacccgt ggatgcagaa cttgacaacg ttaataacgt 1080
tcttaggaca gatttggatg gcctggtcca acagggctat caatccctta atgatatacc 1140
tgacagagta caacgccaaa ccacgactgt cgtagcaggt atcaaaaggg tcttgaattc 1200
cattggttca gatatcgaca atgtaactca gcgtcttcct attcaggata tactctcagc 1260
attctctgtt tatgttaata acactgaaag ttacatccac agaaatttac ctacattgga 1320
agagtatgat tcatactggt ggctgggtgg cctggtcatc tgctctctgc tgaccctcat 1380
cgtgattttt tactacctgg gcttactgtg tggcgtgtgc ggctatgaca ggcatgccac 1440
cccgaccacc cgaggctgtg tctccaacac cggaggcgtc ttcctcatgg ttggagttgg 1500
attaagtttc ctcttttgct ggatattgat gatcattgtg gttcttacct ttgtctttgg 1560
tgcaaatgtg gaaaaactga tctgtgaacc ttacacgagc aaggaattat tccgggtttt 1620
ggatacaccc tacttactaa atgaagactg ggaatactat ctctctggga agctatttaa 1680
taaatcaaaa atgaagctca cttttgaaca agtttacagt gactgcaaaa aaaatagagg 1740
cacttacggc actcttcacc tgcagaacag cttcaatatc agtgaacatc tcaacattaa 1800
tgagcatact ggaagcataa gcagtgaatt ggaaagtctg aaggtaaatc ttaatatctt 1860
tctgttgggt gcagcaggaa gaaaaaacct tcaggatttt gctgcttgtg gaatagacag 1920
aatgaattat gacagctact tggctcagac tggtaaatcc cccgcaggag tgaatctttt 1980

atcatttgca tatgatctag aagcaaaagc aaacagtttg cccccaggaa atttgaggaa 2040
ctccctgaaa agagatgcac aaactattaa aacaattcac cagcaacgag tccttcctat 2100
agaacaatca ctgagcactc tataccaaag cgtcaagata cttcaacgca cagggaatgg 2160
attgttggag agagtaacta ggattctagc ttctctggat tttgctcaga acttcatcac 2220
aaacaatact tcctctgtta ttattgagga aactaagaag tatgggagaa caataatagg 2280
atattttgaa cattatctgc agtggatcga gttctctatc agtgagaaag tggcatcgtg 2340
caaacctgtg gccaccgctc tagatactgc tgttgatgtc tttctgtgta gctacattat 2400
cgaccccttg aatttgtttt ggtttggcat aggaaaagct actgtatttt tacttccggc 2460
tctaattttt gcggtaaaac tggctaagta ctatcgtcga atggattcgg aggacgtgta 2520
cgatgatgtt gaaactatac ccatgaaaaa tatggaaaat ggtaataatg gttatcataa 2580
agatcatgta tatggtattc acaatcctgt tatgacaagc ccatcacaac attgatagct 2640
gatgttgaaa ctgcttgagc atcaggatac tcaaagtgga aaggatcaca gatttttggt 2700
agtttctggg tctacaagga ctttccaaat ccaggagcaa cgccagtggc aacgtagtga 2760
ctcaggcggg caccaaggca acggcaccat tggtctctgg gtagtgcttt aagaatgaac 2820
acaatcacgt tatagtccat ggtccatcac tattcaagga tgactccctc ccttcctgtc 2880
tatttttgtt ttttactttt ttacactgag tttctattta gacactacaa catatggggt 2940
gtttgttccc attggatgca tttctatcaa aactctatca aatgtgatgg ctagattcta 3000
acatattgcc atgtgtggag tgtgctgaac acacaccagt ttacaggaaa gatgcatttt 3060
gtgtacagta aacggtgtat ataccttttg ttaccacaga gttttttaaa caaatgagta 3120
ttataggact ttcttctaaa tgagctaaat aagtcaccat tgacttcttg gtgctgttga 3180
aaataatcca ttttcactaa aagtgtgtga aacctacagc atattcttca cgcagagatt 3240
ttcatctatt atactttatc aaagattggc catgttccac ttggaaatgg catgcaaaag 3300
ccatcataga gaaacctgcg taactccatc tgacaaattc aaaagagaga gagagatctt 3360
gagagagaaa tgctgttcgt tcaaaagtgg agttgtttta acagatgcca attacggtgt 3420
acagtttaac agagttttct gttgcattag gataaacatt aattggagtg cagctaacat 3480
gagtatcatc agactagtat caagtgttct aaaatgaaat atgagaagat cctgtcacaa 3540
ttcttagatc tggtgtccag catggatgaa acctttgagt ttggtcccta aatttgcatg 3600
aaagcacaag gtaaatattc atttgcttca ggagtttcat gttggatctg tcattatcaa 3660
aagtgatcag caatgaagaa ctggtcggac aaaatttaac gttgatgtaa tggaattcca 3720
gatgtaggca ttccccccag gtcttttcat gtgcagattg cagttctgat tcatttgaat 3780
aaaaaggaac ttgg 3794

<210> 40
<211> 2145
<212> DNA
<213> Homo sapiens

<220>
<223>  II.40 (XM_027885)

<400>  40
aaatgaaaaa aaataatagt tcactcaaac acaacttccg ggttgaaggt tcaacgattc      60

tcctcctcac ctccaagtac tgggactaca gacatgcacc acacacccag ctaattctgc     120

atttttagta gagaaggggt ctcaccatgt tgcccaggat ggtctggatc tcctgacctt     180

atggtccgct cgcctcggcc tcccaaagtc ctgggattac aggtgtgacc caccgcgcct     240

ggcccaaagt gctgggatga caggcgtgag acaccatcct gccccacaga aaagatctga     300

gatgggacag cccccgcaga tcaggacgtg ggctctgtta tctggggggt gaccgactca     360

ccctgcctcc tctcgtctct gcaggtggtc tgggaggcgg gcaaagccgg cctggaggag     420

tgtctggtga ctgaagtaca ggtcgtgcag aaaacttgag actggggttc agggcttgtg     480

ggggtctgcc tcaatctccc tggccgggcc aggcgcctgc acagactggc tgctggacct     540

gcgcacgcag cccaggaatg gacattccta acgggtggtg ggcatgggag atgcctgtgt     600

aatttcgtcc gaagctgcca ggaagaagaa cagaactttg tgtgtttatt tcatgataaa     660

gtgatttttt tttttttaac ccactcactg gtcccggtct ctggattcag ccccattcct     720

ccaacactac tagagagact gtttccccgg tttttttttt ggggagatgg agtcacgatc     780

tgtctcccag gttggagtgc agtgatgcaa tctcagctca ctgcaaccgc tgcctcccgg     840

gctcaagcaa ttctcctgcc tcagcctccc aagtaggtgg gattacaggc acctgccacc     900

acccctggct aatttttata ttagcggtct cgaactcctg accttgtgat ctgcccgcct     960

ctgcctccca agtgctggga ttacaggggt gagccaccac acctggcctt ttttctttaa    1020

acttttccaa gcggctgccg aagatggcgg aggtgcaggt cctggtgctt gatggtcgag    1080

gccatctcct gggccgcctg gcggccatcg tggctaaaca ggtactgctg ggccggaagg    1140

tggtggtcgt acgctgtgaa ggcatcaaca tttctggcaa tttctacaga aacaagttga    1200

agtacctggc tttcctccgc aagcggatga acaccaaccc ttcccgaggc ccctaccact     1260

tccgggcccc cagccgcatc ttctggcgga ccgtgcgagg tatgctgccc cacaaaacca    1320

agcgaggcca ggccgctctg gaccgtctca aggtgtttga cggcatccca ccgccctacg    1380

acaagaaaaa gcggatggtg gttcctgctg ccctcaaggt cgtgcgtctg aagcctacaa    1440

gaaagtttgc ctatctgggg cgcctggctc acgaggttgg ctggaagtac caggcagtga    1500

cagccaccct ggaggagaag aggaaagaga aagccaagat ccactaccgg aagaagaaac    1560

agctcatgag gctacggaaa caggccgaga agaacgtgga gaagaaaatt gacaaataca    1620

cagaggtcct caagacccac ggactcctgg tctgagccca ataaagactg ttaattcctc    1680

atgcgttgcc tgcccttcct ccattgttgc cctggaatgt acgggaccca ggggcagcag    1740

cagtccaggt gccacaggca gccctgggac ataggaagct gggagcaagg aaagggtctt    1800

agtcactgcc tcccgaagtt gcttgaaagc actcggagaa ttgtgcaggt gtcatttatc    1860

tatgaccaat aggaagagca accagttact atgagtgaaa gggagccaga agactgattg    1920

gagggcccta tcttgtgagt ggggcatctg ttggactttc cacctggtca tatactctgc 1980

agctgttaga atgtgcaagc acttggggac agcatgagct tgctgttgta cacagggtat 2040

ttctagaagc agaaatagac tgggaagatg cacaaccaag gggttacagg catcgcccat 2100

gctcctcacc tgtattttgt aatcagaaat aaattgcttt taaag 2145

<210> 41
<211> 5725
<212> DNA
<213> Homo sapiens

<220>
<223> II.41 (NM_007289)

<400> 41
cctggaggag ggctctggaa gtcacgtcag gttggctctt caggttcatt tccatagttc 60

cctgcggcct ctgccttggg gagttatgtt ttgttaccga gatccgcgct accagattgc 120

accggggctg atttgggggc tgggaatttg ccattctgct gtacagacac tgattttttt 180

ttcttctttt taaaaagcaa ggtttgtttt cattttggat tttaggtgat gggcaagtca 240

gaaagtcaga tggatataac tgatatcaac actccaaagc caaagaagaa acagcgatgg 300

actcgactgg agatcagcct ctcggtcctt gtcctgctcc tcaccatcat agctgtgaga 360

atgatcgcac tctatgcaac ctacgatgat ggtatttgca agtcatcaga ctgcataaaa 420

tcagctgctc gactgatcca aaacatggat gccaccactg agccttgtag agactttttc 480

aaatatgctt gcggaggctg gttgaaacgt aatgtcattc ccgagaccag ctcccgttac 540

ggcaactttg acattttaag agatgaacta gaagtcgttt tgaaagatgt ccttcaagaa 600

cccaaaactg aagatatagt agcagtgcag aaagcaaaag cattgtacag gtcttgtata 660

aatgaatctg ctattgatag cagaggtgga gaacctctac tcaaactgtt accagacata 720

tatgggtggc cagtagcaac agaaaactgg gagcaaaaat atggtgcttc ttggacagct 780

gaaaaagcta ttgcacaact gaattctaaa tatgggaaaa aagtccttat taatttgttt 840

gttggcactg atgataagaa ttctgtgaat catgtaattc atattgacca acctcgactt 900

ggcctccctt ctagagatta ctatgaatgc actggaatct ataaagaggc ttgtacagca 960

tatgtggatt ttatgatttc tgtggccaga ttgattcgtc aggaagaaag attgcccatc 1020

gatgaaaacc agcttgcttt ggaaatgaat aaagttatgg aattggaaaa agaaattgcc 1080

aatgctacgg ctaaacctga agatcgaaat gatccaatgc ttctgtataa caagatgaga 1140

ttggcccaga tccaaaataa cttttcacta gagatcaatg ggaagccatt cagctggttg 1200

aatttcacaa atgaaatcat gtcaactgtg aatattagta ttacaaatga ggaagatgtg 1260

gttgtttatg ctccagaata tttaaccaaa cttaagccca ttcttaccaa atattctgcc 1320

agagatcttc aaaatttaat gtcctggaga ttcataatgg atcttgtaag cagcctcagc 1380

cgaacctaca aggagtccag aaatgctttc cgcaaggccc tttatggtac aacctcagaa 1440

acagcaactt ggagacgttg tgcaaactat gtcaatggga atatggaaaa tgctgtgggg 1500
aggctttatg tggaagcagc atttgctgga gagagtaaac atgtggtcga ggatttgatt 1560
gcacagatcc gagaagtttt tattcagact ttagatgacc tcacttggat ggatgccgag 1620
acaaaaaaga gagctgaaga aaaggcctta gcaattaaag aaaggatcgg ctatcctgat 1680
gacattgttt caaatgataa caaactgaat aatgagtacc tcgagttgaa ctacaaagaa 1740
gatgaatact tcgagaacat aattcaaaat ttgaaattca gccaaagtaa acaactgaag 1800
aagctccgag aaaaggtgga caaagatgag tggataagtg gagcagctgt agtcaatgca 1860
ttttactctt caggaagaaa tcagatagtc ttcccagccg gcattctgca gccccccttc 1920
tttagtgccc agcagtccaa ctcattgaac tatgggggca tcggcatggt cataggacac 1980
gaaatcaccc atggcttcga tgacaatggc agaaacttta acaaagatgg agacctcgtt 2040
gactggtgga ctcaacagtc tgcaagtaac tttaaggagc aatcccagtg catggtgtat 2100
cagtatggaa acttttcctg ggacctggca ggtggacagc accttaatgg aattaataca 2160
ctgggagaaa acattgctga taatggaggt cttggtcaag catacagagc ctatcagaat 2220
tatattaaaa agaatggcga agaaaaatta cttcctggac ttgacctaaa tcacaaacaa 2280
ctatttttct tgaactttgc acaggtgtgg tgtggaacct ataggccaga gtatgcggtt 2340
aactccatta aaacagatgt gcacagtcca ggcaatttca ggattattgg gactttgcag 2400
aactctgcag agttttcaga agcctttcac tgccgcaaga attcatacat gaatccagaa 2460
aagaagtgcc gggtttggtg atcttcaaaa gaagcattgc agcccttggc tagacttgcc 2520
aacaccacag aaatggggaa ttctctaatc gaaagaaaat gggccctagg ggtcactgta 2580
ctgacttgag ggtgattaac agagagggca ccatcacaat acagataaca ttaggttgtc 2640
ctagaaaggg tgtggaggga ggaagggggt ctaaggtcta tcaagtcaat catttctcac 2700
tgtgtacata atgcttaatt tctaaagata atattactgt ttatttctgt ttctcatatg 2760
gtctaccagt ttgctgatgt ccctagaaaa caatgcaaaa cctttgaggt agaccaggat 2820
ttctaatcaa aagggaaaag aagatgttga agaatagagt taggcaccag aagaagagta 2880
ggtgacacta tagtttaaaa cacattgcct aactactagt ttttactttt atttgcaaca 2940
tttacagtcc ttcaaaatcc ttccaaagaa ttcttataca cattggggcc ttggagctta 3000
catagtttta aactcatttt tgccatacat cagttattca ttctgtgatc atttatttta 3060
agcactctta aagcaaaaaa tgaatgtcta aaattgtttt ttgttgtacc tgctttgact 3120
gatgctgaga ttcttcaggc ttcctgcaat tttctaagca atttcttgct ctatctctca 3180
aaacttggta tttttcagag atttatataa atgtaaaaat aataattttt atatttaatt 3240
attaactaca tttatgagta actattatta taggtaatca atgaatattg aagtttcagc 3300
ttaaaataaa cagttgtgaa ccaagatcta taaagcgata tacagatgaa aatttgagac 3360
tatttaaact tataaatcat attgatgaaa agatttaagc acaaacttta gggtaaaaat 3420
tgcgattgga cagttgtcta gagatatata tacttgtggt tttcaaattg gactttcaaa 3480

attaaatctg tccctgagag tgtctctgat aaaagggcaa atctgcacct atgtagctct 3540
gcatctcctg tcttttcagg tttgtcatca gatggaaata ttttgataat aaattgaaat 3600
tgtgaactca ttgctcccta agactgtgac aactgtctaa ctttagaagt gcatttctga 3660
atagaaatgg gaggcctctg atggaccttc tagaattata agtcacaaag agttctggaa 3720
aagaactgtt tactgcttga taggaattca tcttttgagg cttctgttcc tctcttttcc 3780
tgttgtattg actattttcg ttcattactt gattaagatt ttacaaaaga ggagcacttc 3840
caaaattctt atttttccta acaaaagatg aaagcaggga atttctatct aaatgatgag 3900
tattagttcc ctgtctcttg aaaaatgccc atttgccttt aaaaaaaaaa gttacagaaa 3960
tactataaca tatgtacata aattgcataa agcataagta tacagttcaa taaacttaac 4020
tttaactgaa caatggccct gtagccagca cctgtaagaa acagagcagt accagcgctc 4080
taaaagcacc tccttgtcac tttattactc ccagaacaac aactatcctg acttctaata 4140
tcattcacta gctttgcctg gttttgtctt ttatgcagat agaatcaatc agtatgtatt 4200
cttttgtgcc tggcttcttt ctctcagcct tacatttgtg agattcctct gtattgtgct 4260
gattgtggat cttttcattc tcattgcaga ataatgttct attgtgggac ttattacaat 4320
ttgttcatcc tattgttgat gggcacttga gaactttcca ttttggcgct attacaaata 4380
gtgcaactat gaatgtactg catgttacca tcttacttga gcctttaatg gacttatttc 4440
ttcaaatcct tccaaaaatt attataagca ttgaaattat agtttcaagc caactgtgga 4500
tacccttacc ctttcctcct ttatcacaac caccgttaca agtatactta tatttcccta 4560
aaatacattt aaaacttacc taagtgacat ttgtagttgg agtaatagga gcttccagct 4620
ctaataaaac agctgtctct aacttatttt atttccatca tgtcagagca ggtgaagagc 4680
cagaagtgaa gagtgactag tacaaattat aaaaagccac tagactcttc actgttagct 4740
ttttaaaaca ttaggctccc atccctatgg aggaacaact ctccagtgcc tggatcccct 4800
ctgtctacaa atataagatt ttctgggcct aaaggataga tcaaagtcaa aaatagcaat 4860
gcctccctat ccctcacaca tccagacatc atgaatttta catggtactc ttgttgagtt 4920
ctatagagcc ttctgatgtc tctaaagcac taccgattct ttggagttgt cacatcagat 4980
aagacatatc tctaattcca tccataaatc cagttctact atggctgagt tctggtcaaa 5040
gaaagaaagt ttagaagctg agacacaaag ggttgggagc tgatgaaact cacaaatgat 5100
ggtaggaaga agctctcgac aatacccgtt ggcaaggagt ctgcctccat gctgcagtgt 5160
tcgagtggat tgtaggtgca agatggaaag gattgtaggt gcaagctgtc cagagaaaag 5220
agtccttgtt ccagccctat tctgccactc ctgacagggt gaccttgggt atttgcaata 5280
ttcctttggg cctctgcttc tctcacctaa aaaaagagaa ttagattata ttggtggttc 5340
tcagcaagag aaggagtatg tgtccaatgc tgccttccca tgaatctgtc tcccagttat 5400
gaatcagtgg gcaggataaa ctgaaaactc ccatttaagt gtctgaatcg agtgagacaa 5460
aattttagtc caaataacaa gtaccaaagt tttatcaagt ttgggtctgt gctgctgtta 5520

ctgttaacca tttaagtggg gcaaaacctt gctaattttc tcaaaagcat ttatcattct 5580

tgttgccaca gctggagctc tcaaactaaa agacatttgt tattttggaa agaagaaaga 5640

ctctattctc aaagtttcct aatcagaaat ttttatcagt ttccagtctc aaaaatacaa 5700

aataaaaaca aacgttttta atact 5725

<210> 42
<211> 849
<212> DNA
<213> Homo sapiens

<220>
<223> II.42 (XM_008411)

<400> 42
aaaaggccgg cggaacagcc agaggagcag agaggcaaag aaacattgtg aaatctccaa 60

ctcttaacct tcaacatgaa agtctctgca gtgcttctgt gcctgctgct catgacagca 120

gctttcaacc cccagggact tgctcagcca gatgcactca acgtcccatc tacttgctgc 180

ttcacattta gcagtaagaa gatctccttg cagaggctga agagctatgt gatcaccacc 240

agcaggtgtc cccagaaggc tgtcatcttc agaaccaaac tgggcaagga gatctgtgct 300

gacccaaagg agaagtgggt ccagaattat atgaaacacc tgggccggaa agctcacacc 360

ctgaagactt gaactctgct acccctactg aaatcaagct ggagtacgtg aaatgacttt 420

tccattctcc tctggcctcc tcttctatgc tttggaatac ttctaccata attttcaaat 480

aggatgcatt cggttttgtg attcaaaatg tactatgtgt taagtaatat tggctattat 540

ttgacttgtt gctggtttgg agtttatttg agtattgctg atcttttcta aagcaaggcc 600

ttgagcaagt aggttgctgt ctctaagccc ccttcccttc cactatgagc tgctggcagt 660

gggtttgtat tcggttccca ggggttgaga gcatgcctgt gggagtcatg gacatgaagg 720

gatgctgcaa tgtaggaagg agagctcttt gtgaatgtga ggtgttgcta aatatgttat 780

tgtggaaaga tgaatgcaat agtaggactg ctgacatttt gcagaaaata cattttattt 840

aaaatctcc 849

<210> 43
<211> 2795
<212> DNA
<213> Homo sapiens

<220>
<223> II.43 (XM_055188)

<400> 43
gccaccccag aaggctggag cagggacgcc gtcgctccgg ccgcctgctc ccctcgggtc 60

cccgtgcgag cccacgccgg ccccggtgcc cgcccgcagc cctgccactg gacacaggat 120

aaggcccagc gcacaggccc ccacgtggac agcatggacc gcggcacgct ccctctggct 180

gttgccctgc tgctggccag ctgcagcctc agccccacaa gtcttgcaga aacagtccat 240

tgtgaccttc agcctgtggg ccccgagagg ggcgaggtga catataccac tagccaggtc 300
tcgaagggct gcgtggctca ggcccccaat gccatccttg aagtccatgt cctcttcctg 360
gagttcccaa cgggcccgtc acagctggag ctgactctcc aggcatccaa gcaaaatggc 420
acctggcccc gagaggtgct tctggtcctc agtgtaaaca gcagtgtctt cctgcatctc 480
caggccctgg gaatcccact gcacttggcc tacaattcca gcctggtcac cttccaagag 540
cccccggggg tcaacaccac agagctgcca tccttcccca agacccagat ccttgagtgg 600
gcagctgaga ggggccccat cacctctgct gctgagctga atgaccccca gagcatcctc 660
ctccgactgg gccaagccca ggggtcactg tccttctgca tgctggaagc cagccaggac 720
atgggccgca cgctcgagtg gcggccgcgt actccagcct tggtccgggg ctgccacttg 780
gaaggcgtgg ccggccacaa ggaggcgcac atcctgaggg tcctgccggg ccactcggcc 840
gggccccgga cggtgacggt gaaggtggaa ctgagctgcg cacccgggga tctcgatgcc 900
gtcctcatcc tgcagggtcc cccctacgtg tcctggctca tcgacgccaa ccacaacatg 960
cagatctgga ccactggaga atactccttc aagatctttc cagagaaaaa cattcgtggc 1020
ttcaagctcc cagacacacc tcaaggcctc ctgggggagg cccggatgct caatgccagc 1080
attgtggcat ccttcgtgga gctaccgctg gccagcattg tctcacttca tgcctccagc 1140
tgcggtggta ggctgcagac ctcacccgca ccgatccaga ccactcctcc caaggacact 1200
tgtagcccgg agctgctcat gtccttgatc cagacaaagt gtgccgacga cgccatgacc 1260
ctggtactaa agaaagagct tgttgcgcat ttgaagtgca ccatcacggg cctgaccttc 1320
tgggacccca gctgtgaggc agaggacagg ggtgacaagt ttgtcttgcg cagtgcttac 1380
tccagctgtg gcatgcaggt gtcagcaagt atgatcagca atgaggcggt ggtcaatatc 1440
ctgtcgagct catcaccaca gcggaaaaag gtgcactgcc tcaacatgga cagcctctct 1500
ttccagctgg gcctctacct cagcccacac ttcctccagg cctccaacac catcgagccg 1560
gggcagcaga gctttgtgca ggtcagagtg tccccatccg tctccgagtt cctgctccag 1620
ttagacagct gccacctgga cttggggcct gagggaggca ccgtggaact catccagggc 1680
cgggcggcca agggcaactg tgtgagcctg ctgtccccaa gccccgaggg tgacccgcgc 1740
ttcagcttcc tcctccactt ctacacagta cccataccca aaaccggcac cctcagctgc 1800
acggtagccc tgcgtcccaa gaccgggtct caagaccagg aagtccatag gactgtcttc 1860
atgcgcttga acatcatcag ccctgacctg tctggttgca caagcaaagg cctcgtcctg 1920
cccgccgtgc tgggcatcac ctttggtgcc ttcctcatcg gggccctgct cactgctgca 1980
ctctggtaca tctactcgca cacgcgttcc cccagcaagc gggagcccgt ggtggcggtg 2040
gctgccccgg cctcctcgga gagcagcagc accaaccaca gcatcgggag cacccagagc 2100
accccctgct ccaccagcag catggcatag ccccggcccc ccgcgctcgc ccagcaggag 2160
agactgagca gccgccagct gggagcactg gtgtgaactc accctgggag ccagtcctcc 2220
actcgaccca gaatggagcc tgctctccgc gcctaccctt cccgcctccc tctcagaggc 2280

ctgctgccag tgcagccact ggcttggaac accttggggt ccctccaccc cacagaacct 2340

tcaacccagt gggtctggga tatggctgcc caggagacag accacttgcc acgctgttgt 2400

aaaaacccaa gtccctgtca tttgaacctg gatccagcac tggtgaactg agctgggcag 2460

gaagggagaa cttgaaacag attcaggcca gcccagccag gccaacagca cctccccgct 2520

gggaagagaa gagggcccag cccagagcca cctggatcta tccctgcggc ctccacacct 2580

gaacttgcct aactaactgg caggggagac aggagcctag cggagcccag cctgggagcc 2640

cagagggtgg caagaacagt gggcgttggg agcctagctc ctgccacatg gagccccctc 2700

tgccggtcgg gcagccagca gagggggagt agccaagctg cttgtcctgg gcctgcccct 2760

gtgtattcac caccaataaa tcagaccatg aaacc 2795

<210> 44
<211> 2207
<212> DNA
<213> Homo sapiens

<220>
<223> II.44 (NM_021073)

<400> 44
ctcttgaaga gggctggtat atttgtgcct gctggaggtg gaattaacag taagaaggag 60

aaagggattg aatggactta caggaaggat ttcaagtaaa ttcagggaaa cacatttact 120

tgaatagtac aacctagagt attattttac actaagacga cacaaaagat gttaaagtta 180

tcaccaagct gccggacaga tatatattcc aacaccaagg tgcagatcag catagatctg 240

tgattcagaa atcaggattt gttttggaaa gagctcaagg gttgagaaga actcaaaagc 300

aagtgaagat tactttggga actacagttt atcagaagat caacttttgc taattcaaat 360

accaaaggcc tgattatcat aaattcatat aggaatgcat aggtcatctg atcaaataat 420

attagccgtc ttctgctaca tcaatgcagc aaaaactctt aacaactgtg gataattgga 480

aatctgagtt tcagctttct tagaaataac tactcttgac atattccaaa atatttaaaa 540

taggacagga aaatcggtga ggatgttgtg ctcagaaatg tcactgtcat gaaaaatagg 600

taaatttgtt ttttcagcta ctgggaaact gtacctccta gaaccttagg tttttttttt 660

ttttaagagg acaagaagga ctaaaaatat caacttttgc ttttggacaa aaatgcatct 720

gactgtattt ttacttaagg gtattgtggg tttcctctgg agctgctggg ttctagtggg 780

ttatgcaaaa ggaggtttgg gagacaatca tgttcactcc agttttattt atagaagact 840

acggaaccac gaaagacggg aaatacaaag ggaaattctc tctatcttgg gtttgcctca 900

cagacccaga ccattttcac ctggaaaaca agcgtcctct gcacctctct ttatgctgga 960

tctctacaat gccatgacca atgaagaaaa tcctgaagag tcggagtact cagtaagggc 1020

atccttggca gaagagacca gaggggcaag aaagggatac ccagcctctc ccaatgggta 1080

tcctcgtcgc atacagttat ctcggacgac tcctctgacc acccagagtc ctcctctagc 1140

cagcctccat gataccaact ttctgaatga tgctgacatg gtcatgagct ttgtcaactt 1200

agttgaaaga gacaaggatt tttctcacca gcgaaggcat tacaaagaat ttcgatttga 1260

tcttacccaa attcctcatg gagaggcagt gacagcagct gaattccgga tatacaagga 1320

ccggagcaac aaccgatttg aaaatgaaac aattaagatt agcatatatc aaatcatcaa 1380

ggaatacaca aatagggatg cagatctgtt cttgttagac acaagaaagg cccaagcttt 1440

agatgtgggt tggcttgtct ttgatatcac tgtgaccagc aatcattggg tgattaatcc 1500

ccagaataat ttgggcttac agctctgtgc agaaacaggg gatggacgca gtatcaacgt 1560

aaaatctgct ggtcttgtgg gaagacaggg acctcagtca aaacaaccat tcatggtggc 1620

cttcttcaag gcgagtgagg tacttcttcg atccgtgaga gcagccaaca aacgaaaaaa 1680

tcaaaaccgc aataaatcca gctctcatca ggactcctcc agaatgtcca gtgttggaga 1740

ttataacaca agtgagcaaa aacaagcctg taagaagcac gaactctatg tgagcttccg 1800

ggatctggga tggcaggact ggattatagc accagaagga tacgctgcat tttattgtga 1860

tggagaatgt tcttttccac ttaacgccca tatgaatgcc accaaccacg ctatagttca 1920

gactctggtt catctgatgt ttcctgacca cgtaccaaag ccttgttgtg ctccaaccaa 1980

attaaatgcc atctctgttc tgtactttga tgacagctcc aatgtcattt tgaaaaaata 2040

tagaaatatg gtagtacgct catgtggctg ccactaatat taaataatat tgataataac 2100

aaaaagatct gtattaaggt ttatggctgc aataaaaagc atactttcag acaaacgggg 2160

aatttcctaa aaaaaaaaaa aaaaaaaaaa aaaaaaaaaa aaaaaaa 2207

<210> 45
<211> 2580
<212> DNA
<213> Homo sapiens

<220>
<223> II.45 (XM_007417)

<400> 45
   cctgtttaga cacatggaca agaatcccag cgctacaagg cacacagtcc gcttcttcgt 60
   cctcagggtt gccagcgctt cctggaagtc ctgaagctct cgcagtgcag tgagttcatg 120
   caccttcttg ccaagcctca gtctttggga tctggggagg ccgcctggtt ttcctccctc 180
   cttctgcacg tctgctgggg tctcttcctc tccaggcctt gccgtccccc tggcctctct 240
   tcccagctca cacatgaaga tgcacttgca aagggctctg gtggtcctgg ccctgctgaa 300
   ctttgccacg gtcagcctct ctctgtccac ttgcaccacc ttggacttcg gccacatcaa 360
   gaagaagagg gtggaagcca ttaggggaca gatcttgagc aagctcaggc tcaccagccc 420
   ccctgagcca acggtgatga cccacgtccc ctatcaggtc ctggcccttt acaacagcac 480
   ccgggagctg ctggaggaga tgcatgggga gagggaggaa ggctgcaccc aggaaaacac 540
   cgagtcggaa tactatgcca aagaaatcca taaattcgac atgatccagg ggctggcgga 600
   gcacaacgaa ctggctgtct gccctaaagg aattacctcc aaggtttttc gcttcaatgt 660
   gtcctcagtg gagaaaaata gaaccaacct attccgagca gaattccggg tcttgcgggt 720
   gcccaacccc agctctaagc ggaatgagca gaggatcgag ctcttccaga tccttcggcc 780
   agatgagcac attgccaaac agcgctatat cggtggcaag aatctgccca cacggggcac 840
   tgccgagtgg ctgtcctttg atgtcactga cactgtgcgt gagtggctgt tgagaagaga 900
   gtccaactta ggtctagaaa tcagcattca ctgtccatgt cacacctttc agcccaatgg 960
   agatatcctg gaaaacattc acgaggtgat ggaaatcaaa ttcaaaggcg tggacaatga 1020
   ggatgaccat ggccgtggag atctggggcg cctcaagaag cagaaggatc accacaaccc 1080
   tcatctaatc ctcatgatga ttcccccaca ccggctcgac aacccgggcc aggggggtca 1140
   gaggaagaag cgggctttgg acaccaatta ctgcttccgc aacttggagg agaactgctg 1200
   tgtgcgcccc ctctacattg acttccgaca ggatctgggc tggaagtggg tccatgaacc 1260
   taagggctac tatgccaact tctgctcagg cccttgccca tacctccgca gtgcagacac 1320
   aacccacagc acggtgctgg gactgtacaa cactctgaac cctgaagcat ctgcctcgcc 1380
   ttgctgcgtg ccccaggacc tggagcccct gaccatcctg tactatgttg ggaggacccc 1440
   caaagtggag cagctctcca acatggtggt gaagtcttgt aaatgtagct gagaccccac 1500
   gtgcgacaga gagaggggag agagaaccac cactgcctga ctgcccgctc ctcgggaaac 1560
   acacaagcaa caaacctcac tgagaggcct ggagcccaca accttcggct ccgggcaaat 1620
   ggctgagatg gaggtttcct tttggaacat ttctttcttg ctggctctga gaatcacggt 1680
   ggtaaagaaa gtgtgggttt ggttagagga aggctgaact cttcagaaca cacagacttt 1740
   ctgtgacgca gacagagggg atggggatag aggaaaggga tggtaagttg agatgttgtg 1800
   tggcaatggg atttgggcta ccctaaaggg agaaggaagg gcagagaatg gctgggtcag 1860
   ggccagactg gaagacactt cagatctgag gttggatttg ctcattgctg taccacatct 1920
   gctctaggga atctggatta tgttatacaa ggcaagcatt tttttttttt ttttaaagac 1980
   aggttacgaa gacaaagtcc cagaattgta tctcatactg tctgggatta agggcaaatc 2040
   tattactttt gcaaactgtc ctctacatca attaacatcg tgggtcacta cagggagaaa 2100
   atccaggtca tgcagttcct ggcccatcaa ctgtattggg ccttttggat atgctgaacg 2160
   cagaagaaag ggtggaaatc aaccctctcc tgtctgccct ctgggtccct cctctcacct 2220
   ctccctcgat catatttccc cttggacact tggttagacg ccttccaggt caggatgcac 2280
   atttctggat tgtggttcca tgcagccttg gggcattatg ggttcttccc ccacttcccc 2340
   tccaagaccc tgtgttcatt tggtgttcct ggaagcaggt gctacaacat gtgaggcatt 2400
   cggggaagct gcacatgtgc cacacagtga cttggcccca gacgcataga ctgaggtata 2460
   aagacaagta tgaatattac tctcaaaatc tttgtataaa taaatatttt tggggcatcc 2520
   tggatgattt catcttctgg aatattgttt ctagaacagt aaaagcctta ttctaaggtg 2580

<210> 46
<211> 2898
<212> DNA

<213>  Homo sapiens

<220>
<223>  II.46 (NM_001495)

<400>  46
   tttgattgta gcgggcaagg ggggcgtcga gaggcagcag cccatcgccc gcctctcacc      60
   ccaccccctc cagccagagg cgagaatcgc aggactcggg atcttcatcg ggtggactag     120
   ctgggatctc cgcattggat ttggggctga ttaccactgc ttgcctatta ttattgttgt     180
   tgttactact attatttttt tttacccaag ggagaaagac aaaaaaacgg tgggatttat     240
   ttaacatgat cttggcaaac gtcttctgcc tcttcttctt tctagacgag accctccgct     300
   ctttggccag cccttcctcc ctgcagggcc ccgagctcca cggctggcgc cccccagtgg     360
   actgtgtccg ggccaatgag ctgtgtgccg ccgaatccaa ctgcagctct cgctaccgca     420
   ctctgcggca gtgcctggca ggccgcgacc gcaacaccat gctggccaac aaggagtgcc     480
   aggcggcctt ggaggtcttg caggagagcc cgctgtacga ctgccgctgc aagcggggca     540
   tgaagaagga gctgcagtgt ctgcagatct actggagcat ccacctgggg ctgaccgagg     600
   gtgaggagtt ctacgaagcc tccccctatg agccggtgac ctcccgcctc tcggacatct     660
   tcaggcttgc ttcaatcttc tcagggacag gggcagaccc ggtggtcagc gccaagagca     720
   accattgcct ggatgctgcc aaggcctgca acctgaatga caactgcaag aagctgcgct     780
   cctcctacat ctccatctgc aaccgcgaga tctcgcccac cgagcgctgc aaccgccgca     840
   agtgccacaa ggccctgcgc cagttcttcg accgggtgcc cagcgagtac acctaccgca     900
   tgctcttctg ctcctgccaa gaccaggcgt gcgctgagcg ccgccggcaa accatcctgc     960
   ccagctgctc ctatgaggac aaggagaagc ccaactgcct ggacctgcgt ggcgtgtgcc    1020
   ggactgacca cctgtgtcgg tcccggctgg ccgacttcca tgccaattgt cgagcctcct    1080
   accagacggt caccagctgc cctgcggaca attaccaggc gtgtctgggc tcttatgctg    1140
   gcatgattgg gtttgacatg acacctaact atgtggactc cagccccact ggcatcgtgg    1200
   tgtccccctg gtgcagctgt cgtggcagcg ggaacatgga ggaggagtgt gagaagttcc    1260
   tcagggactt caccgagaac ccatgcctcc ggaacgccat ccaggccttt ggcaacggca    1320
   cggacgtgaa cgtgtcccca aaaggcccct cgttccaggc cacccaggcc cctcgggtgg    1380
   agaagacgcc ttctttgcca gatgacctca gtgacagtac cagcttgggg accagtgtca    1440
   tcaccacctg cacgtctgtc caggagcagg ggctgaaggc caacaactcc aaagagttaa    1500
   gcatgtgctt cacagagctc acgacaaata tcatcccagg gagtaacaag gtgatcaaac    1560
   ctaactcagg ccccagcaga gccagaccgt cggctgcctt gaccgtgctg tctgtcctga    1620
   tgctgaaaca ggccttgtag gctgtgggaa ccgagtcaga atatttttga aagctacgca    1680
   gacaagaaca gccgcctgac gaaatggaaa cacacacaga cacacacaca ccttgcaaaa    1740
   aaaaaattgt ttttcccacc ttgtcgctga acctgtctcc tcccaggttt cttctctgga    1800
   gaagtttttg taaaccaaac agacaagcag gcaggcagcc tgagagctgg cccaggggtc    1860
   ccctggcagg ggaaactctg gtgccgggga gggcacgagg ctctagaaat gcccttcact 1920
   ttctcctggt gtttttctct ctggaccctt ctgaagcaga gaccggacaa gagcctgcag 1980
   cggaagggac tctgggctgt gcctgaggct ggctgggggc aggacaacac agctgcttcc 2040
   ccaggctgcc cactctgggg acccgctggg ggctggcaga gggcatcggt cagcggggca 2100
   gcggggctgg ccatgagggt ccaccttcag ccctttggct tcaaggatgg agatggtttt 2160
   gccctccctc tctgccctcg ggtggggctg gtgggtctgc agctggtgtg ggaacttccc 2220
   cacggatggc ggtggagggg gttcgcaccg tgctgggctc cccctgactg tagcacggag 2280
   tgttggggct gggggccagc tccaggaggg cttgagagct cagcctgcct gggagagccc 2340
   ttgtggcgag gcattaaaac ttgggcacca gcttctttct cggtggcaga aattttgaag 2400
   tcagagagaa acggtccttt gttggcttct ttgctttctc gtgggtcctt tggcaggcct 2460
   ccctttgggg agagggaggg gagagaccac agccgggtgt gtgtctgcag caccgtgggc 2520
   cctcaagctt tcctgctgtc ttctccctcc tcctcctttc ccctttctct ttcctcattt 2580
   cctagacgta cgtcaactgt atgtacatac cggggctcct ctcctaacat atatgtatat 2640
   acacatccat atacatatat tgtgtggttt cccctttctt tccttttttt aagcaacaaa 2700
   actatggaaa taatacccca acagatgagc gaaaatgtat tattgtaaag tttatttttt 2760
   ttaatactgt tgtctataat ggggaaaaag gacattggcc ccgcagtgcc ctgccccagt 2820
   cagcctggct gggctctggt gggggctcct gatccgcatc caagcttaac caaggctcca 2880
   ataaacgtgc taggaagc 2898

<210> 47
<211> 2173
<212> DNA
<213> Homo sapiens

<220>
<223> II.47 (XM_009475)

<400> 47
aaaggcgtcg gcgccacgcg catatccctg ctcggcgctg cccgcccagt tcctgttccc 60
agactgaggc ccagccccct tcgcccgttt ccatcacgag tgccgccagc atgtctgaca 120
aactgcccta caaagtcgcc gacatcggcc tggctgcctg gggacgcaag gccctggaca 180
ttgctgagaa cgagatgccg ggcctgatgc gtatgcggga gcggtactcg gcctccaagc 240
cactgaaggg cgcccgcatc gctggctgcc tgcacatgac cgtggagacg gccgtcctca 300
ttgagaccct cgtcaccctg ggtgctgagg tgcagtggtc cagctgcaac atcttctcca 360
cccaggacca tgcggcggct gccattgcca aggctggcat tccggtgtat gcctggaagg 420
gcgaaacgga cgaggagtac ctgtggtgca ttgagcagac cctgtacttc aaggacgggc 480
ccctcaacat gattctggac gacgggggcg acctcaccaa cctcatccac accaagtacc 540
cgcagcttct gccaggcatc cgaggcatct ctgaggagac cacgactggg gtccacaacc 600

tctacaagat gatggccaat gggatcctca aggtgcctgc catcaatgtc aatgactccg 660

tcaccaagag caagtttgac aacctctatg gctgccggga gtccctcata gatggcatca 720

agcgggccac agatgtgatg attgccggca aggtagcggt ggtagcaggc tatggtgatg 780

tgggcaaggg ctgtgcccag gccctgcggg gtttcggagc ccgcgtcatc atcaccgaga 840

ttgaccccat caacgcactg caggctgcca tggagggcta tgaggtgacc accatggatg 900

aggcctgtca ggagggcaac atctttgtca ccaccacagg ctgtattgac atcatccttg 960

gccggcactt tgagcagatg aaggatgatg ccattgtgtg taacattgga cactttgacg 1020

tggagatcga tgtcaagtgg ctcaacgaga acgccgtgga gaaggtgaac atcaagccgc 1080

aggtggaccg gtatcggttg aagaatgggc gccgcatcat cctgctggcc gagggtcggc 1140

tggtcaacct gggttgtgcc atgggccacc ccagcttcgt gatgagtaac tccttcacca 1200

accaggtgat ggcgcagatc gagctgtgga cccatccaga caagtacccc gttggggttc 1260

atttcctgcc caagaagctg gatgaggcag tggctgaagc ccacctgggc aagctgaatg 1320

tgaagttgac caagctaact gagaagcaag cccagtacct gggcatgtcc tgtgatggcc 1380

ccttcaagcc ggatcactac cgctactgag agccaggtct gcgtttcacc ctccagctgc 1440

tgtccttgcc caggccccac ctctcctccc taagagctaa tggcaccaac tttgtgattg 1500

gtttgtcagt gtcccccatc gactctctgg ggctgatcac ttagtttttg gcctctgctg 1560

cagccgtcat actgttccaa atgtggcagc gggaacagag taccctcttc aagccccggt 1620

catgatggag gtcccagcca cagggaacca tgagctcagt ggtcttggaa cagctcacta 1680

agtcagtcct tccttagcct ggaagtcagt agtggagtca caaagcccat gtgttttgcc 1740

atctaggcct tcacctggtc tgtggactta tacctgtgtg cttggtttac aggtccagtg 1800

gttcttcagc ccatgacaga tgagaagggg ctatattgaa gggcaaagag gaactgttgt 1860

ttgaattttc ctgagagcct ggcttagtgc tgggccttct cttaaacctc attacaatga 1920

ggttagtact tttagtccct gttttacagg ggttagaata gactgttaag gggcaactga 1980

gaaagaacag agaagtgaca gctaggggtt gagaggggcc agaaaaacat gaatgcaggc 2040

agatttcgtg aaatctgcca ccactttata accagatggt tcctttcaca accctgggtc 2100

aaaaagagaa taatttggcc tataatgtta aaagaaagca ggaaggtggg taaataaaaa 2160

tcttggtgcc tgg 2173

<210> 48
<211> 2485
<212> DNA
<213> Homo sapiens

<220>
<223> II.48 (XM_006738)

<400> 48
agaacgtgcg catcgacccc agtagcctgt ccttcaacat gtggaaggag atccctatcc 60

ccttctatct ctccgtctac ttctttgacg tcatgaaccc cagcgagatc ctgaagggcg 120

agaagccgca ggtgcgggag cgcgggccct acgtgtacag ggagttcagg cacaaaagca 180
acatcacctt caacaacaac gacaccgtgt ccttcctcga gtaccgcacc ttccagttcc 240
agccctccaa gtcccacggc tcggagagcg actacatcgt catgcccaac atcctggtct 300
tgggtgcggc ggtgatgatg gagaataagc ccatgaccct gaagctcatc atgaccttgg 360
cattcaccac cctcggcgaa cgtgccttca tgaaccgcac tgtgggtgag atcatgtggg 420
gctacaagga cccccttgtg aatctcatca acaagtactt tccaggcatg ttccccttca 480
aggacaagtt cggattattt gctgagctca acaactccga ctctgggctc ttcacggtgt 540
tcacgggggt ccagaacatc agcaggatcc acctcgtgga caagtggaac gggctgagca 600
aggttgactt ctggcattcc gatcagtgca acatgatcaa tggaacttct gggcaaatgt 660
ggccgccctt catgactcct gagtcctcgc tggagttcta cagcccggag gcctgccgat 720
ccatgaagct aatgtacaag gagtcagggg tgtttgaagg catccccacc tatcgcttcg 780
tggctcccaa aaccctgttt gccaacgggt ccatctaccc acccaacgaa ggcttctgcc 840
cgtgcctgga gtctggaatt cagaacgtca gcacctgcag gttcagtgcc cccttgtttc 900
tctcccatcc tcacttcctc aacgctgacc cggttctggc agaagcggtg actggcctgc 960
accctaacca ggaggcacac tccttgttcc tggacatcca cccggtcacg ggaatcccca 1020
tgaactgctc tgtgaaactg cagctgagcc tctacatgaa atctgtcgca ggcattggac 1080
aaactgggaa gattgagcct gtggtcctgc cgctgctctg gtttgcagag agcggggcca 1140
tggaggggga gactcttcac acattctaca ctcagctggt gttgatgccc aaggtgatgc 1200
actatgccca gtacgtcctc ctggcgctgg gctgcgtcct gctgctggtc cctgtcatct 1260
gccaaatccg gagccaagta ggtgctggcc agagggcagc ccgggctgac agccattcgc 1320
ttgcctgctg ggggaaaggg gcctcagatc ggaccctctg gccaaccgca gcctggagcc 1380
cacctccagc agcagtcctg cgtctctgcc ggagtgggag cggtcactgc tgggggctgc 1440
gcagcacgct tgcgtctttt gcatgccgcg ttgccactac tctgcctgtt ctggaaggcc 1500
tgggaccctc ccttggaggg ggcacagggt cctgaggaca ccgtgagcca gccaggcctg 1560
gccgctgggc ctgaccggcc ccccagcccc tacaccccgc ttctcccgga ctctcccagc 1620
ggacagcccc ccagccccac agcctgagcc tcccagctgc catgtgcctg ttgcacacct 1680
gcacacacgc cctggcacac atacacacat gcgtgcaggc ttgtgcagac actcagggat 1740
ggagctgctg ctgaagggac ttgtagggag aggctcgtca acaagcactg ttctggaacc 1800
ttctctccac gtggcccaca ggcctgacca caggggctgt gggtcctgcg tccccttcct 1860
cgggtgagcc tggcctgtcc cgttcagccg ttgggcccag gcttcctccc ctccaaggtg 1920
aaacactgca gtcccggtgt ggtggctccc catgcaggac gggccaggct gggagtgccg 1980
ccttcctgtg ccaaattcag tggggactca gtgcccaggc cctggccacg agctttggcc 2040
ttggtctacc tgccaggcca ggcaaagcgc ctttacacag gcctcggaaa acaatggagt 2100
gagcacaaga tgccctgtgc agctgcccga gggtctccgc ccaccccggc cggactttga 2160

tccccccgaa gtcttcacag gcactgcatc gggttgtctg gcgccctttt cctccagcct 2220

aaactgacat catcctatgg actgagccgg ccactctctg gccgaagtgg ccgcaggctg 2280

tgcccccgag ctgcccccac cccctcacag ggtccctcag attataggtg cccaggctga 2340

ggtgaagagg cctgggggcc ctgccttccg ggcgctcctg gaccctgggg caaacctgtg 2400

acccttttct actggaatag aaatgagttt tatcatcttt gaaaaataat tcactcttga 2460

agtaataaac gtttaaaaaa atggg 2485

<210> 49
<211> 1437
<212> DNA
<213> Homo sapiens

<220>
<223> II.49 (NM_001772)

<400> 49
gcttcctcag acatgccgct gctgctactg ctgcccctgc tgtgggcagg ggccctggct 60

atggatccaa atttctggct gcaagtgcag gagtcagtga cggtacagga gggtttgtgc 120

gtcctcgtgc cctgcacttt cttccatccc ataccctact acgacaagaa ctccccagtt 180

catggttact ggttccggga aggagccatt atatccgggg actctccagt ggccacaaac 240

aagctagatc aagaagtaca ggaggagact cagggcagat tccgcctcct tggggatccc 300

agtaggaaca actgctccct gagcatcgta gacgccagga ggagggataa tggttcatac 360

ttctttcgga tggagagagg aagtaccaaa tacagttaca aatctcccca gctctctgtg 420

catgtgacag acttgaccca caggcccaaa atcctcatcc ctggcactct agaacccggc 480

cactccaaaa accttacctg ctctgtgtcc tgggcctgtg agcagggaac acccccgatc 540

ttctcctggt tgtcagctgc ccccacctcc ctgggcccca ggactactca ctcctcggtg 600

ctcataatca ccccacggcc ccaggaccac ggcaccaacc tgacctgtca ggtgaagttc 660

gctggagctg gtgtgactac ggagagaacc atccagctca acgtcaccta tgttccacag 720

aacccaacaa ctggtatctt tccaggagat ggctcaggga aacaagagac cagagcagga 780

ctggttcatg gggccattgg aggagctggt gttacagccc tgctcgctct ttgtctctgc 840

ctcatcttct tcatagtgaa gacccacagg aggaaagcag ccaggacagc agtgggcagc 900

aatgacaccc accctaccac agggtcagcc tccccgaaac accagaagaa ctccaagtta 960

catggcccca ctgaaacctc aagctgttca ggtgccgccc ctactgtgga gatggatgag 1020

gagctgcatt atgcttccct caactttcat gggatgaatc cttccaagga cacctccacc 1080

gaatactcag aggtcaggac ccagtgagga accctcaaga gcatcaggct cagctagaag 1140

atccacatcc tctacaggtc ggggaccaaa ggctgattct tggagattta actccccaca 1200

ggcaatgggt ttatagacat tatgtgagtt tcctgctata ttaacatcat cttgagactt 1260

tgcaagcaga gagtcgtgga atcaaatctg tgctctttca tttgctaagt gtatgatgtc 1320

acacaagctc cttaaccttc catgtctcca ttttcttctc tgtgaagtag gtataagaag 1380

tcctatctca tagggatgct gtgagcatta aataaaggta cacatggaaa acaccag 1437

<210> 50
<211> 3404
<212> DNA
<213> Homo sapiens

<220>
<223> II.50 (NM_057158)

<400> 50
gcagttcaga cccccccaca cccatcaaag agccgctcct cccccccgca ggcgccttcg 60

ccgcctccct cccttccttt cctttccgct cctcttccga cctgtccacc cgggaggaag 120

ggagctggaa agggggcgga aacctctccc ctccaaaaag cacaacaaaa ctgttcagtg 180

cggaggagcc gggttcgccc ctgccggaca gcggggggct ttgttccccg cagttgtttc 240

ctgcccattt gacctgtcag ctgctgggga aacgctgctg ttgacctttg gttgaactgc 300

taaggcgatt ttgctgattt ttctttcttt ttccgcgagg gctgtctttt gctcctccaa 360

atgagcccag tccccctccc ttctccccaa agcgctccaa gagaaagtgc caggaagggg 420

cttgtcccgg aaggcctggc ggctgagcgg ggccaggtcc tggttaggcc accagggtgg 480

gcgtccgcgc cattgtttga gcttgtcggc gctggtggga gagatgaggg caattcctct 540

gggacgcaag tcccctcgaa tggccggggc tggccgggat gttccccgca cggcgctgcc 600

ctcgagtccc cccgatggag agcgcgggcg cgccttcctt cgctggcgtc caaacccggg 660

accagctaga acacagcagg gctgggactg ggttccagcc ccacgtggag tctggatttg 720

ttttgttgtg ttttgctttc cttcctggaa gaaatcccga ggggaccgcc ctagagcggc 780

agctccagga cctcggccct tgggcttccg ggggtgcagc cacttaggcc ccgctcccgg 840

ggagagaggg attatttttt aagatttatc cccagggcgc gcggcatttc cctgtccctc 900

gtgaatcccg ttgagagtcc tccctcccca acctcctcca tttccccagc cagaccgatt 960

cgagagccct ggagattctg ggcgaggcta gtgactgggt agtacaggcc tctagcccca 1020

ccattgctct ctctgtcttc agttccccag gagggcaatg gcatcaaaca gcacagctct 1080

gggggatgtc aatattgcat accttttcta cctaaaggga aaatgactcg cttttctgct 1140

tgcaaatatg gtagtttctg cttacaaatg taatacaatg cccatgacag ccaaggactg 1200

gaagcataag ttgctaggtc ttacaggtga ttttttacaa tgaagcaaac tcactatgtt 1260

agacaccatt tacattggat gtctccaact aacaaaagta actaaagaca gatgtaggtg 1320

taaattgaga gtgaaatttg accctttaga ccgtcacaac ttccttgggc ttatcctggg 1380

tgcttatagg agaggtgggc tccacccaca aaaatggact gctcagaaaa atgagggaga 1440

gagaaagggt ggccactttc ccgagccaag aaattccttg aaaaaaaatc agaacatctg 1500

aaaccagaga gccgatttcc ttaccgggag gcagttcctg gctaacgaag aggaagcacg 1560

atgggaagaa aagttcactc caacggaagc cagtttgctg aacatagcag atcgcccagg 1620

aggactggga gagactgcaa accagttcga gcccccagca tggcgttagg tgtcagccag 1680
ctggcaggaa ggtccaggtg tctgtgttca gagtctcaag gcggctatga gaggttttcc 1740
tccgagtacc cagaattctg ttctaaaacc aaggccctgg cagccatccc acccccggtt 1800
ccccccagtg ccacagagcc cttggacctg ggctgcagct cctgtgggac cccactacac 1860
gaccaggggg gtcctgtgga gatccttccc ttcctctacc tcggcagtgc ctaccatgct 1920
gcccggagag acatgctgga cgccctgggc atcacggctc tgttgaatgt ctcctcggac 1980
tgcccaaacc actttgaagg acactatcag tacaagtgca tcccagtgga agataaccac 2040
aaggccgaca tcagctcctg gttcatggaa gccatagagt acatcgatgc cgtgaaggac 2100
tgccgtgggc gcgtgctggt gcactgccag gcgggcatct cgcggtcggc caccatctgc 2160
ctggcctacc tgatgatgaa gaaacgggtg aggctggagg aggccttcga gttcgttaag 2220
cagcgccgca gcatcatctc gcccaacttc agcttcatgg ggcagctgct gcagttcgag 2280
tcccaggtgc tggccacgtc ctgtgctgcg gaggctgcta gcccctcggg acccctgcgg 2340
gagcggggca agacccccgc cacccccacc tcgcagttcg tcttcagctt tccggtctcc 2400
gtgggcgtgc actcggcccc cagcagcctg ccctacctgc acagccccat caccacctct 2460
cccagctgtt agagccgccc tgggggcccc agaaccagag ctggctccca gcaagggtag 2520
gacgggccgc atgcgggcag aaagttggga ctgagcagct gggagcaggc gaccgagctc 2580
cttccccatc atttctcctt ggccaacgac gaggccagcc agaatggcaa taaggactcc 2640
gaatacataa taaaagcaaa cagaacactc caacttagag caataacggc tgccgcagca 2700
gccagggaag accttggttt ggtttatgtg tcagtttcac ttttccgata gaaatttctt 2760
acctcatttt tttaagcagt aaggcttgaa gtgatgaaac ccacagatcc tagcaaatgt 2820
gcccaaccag ctttactaaa gggggaggaa gggagggcaa agggatgaga agacaagttt 2880
cccagaagtg cctggttctg tgtacttgtc cctttgttgt cgttgttgta gttaaaggaa 2940
tttcattttt taaaagaaat cttcgaaggt gtggttttca tttctcagtc accaacagat 3000
gaataattat gcttaataat aaagtattta ttaagacttt cttcagagta tgaaagtaca 3060
aaaagtctag ttacagtgga tttagaatat atttatgttg atgtcaaaca gctgagcacc 3120
gtagcatgca gatgtcaagg cagttaggaa gtaaatggtg tcttgtagat atgtgcaagg 3180
tagcatgatg agcaacttga gtttgttgcc actgagaagc aggcgggttg ggtgggagga 3240
ggaagaaagg gaagaattag gtttgaattg ctttttaaaa aaaaaagaaa agaaaaagac 3300
agcatctcac tatgttgcca aggctcatct tgagaagcag gcgggttggg tgggaggagg 3360
aagaaaggga agaattaggt ttgaattgct tttttaaaaa aaaa 3404

<210> 51
<211> 2202
<212> DNA
<213> Homo sapiens

<220>
<223>  II.51 (XM_058179)

<400>  51
aggatgccag ggatcagctg accatgtggt tagcatctcg ggagtgcctc ttcagttaca      60

accaaacagc atacagacga aggttgacag cattgcatgg aagaagttgc tgccctcaca     120

aaatggattt catcacatat tgaagtggga gaatggctct ttgccttcca atacttccaa     180

tgatagattc agttttatag tcaagaactt gagtcttctc atcaaggcag ctcagcagca     240

ggacagtggc ctctactgcc tggaggtcac cagtatatct ggaaaagttc agacagccac     300

gttccaggtt tttgtatttg ataaagttga gaaaccccgc ctacaggggc aggggaagat     360

cctggacaga gggagatgcc aagtggctct gtcttgcttg gtctccaggg atggcaatgt     420

gtcctatgct tggtacagag ggagcaagct gatccagaca gcagggaacc tcacctacct     480

ggacgaggag gttgacatta atggcactca cacatatacc tgcaatgtca gcaatcctgt     540

tagctgggaa agccacaccc tgaatctcac tcaggactgt cagaatgccc atcaggaatt     600

cagattttgg ccgtttttgg tgatcatcgt gattctaagc gcactgttcc ttggcaccct     660

tgcctgcttc tgtgtgtgga ggagaaagag gaaggagaag cagtcagaga ccagtcccaa     720

ggaatttttg acaatttacg aagatgtcaa ggatctgaaa accaggagaa atcacgagca     780

ggagcagact tttcctggag gggggagcac catctactct atgatccagt cccagtcttc     840

tgctcccacg tcacaagaac ctgcatatac attatattca ttaattcagc cttccaggaa     900

gtctggatcc aggaagagga accacagccc ttccttcaat agcactatct atgaagtgat     960

tggaaagagt caacctaaag cccagaaccc tgctcgattg agccgcaaag agctggagaa    1020

ctttgatgtt tattcctagt tgctgcagca attctcacct ttcttgcaca tcagcatctg    1080

ctttgggaat tggcacagtg gatgacggca caggagtctc tatagaacac ttcctagtct    1140

ggagaggata tggaaatttg ttcttgttct atattttgtt ttgaaaatga tgtctaacaa    1200

ccatgataag agcaaggctg ttaaataata tcttccaatt tacagatcag acatgaatgg    1260

gtggaggggt taggttgttc acaaaaggcc acattccaag tatttgtaat ctagaaagtg    1320

ttatgtaagt gatgttatta gcatcgagat tccctccacc tgattttcaa gctgtcactt    1380

gtttcctttt ctcccctctc tgggttgact gcatttctag actctcgccg gcccaggccc    1440

atcttccaaa gcaagaggaa ggaatgataa tggtgactca ggggaagaag aaacagccct    1500

cctctgaaag cctggactgt ccggctgtga actggctggc aggttctgca cgtgggtggg    1560

ggccagggcc tgggctttac tcaattgcag agaaaaaact ttctccctgc atctcatacc    1620

tttacctctg gccagttggc caccaggggg agtgggctga agggagagta gatggtgcaa    1680

agcaagccca tctctgagta gaaaaatcac ccagagcaca tgctgacctg ataactgggg    1740

tgttgagacc agctttgtcc atggtatgat gtttgattta tgaagacgca ttgttagaaa    1800

tccatttggc ttcttcatag aagtggcttc ccagaggaag aggcctctca gaaaccatgt    1860

tctatttaag ttctgagtcc tgatgagtgt tccccaggat gcacattgaa gggagggctc    1920

aggcagctga gggctgagaa tgaggcagtt ggaatctaga cactatgctg ggttccctga 1980

gtcgtcaggc cagacatttc aacaaggctg tggggagcag ggctgtgact ctggctgagc 2040

ccaggaaagc gacaagggtg aactgggaga ggacttactc agagacccca acaggtgata 2100

ctgcacaaag cctggttctt caattttcct accctgtatc taacatagga gtttcatata 2160

aaacggtgat atcatgcaga tgcagtctga attccttgcc tg 2202

<210> 52
<211> 1932
<212> DNA
<213> Homo sapiens

<220>
<223> II.52 (NM_001770)

<400> 52
gcatcccctg cgcgaagctg ggtgccccgg agagtctgac caccatgcca cctcctcgcc 60

tcctcttctt cctcctcttc ctcaccccca tggaagtcag gcccgaggaa cctctagtgg 120

tgaaggtgga agagggagat aacgctgtgc tgcagtgcct caaggggacc tcagatggcc 180

ccactcagca gctgacctgg tctcgggagt ccccgcttaa acccttctta aaactcagcc 240

tggggctgcc aggcctggga atccacatga ggcccctggc catctggctt ttcatcttca 300

acgtctctca acagatgggg ggcttctacc tgtgccagcc ggggcccccc tctgagaagg 360

cctggcagcc tggctggaca gtcaatgtgg agggcagcgg ggagctgttc cggtggaatg 420

tttcggacct aggtggcctg ggctgtggcc tgaagaacag gtcctcagag ggccccagct 480

ccccttccgg gaagctcatg agccccaagc tgtatgtgtg ggccaaagac cgccctgaga 540

tctgggaggg agagcctccg tgtgtcccac cgagggacag cctgaaccag agcctcagcc 600

aggacctcac catggcccct ggctccacac tctggctgtc ctgtggggta ccccctgact 660

ctgtgtccag ggggcccctc tcctggaccc atgtgcaccc caaggggcct aagtcattgc 720

tgagcctaga gctgaaggac gatcgcccgg ccagagatat gtgggtaatg gagacgggtc 780

tgttgttgcc ccgggccaca gctcaagacg ctggaaagta ttattgtcac cgtggcaacc 840

tgaccatgtc attccacctg gagatcactg ctcggccagt actatggcac tggctgctga 900

ggactggtgg ctggaaggtc tcagctgtga ctttggctta tctgatcttc tgcctgtgtt 960

cccttgtggg cattcttcat cttcaaagag ccctggtcct gaggaggaaa agaaagcgaa 1020

tgactgaccc caccaggaga ttcttcaaag tgacgcctcc cccaggaagc gggccccaga 1080

accagtacgg gaacgtgctg tctctcccca cacccacctc aggcctcgga cgcgcccagc 1140

gttgggccgc aggcctgggg ggcactgccc cgtcttatgg aaacccgagc agcgacgtcc 1200

aggcggatgg agccttgggg tcccggagcc cgccgggagt gggcccagaa gaagaggaag 1260

gggagggcta tgaggaacct gacagtgagg aggactccga gttctatgag aacgactcca 1320

accttgggca ggaccagctc tcccaggatg gcagcggcta cgagaaccct gaggatgagc 1380

ccctgggtcc tgaggatgaa gactccttct ccaacgctga gtcttatgag aacgaggatg 1440

aagagctgac ccagccggtc gccaggacaa tggacttcct gagccctcat gggtcagcct 1500

gggaccccag ccgggaagca acctccctgg ggtcccagtc ctatgaggat atgagaggaa 1560

tcctgtatgc agccccccag ctccgctcca ttcggggcca gcctggaccc aatcatgagg 1620

aagatgcaga ctcttatgag aacatggata atcccgatgg gccagaccca gcctggggag 1680

gagggggccg catgggcacc tggagcacca ggtgatcctc aggtggccag cctggatctc 1740

ctcaagtccc caagattcac acctgactct gaaatctgaa gacctcgagc agatgatgcc 1800

aacctctgga gcaatgttgc ttaggatgtg tgcatgtgtg taagtgtgtg tgtgtgtgtg 1860

tgtgtgtata catgccagtg acacttccag tcccctttgt attccttaaa taaactcaat 1920

gagctcttcc aa 1932

<210> 53
<211> 1410
<212> DNA
<213> Homo sapiens

<220>
<223> II.53 (NM_004931)

<400> 53
gcgactgtct ccgccgagcc cccggggcca ggtgtcccgg gcgcgccacg atgcggccgc 60

ggctgtggct cctcctggcc gcgcagctga cagttctcca tggcaactca gtcctccagc 120

agacccctgc atacataaag gtgcaaacca acaagatggt gatgctgtcc tgcgaggcta 180

aaatctccct cagtaacatg cgcatctact ggctgagaca gcgccaggca ccgagcagtg 240

acagtcacca cgagttcctg gccctctggg attccgcaaa agggactatc cacggtgaag 300

aggtggaaca ggagaagata gctgtgtttc gggatgcaag ccggttcatt ctcaatctca 360

caagcgtgaa gccggaagac agtggcatct acttctgcat gatcgtcggg agccccgagc 420

tgaccttcgg gaagggaact cagctgagtg tggttgattt ccttcccacc actgcccagc 480

ccaccaagaa gtccaccctc aagaagagag tgtgccggtt acccaggcca gagacccaga 540

agggcccact ttgtagcccc atcacccttg gcctgctggt ggctggcgtc ctggttctgc 600

tggtttccct gggagtggcc atccacctgt gctgccggcg gaggagagcc cggcttcgtt 660

tcatgaaaca attttacaaa tgagcagaga atacggtttt ggtgtcctgc tacaaaaaga 720

catcggtcag taacgagcac gatgtggaaa aatgagagaa gggacacatt caaccctgga 780

gagttcaatg gctgctgaag ctgcctgctt ttcactgctg caaggccttt ctgtgtgtga 840

tgtgcatggg agcaacttgt tcgtgggtca tcgggaatac tagggagaag gtttcattgc 900

ccccagggca cttcacagag tgtgctggag gactgagtaa gaaatgctgc ccatgccacc 960

gcttccggct cctgtgcttt ccctgaactg ggacctttag tggtggccat ttagccacca 1020

tctttgcagg ttgctttgcc ctggtagggc agtaacattg ggtcctgggt ctttcatggg 1080

gtgatgctgg gctggctccc tcttggtctt cccaggctgg ggctgacctt cctcgcagag 1140

aggccaggtg caggttggga atgaggcttg ctgagagggg ctgtccagtt cccagaaggc 1200

atatcagtct ctgagggctt cctttggggc cgggaacttg cgggtttgag gataggagtt 1260

cacttcatct tctcagctcc catttctact cttaagtttc tcagctccca tttctactct 1320

cccatggctt aatgcttctt tcattttctg tttgttttat acaaatgtct tagttgtaca 1380

aataaagtcc caggttaaag ataaaaaaaa 1410

<210> 54
<211> 823
<212> DNA
<213> Homo sapiens

<220>
<223> II.54 (XM_006454)

<400> 54
gggctgctcc acgcttttgc cggaggacag agactgacat ggaacagggg aagggcctgg 60

ctgtcctcat cctggctatc attcttcttc aaggtacttt ggcccagtca atcaaaggaa 120

accacttggt taaggtgtat gactatcaag aagatggttc ggtacttctg acttgtgatg 180

cagaagccaa aaatatcaca tggtttaaag atgggaagat gatcggcttc ctaactgaag 240

ataaaaaaaa atggaatctg ggaagtaatg ccaaggaccc tcgagggatg tatcagtgta 300

aaggatcaca gaacaagtca aaaccactcc aagtgtatta cagaatgtgt cagaactgca 360

ttgaactaaa tgcagccacc atatctggct ttctctttgc tgaaatcgtc agcattttcg 420

tccttgctgt tggggtctac ttcattgctg gacaggatgg agttcgccag tcgagagctt 480

cagacaagca gactctgttg cccaatgacc agctctacca gcccctcaag gatcgagaag 540

atgaccagta cagccacctt caaggaaacc agttgaggag gaattgaact caggactcag 600

agtagtccag gtgttctcct cctattcagt tcccagaatc aaagcaatgc attttggaaa 660

gctcctagca gagagacttt cagccctaaa tctagactca aggttcccag agatgacaaa 720

tggagaagaa aggccatcag agcaaatttg ggggtttctc aaataaaata aaaataaaaa 780

caaatactgt gtttcagaag cgccacctat tggggaaaat tgt 823

<210> 55
<211> 1581
<212> DNA
<213> Homo sapiens

<220>
<223> II.55 (XM_041847)

<400> 55
ccctgacaag ctgccaggca ggttctcttc ctctcacata ctgacccacg gctccaccct 60

ctctcccctg gaaaggacac catgagcact gaaagcatga tccgggacgt ggagctggcc 120

gaggaggcgc tccccaagaa gacagggggg ccccagggct ccaggcggtg cttgttcctc 180

agcctcttct ccttcctgat cgtggcaggc gccaccacgc tcttctgcct gctgcacttt 240

ggagtgatcg gcccccagag ggaagagttc cccagggacc tctctctaat cagccctctg 300

gcccaggcag tcagatcatc ttctcgaacc ccgagtgaca agcctgtagc ccatgttgta 360

gcaaaccctc aagctgaggg gcagctccag tggctgaacc gccgggccaa tgccctcctg 420

gccaatggcg tggagctgag agataaccag ctggtggtgc catcagaggg cctgtacctc 480

atctactccc aggtcctctt caagggccaa ggctgcccct ccacccatgt gctcctcacc 540

cacaccatca gccgcatcgc cgtctcctac cagaccaagg tcaacctcct ctctgccatc 600

aagagcccct gccagaggga gaccccagag ggggctgagg ccaagccctg gtatgagccc 660

atctatctgg gaggggtctt ccagctggag aagggtgacc gactcagcgc tgagatcaat 720

cggcccgact atctcgactt tgccgagtct gggcaggtct actttgggat cattgccctg 780

tgaggaggac gaacatccaa ccttcccaaa cgcctcccct gccccaatcc ctttattacc 840

ccctccttca gacaccctca acctcttctg gctcaaaaag agaattgggg gcttagggtc 900

ggaacccaag cttagaactt taagcaacaa gaccaccact tcgaaacctg ggattcagga 960

atgtgtggcc tgcacagtga agtgctggca accactaaga attcaaactg gggcctccag 1020

aactcactgg ggcctacagc tttgatccct gacatctgga atctggagac cagggagcct 1080

ttggttctgg ccagaatgct gcaggacttg agaagacctc acctagaaat tgacacaagt 1140

ggaccttagg ccttcctctc tccagatgtt tccagacttc cttgagacac ggagcccagc 1200

cctccccatg gagccagctc cctctattta tgtttgcact tgtgattatt tattatttat 1260

ttattattta tttatttaca gatgaatgta tttatttggg agaccggggt atcctggggg 1320

acccaatgta ggagctgcct tggctcagac atgttttccg tgaaaacgga gctgaacaat 1380

aggctgttcc catgtagccc cctggcctct gtgccttctt ttgattatgt tttttaaaat 1440

atttatctga ttaagttgtc taaacaatgc tgatttggtg accaactgtc actcattgct 1500

gagcctctgc tccccagggg agttgtgtct gtaatcgccc tactattcag tggcgagaaa 1560

taaagtttgc ttagaaaaga a 1581

<210> 56
<211> 3121
<212> DNA
<213> Homo sapiens

<220>
<223> II.56 (NM_145319)

<400> 56
gcggccgccc ccgcagctgc gcagcctgcc cttcgggacg ctggagctag gcgacaccgc 60

ggctctggat gccttctaca acgcggatgt ggtggtgctg gaggtgagca gctcgctggt 120

acagccctcc ctgttctacc accttggtgt gcgtgagagc ttcagcatga ccaacaatgt 180

gctcctctgc tcccaggccg acctccctga cctgcaggcc ctgcgggagg atgttttcca 240

gaagaactcg gattgcgttg gcagctacac actgatcccc tatgtggtga cggccactgg 300

tcgggtgctg tgtggtgatg caggccttct gcggggcctg gctgatgggc tggtacaggc 360

tggagtgggg accgaggccc tgctcactcc cctggtgggc cggcttgccc gcctgctgga 420
ggccacaccc acagactctt gtggctattt ccgggagacc attcggcggg acatccggca 480
ggcgcgggag cggttcagtg ggccacagct gcggcaggag ctggctcgcc tgcagcggag 540
actggacagc gtggagctgc tgagccccga catcatcatg aacttgctgc tctcctaccg 600
cgatgtgcag gactactcgg ccatcattga gctggtggag acgctgcagg ccttgcccac 660
ctgtgatgtg gccgagcagc ataatgtctg cttccactac acttttgccc tcaaccggag 720
gaacaggcct ggggaccggg cgaaggccct gtctgtgctg ctgccgctgg tacagcttga 780
gggctctgtg gcgcccgatc tgtactgcat gtgtggccgt atctacaagg acatgttctt 840
cagctcgggt ttccaggatg ctgggcaccg ggagcaggcc tatcactggt atcgcaaggc 900
ttttgacgta gagcccagcc ttcactcagg catcaatgca gctgtgctcc tcattgctgc 960
cgggcagcac tttgaggatt ccaaagagct ccggctaata ggcatgaagc tgggctgcct 1020
gctggcccgc aaaggctgcg tggagaagat gcagtattac tgggatgtgg gtttctacct 1080
gggagcccag atcctcgcca atgaccccac ccaggtggtg ctggctgcag agcagctgta 1140
taagctcaat gcccccatat ggtacctggt gtccgtgatg gagaccttcc tgctctacca 1200
gcacttcagg cccacgccag agccccctgg agggccacca cgccgtgccc acttctggct 1260
ccacttcttg ctacagtcct gccaaccatt caagacagcc tgtgcccagg gcgaccagtg 1320
cttggtgctg gtcctggaga tgaacaaggt gctgctgcct gcaaagctcg aggttcgggg 1380
tactgaccca gtaagcacag tgaccctgag cctgctggag cctgagaccc aggacattcc 1440
ctccagctgg accttcccag tcgcctccat atgcggagtc agcgcctcaa agcgcgacga 1500
gcgctgctgc ttcctctatg cactcccccc ggctcaggac gtccagctgt gcttccccag 1560
cgtagggcac tgccagtggt tctgcggcct gatccaggcc tgggtgacga acccggattc 1620
cacggcgccc gcggaggagg cggagggcgc gggggagatg ttggagtttg attatgagta 1680
cacggagacg ggcgagcggc tggtgctggg caagggcacg tatggggtgg tgtacgcggg 1740
ccgcgatcgc cacacgaggg tgcgcatcgc catcaaggag atcccggagc gggacagcag 1800
gttctctcag cccctgcatg aagagatcgc tcttcacaga cgcctgcgcc acaagaacat 1860
agtgcgctat ctgggctcag ctagccaggg cggctacctt aagatcttca tggaggaagt 1920
gcctggaggc agcctgtcct ccttgctgcg gtcggtgtgg ggacccctga aggacaacga 1980
gagcaccatc agtttctaca cccgccagat cctgcaggga cttggctact tgcacgacaa 2040
ccacatcgtg cacagggaca taaaagggga caatgtgctg atcaacacct tcagtgggct 2100
gctcaagatt tctgacttcg gcacctccaa gcggctggca ggcatcacac cttgcactga 2160
gaccttcaca ggaactctgc agtatatggc cccagaaatc attgaccagg gcccacgcgg 2220
gtatgggaaa gcagctgaca tctggtcact gggctgcact gtcattgaga tggccacagg 2280
tcgccccccc ttccacgagc tcgggagccc acaggctgcc atgtttcagg tgggtatgta 2340
caaggtccat ccgccaatgc ccagctctct gtcggccgag gcccaagcct ttctcctccg 2400

aacttttgag ccagaccccc gcctccgagc cagcgcccag acactgctgg gggacccctt 2460

cctgcagcct gggaaaagga gccgcagccc cagctcccca cgacatgctc cacggccctc 2520

agatgcccct tctgccagtc ccactccttc agccaactca accacccagt ctcagacatt 2580

cccgtgccct caggcaccct ctcagcaccc acccagcccc ccgaagcgct gcctcagtta 2640

tgggggcacc agccagctcc gggtgcccga ggagcctgcg gccgaggagc ctgcgtctcc 2700

ggaggagagt tcggggctga gcctgctgca ccaggagagc aagcgtcggg ccatgctggc 2760

cgcagtattg gagcaggagc tgccagcgct ggcggagaat ctgcaccagg agcagaagca 2820

agagcagggg gcccgtctgg gcagaaacca tgtggaagag ctgctgcgct gcctcggggc 2880

acacatccac actcccaacc gccggcagct cgcccaggag ctgcgggcgc tgcaaggatg 2940

gatgaatgga gaggacaaag gcagcttctg acacaccagc cccaggacct ggggcgactg 3000

gaggaagcca ggcgagtggg gcccaggact ggttccagtg agagaaacca accacaggca 3060

cccaagcact accagacaaa gcgtattaaa cagaacactt ttgaaaaaaa aaaaaaaaaa 3120

a 3121

<210> 57
<211> 3334
<212> DNA
<213> Homo sapiens

<220>
<223> II.57 (XM_045985)

<400> 57
attcctgcct gggaggttgt ggaagaagga agatggccag agctttgtgt ccactgcaag 60

ccctctggct tctggagtgg gtgctgctgc tcttgggacc ttgtgctgcc cctccagcct 120

gggccttgaa cctggaccca gtgcagctca ccttctatgc aggccccaat ggcagccagt 180

ttggattttc actggacttc cacaaggaca gccatgggag agtggccatc gtggtgggcg 240

ccccgcggac cctgggcccc agccaggagg agacgggcgg cgtgttcctg tgcccctgga 300

gggccgaggg cggccagtgc ccctcgctgc tctttgacct ccgtgatgag acccgaaatg 360

taggctccca aactttacaa accttcaagg cccgccaagg actgggggcg tcggtcgtca 420

gctggagcga cgtcattgtg gcctgcgccc cctggcagca ctggaacgtc ctagaaaaga 480

ctgaggaggc tgagaagacg cccgtaggta gctgcttttt ggctcagcca gagagcggcc 540

gccgcgccga gtactccccc tgtcgcggga acaccctgag ccgcatttac gtggaaaatg 600

attttagctg ggacaagcgt tactgtgaag cgggcttcag ctccgtggtc actcaggccg 660

gagagctggt gcttggggct cctggcggct attatttctt aggtctcctg gcccaggctc 720

cagttgcgga tattttctcg agttaccgcc caggcatcct tttgtggcac gtgtcctccc 780

agagcctctc ctttgactcc agcaacccag agtacttcga cggctactgg gggtactcgg 840

tggccgtggg cgagttcgac ggggatctca acactacaga atatgtcgtc ggtgccccca 900

cttggagctg gaccctggga gcggtggaaa ttttggattc ctactaccag aggctgcatc 960
ggctgcgcgg agagcagatg gcgtcgtatt ttgggcattc agtggctgtc actgacgtca 1020
acggggatgg gaggcatgat ctgctggtgg gcgctccact gtatatggag agccgggcag 1080
accgaaaact ggccgaagtg gggcgtgtgt atttgttcct gcagccgcga ggcccccacg 1140
cgctgggtgc ccccagcctc ctgctgactg gcacacagct ctatgggcga ttcggctctg 1200
ccatcgcacc cctgggcgac ctcgaccggg atggctacaa tgacattgca gtggctgccc 1260
cctacggggg tcccagtggc cggggccaag tgctggtgtt cctgggtcag agtgaggggc 1320
tgaggtcacg tccctcccag gtcctggaca gccccttccc cacaggctct gcctttggct 1380
tctcccttcg aggtgccgta gacatcgatg acaacggata cccagacctg atcgtgggag 1440
cttacggggc caaccaggtg gctgtgtaca gagctcagcc agtggtgaag gcctctgtcc 1500
agctactggt gcaagattca ctgaatcctg ctgtgaagag ctgtgtccta cctcagacca 1560
agacacccgt gagctgcttc aacatccaga tgtgtgttgg agccactggg cacaacattc 1620
ctcagaagct atccctaaat gccgagctgc agctggaccg gcagaagccc cgccagggcc 1680
ggcgggtgct gctgctgggc tctcaacagg caggcaccac cctgaacctg gatctgggcg 1740
gaaagcacag ccccatctgc cacaccacca tggccttcct tcgagatgag gcagacttcc 1800
gggacaagct gagccccatt gtgctcagcc tcaatgtgtc cctaccgccc acggaggctg 1860
gaatggcccc tgctgtcgtg ctgcatggag acacccatgt gcaggagcag acacgaatcg 1920
tcctggactg tggggaagat gacgtatgtg tgccccagct tcagctcact gccagcgtga 1980
cgggctcccc gctcctagtt ggggcagata atgtcctgga gctgcagatg gacgcagcca 2040
acgagggcga gggggcctat gaagcagagc tggccgtgca cctgccccag ggcgcccact 2100
acatgcgggc cctaagcaat gtcgagggct ttgagagact catctgtaat cagaagaagg 2160
agaatgagac cagggtggtg ctgtgtgagc tgggcaaccc catgaagaag aacgcccaga 2220
taggaatcgc gatgttggtg agcgtgggga atctggaaga ggctggggag tctgtgtcct 2280
tccagctgca gatacggagc aagaacagcc agaatccaaa cagcaagatt gtgctgctgg 2340
acgtgccggt ccgggcagag gcccaagtgg agctgcgagg gaactccttt ccagcctccc 2400
tggtggtggc agcagaagaa ggtgagaggg agcagaacag cttggacagc tggggaccca 2460
aagtggagca cacctatgag ctccacaaca atggccctgg gactgtgaat ggtcttcacc 2520
tcagcatcca ccttccggga cagtcccagc cctccgacct gctctacatc ctggatatac 2580
agccccaggg gggccttcag tgcttcccac agcctcctgt caaccctctc aaggtggact 2640
gggggctgcc catccccagc ccctccccca ttcacccggc ccatcacaag cgggatcgca 2700
gacagatctt cctgccagag cccgagcagc cctcgaggct tcaggatcca gttctcgtaa 2760
gctgcgactc ggcgccctgt actgtggtgc agtgtgacct gcaggagatg gcgcgcgggc 2820
agcgggccat ggtcacggtg ctggccttcc tgtggctgcc cagcctctac cagaggcctc 2880
tggatcagtt tgtgctgcag tcgcacgcat ggttcaacgt gtcctccctc ccctatgcgg 2940

tgcccccgct cagcctgccc cgaggggaag ctcaggtgtg gacacagctg ctccgggcct 3000

tggaggagag ggccattcca atctggtggg tgctggtggg tgtgctgggt ggcctgctgc 3060

tgctcaccat cctggtcctg gccatgtgga aggtcggctt cttcaagcgg aaccggccac 3120

ccctggaaga agatgatgaa gagggggagt gatggtgcag cctacactat tctagcagga 3180

gggttgggcg tgctacctgc accgcccctt ctccaacaag ttgcctccaa gctttgggtt 3240

ggagctgttc cattgggtcc tcttggtgtc gtttccctcc caacagagct gggctacccc 3300

ccctcctgct gcctaataaa gagactgagc cctg 3334

<210> 58
<211> 866
<212> DNA
<213> Homo sapiens

<220>
<223> II.58 (XM_055756)

<400> 58
gtaatgcatc caggaagcct ggaggcctgt ggtttccgca cccgctgcca cccccgcccc 60

tagcgtggac atttatcctc tagcgctcag gccctgccgc catcgccgca gatccagcgc 120

ccagagagac accagagaac ccaccatggc cccctttgag cccctggctt ctggcatcct 180

gttgttgctg tggctgatag cccccagcag ggcctgcacc tgtgtcccac cccacccaca 240

gacggccttc tgcaattccg acctcgtcat cagggccaag ttcgtgggga caccagaagt 300

caaccagacc accttatacc agcgttatga gatcaagatg accaagatgt ataaagggtt 360

ccaagcctta ggggatgccg ctgacatccg gttcgtctac acccccgcca tggagagtgt 420

ctgcggatac ttccacaggt cccacaaccg cagcgaggag tttctcattg ctggaaaact 480

gcaggatgga ctcttgcaca tcactacctg cagttttgtg gctccctgga acagcctgag 540

cttagctcag cgccggggct tcaccaagac ctacactgtt ggctgtgagg aatgcacagt 600

gtttccctgt ttatccatcc cctgcaaact gcagagtggc actcattgct tgtggacgga 660

ccagctcctc caaggctctg aaaagggctt ccagtcccgt caccttgcct gcctgcctcg 720

ggagccaggg ctgtgcacct ggcagtccct gcggtcccag atagcctgaa tcctgcccgg 780

agtggaagct gaagcctgca cagtgtccac cctgttccca ctcccatctt tcttccggac 840

aatgaaataa agagttacca cccagc 866

<210> 59
<211> 817
<212> DNA
<213> Homo sapiens

<220>
<223> II.59 (NM_004740)

<400> 59
tgctgtcttt ctacttttta atttaaatac ccaacctctc catcacagct gcatccctga 60

gagtgggagg gggctgtagt ggtagctggg gctcccaaga acgactcggg aatgtcatct 120

ccatcttcac ccttcagaga gcagtccttt ctctgtgcag ctggagacgc tggtgaggag 180

agccgggtcc aggttcttaa gaatgaggtg cggaggggct ctccggtgct gctgggctgg 240

gttgagcaag cctacgcaga caagtgtgtg tgtggaccat ccgcacctcc agcccccacc 300

ccaccctctt tgtctcagcg tgttatgtgc aatgacctat ttaaggtaaa cccattccaa 360

ctacagcagt tcagggctga tccaagcact gcctccctcc tgctctgtcc aggtggtctg 420

gaccataaac tcaacttgag agggaaggct tggggttgag gacttgtgat cagaaaaact 480

gaagatggaa gttttggccg gtgctcatta gacatgagtc ctcactctgt gtcctgagcc 540

cgtgtcattc ttccaacctc cctgccccca cacacttatc ccagacacaa caccatgtgg 600

tctggaggtc ccagccccca ccctaaaaag gttatccctg agaactccac cagacttggg 660

agcccaagtg cagtgcctgg tgctgctccc atctgccgcc ccccttctct cctgcaattg 720

gtttgtactc actgggctgt gctctcccct gtttacccga tgtatggaaa taaaggccct 780

tttcctcctg aaaaaaaaaa aaaaaaaaaa aaaaaaa 817

<210> 60
<211> 4490
<212> DNA
<213> Homo sapiens

<220>
<223> II.60 (XM_008432)

<400> 60
   aggtgaacag gtcctcacgc ccagctccgc gccctcacgc gctctcgccg ggaccccgct 60
   tccgctggca gccatgggcc ccggccccag ccgcgcgccc cgcgccccac gcctgatgct 120
   ctgtgcgctc gccttgatgg tggcggccgg cggctgcgtc gtctccgcct tcaacctgga 180
   tacccgattc ctggtagtga aggaggccgg gaacccgggc agcctcttcg gctactcggt 240
   cgccctccat cggcagacag agcggcagca gcgctacctg ctcctggctg gtgccccccg 300
   ggagctcgct gtgcccgatg gctacaccaa ccggactggt gctgtgtacc tgtgcccact 360
   cactgcccac aaggatgact gtgagcggat gaacatcaca gtgaaaaatg accctggcca 420
   tcacattatt gaggacatgt ggcttggagt gactgtggcc agccagggcc ctgcaggcag 480
   agttctggtc tgtgcccacc gctacaccca ggtgctgtgg tcagggtcag aagaccagcg 540
   gcgcatggtg ggcaagtgct acgtgcgagg caatgaccta gagctggact ccagtgatga 600
   ctggcagacc taccacaacg agatgtgcaa tagcaacaca gactacctgg agacgggcat 660
   gtgccagctg ggcaccagcg gtggcttcac ccagaacact gtgtacttcg gcgcccccgg 720
   tgcctacaac tggaaaggaa acagctacat gattcagcgc aaggagtggg acttatctga 780
   gtatagttac aaggacccag aggaccaagg aaacctctat attgggtaca cgatgcaggt 840
   aggcagcttc atcctgcacc ccaaaaacat caccattgtg acaggtgccc cacggcaccg 900
   acatatgggc gcggtgttct tgctgagcca ggaggcaggc ggagacctgc ggaggaggca 960
   ggtgctggag ggctcgcagg tgggcgccta ttttggcagc gccattgccc tggcagacct 1020
   gaacaatgat gggtggcagg acctcctggt gggcgccccc tactacttcg agaggaaaga 1080
   ggaagtaggg ggtgccatct atgtcttcat gaaccaggcg ggaacctcct tccctgctca 1140
   cccctcactc cttcttcatg gccccagtgg ctctgccttt ggtttatctg tggccagcat 1200
   tggtgacatc aaccaggatg gatttcagga tattgctgtg ggagctccgt ttgaaggctt 1260
   gggcaaagtg tacatctatc acagtagctc taaggggctc cttagacagc cccagcaggt 1320
   aatccatgga gagaagctgg gactgcctgg gttggccacc ttcggctatt ccctcagtgg 1380
   gcagatggat gtggatgaga acttctaccc agaccttcta gtgggaagcc tgtcagacca 1440
   cattgtgctg ctgcagggcc cggcccgtca tcaacatcgt ccacaagacc ttggtgccca 1500
   ggccagctgt gctggaccct gcactttgca cggccacctc ttgtgtgcaa gtggagctgt 1560
   gctttgctta caaccagagt gccgggaacc ccaactacag gcgaaacatc accctggcct 1620
   acactctgga ggctgacagg gaccgccggc cgccccggct ccgctttgcc ggcagtgagt 1680
   ccgctgtctt ccacggcttc ttctccatgc ccgagatgcg ctgccagaag ctggagctgc 1740
   tcctgatgga caacctccgt gacaaactcc gccccatcat catctccatg aactactctt 1800
   tacctttgcg gatgcccgat cgcccccggc tggggctgcg gtccctggac gcctacccga 1860
   tcctcaacca ggcacaggct ctggagaacc acactgaggt ccagttccag aaggagtgcg 1920
   ggcctgacaa caagtgtgag agcaacttgc agatgcgggc agccttcgtg tcagagcagc 1980
   agcagaagct gagcaggctc cagtacagca gagacgtccg gaaattgctc ctgagcatca 2040
   acgtgacgaa cacccggacc tcggagcgct ccggggagga cgcccacgag gcgctgctca 2100
   ccctggtggt gcctcccgcc ctgctgctgt cctcagtgcg cccccccggg gcctgccaag 2160
   ctaatgagac catcttttgc gagctgggga accccttcaa acggaaccag aggatggagc 2220
   tgctcatcgc ctttgaggtc atcggggtga ccctgcacac aagggacctt caggtgcagc 2280
   tgcagctctc cacgtcgagt caccaggaca acctgtggcc catgatcctc actctgctgg 2340
   tggactatac actccagacc tcgcttagca tggtaaatca ccggctacaa agcttctttg 2400
   gggggacagt gatgggtgag tctggcatga aaactgtgga ggatgtagga agccccctca 2460
   agtatgaatt ccaggtgggc ccaatggggg aggggctggt gggcctgggg accctggtcc 2520
   taggtctgga gtggccctac gaagtcagca atggcaagtg gctgctgtat cccacggaga 2580
   tcaccgtcca tggcaatggg tcctggccct gccgaccacc tggagacctt atcaaccctc 2640
   tcaacctcac tctttctgac cctggggaca ggccatcatc cccacagcgc aggcggcgac 2700
   agctggatcc agggggaggc cagggccccc cacctgtcac tctggctgct gccaaaaaag 2760
   ccaagtctga gactgtgctg acctgtgcca cagggcgtgc ccactgtgtg tggctagagt 2820
   gccccatccc tgatgccccc gttgtcacca acgtgactgt gaaggcacga gtgtggaaca 2880
   gcaccttcat cgaggattac agagactttg accgagtccg ggtaaatggc tgggctaccc 2940
   tattcctccg aaccagcatc cccaccatca acatggagaa caagaccacg tggttctctg 3000
   tggacattga ctcggagctg gtggaggagc tgccggccga aatcgagctg tggctggtgc 3060
   tggtggccgt gggtgcaggg ctgctgctgc tggggctgat catcctcctg ctgtggaagt 3120
   gtgacttctt taagcggacc cgctattatc agatcatgcc caagtaccac gcagtgcgga 3180
   tccgggagga ggagcgctac ccacctccag ggagcaccct gcccaccaag aagcactggg 3240
   tgaccagctg gcagactcgg gaccaatact actgacgtcc tccctgatcc caccccctcc 3300
   tcccccagtg tcccctttct tcctatttat cataagttat gcctctgaca gtccacaggg 3360
   gccaccacct ttggctggta gcagcaggct caggcacata cacctcgtca agagcatgca 3420
   catgctgtct ggccctgggg atcttcccac aggagggcca gcgctgtgga ccttacaacg 3480
   ccgagtgcac tgcattcctg tgccctagat gcacgtgggg cccactgctc gtggactgtg 3540
   ctggtgcatc acggatggtg catgggctcg ccgtgtctca gcctctgcca gcgccaaaac 3600
   aagccaaaga gcctcccacc agagccggga ggaaaaggcc cctgcaatgt ggtgacacct 3660
   ccccctttca cactggatcc atcttgagcc acagtcactg gattgacttt gctgtcaaaa 3720
   ctactgacag ggagcagccc ccgggccgct ggctggtggg cccccaatga cacccatgcc 3780
   agagaggtgg ggatcctgcc taaggttgtc tacgggggca cttggaggac ctggcgtgct 3840
   cagacccaac agcaaaggaa ctagaaagaa ggacccagaa cggcttgctt tcctgcatct 3900
   ctgtgaagcc tctctccttg gccacagact gaactcgcag ggaatgcagc aggaaggaac 3960
   aaagacaggc aaacggcaac gtagcctggg ctcactgtgc tggggcacgg cgggatcctc 4020
   cacagagagg aggggaccaa ttctggacag acagatgttg ggaggataca gaggagatgc 4080
   cacttctcac tcaccactac cagccagcct cagaaggccc cagagagacc ctgcaagacc 4140
   acggagggag cgacacttga atgtagaata ggcagggggc cctgccccac cccatccagc 4200
   cagaccccac gctgaccatg cgtcaggggc ctagaggtgg agttcttagc tatccttggc 4260
   tttcagagcc agcctggctc tgccccctcc cccatgggct gtgtcctaag gcccatttga 4320
   gaagctgagg ctagttccag aaaacctctc ctgacccctg cctgttggca ggcccactcc 4380
   ccagccccag ccccttccat ggtactgtag caggggaatt ccctccccct ccttgtgcct 4440
   tctttgtata taggcttctc acggcgacca ataaacagct cccagtttgt 4490

<210> 61
<211> 5580
<212> DNA
<213> Homo sapiens

<220>
<223> II.61 (XM_034770)

<400> 61
   agctcctgtg acagacggag ctggagcggc ggggcggcgg cggagtccgg cggccgggag 60
   agcgagtgag cgagcggagg agcagcgaca cgggagtcta gggagcgaga aggagaagga 120
   ggggagcgct cgggcgcgag cgagagaaac cgcgagcgcc gagcttggac tcgagccccg 180
   gaacggctga ggagcccgcc cgctcccctc ccctccccct ccccgggccc gggcccagcg 240
   cgccatcctc ccccctcctt ccctccctcc ctccttcctc cctcccctct ccctccccct 300
   cccccgccgg tggatgggag tgaaggacgg aagaggccct gcggaggcgg cggtgcagcg 360
   ctccggtgga atgaatctta cttgttgaat atcttctggt tactagttgg attcatttgt 420
   gaaagaatca ttttcccctg tgtggaagac acttagtggc atatttaaat tataagtcca 480
   cggatcaaaa agctttttga tttcccaaag gagggacata ccactatatc agataagctt 540
   gacattacag ccaagatggt gctgtcccag agacaacgag atgaactaaa tcgagctata 600
   gcagattatc ttcgttcaaa tggctatgaa gaggcatatt cagtttttaa aaaggaagct 660
   gaattagatg tgaatgaaga attagataaa aagtatgctg gtcttttgga aaaaaaatgg 720
   acatctgtta ttagattaca aaagaaggtt atggaattag aatcaaagct aaatgaagca 780
   aaagaagaat ttacgtcagg tggacctctt ggtcagaaac gagacccaaa agaatggatt 840
   ccccgtccgc cagaaaaata tgcattgagt ggtcacagga gtccagtcac tcgagtcatt 900
   ttccatcctg tgttcagtgt tatggtctct gcttcagagg atgctacaat taaggtgtgg 960
   gattatgaga ctggagattt tgaacgaact cttaaaggac atacagactc tgtacaggac 1020
   atttcattcg accacagcgg caagcttctg gcttcctgtt ctgcagatat gaccattaaa 1080
   ctatgggatt ttcagggctt tgaatgcatc agaaccatgc acggccatga ccacaatgtt 1140
   tcttcagtag ccatcatgcc caatggagat catatagtgt ctgcctcaag ggataaaact 1200
   ataaaaatgt gggaagtgca aactggctac tgtgtgaaga cattcacagg acacagagaa 1260
   tgggtacgta tggtacggcc aaatcaagat ggcactctga tagccagctg ttccaatgac 1320
   cagactgtgc gtgtatgggt cgtagcaaca aaggaatgca aggctgagct ccgagagcat 1380
   gagcatgtgg tagaatgcat ttcctgggct ccagaaagct catattcctc catctctgaa 1440
   gcaacaggat ctgagactaa aaaaagtggt aaacctgggc cattcttgct gtctggatcc 1500
   agagacaaga ctattaagat gtgggatgtc agtactggca tgtgccttat gaccctcgtg 1560
   ggtcatgata actgggtacg tggagttctg ttccattctg gggggaagtt tattttgagt 1620
   tgtgctgatg acaagaccct acgcgtatgg gattacaaga acaagcgatg catgaagacc 1680
   ctcaatgcgc atgaacactt tgttacctcc ttggatttcc acaagacggc accctatgtc 1740
   gtcactggca gcgtagatca aacagtaaaa gtgtgggagt gccgttgatt gtgtctcctt 1800
   cggcccctcc tccctctttt cctctggatg cactctgatg ataccatggt taccccattg 1860
   agctctgttt aaataaatat tgtcctttca tgtaaattat tctggatgta gattgagctt 1920
   attaaatgtt acacacaaag tattcatgca tggtgaatcc aaattgtata ctgtaaattt 1980
   acatacgttg tctagaagta ccatagggtt taaaaacctg ggctggcatt ggtcacacca 2040
   ggcctaagaa ggcagaagtt gaatcaattg aactagggca ctaaactgaa tagttgacag 2100
   tgtcatttta tgttggatta ttaattcctg tttttctttc tgctatctgt tggtgcctga 2160
   cttgatggcc tcatttgggg aaaagtggtg gttattaggg ctttttctga aatgtgtatc 2220
   tatgtaacat cacttaagtg tgcttaataa atcttctgta aggattttag atgataaggc 2280
   tacaattcag aatcttctga accatctatg taatgaatgg ggattataca ttggaatttt 2340
   tgtcatgaca catttgccaa atcagtagga tatatttgtt ttggcagcct atcacgcaga 2400
   ggctagtggt atatttatgt aagaaaatga ctgtaaatct caagaaaaat ctcagcagct 2460
   aatagcaact catttatttc attttggtct taatgctttg taaacaggtc aaaaaatact 2520
   gtcatactct aagcttctat tttccacact ggacatactt ctagttgtat tctccatact 2580
   attagactgt gtagtgatgt gacttccaag tagaatttaa tctccccatt gagtgtgtca 2640
   tggtacaaat cactattcgt tttggtgttt ttttagggat gtgcaatgtg cattacataa 2700
   tgacagaaat actgagaagg ttctgtgtgc ccatttgaaa ggagtgggag gaatacagca 2760
   gtttgttttt caacatgaat ctgatattga tttaaactgt gtttcactta caagttttaa 2820
   aaaaatgaca gggtttaatg gagcgtgcat aaaaatgtac tgttttcacc ttttgtttat 2880
   atgtaaatgt ttgtaagtat atgggcctat ctgtaagtgg ataagtctgt atgtgtgtat 2940
   catacacatc aacctccatg tccttagtcc tgggtttttg aaaaagtgct aaaacggaca 3000
   agtagaataa atgttgctgt ggaatgccat gctttagaac aaaccctttt tgatcttaat 3060
   gcttctgaaa actaggtctg actctgggga tttttttcca gccgaaggaa aatcacttcc 3120
   gttatgtccc cctctaattt agccgctcga cattttacac aacccggata tgttgtatat 3180
   tttgacccaa agttacaggt aggtttaaga gaatttttag ccatgacttt tggagcacta 3240
   ttccattgtc agttattaat aaagaattcc attgcttagc taaccaacag gttttttttg 3300
   tttccaagag agttatttga aaagttaaca gaacaatgag ataacagtga cagtttaaca 3360
   aagataaaat tctgaactgc gttttattca tttgtgtact atgtgatttt ttaaatgtcc 3420
   cctttagtat ttaatggaaa attggttcct gcaaaagaca aagggtgaga gttagcgtcc 3480
   tgtagataca cacagagact aggccgtata ttaactagaa gcagctttat gtctagcttg 3540
   tgtctttttg tttgtttgct tgtttgtttt tagattcctg agagatgtct ctggaaggga 3600
   aagttttgag aactaatggc tatttttgag gacaaaaatt acatcttaag ctaattcctt 3660
   aaatacatac agtaggtgaa ttttcaggac aatattgcct cacaaccctg cttacattga 3720
   aaagtctttt tcccttagct cttctgactg gatttttcta caaaaactat ggaaaatatc 3780
   tttgttcttg tttgctgcta ttttctgtcc tattttgaga aatataaata catagaaatg 3840
   gtgcatctta acatttgttt gtacatgtat aaatgtcttg tattttaatt catttttagc 3900
   atgaattgtt taagggtaag ccacaacatc tagaaatcac tcatagatat tgaacaataa 3960
   aggagaatgg taccgatgca ggaggaagca agcgtgtctt cccctgcagc acacagcgac 4020
   ttgcgttgac aaaggaggag gaaacgatta ctctgtaaac aaagttatcc ttacttggga 4080
   gattgccaca gcctgctgct gagttgagtt accagacatc ctccatgtga gaagcagcga 4140
   acattgaatc tcagggatgg cccacaactg ggtccacatg taatgagccc tgtttaataa 4200
   cgaaggggtg ggggagagca gtccgtctac aacctggaat cagatttgca aaatttcctg 4260
   cactgctgtc tgacactgtc ctgttgatgc cctttctgac tgtgttctct gttttctctg 4320
   tctgctgtct aaccctgtgc cttgcctggg ataaggacaa tgatgaggtt actggtttgg 4380
   attgtaagta gaggactttt attaattggt ttagaggttc actgctgctt tgtcactttc 4440
   tcaatcaaat tggccactta agaaataaag agctggtaga attgcatcct cagatgatta 4500
   ttgactgtgt gtgtgtgtga aaacagacat tccagtgcca cccaaatata tatctgtaac 4560
   gtgcccaaga aatcctagct gcgctcttga gagtgcatgc catggagact ggtttagaca 4620
   ccgcgtggag cctagttgcc tgttgtcacg gcatcttgca ctttaggaga ctaagaccgt 4680
   cctggttcgt ctgtgtgtgg tgtgaccaat ggtgtgccca gagcactact ctcaaaatca 4740
   ctagtgttag caagtcgtcc cgggctgggg agcgttcgcc gtagtctttg gaagctttgg 4800
   ctttagattt accaagcccc gcctccccgc tgccagtgcc ctgctctccc gttcgcctct 4860
   ttctgtttct gtgtgaactt tcccggtaat atcactcgtt aaataggttt tctttaaact 4920
   taattaaaga aaaactattt aaaggtaaag gatattttgt tgacatcggt ggctcgatca 4980
   tccttaagca actgaagtta aaattgttga aggaaaaggc acttaaattg gttactttca 5040
   tgtccagctg tatataagtc cagtgtgttc atctagatga cgcaaagaat ctcctggtag 5100
   agaagcgaca tgtaaaaaac tggtggaaaa aggttttgga ttttttttcc agtggggtgg 5160
   ggggagggca agctggattt acaggtcacg gctggactga atgggccttt ttatcttccc 5220
   actgtatcat ggaagtagct gcttgcttgt actgtccatc cttcaggcat ccctaaagct 5280
   cactctgaag atgttagaga caaacacaaa ctcttcgagt taaagttgat cctgacactg 5340
   acatgaaggc aagccttgat ttcgtatgaa cgttgctgaa gtggtaattg aggaaaacag 5400
   ttccccagat tgttaagagt tcactgaaga tattgacaca attttaaaaa atcagtaaag 5460
   gaatgtatat aatattgctc tcgtgtttta cagtaagatt tgttgctctc agactgtgta 5520
   aaacaaaatt tattcatgtt ttctgcatat taaaaaatct tattgtacca actggtaaac 5580

<210> 62
<211> 2644
<212> DNA
<213> Homo sapiens

<220>
<223> II.62 (NM_014326)

<400> 62
gaccgcggca gctcagcctc ccgccgattg tatgttccag gcctcaatga ggagtccaaa 60

catggagcca ttcaagcagc agaaggtgga ggacttttat gacatcggag aggagctggg 120

gagtggccag tttgccatcg tgaagaagtg ccgggagaag agcacggggc ttgagtatgc 180

agccaagttc atcaagaagc ggcagagccg ggcgagccgg cgcggtgtga gccgggagga 240

gatcgagcgg gaggtgagca tcctgcggca ggtgctgcac cacaatgtca tcacgctgca 300

cgacgtctat gagaaccgca ccgacgtggt gctcatcctt gagctagtgt ctggaggaga 360

gctcttcgat ttcctggccc agaaggagtc actgagtgag gaggaggcca ccagcttcat 420

taagcagatc ctggatgggg tgaactacct tcacacaaag aaaattgctc actttgatct 480
caagccagaa aacattatgt tgttagacaa gaatattccc attccacaca tcaagctgat 540
tgactttggt ctggctcacg aaatagaaga tggagttgaa tttaagaata tttttgggac 600
gccggaattt gttgctccag aaattgtgaa ctacgagccc ctgggtctgg aggctgacat 660
gtggagcata ggcgtcatca cctacatcct cttaagtgga gcatcccctt tcctgggaga 720
cacgaagcag gaaacactgg caaatatcac agcagtgagt tacgactttg atgaggaatt 780
cttcagccag acgagcgagc tggccaagga ctttattcgg aagcttctgg ttaaagagac 840
ccggaaacgg ctcacaatcc aagaggctct cagacacccc tggatcacgc cggtggacaa 900
ccagcaagcc atggtgcgca gggagtctgt ggtcaatctg gagaacttca ggaagcagta 960
tgtccgcagg cggtggaagc tttccttcag catcgtgtcc ctgtgcaacc acctcacccg 1020
ctcgctgatg aagaaggtgc acctgaggcc ggatgaggac ctgaggaact gtgagagtga 1080
cactgaggag gacatcgcca ggaggaaagc cctccaccca cggaggagga gcagcacctc 1140
ctaactggcc tgacctgcag tggccgccag ggaggtctgg gcccagcggg gctcccttct 1200
gtgcagactt ttggacccag ctcagcacca gcacccgggc gtcctgagca ctttgcaaga 1260
gagatgggcc caaggaattc agaagagctt gcaggcaagc caggagaccc tgggagctgt 1320
ggctgtcttc tgtggaggag gctccagcat tcccaaagct cttaattctc cataaaatgg 1380
gctttcctct gtctgccatc ctcagagtct ggggtgggag tgtggactta ggaaaacaat 1440
ataaaggaca tcctcatcat cacggggtga aggtcagact aaggcagcct tcttcacagg 1500
ctgagggggt tcagaaccag cctggccaaa aattacacca gagagacaga gtcctcccca 1560
ttgggaacag ggtgattgag gaaagtgaac cttgggtgtg agggaccaat cctgtgacct 1620
cccagaacca tggaagccag gacgtcaggc tgaccaacac ctcagacctt ctgaagcagc 1680
ccattgctgg cccgccatgt tgtaattttg ctcattttta ttaaacttct ggtttacctg 1740
atgcttggct tcttttaggg ctacccccat ctcatttcct ttagcccgtg tgcctgtaac 1800
tctgaggggg ggcacccagt ggggtgctga gtgggcagaa tctcagaagg tcctcctgaa 1860
ccgtccgcgc aggcctgcag tgggcctgcc tcctccttgc ttccctaaca ggaaggtgtc 1920
cagttcaaga gaacccaccc agagactggg agtggtggct cacgcctata atccctgcgc 1980
tttggcagtc cgaggcaggg gaattgcttg aactcaggag ttggagacca gcctgggcaa 2040
catggcaaaa ctcagtctgt acaaaaaata caaaaaatta gccaggtgta ggggtaggca 2100
cctggcatcc cagctactcc aggggctgag gtgacagcat tgcttaagcc cagaaggtcg 2160
aggctgcagt gagctgagat cacgccactg cactccagtc tgggtgacag agagagacca 2220
tatccaaaaa aaaaaaaagt tgccagagac gagtatgccc atgctccctc tacctcactg 2280
ccaccactcc tgcttttagg agctgagtgt gtctccctaa aatttctatg ttgaagtctt 2340
aacccttggt accacagaat atcactgtat ttggagatgg ggtctttaga aaggcactta 2400
aattaaaatg agctcactga tatgggcccc gatgcaatat aattggtgtc cttataagaa 2460

ggggaggtta ggacacgcag gaaagaccac atgaaggccc aggagtggga gggggaatag 2520

ccatcgacaa actaaggggg cctcagagga aaccaaccct gctgacacct caatcttaga 2580

ctctggcctc aaaaattgta agaaaataaa cttctgtctt ttaagccaaa aaaaaaaaaa 2640

aaaa 2644

<210> 63
<211> 2470
<212> DNA
<213> Homo sapiens

<220>
<223> II.63 (XM_043864)

<400> 63
gacgagccga gccgagccaa gcggagctgg gccactgtgc acgcccggag ggtcctggcg 60

gcgcccccgc tcctgcgcgc actctcggcg ccggacacga gcactgcctg ccgggaacag 120

gctgggggga ggtgcggggg cttggcccac ttggtggaag aacagctttg ggattttttt 180

ttttttcatt gtcggataca ggcatttcaa agggaaaccg ttgaaatgca taacctgcaa 240

acactgcctc ctaaaccacc aaaacctact actgtagcca acaacggtat gaataacaat 300

atgtccttac aagatgctga atggtactgg ggagatatct cgagggaaga agtgaatgaa 360

aaacttcgag atacagcaga cgggaccttt ttggtacgag atgcgtctac taaaatgcat 420

ggtgattata ctcttacact aaggaaaggg ggaaataaca aattaatcaa aatatttcat 480

cgagatggga aatatggctt ctctgaccca ttaaccttca gttctgtggt tgaattaata 540

aaccactacc ggaatgaatc tctagctcag tataatccca aattggatgt gaaattactt 600

tatccagtat ccaaatacca acaggatcaa gttgtcaaag aagataatat tgaagctgta 660

gggaaaaaat tacatgaata taacactcag tttcaagaaa aaagtcgaga atatgataga 720

ttatatgaag aatatacccg cacatcccag gaaatccaaa tgaaaaggac agctattgaa 780

gcatttaatg aaaccataaa aatatttgaa gaacagtgcc agacccaaga gcggtacagc 840

aaagaataca tagaaaagtt taaacgtgaa ggcaatgaga aagaaataca aaggattatg 900

cataattatg ataagttgaa gtctcgaatc agtgaaatta ttgacagtag aagaagattg 960

gaagaagact tgaagaagca ggcagctgag tatcgagaaa ttgacaaacg tatgaacagc 1020

attaaaccag accttatcca gctgagaaag acgagagacc aatacttgat gtggttgact 1080

caaaaaggtg ttcggcaaaa gaagttgaac gagtggttgg gcaatgaaaa cactgaagac 1140

caatattcac tggtggaaga tgatgaagat ttgccccatc atgatgagaa gacatggaat 1200

gttggaagca gcaaccgaaa caaagctgaa aacctgttgc gagggaagcg agatggcact 1260

tttcttgtcc gggagagcag taaacagggc tgctatgcct gctctgtagt ggtggacggc 1320

gaagtaaagc attgtgtcat aaacaaaaca gcaactggct atggctttgc cgagccctat 1380

aacttgtaca gctctctgaa agaactggtg ctacattacc aacacacctc ccttgtgcag 1440

cacaacgact ccctcaatgt cacactagcc tacccagtat atgcacagca gaggcgatga 1500

agcgcttact ctttgatcct tctcctgaag ttcagccacc ctgaggcctc tggaaagcaa 1560

agggctcctc tccagtctga tctgtgaatt gagctgcaga aacgaagcca tctttctttg 1620

gatgggacta gagctttctt tcacaaaaaa gaagtagggg aagacatgca gcctaaggct 1680

gtatgatgac cacacgttcc taagctggag tgcttatccc ttctttttct tttttctttt 1740

ggtttaattt aaagccacaa ccacatacaa cacaaagaga aaaagaaatg caaaaatctc 1800

tgcgtgcagg gacaaagagg cctttaacca tggtgcttgt taatgctttc tgaagcttta 1860

ccagctgaaa gttgggactc tggagagcgg aggagagaga ggcagaagaa ccctggcctg 1920

agaaggtttg gtccagcctg gtttagcctg gatgttgctg tgcacggtgg acccagacac 1980

atcgcactgt ggattatttc attttgtaac aaatgaacga tatgtagcag aaaggcacgt 2040

ccactcacaa gggacgcttt gggagaatgt cagttcatgt atgttcagaa gaaattctgt 2100

catagaaagt gccagaaagt gtttaacttg tcaaaaaaca aaaacccagc aacagaaaaa 2160

tggagtttgg aaaacaggac ttaaaatgac attcagtata taaaatatgt acataatatt 2220

ggatgactaa ctatcaaata gatggatttg tatcaatacc aaatagcttc tgttttgttt 2280

tgctgaaggc taaattcaca gcgctatgca attcttaatt ttcattaagt tgttatttca 2340

gttttaaatg taccttcaga ataagcttcc ccaccccagt ttttgttgct tgaaaatatt 2400

gttgtcccgg atttttgtta atattcattt ttgttatcct tttttaaaag taaatgtaca 2460

ggatgccagt 2470

<210> 64
<211> 3383
<212> DNA
<213> Homo sapiens

<220>
<223> II.64 (U54994)

<400> 64
   agaagagctg agacatccgt tcccctacaa gaaactctcc ccgggtggaa caagatggat 60
   tatcaagtgt caagtccaat ctatgacatc aattattata catcggagcc ctgccaaaaa 120
   atcaatgtga agcaaatcgc agcccgcctc ctgcctccgc tctactcact ggtgttcatc 180
   tttggttttg tgggcaacat gctggtcatc ctcatcctga taaactgcaa aaggctgaag 240
   agcatgactg acatctacct gctcaacctg gccatctctg acctgttttt ccttcttact 300
   gtccccttct gggctcacta tgctgccgcc cagtgggact ttggaaatac aatgtgtcaa 360
   ctcttgacag ggctctattt tataggcttc ttctctggaa tcttcttcat catcctcctg 420
   acaatcgata ggtacctggc tgtcgtccat gctgtgtttg ctttaaaagc caggacggtc 480
   acctttgggg tggtgacaag tgtgatcact tgggtggtgg ctgtgtttgc gtctctccca 540
   ggaatcatct ttaccagatc tcaaaaagaa ggtcttcatt acacctgcag ctctcatttt 600
   ccatacagtc agtatcaatt ctggaagaat ttccagacat taaagatagt catcttgggg 660
   ctggtcctgc cgctgcttgt catggtcatc tgctactcgg gaatcctaaa aactctgctt 720
   cggtgtcgaa atgagaagaa gaggcacagg gctgtgaggc ttatcttcac catcatgatt 780
   gtttattttc tcttctgggc tccctacaac attgtccttc tcctgaacac cttccaggaa 840
   ttctttggcc tgaataattg cagtagctct aacaggttgg accaagctat gcaggtgaca 900
   gagactcttg ggatgacgca ctgctgcatc aaccccatca tctatgcctt tgtcggggag 960
   aagttcagaa actacctctt agtcttcttc caaaagcaca ttgccaaacg cttctgcaaa 1020
   tgctgttcta ttttccagca agaggctccc gagcgagcaa gctcagttta cacccgatcc 1080
   actggggagc aggaaatatc tgtgggcttg tgacacggac tcaagtgggc tggtgaccca 1140
   gtcagagttg tgcacatggc ttagttttca tacacagcct gggctggggg tggggtggga 1200
   gaggtctttt ttaaaaggaa gttactgtta tagagggtct aagattcatc catttatttg 1260
   gcatctgttt aaagtagatt agatctttta agcccatcaa ttatagaaag ccaaatcaaa 1320
   atatgttgat gaaaaatagc aaccttttta tctccccttc acatgcatca agttattgac 1380
   aaactctccc ttcactccga aagttcctta tgtatattta aaagaaagcc tcagagaatt 1440
   gctgattctt gagtttagtg atctgaacag aaataccaaa attatttcag aaatgtacaa 1500
   ctttttacct agtacaaggc aacatatagg ttgtaaatgt gtttaaaaca ggtctttgtc 1560
   ttgctatggg gagaaaagac atgaatatga ttagtaaaga aatgacactt ttcatgtgtg 1620
   atttcccctc caaggtatgg ttaataagtt tcactgactt agaaccaggc gagagacttg 1680
   tggcctggga gagctgggga agcttcttaa atgagaagga atttgagttg gatcatctat 1740
   tgctggcaaa gacagaagcc tcactgcaag cactgcatgg gcaagcttgg ctgtagaagg 1800
   agacagagct ggttgggaag acatggggag gaaggacaag gctagatcat gaagaacctt 1860
   gacggcattg ctccgtctaa gtcatgagct gagcagggag atcctggttg gtgttgcaga 1920
   aggtttactc tgtggccaaa ggagggtcag gaaggatgag catttagggc aaggagacca 1980
   ccaacagccc tcaggtcagg gtgaggatgg cctctgctaa gctcaaggcg tgaggatggg 2040
   aaggagggag gtattcgtaa ggatgggaag gagggaggta ttcgtgcagc atatgaggat 2100
   gcagagtcag cagaactggg gtggatttgg tttggaagtg agggtcagag aggagtcaga 2160
   gagaatccct agtcttcaag cagattggag aaacccttga aaagacatca agcacagaag 2220
   gaggaggagg aggtttaggt caagaagaag atggattggt gtaaaaggat gggtctggtt 2280
   tgcagagctt gaacacagtc tcacccagac tccaggctgt ctttcactga atgcttctga 2340
   cttcatagat ttccttccca tcccagctga aatactgagg ggtctccagg aggagactag 2400
   atttatgaat acacgaggta tgaggtctag gaacatactt cagctcacac atgagatcta 2460
   ggtgaggatt gattacctag tagtcatttc atgggttgtt gggaggattc tatgaggcaa 2520
   ccacaggcag catttagcac atactacaca ttcaataagc atcaaactct tagttactca 2580
   ttcagggata gcactgagca aagcattgag caaaggggtc ccatataggt gagggaagcc 2640
   tgaaaaacta agatgctgcc tgcccagtgc acacaagtgt aggtatcatt ttctgcattt 2700
   aaccgtcaat aggcaaaggg gggaagggac atattcattt ggaaataagc tgccttgagc 2760
   cttaaaaccc acaaaagtac aatttaccag cctccgtatt tcagactgaa tgggggtggg 2820
   gggggcgcct taggtactta ttccagatgc cttctccaga caaaccagaa gcaacagaaa 2880
   aaatcgtctc tccctccctt tgaaatgaat atacccctta gtgtttgggt atattcattt 2940
   caaagggaga gagagaggtt tttttctgtt ctttctcata tgattgtgca catacttgag 3000
   actgttttga atttggggga tggctaaaac catcatagta caggtaaggt gagggaatag 3060
   taagtggtga gaactactca gggaatgaag gtgtcagaat aataagaggt gctactgact 3120
   ttctcagcct ctgaatatga acggtgagca ttgtggctgt cagcaggaag caacgaaggg 3180
   aaatgtcttt ccttttgctc ttaagttgtg gagagtgcaa cagtagcata ggaccctacc 3240
   ctctgggcca agtcaaagac attctgacat cttagtattt gcatattctt atgtatgtga 3300
   aagttacaaa ttgcttgaaa gaaaatatgc atctaataaa aaacaccttc taaaataaaa 3360
   aaaaaaaaaa aaaaaaaaaa aaa 3383

<210> 65
<211> 420
<212> DNA
<213> Homo sapiens

<220>
<223> II.65 (NM_004089)

<400> 65
gggggctggc cgagcgccgt gcgcgcttgg gagaaggccg gaagcttacc agccgagaag 60

gaattcctag ctagcttcag agccggtgcc tccggagcca gcgtggtggc catagacaac 120

aagttcgaac aggccatgga tctggtgaag aatcatctga tgtatgctgt gagagaggag 180

gtggagatcc tgaaggagca gatccgagag ctggtggaga agaactccca gctagagcgt 240

gagaacaccc tgttgaagac cctggcaagc ccagagcagc tggagaagtt ccagtcctgt 300

ctgagccctg aagagccagc tcccgaatcc ccacaagtgc ccgaggcccc tggtggttct 360

gcggtgtaag tcgctctgtc ctcagggtgg gcagagccac taaacttgtt ttacctaggg 420

<210> 66
<211> 3590
<212> DNA
<213> Homo sapiens

<220>
<223> II.66 (XM_037260)

<400> 66
ggggcgcaca gagccagagg ggcttgcgag cggcggctga gggaccgcgg ggagggggcg 60

ccgagcggct ccagcgcaga gactctcact gcacgccgga gggcgccctt cctcgctcgc 120

gcccgcgcga ccgcgcgccc cagtcccgcc ccgccccgct aaccgcccca gacacagcgc 180

tcgccgaggg tcgcttggac cctgatctta cccgtgggca ccctgcgctc tgcctgccgc 240

gaagaccggc tccccgaccc gcagaagtca ggagagaggg tgaagcggag cagcccgagg 300
cggggcagcc tcccggagca gcgccgcgca gagcccggga caatggggcc gcggcggctg 360
ctgctggtgg ccgcctgctt cagtctgtgc ggcccgctgt tgtctgcccg cacccgggcc 420
cgcaggccag aatcaaaagc aacaaatgcc accttagatc cccggtcatt tcttctcagg 480
aaccccaatg ataaatatga accattttgg gaggatgagg agaaaaatga aagtgggtta 540
actgaataca gattagtctc catcaataaa agcagtcctc ttcaaaaaca acttcctgca 600
ttcatctcag aagatgcctc cggatatttg accagctcct ggctgacact ctttgtccca 660
tctgtgtaca ccggagtgtt tgtagtcagc ctcccactaa acatcatggc catcgttgtg 720
ttcatcctga aaatgaaggt caagaagccg gcggtggtgt acatgctgca cctggccacg 780
gcagatgtgc tgtttgtgtc tgtgctcccc tttaagatca gctattactt ttccggcagt 840
gattggcagt ttgggtctga attgtgtcgc ttcgtcactg cagcatttta ctgtaacatg 900
tacgcctcta tcttgctcat gacagtcata agcattgacc ggtttctggc tgtggtgtat 960
cccatgcagt ccctctcctg gcgtactctg ggaagggctt ccttcacttg tctggccatc 1020
tgggctttgg ccatcgcagg ggtagtgcct ctgctcctca aggagcaaac catccaggtg 1080
cccgggctca acatcactac ctgtcatgat gtgctcaatg aaaccctgct cgaaggctac 1140
tatgcctact acttctcagc cttctctgct gtcttctttt ttgtgccgct gatcatttcc 1200
acggtctgtt atgtgtctat cattcgatgt cttagctctt ccgcagttgc caaccgcagc 1260
aagaagtccc gggctttgtt cctgtcagct gctgttttct gcatcttcat catttgcttc 1320
ggacccacaa acgtcctcct gattgcgcat tactcattcc tttctcacac ttccaccaca 1380
gaggctgcct actttgccta cctcctctgt gtctgtgtca gcagcataag ctgctgcatc 1440
gaccccctaa tttactatta cgcttcctct gagtgccaga ggtacgtcta cagtatctta 1500
tgctgcaaag aaagttccga tcccagcagt tataacagca gtgggcagtt gatggcaagt 1560
aaaatggata cctgctctag taacctgaat aacagcatat acaaaaagct gttaacttag 1620
gaaaagggac tgctgggagg ttaaaaagaa aagtttataa aagtgaataa cctgaggatt 1680
ctattagtcc ccacccaaac tttattgatt cacctcctaa aacaacagat gtacgacttg 1740
catacctgct ttttatggga gctgtcaagc atgtattttt gtcaattacc agaaagataa 1800
caggacgaga tgacggtgtt attccaaggg aatattgcca atgctacagt aataaatgaa 1860
tgtcacttct ggatatagct aggtgacata tacatactta catgtgtgta tatgtagatg 1920
tatgcacaca catatattat ttgcagtgca gtatagaata ggcactttaa aacactcttt 1980
ccccgcaccc cagcaattat gaaaataatc tctgattccc tgatttaata tgcaaagtct 2040
aggttggtag agtttagccc tgaacatttc atggtgttca tcaacagtga gagactccat 2100
agtttgggct tgtaccactt ttgcaaataa gtgtattttg aaattgtttg acggcaaggt 2160
ttaagttatt aagaggtaag acttagtact atctgtgcgt agaagttcta gtgttttcaa 2220
ttttaaacat atccaagttt gaattcctaa aattatggaa acagatgaaa agcctctgtt 2280

ttgatatggg tagtattttt tacattttac acactgtaca cataagccaa aactgagcat 2340

aagtcctcta gtgaatgtag gctggctttc agagtaggct attcctgaga gctgcatgtg 2400

tccgcccccg atggaggact ccaggcagca gacacatgcc agggccatgt cagacacaga 2460

ttggccagaa accttcctgc tgagcctcac agcagtgaga ctggggccac tacatttgct 2520

ccatcctcct gggattggct gtgaactgat catgtttatg agaaactggc aaagcagaat 2580

gtgatatcct aggaggtaat gaccatgaaa gacttctcta cccatcttaa aaacaacgaa 2640

agaaggcatg gacttctgga tgcccatcca ctgggtgtaa acacatctag tagttgttct 2700

gaaatgtcag ttctgatatg gaagcaccca ttatgcgctg tggccactcc aataggtgct 2760

gagtgtacag agtggaataa gacagagacc tgccctcaag agcaaagtag atcatgcata 2820

gagtgtgatg tatgtgtaat aaatatgttt cacacaaaca aggcctgtca gctaaagaag 2880

tttgaacatt tgggttacta tttcttgtgg ttataactta atgaaaacaa tgcagtacag 2940

gacatatatt ttttaaaata agtctgattt aattgggcac tatttattta caaatgtttt 3000

gctcaataga ttgctcaaat caggttttct tttaagaatc aatcatgtca gtctgcttag 3060

aaataacaga agaaaataga attgacattg aaatctagga aaattattct ataatttcca 3120

tttacttaag acttaatgag actttaaaag cattttttaa cctcctaagt atcaagtata 3180

gaaaatcttc atggaattca caaagtaatt tggaaattag gttgaaacat atctcttatc 3240

ttacgaaaaa atggtagcat tttaaacaaa atagaaagtt gcaaggcaaa tgtttattta 3300

aaagagcagg ccaggcgcgg tggctcacgc ctgtaatccc agcactttgg gaggctgagg 3360

cgggtggatc acgaggtcag gagatcgaga ccatcctggc taacacggtg aaacccgtct 3420

ctactaaaaa tgcaaaaaaa attagccggg cgtggtggca ggcacctgta gtcccagcta 3480

ctcgggaggc tgaggcagga gactggcgtg aacccaggag gcggaccttg tagtgagccg 3540

agatcgcgcc actgtgctcc agcctgggca acagagcaag actccatctc 3590

<210> 67
<211> 8615
<212> DNA
<213> Homo sapiens

<220>
<223> II.67 (NM_172217)

<400> 67
gagtttgcag aagagagtct tcccgagaag gcagaacgag ggtgtgtgga gcactgcggg 60

cgagatggag ctgagggaag gcagagctgg gtctcactct gttgcccagg ctggagttca 120

gcggtgcaat cccggctcac tgcagccttg actccgtgag ctcagggact catgaagtgg 180

cagctaagcc ctgtccagtg gccacccgtc agccaagggc cagagaccag gaaaggaaga 240

aaggcagctt cacttcctct ttgaggatgg agtcgcacag ccgcgctgga aagagcagaa 300

aatctgcaaa atttcggtcc atctccaggt ccctgatgct ctgtaatgct aagaccagtg 360

atgatggctc tagccctgat gagaaatatc ctgatccctt tgagatttcc ttggcccagg 420
gcaaggaggg aattttccac tcatctgtgc agctggcaga cacatcggag gctgggccca 480
gcagtgttcc tgatctagca ctggcctcgg aggctgctca actccaagca gctgggaatg 540
atcgaggcaa gacctgtagg aggatattct tcatgaagga atcttccaca gcttcctctc 600
gagaaaagcc tggaaaacta gaagcacaaa gtagtaactt cctgtttcct aaagcctgcc 660
accaaagggc acgcagcaac tcaaccagtg ttaatcccta ttgcacaaga gaaattgatt 720
ttccaatgac caagaaatct gcagcgccca cggacaggca gccttactct ctctgcagta 780
acaggaagtc cctctctcaa caattggact gtccagcagg aaaggctgcg ggaacttcga 840
gaccaacacg gtccctgagc acagctcagc tcgtgcagcc atctgggggc ctccaggctt 900
cagtcatctc caacatcgtg ctgatgaagg gccaggctaa gggtctgggc ttcagcatcg 960
ttgggggaaa agacagcatt tatggcccca ttgggattta cgtcaaaacc atttttgcag 1020
ggggagcagc agcagccgat ggaaggctac aggaaggtga tgaaattctg gagctcaatg 1080
gtgaatcaat ggctggacta acacatcagg atgctttgca gaagttcaag caagccaaaa 1140
aggggctcct caccctcacc gtgagaaccc gcctgacggc gcctccttcc ctgtgcagcc 1200
acctgtctcc cccactgtgc cgctccctga gctccagcac ttgtatcacc aaggacagca 1260
gctccttcgc cttggaaagc ccctcggctc ccatcagcac cgccaagccc aattacagaa 1320
tcatggtgga ggtttctctg cagaaagagg ccggcgtggg cctgggcatc ggcctgtgca 1380
gcgttcccta cttccaatgc atctctggca ttttcgtcca cacgctgtca ccaggatccg 1440
tggcgcacct ggacggacgt ctccggtgtg gggacgagat tgtggaaatc agtgattccc 1500
ctgtgcactg cctgacgctc aatgaagtct acacgatcct gagtcactgt gatcccggtc 1560
cagtccccat cattgttagc cgacatccag acccacaggt ctctgaacag caactcaaag 1620
aagctgtggc ccaggctgtg gaaaacacca agtttggaaa ggagaggcat cagtggagtc 1680
tggaaggtgt caaaaggctg gaaagcagtt ggcacgggcg gcccaccttg gagaaggaac 1740
gagagaagaa ctcagcaccc ccgcatcgca gggctcagaa ggtcatgatc cgctccagca 1800
gtgacagcag ctacatgtct gggtccccag ggggaagtcc tgggagtggc agtgctgaga 1860
agccgtcctc tgacgtggac atcagcacac acagccccag cttgcctctg gcacgggagc 1920
cagtggtgct ttctatagca tcctccaggc tgccccagga gagcccaccc ctcccagaga 1980
gccgggacag ccacccgccg ctgagactga agaaatcctt tgagattttg gaagcgagag 2040
agctgctgcc actgctgcta ccacaggaag acacagcagg gagaagccct agtgcctctg 2100
ccggctgccc aggacctggt atcggcccac agaccaagtc ctccacagag ggcgagccag 2160
ggtggagaag agccagccca gtgacccaaa catccccgat aaaacaccca ctgcttaaga 2220
ggcaggctcg gatggactat agctttgata ccacagccga agacccttgg gttaggattt 2280
ctgactgcat caaaaactta tttagcccca tcatgagtga gaaccatggc cacatgcctc 2340
tacagcccaa tgccagcctg aatgaagaag aagggacaca gggccaccca gatgggaccc 2400

caccaaagct ggacaccgcc aatggcactc ccaaagttta caagtcagca gacagcagca 2460
ctgtgaagaa aggtcctcct gtggctccca agccagcctg gtttcgccaa agcttgaaag 2520
gtttgaggaa tcgtgcttca gacccaagag ggctccctga tcctgccttg tccacccagc 2580
cagcacctgc ttccagggag cacctaggat cacacatccg ggcctcctcc tcctcctcct 2640
ccatcaggca gagaatcagc tcctttgaaa cctttggctc ctctcaactg cctgacaaag 2700
gagcccagag actgagcctc cagccctcct ctggggaggc agcaaaacct cttgggaagc 2760
atgaggaagg acggttttct ggactcttgg ggcgaggggc tgcacccact cttgtgcccc 2820
agcagcctga gcaagtactg tcctcggggt cccctgcagc ctccgaggcc agagacccag 2880
gtgtgtctga gtcccctccc ccagggcggc agcccaatca gaaaactctc ccccctggcc 2940
cggacccgct cctaaggctg ctgtcaacac aggctgagga atctcaaggc ccagtgctca 3000
agatgcctag ccagcgagca cggagcttcc ccctgaccag gtcccagtcc tgtgagacga 3060
agctacttga cgaaaagacc agcaaactct attctatcag cagccaagtg tcatcggctg 3120
tcatgaaatc cttgctgtgc cttccatctt ctatctcctg tgcccagact ccctgcatcc 3180
ccaaggaagg ggcatctcca acatcatcat ccaacgaaga ctcagctgca aatggttctg 3240
ctgaaacatc tgccttggac acggggttct cgctcaacct ttcagagctg agagaatata 3300
cagagggtct cacggaagcc aaggaagacg atgatgggga ccacagttcc cttcagtctg 3360
gtcagtccgt tatctccctg ctgagctcag aagaattaaa aaaactcatc gaggaggtga 3420
aggttctgga tgaagcaaca ttaaagcaat tagacggcat ccatgtcacc atcttacaca 3480
aggaggaagg tgctggtctt gggttcagct tggcaggagg agcagatcta gaaaacaagg 3540
tgattacggt tcacagagtg tttccaaatg ggctggcctc ccaggaaggg actattcaga 3600
agggcaatga ggttctttcc atcaacggca agtctctcaa ggggaccacg caccatgatg 3660
ccttggccat cctccgccaa gctcgagagc ccaggcaagc tgtgattgtc acaaggaagc 3720
tgactccaga ggccatgccc gacctcaact cctccactga ctctgcagcc tcagcctctg 3780
cagccagtga tgtttctgta gaatctacag cagaggccac agtctgcacg gtgacactgg 3840
agaagatgtc ggcagggctg ggcttcagcc tggaaggagg gaagggctcc ctacacggag 3900
acaagcctct caccattaac aggattttca aaggtggaca cagaggactt cgtgccagag 3960
gcaagatccc tgtaaatatt gttgcacaaa aatctcacta gctctcttcc cataccaccc 4020
aatgctgatg tcctcaccac atgcggagaa caaatgtgaa gggagtagga tattgggtca 4080
gtgtccaaag cagggtctgg gcaggactca gctccccaga gtcctctatg aactatggac 4140
ggtgctccag gcaggctaag gcgtggagct gcctgatatt tccctcccct ggggacagca 4200
agggctatcc ctttccaaag gccatggaga gctggagcct ggtgccctaa cctctgagtc 4260
accatcttaa gagatgcctc attttagaac caccaacaag caagctccca agggatggtg 4320
ccctgttctc taccaagcta tcctggctct ttggagatca aggagaggag gcaactttcc 4380
ttgttcccca tcatctgtgg aacccattac cttctccctc atttcaggag cagcctcaga 4440

acaaagtgag acagtccagc ctggagatga aatcttgcag ctgggtggca ctgccatgca 4500
gggcctcaca cggtttgaag cctggaacat catcaaggca ctgcctgatg gacctgtcac 4560
gattgtcatc aggagaaaaa gcctccagtc caaggaaacc acagctgctg gagactccta 4620
ggcaggacat gctgaagcca aagccaataa cacacagcta acacacagct cccataaccg 4680
ctgattctca gggtctctgc tgccgcccca cccagatggg ggaaagcaca ggtgggcttc 4740
ccagtggctg ctgcccaggc ccagaccttc taggacgcca cccagcaaaa ggttgttcct 4800
aaaataaggg cagagtcaca cgggggcagc tgatacaaat tgcagactgt gtaaaaagag 4860
agcttaatga taatattgtg gtgccacaaa taaaatggat ttattagaat ttcatatgac 4920
attcatgcct ggcttcgcaa aatgtttcaa gtactgtaac tgtgtcatga ttcaccccca 4980
aacagtgaca tttatttttc tcatgaatct gcaatgtggg cagagattgg aatgggcagc 5040
tcatctctgt cccacttggc atcagctggc gtcatgcaaa gtcatgcaaa ggctgggacc 5100
acgtgagatc attcactcat acatctggcc gttgatgttg gctgggaact cacctggggc 5160
tgctggcctg aatgcttata ggtggcctct ccttgtggcc tgggcctcct cacaacatgg 5220
tgtctggatt cccaggatga gcatcccagg atcgcaagag ccatgtagaa gctgcatctt 5280
gtttatacct ttgccttgga agttgcatgg catcacctcc accatactcc atcagttaga 5340
gctgacacaa acctgcctgg gtttaagggg agaggaaata ttgctggggt catttatgaa 5400
aaatacagtt tgtcacatga aacatttgca aaattgtttt tggttggatt ggagaagtaa 5460
tcctagggaa gggtggtgga gccagtaaac agaggagtac aggtgaagca ccaagctcaa 5520
agcgtggaca ggtgtgccga cagaaggaac cagcgtgtat atgagggtat caaataaaat 5580
tgctactact tacctaccac atgccaagca ctgtgctcag ggctaaacgg gcattgcctt 5640
tagtgatcac agcagcttct acggtgtatg gttctgtgcc aatgtattga taagagggca 5700
cacactgtgt acagtaaatg gcttatccag ctggtgagtg atgcggcaga ttgagttttc 5760
ttctgtgatt cggtggagac tatcagccca agatgcttta agtgcacaac attacaggga 5820
atgcctgagt gcctggccaa agggatattt ggtttggcca tctctggatg cctgattgcc 5880
aagctcagga ccaggcaatg tgactttgca tcagcaacaa ccagcatccc ttgaccaggc 5940
ctgggccaga gtattggtct cctctcagcc cctgatcctg tgaagtaagg atgtggggga 6000
agacctggca aggacacaga tgaaacacaa acaatagtaa ttctcaggcc atcatcagtg 6060
gagccatgtt aatgtaatct gatggcttct ccagggtcca caggaagtga agaatctgtt 6120
tcccagcagt ggactcaaaa cccatctggg ctcctaacct tcctgtaaac ccctttagtg 6180
gcttcattag agcaggcgtt cagctcactg ttctattcat ctcaaggaat aatgggctta 6240
gagcagtttc tgtcctgctg gttaacttgt ttggcctatt ccattctgga ttttgtcaag 6300
cagtagacaa gcaattagac aagaacttgg aggcaccatt tgtatccact ttttagactt 6360
aatagaaaca ttgaagatga acataatcta ccaacgaaag acgtgattca attcaacact 6420
cccttcccat gacccaggct gggcaaggag gccacgtgat gtggagggca cattccttgc 6480

ctgcacaaac tcaccatctg tgcacgcagt ggccttccct aaaatcaggg aattgtttta 6540
agtcttatca agcagccaag ggatgaaaga gaaggtgggt tttcatcaag actggaaggt 6600
ggggacaggg atgagcatgg agctggccgt gggcctgggg taccaagaga ctccttgaga 6660
gaccaggcaa agcaagtgat tgggacagag gttatctgtc ccaggttatc tgggcataga 6720
tgcaggtgag cccatggccc tcccagtacc tcctgtctct ggcctgtttt agaaggttct 6780
ctcctcccca aggagacaca acaactccta gggccactga agatataact attgcccagg 6840
tttctggtct ctaggctggg gaagtcctct gggtaggaat cagcaagaag atcctaaaac 6900
aaaagctcat ccatttgcgt tccatgatgc tgggatttac acttgaggct tagctttgct 6960
ctgccaactt cttcagagct gacacaggat gaaggcaatg ccatcctcaa acactgcagg 7020
catcacagct aacaattgtg aagtcgtctt aactcaccat aaaaaggaat ccactcccag 7080
gcagccctac ttctttgctt tgcccagcat tttactgatt catacattat ctcacttgtg 7140
ccaacactca agaagcaggc tacactgaca ctggtattcc tgcctccata ttttctttaa 7200
aagacaaatc aaagcagata tattaagtga ctgttcaaga gcacacttgg cccaagtggc 7260
agagcttgga ctggatgcat gttttccagc tcctcatcca gggctctgac cagtttaacc 7320
tgatgcagtc acgtggagga gcagtgcagg cacagtatgt cccataggcc cagtgagatg 7380
cattcttggt tggctggcct tccacttggc tacacaggga tgtacaaggc gatcccatct 7440
tgataagacc accacctcag agtatggagc tcagagaggg caggcatgaa gtttccttgg 7500
ctggtgcacc tagaattggc tgaactcatg agaagttgat atagaacagt gcttgccaca 7560
gagcggggac tcggtaagca cttaatgaat gaatgaattc taagtcaatc caagagtctg 7620
atgatttctt gaaaagggtg ttagctaaag gatcttaggc atgactgtag aatttgtagt 7680
tgcaatagaa cagagaaaga ggaagctttc tgtctcctta acactgagct gtcatgtttt 7740
aaagcttgct cacatcttgg cacatttaag agacagtcac cccaggactc aaaaataggg 7800
aagtaacagt aacgcagggg aaacgttttc tgtttggagg agcaaaggct gagaacactg 7860
tgaaaacatt ttgcgcgcac aatagtaacc tgggtaaatg cagcgtgaag ggattttagt 7920
cacacgtggt ctttcttaca aggaaggtgg tgggggtgca gatgaggttg ctagagaatg 7980
ttagaggatc cctctctgga ttggagatag ggaaagaaag ttgcacggct gctgaggccc 8040
cttctaggtg gcaaggctgt gctccctggt tctgatgatg tgcctgggtg gacatggccc 8100
ctgtgagttt gtacagtctt gcagcaggat ctagaggggg gatttccagc cagggctgct 8160
agacggaggc ctactcttcc atctttcctg atggcaggat ggcctggcca gggcctggaa 8220
gacagagacc tcctgcctcc gcctcagtaa gacgacaagg aaaggcaaat gcccaaggga 8280
aagaaaagga aggctcttct ccccagagtt ccccatgcag acatgagtgc gtgctcagtt 8340
cagaatcact tctgagaact catccctaat gctgcagatt tgggctggaa cagattcaca 8400
ctgtctggtt tcaccgagga catgaaactc caccttgcgg ggataaagag agaaaaacaa 8460
attcatcaaa tggaagacac attgaaagtg tttttcctta atgcttatcc tgtttttaaa 8520

ccattatttc caagttgaca ccttttttaa ggaaaaataa atattttgcg gcattaaagc 8580

cataaaaaaa aaaaaaaaaa aaaaaaaaaa aaaaa 8615

<210> 68
<211> 2448
<212> DNA
<213> Homo sapiens

<220>
<223> II.68 (AF244129)

<400> 68
cttcagtctc agttctgaaa atagatcatc atggtggcac caaagagtca cacagatgac 60

tgggctcctg ggcctttctc cagtaagcca cagaggagtc agctgcaaat attctcttct 120

gttctacaga cctctctcct cttcctgctc atgggactaa gagcctctgg aaaggactca 180

gccccaacag tggtgtcagg gatcctaggg ggttccgtga ctctccccct aaacatctca 240

gtagacacag agattgagaa cgtcatctgg attggtccca aaaatgctct tgctttcgca 300

cgtcccaaag aaaatgtaac cattatggtc aaaagctacc tgggccgact agacatcacc 360

aagtggagtt actccctgtg catcagcaat ctgactctga atgatgcagg atcctacaaa 420

gcccagataa accaaaggaa ttttgaagtc accactgagg aggaattcac cctgttcgtc 480

tatgagcagc tgcaggagcc ccaagtcacc atgaagtctg tgaaggtgtc tgagaacttc 540

tgtaacatca ctctaatgtg ctccgtgaag ggggcagaga aaagtgttct gtacagctgg 600

accccaaggg aaccccatgc ttctgagtcc aatggaggct ccattcttac cgtctcccga 660

acaccatgtg acccagacct gccatacatc tgcacagccc agaaccccgt cagccagaga 720

agctccctcc ctgtccatgt tgggcagttc tgtacagatc caggagcctc cagaggagga 780

acaacggggg agactgtggt aggggtcctg ggagagccag tcaccctgcc acttgcactc 840

ccagcctgcc gggacacaga gaaggttgtc tggttgttta acacatccat cattagcaaa 900

gagagggaag aagcagcaac ggcagatcca ctcattaaat ccagggatcc ttacaagaac 960

agggtgtggg tctccagcca ggactgctcc ctgaagatca gccagctgaa gatagaggac 1020

gccggcccct accatgccta cgtgtgctca gaggcctcca gcgtcaccag catgacacat 1080

gtcaccctgc tcatctaccg caggctgagg aagcccaaaa tcacgtggag cctcaggcac 1140

agtgaggatg gcatctgcag gatcagcctg acctgctccg tggaggacgg gggaaacact 1200

gtcatgtaca catggacccc gctgcagaag gaagctgttg tgtcccaagg ggaatcacac 1260

ctcaatgtct catggagaag cagtgaaaat caccccaacc tcacatgcac agccagcaac 1320

cctgtcagca ggagttccca ccagtttctt tctgagaaca tctgttcagg acctgagaga 1380

aacacaaagc tttggattgg gttgttcctg atggtttgcc ttctgtgcgt tgggatcttc 1440

agctggtgca tttggaagcg aaaaggacgg tgttcagtcc cagccttctg ttccagccaa 1500

gctgaggccc cagcggatac accagaaccc acagctggcc acacgctata ctctgtgctc 1560

tcccaaggat atgagaagct ggacactccc ctcaggcctg ccaggcaaca gcctacaccc 1620

acctcagaca gcagctctga cagcaacctc acaactgagg aggatgagga caggcctgag 1680

gtgcacaagc ccatcagtgg aagatatgag gtgtttgacc aggtcactca ggagggcgct 1740

ggacatgacc cagcccctga gggccaagca gactatgatc ccgtcactcc atatgtcacg 1800

gaagttgagt ctgtggttgg agagaacacc gtgtatgcac aagtgttcaa cttacaggga 1860

aagaccccag tttctcagaa ggaagagagc tcagccacaa tctactgctc catacggaaa 1920

cctcaggtgg tgccaccacc acaacagaat gatcttgaga ttcctgaaag tcctacctat 1980

gaaaatttca cctgaaagga aaagcagctg ctgcctctct cctgggaccg tggggttgga 2040

aagtcagctg gacctcatgg ggcctggggc tcgctgacag aagcacctca gaatttcctt 2100

cagtgcctca gagatgcctg gatgtggccc ctccccctcc ttctcaccct taaggactcc 2160

caaacccatt aatagttcag acacaggctc cttcttggag cctatgggct tcagatgtct 2220

ttgccccatt tgtcacctcg cacacttata gcgtttcctc ctcgaaattc taccaagact 2280

ggtcaaatgt tgctgagggg cctggaccag ctgtccttta caccaccttc tcaacactgc 2340

tgaaaagaac cccaagagaa ttgtcacaca tgacacaaga tgtacataat atcatgctca 2400

cgcctggagt gttatttaaa ataaaaggca ggaaataaaa aaaaaaaa 2448

<210> 69
<211> 1566
<212> DNA
<213> Homo sapiens

<220>
<223> II.69 (NM_003775)

<400> 69
gagtcagccc ccgggggagg ccatgaacgc cacggggacc ccggtggccc ccgagtcctg 60

ccaacagctg gcggccggcg ggcacagccg gctcattgtt ctgcactaca accactcggg 120

ccggctggcc gggcgcgggg ggccggagga tggcggcctg ggggccctgc gggggctgtc 180

ggtggccgcc agctgcctgg tggtgctgga gaacttgctg gtgctggcgg ccatcaccag 240

ccacatgcgg tcgcgacgct gggtctacta ttgcctggtg aacatcacgc tgagtgacct 300

gctcacgggc gcggcctacc tggccaacgt gctgctgtcg ggggcccgca ccttccgtct 360

ggcgcccgcc cagtggttcc tacgggaggg cctgctcttc accgccctgg ccgcctccac 420

cttcagcctg ctcttcactg caggggagcg ctttgccacc atggtgcggc cggtggccga 480

gagcggggcc accaagacca gccgcgtcta cggcttcatc ggcctctgct ggctgctggc 540

cgcgctgctg gggatgctgc ctttgctggg ctggaactgc ctgtgcgcct ttgaccgctg 600

ctccagcctt ctgcccctct actccaagcg ctacatcctc ttctgcctgg tgatcttcgc 660

cggcgtcctg gccaccatca tgggcctcta tggggccatc ttccgcctgg tgcaggccag 720

cgggcagaag gccccacgcc cagcggcccg ccgcaaggcc cgccgcctgc tgaagacggt 780

gctgatgatc ctgctggcct tcctggtgtg ctggggccca ctcttcgggc tgctgctggc 840

cgacgtcttt ggctccaacc tctgggccca ggagtacctg cggggcatgg actggatcct 900

ggccctggcc gtcctcaact cggcggtcaa ccccatcatc tactccttcc gcagcaggga 960

ggtgtgcaga gccgtgctca gcttcctctg ctgcgggtgt ctccggctgg gcatgcgagg 1020

gcccggggac tgcctggccc gggccgtcga ggctcactcc ggagcttcca ccaccgacag 1080

ctctctgagg ccaagggaca gctttcgcgg ctcccgctcg ctcagctttc ggatgcggga 1140

gcccctgtcc agcatctcca gcgtgcggag catctgaagt tgcagtcttg cgtgtggatg 1200

gtgcagccac cgggtgcgtg ccaggcaggc cctcctgggg tacaggaagc tgtgtgcacg 1260

cagcctcgcc tgtatgggga gcagggaacg ggacaggccc ccatggtctt cccggtggcc 1320

tctcggggct tctgacgcca aatgggcttc ccatggtcac cctggacaag gaggtaacca 1380

ccccacctcc ccgtaggagc agagagcacc ctggtgtggg ggcgagtggt tccccacaac 1440

cccgcttctg tgtgattctg gggaagtccc ggcccctctc tgggcctcag tagggctccc 1500

aggctgcaag gggtggactg tgggatgcat gccctggcaa cattgaagtt cgatcatggt 1560

aaaaaa 1566

<210> 70
<211> 1702
<212> DNA
<213> Homo sapiens

<220>
<223> II.70 (NM_001781)

<400> 70
agactcaaca agagctccag caaagacttt cactgtagct tgacttgacc tgagattaac 60

tagggaatct tgagaataaa gatgagctct gaaaattgtt tcgtagcaga gaacagctct 120

ttgcatccgg agagtggaca agaaaatgat gccaccagtc cccatttctc aacacgtcat 180

gaagggtcct tccaagttcc tgtcctgtgt gctgtaatga atgtggtctt catcaccatt 240

ttaatcatag ctctcattgc cttatcagtg ggccaataca attgtccagg ccaatacaca 300

ttctcaatgc catcagacag ccatgtttct tcatgctctg aggactgggt tggctaccag 360

aggaaatgct actttatttc tactgtgaag aggagctgga cttcagccca aaatgcttgt 420

tctgaacatg gtgctactct tgctgtcatt gattctgaaa aggacatgaa ctttctaaaa 480

cgatacgcag gtagagagga acactgggtt ggactgaaaa aggaacctgg tcacccatgg 540

aagtggtcaa atggcaaaga atttaacaac tggttcaacg ttacagggtc tgacaagtgt 600

gtttttctga aaaacacaga ggtcagcagc atggaatgtg agaagaattt atactggata 660

tgtaacaaac cttacaaata ataaggaaac atgttcactt attgactatt atagaatgga 720

actcaaggaa atctgtgtca gtggatgctg ctctgtggtc cgaagtcttc catagagact 780

ttgtgaaaaa aaattttata gtgtcttggg aattttcttc caaacagaac tatggaaaaa 840

aaggaagaaa ttccaggaaa atctgcactg tgggctttta ttgccatgag ctagaagcat 900

cacaggttga ccaataacca tgcccaagaa tgagaagaat gactatgcaa cctttggatg 960

cactttatat tattttgaat ccagaaataa tgaaataact aggcgtggac ttactattta 1020

ttgctgaatg actaccaaca gtgagagccc ttcatgcatt tgcactactg gaaggagtta 1080

gatgttggta ctagatactg aatgtaaaca aaggaattat ggctggtaac ataggttttt 1140

agtctaattg aatcccttaa actcagggag catttataaa tggacaaatg cttatgaaac 1200

taagatttgt aatatttctc tctttttaga gaaatttgcc aatttacttt gttatttttc 1260

cccaaaaaga atgggatgat cgtgtattta tttttttact tcctcagctg tagacaggtc 1320

cttttcgatg gtacatattt ctttgccttt ataatctttt atacagtgtc ttacagagaa 1380

aagacataag caaagactat gaggaatatt tgcaagacat agaatagtgt tggaaaatgt 1440

gcaatatgtg atgtggcaaa tctctattag gaaatattct gtaatcttca gacctagaat 1500

aatactagtc ttataatagg tttgtgactt tcctaaatca attctattac gtgcaatact 1560

tcaatacttc atttaaaata tttttatgtg caataaaatg tatttgtttg tattttgtgt 1620

tcagtacaat tataagctgt ttttatatat gtgaaataaa agtagaataa acacaaaaaa 1680

aaaaaaaaaa aaaaaaaaaa aa 1702

<210> 71
<211> 1349
<212> DNA
<213> Homo sapiens

<220>
<223> II.71 (NM_019846)

<400> 71
   cctgatcgaa cagcctcact tgtgttgctg tcagtgccag tagggcaggc aggaatgcag 60
   cagagaggac tcgccatcgt ggccttggct gtctgtgcgg ccctacatgc ctcagaagcc 120
   atacttccca ttgcctccag ctgttgcacg gaggtttcac atcatatttc cagaaggctc 180
   ctggaaagag tgaatatgtg tcgcatccag agagctgatg gggattgtga cttggctgct 240
   gtcatccttc atgtcaagcg cagaagaatc tgtgtcagcc cgcacaacca tactgttaag 300
   cagtggatga aagtgcaagc tgccaagaaa aatggtaaag gaaatgtttg ccacaggaag 360
   aaacaccatg gcaagaggaa cagtaacagg gcacatcagg ggaaacacga aacatacggc 420
   cataaaactc cttattagag agtctacaga taaatctaca gagacaattc ctcaagtgga 480
   cttggccatg attggttagt ctcactctat cacacaggct ggagggcagt ggcaggatct 540
   cagttcaccc caacctttgc ctcacgggtt caagggattc tcgtgcctca gccttccaag 600
   tagctgggat tacaggtgtg cgccagtacg cctggctagt tttagtattt tttgttacag 660
   acggggtttc accatgttgg ctgggctggt ctcaaacttc tgatctcaag tgatccatct 720
   gccttggcct cccaaagtgc tgggattaca ggcatgagcc actgcacctg gcctacgtat 780
   taggctttat gtaagatgta catagtactc catactcctt tccttttcct cctattgaaa 840
   ctgttcctat aaactttata aaattaatca gagaagaaga gagggtgaga aacataaata 900
   atccaagctt gcaccacatt cagcattaat cactaggtca ctttgcttgc tgaccccttc 960
   ctcatagttg tttggctatt gcttcagaat tatgtggagt ctgttacaag attatagttc 1020
   cccttaactg ctctatagat aacagcttga acattatgaa atgctaagtt ttcccttcga 1080
   gacattcttt caggtcctgc atactgatga aactactgat gtcagctggt ctgaaggacc 1140
   ctaccagaag ctaaatcatc aaagaatgca atttccatat cctaatgatt caatctccct 1200
   taccctgacc aatcagtggc ccaaattttc cagccccttg cctcccagaa ccccagccca 1260
   gaactcttca gagatttaag aatctcctcc tacctcctga ctcagcacca tgtaatcatt 1320
   aaactctctg ctgcaaaaaa aaaaaaaaa 1349

<210> 72
<211> 1103
<212> DNA
<213> Homo sapiens

<220>
<223> II.72 (NM_001511)

<400> 72
cacagagccc gggccgcagg cacctcctcg ccagctcttc cgctcctctc acagccgcca 60

gacccgcctg ctgagcccca tggcccgcgc tgctctctcc gccgccccca gcaatccccg 120

gctcctgcga gtggcactgc tgctcctgct cctggtagcc gctggccggc gcgcagcagg 180

agcgtccgtg gccactgaac tgcgctgcca gtgcttgcag accctgcagg gaattcaccc 240

caagaacatc caaagtgtga acgtgaagtc ccccggaccc cactgcgccc aaaccgaagt 300

catagccaca ctcaagaatg ggcggaaagc ttgcctcaat cctgcatccc ccatagttaa 360

gaaaatcatc gaaaagatgc tgaacagtga caaatccaac tgaccagaag ggaggaggaa 420

gctcactggt ggctgttcct gaaggaggcc ctgcccttat aggaacagaa gaggaaagag 480

agacacagct gcagaggcca cctggattgt gcctaatgtg tttgagcatc gcttaggaga 540

agtcttctat ttatttattt attcattagt tttgaagatt ctatgttaat attttaggtg 600

taaaataatt aagggtatga ttaactctac ctgcacactg tcctattata ttcattcttt 660

ttgaaatgtc aaccccaagt tagttcaatc tggattcata tttaatttga aggtagaatg 720

ttttcaaatg ttctccagtc attatgttaa tatttctgag gagcctgcaa catgccagcc 780

actgtgatag aggctggcgg atccaagcaa atggccaatg agatcattgt gaaggcaggg 840

gaatgtatgt gcacatctgt tttgtaactg tttagatgaa tgtcagttgt tatttattga 900

aatgatttca cagtgtgtgg tcaacatttc tcatgttgaa actttaagaa ctaaaatgtt 960

ctaaatatcc cttggacatt ttatgtcttt cttgtaaggc atactgcctt gtttaatggt 1020

agttttacag tgtttctggc ttagaacaaa ggggcttaat tattgatgtt ttcatagaga 1080

atataaaaat aaagcactta tag 1103

<210> 73
<211> 3215

<212>  DNA
<213>  Homo sapiens

<220>
<223>  II.73 (NM_006505)

<400>  73
cttgaagaag tgggtattcc ccttcccacc ccaggcactg gaggagcggc cccccgggga      60

ttccaggacc tgagctccgg gagctggact cgcagcgacc gcggcagagc gagctggcgc     120

cgggaagcga ggagacgccc gcgggaggcc cagctgctcg gagcaactgg catggcccga     180

gccatggccg ccgcgtggcc gctgctgctg gtggcgctac tggtgctgtc ctggccaccc     240

ccaggaaccg gggacgtcgt cgtgcaggcg cccacccagg tgcccggctt cttgggcgac     300

tccgtgacgc tgccctgcta cctacaggtg cccaacatgg aggtgacgca tgtgtcacag     360

ctgacttggg cgcggcatgg tgaatctggc agcatggccg tcttccacca aacgcagggc     420

cccagctatt cggagtccaa acggctggaa ttcgtggcag ccagactggg cgcggagctg     480

cggaatgcct cgctgaggat gttcgggttg cgcgtagagg atgaaggcaa ctacacctgc     540

ctgttcgtca cgttcccgca gggcagcagg agcgtggata tctggctccg agtgcttgcc     600

aagccccaga acacagctga ggttcagaag gtccagctca ctggagagcc agtgcccatg     660

gcccgctgcg tctccacagg gggtcgcccg ccagcccaaa tcacctggca ctcagacctg     720

ggcgggatgc ccaatacgag ccaggtgcca gggttcctgt ctggcacagt cactgtcacc     780

agcctctgga tattggtgcc ctcaagccag gtggacggca agaatgtgac ctgcaaggtg     840

gagcacgaga gctttgagaa gcctcagctg ctgactgtga acctcaccgt gtactacccc     900

ccagaggtat ccatctctgg ctatgataac aactggtacc ttggccagaa tgaggccacc     960

ctgacctgcg atgctcgcag caacccagag cccacaggct ataattggag cacgaccatg    1020

ggtcccctgc caccctttgc tgtggcccag ggcgcccagc tcctgatccg tcctgtggac    1080

aaaccaatca acacaacttt aatctgcaac gtcaccaatg ccctaggagc tcgccaggca    1140

gaactgaccg tccaggtcaa agagggacct cccagtgagc actcaggcat gtcccgtaac    1200

gccatcatct tcctggttct gggaatcctg gtttttctga tcctgctggg gatcgggatt    1260

tatttctatt ggtccaaatg ttcccgtgag gtcctttggc actgtcatct gtgtccctcg    1320

agtacagagc atgccagcgc ctcagctaat gggcatgtct cctattcagc tgtgagcaga    1380

gagaacagct cttcccagga tccacagaca gagggcacaa ggtgacagcg tcgggactga    1440

gaggggagag agactggagc tggcaaggac gtgggcctcc agagttggac ccgaccccaa    1500

tggatgaaga ccccctccaa agagaccagc ctccctccct gtgccagacc tcaaaacgac    1560

gggggcaggt gcaagttcat aggtctccaa gaccaccctc ctttcatttg ctagaaggac    1620

tcactagact caggaaagct gttaggctca cagttacagt ttattacagt aaaaggacag    1680

agattaagat cagcaaaggg aggaggtgca cagcacacgt tccacgacag atgaggcgac    1740

ggcttccatc tgccctctcc cagtggagcc atataggcag cacctgattc tcacagcaac    1800

atgtgacaac atgcaagaag tactgccaat actgccaacc agagcagctc actcgagatc 1860

tttgtgtcca gagttttttg tttgtcttga gacagggtct ggctctgttg gcagactaga 1920

gtacagtggt gagatcacag ttcattgcag ccttgacttc tcaacgccaa gtcatcctcc 1980

cacctcagcc tcctgagtag ctatgactac aggtatgtgc caccacgtct ggctaatctt 2040

tttattattt gtaaagtcga ggtttccctg tgttgcccag gctggtcttg aactcttggc 2100

tccaagtgat acttctgcct tggcctccca aagtgctgaa ttaagcagct caccatccac 2160

acggctgacc tcatacatca agccaatacc gtgtggccca agacccccac cataaatcac 2220

atcattagca tgaaccaccc agagtggccc aagactccca gatcagctac caggcaggat 2280

attccaaggg cttagagatg aatgcccagg agctgaggat aaagggcccg atctttcttt 2340

gggcaaggtt aagcctttac tgcatagcag accacacaga agggtgtggg ccaccagaga 2400

attttggtaa aaatttggcc tctggccttg agcttctaaa tctctgtatc cgtcagatct 2460

ctgtggttac aagaaacagc cactgaccct ggtcaccaga ggctgcaatt caggccgcaa 2520

gcagctgcct agggggtgtc caaggagcag agaaaactac tagatgtgaa cttgaagaag 2580

gttgtcagct gcagccactt tctgccagca tctgcagcca ctttctgcca gcatctgcag 2640

ccagcaagct gggactggca ggaaataacc cacaaaagaa gcaaatgcaa tttccaacac 2700

aagggggaag ggatgcaggg ggaggcagcg ctgcagttgc tcaggacacg ctcctatagg 2760

accaagatgg atgcgaccca agacccagga ggcccagctg ctcagtgcaa ctgacaagtt 2820

aaaaaggtct atgatcttga gggcagacag cagaattcct cttataaaga aaactgtttg 2880

ggaaaatacg ttgagggaga gaagaccttg ggccaagatg ctaaatggga atgcaaagct 2940

tgagctgctc tgcaagagaa aataagcagg acagaggatt tgctctggac agagatggaa 3000

gagccgggaa cagagaagtg tggggaagag ataggaacca gcaggatggc aggggcaaag 3060

ggctcaaggg tgaggaggcc agtgggaccc cacagagttg gggagataaa ggaacattgg 3120

ttgctttggt ggcacgtaag ctccttgtct gtctccagca cccagaatct cattaaagct 3180

tatttattgt acctccaaaa aaaaaaaaaa aaaaa 3215

<210> 74
<211> 1474
<212> DNA
<213> Homo sapiens

<220>
<223> II.74 (NM_000075)

<400> 74
agccctccca gtttccgcgc gcctctttgg cagctggtca catggtgagg gtgggggtga 60

gggggcctct ctagcttgcg gcctgtgtct atggtcgggc cctctgcgtc cagctgctcc 120

ggaccgagct cgggtgtatg gggccgtagg aaccggctcc ggggccccga taacgggccg 180

cccccacagc accccgggct ggcgtgaggg tctcccttga tctgagaatg gctacctctc 240

gatatgagcc agtggctgaa attggtgtcg gtgcctatgg gacagtgtac aaggcccgtg 300

atccccacag tggccacttt gtggccctca agagtgtgag agtccccaat ggaggaggag 360

gtggaggagg ccttcccatc agcacagttc gtgaggtggc tttactgagg cgactggagg 420

cttttgagca tcccaatgtt gtccggctga tggacgtctg tgccacatcc cgaactgacc 480

gggagatcaa ggtaaccctg gtgtttgagc atgtagacca ggacctaagg acatatctgg 540

acaaggcacc cccaccaggc ttgccagccg aaacgatcaa ggatctgatg cgccagtttc 600

taagaggcct agatttcctt catgccaatt gcatcgttca ccgagatctg aagccagaga 660

acattctggt gacaagtggt ggaacagtca agctggctga ctttggcctg gccagaatct 720

acagctacca gatggcactt acacccgtgg ttgttacact ctggtaccga gctcccgaag 780

ttcttctgca gtccacatat gcaacacctg tggacatgtg gagtgttggc tgtatctttg 840

cagagatgtt tcgtcgaaag cctctcttct gtggaaactc tgaagccgac cagttgggca 900

aaatctttga cctgattggg ctgcctccag aggatgactg gcctcgagat gtatccctgc 960

cccgtggagc ctttcccccc agagggcccc gcccagtgca gtcggtggta cctgagatgg 1020

aggagtcggg agcacagctg ctgctggaaa tgctgacttt taacccacac aagcgaatct 1080

ctgcctttcg agctctgcag cactcttatc tacataagga tgaaggtaat ccggagtgag 1140

caatggagtg gctgccatgg aaggaagaaa agctgccatt tcccttctgg acactgagag 1200

ggcaatcttt gcctttatct ctgaggctat ggagggtcct cctccatctt tctacagaga 1260

ttactttgct gccttaatga cattcccctc ccacctctcc ttttgaggct tctccttctc 1320

cttcccattt ctctacacta aggggtatgt tccctcttgt ccctttccct acctttatat 1380

ttggggtcct tttttataca ggaaaaacaa aacaaagaaa taatggtctt tttttttttt 1440

ttaaaaaaaa aaaaaaaaaa aaaaaaaaaa aaaa 1474

<210> 75
<211> 4689
<212> DNA
<213> Homo sapiens

<220>
<223> II.75 (XM_042066)

<400> 75
cgagccctga ggcaggcggc ggcggaggag ccctcaaggc gagcagcgcg cccgcggctg 60

ccgcgggact gctgcgggag gcgggcagcg ggggccgcga gcgggcggac tggcggcggc 120

ggcagctgcg caaagtgcgg agtgtggagc tggaccagct gcctgagcag ccgctcttcc 180

ttgccgcctc accgccggcc tcctcgactt ccccgtcgcc ggagcccgcg gacgcagcgg 240

ggagtgggac cggcttccag cctgtggcgg tgccgccgcc ccacggagcc gcgagccgcg 300

gcggcgccca ccttaccgag tcggtggcgg cgccggacag cggcgcctcg agtcccgcag 360

cggccgagcc cggggagaag cgggcgcccg ccgccgagcc gtctcctgca gcggcccccg 420

ccggtcgtga gatggagaat aaagaaactc tcaaagggtt gcacaagatg gatgatcgtc 480

cagaggaacg aatgatcagg gagaaactga aggcaacctg tatgccagcc tggaagcacg 540
aatggttgga aaggagaaat aggcgagggc ctgtggtggt aaaaccaatc ccagttaaag 600
gagatggatc tgaaatgaat cacttagcag ctgagtctcc aggagaggtc caggcaagtg 660
cggcttcacc agcttccaaa ggccgacgca gtccttctcc tggcaactcc ccatcaggtc 720
gcacagtgaa atcagaatct ccaggagtaa ggagaaaaag agtttcccca gtgccttttc 780
agagtggcag aatcacacca ccccgaagag ccccttcacc agatggcttc tcaccatata 840
gccctgagga aacaaaccgc cgtgttaaca aagtgatgcg ggccagactg tacttactgc 900
agcagatagg gcctaactct ttcctgattg gaggagacag cccagacaat aaataccggg 960
tgtttattgg gcctcagaac tgcagctgtg cacgtggaac attctgtatt catctgctat 1020
ttgtgatgct ccgggtgttt caactagaac cttcagaccc aatgttatgg agaaaaactt 1080
taaagaattt tgaggttgag agtttgttcc agaaatatca cagtaggcgt agctcaagga 1140
tcaaagctcc atctcgtaac accatccaga agtttgtttc acgcatgtca aattctcata 1200
cattgtcatc atctagtact tctacgtcta gttcagaaaa cagcataaag gatgaagagg 1260
aacagatgtg tcctatttgc ttgttgggca tgcttgatga agaaagtctt acagtgtgtg 1320
aagacggctg caggaacaag ctgcaccacc actgcatgtc aatttgggca gaagagtgta 1380
gaagaaatag agaaccttta atatgtcccc tttgtagatc taagtggaga tctcatgatt 1440
tctacagcca cgagttgtca agtcctgtgg attccccttc ttccctcaga gctgcacagc 1500
agcaaaccgt acagcagcag cctttggctg gatcacgaag gaatcaagag agcaatttta 1560
accttactca ttatggaact cagcaaatcc ctcctgctta caaagattta gctgagccat 1620
ggattcaggt gtttggaatg gaactcgttg gctgcttatt ttctagaaac tggaatgtga 1680
gagagatggc cctcaggcgt ctttcccatg atgtcagtgg ggccctgctg ttggcaaatg 1740
gggagagcac tggaaattct gggggcagca gtggaagcag cccgagtggg ggagccacca 1800
gtgggtcttc ccagaccagt atctcaggag atgtggtgga ggcatgctgc agcgttctgt 1860
caatggtctg tgctgaccct gtctacaaag tgtacgttgc tgctttaaaa acattgagag 1920
ccatgctggt atatactcct tgccacagtt tagcggaaag aatcaaactt cagagacttc 1980
tccagccagt tgtagacacc atcctagtca aatgtgcaga tgccaatagc cgcacaagtc 2040
agctgtccat atcaacactg ttggaactgt gcaaaggcca agcaggagag ttggcagttg 2100
gcagagaaat actaaaagct ggatccattg gtattggtgg tgttgattat gtcttaaatt 2160
gtattcttgg aaaccaaact gaatcaaaca attggcaaga acttcttggc cgcctttgtc 2220
ttatagatag actgttgttg gaatttcctg ctgaatttta tcctcatatt gtcagtactg 2280
atgtttcaca agctgagcct gttgaaatca ggtataagaa gctgctgtcc ctcttaacct 2340
ttgctttgca gtccattgat aattcccact caatggttgg caaactttcc agaaggatct 2400
acttgagttc tgcaagaatg gttactacag taccccatgt gttttcaaaa ctgttagaaa 2460
tgctgagtgt ttccagttcc actcacttca ccaggatgcg tcgccgtttg atggctattg 2520

cagatgaggt ggaaattgcc gaagccatcc agttgggcgt agaagacact ttggatggtc 2580
aacaggacag cttcttgcag gcatctgttc ccaacaacta tctggaaacc acagagaaca 2640
gttcccctga gtgcacagtc catttagaga aaactggaaa aggattatgt gctacaaaat 2700
tgagtgccag ttcagaggac atttctgaga gactggccag catttcagta ggaccttcta 2760
gttcaacaac aacaacaaca acaacaacag agcaaccaaa gccaatggtt caaacaaaag 2820
gcagacccca cagtcagtgt ttgaactcct ctcctttatc tcatcattcc caattaatgt 2880
ttccagcctt gtcaacccct tcttcttcta ccccatctgt accagctggc actgcaacag 2940
atgtctctaa gcatagactt cagggattca ttccctgcag aataccttct gcatctcctc 3000
aaacacagcg caagttttct ctacaattcc acagaaactg tcctgaaaac aaagactcag 3060
ataaactttc cccagtcttt actcagtcaa gacccttgcc ctccagtaac atacacaggc 3120
caaagccatc tagacctacc ccaggtaata caagtaaaca gggagatccc tcaaaaaata 3180
gcatgacact tgatctgaac agtagttcca aatgtgatga cagctttggc tgtagcagca 3240
atagtagtaa tgctgttata cccagtgacg agacagtgtt caccccagta gaggagaaat 3300
gcagattaga tgtcaataca gagctcaact ccagtattga ggaccttctt gaagcatcta 3360
tgccttcaag tgatacaaca gtaactttta agtcagaagt tgctgtcctg tctcctgaaa 3420
aggctgaaaa tgatgatacc tacaaagatg atgtgaatca taatcaaaag tgcaaagaga 3480
agatggaagc tgaagaagaa gaagctttag caattgccat ggcaatgtca gcgtctcagg 3540
atgccctccc catagttcct cagctgcagg ttgaaaatgg agaagatatc atcattattc 3600
aacaggatac accagagact ctaccaggac ataccaaagc aaaacaaccg tatagagaag 3660
acactgaatg gctgaaaggt caacagatag gccttggagc attttcttct tgttatcagg 3720
ctcaagatgt gggaactgga actttaatgg ctgttaaaca ggtgacttat gtcagaaaca 3780
catcttctga gcaagaagaa gtagtagaag cactaagaga agagataaga atgatgagcc 3840
atctgaatca tccaaacatc attaggatgt tgggagccac gtgtgagaag agcaattaca 3900
atctcttcat tgaatggatg gcagggggat cggtggctca tttgctgagt aaatatggag 3960
ccttcaaaga atcagtagtt attaactaca ctgaacagtt actccgtggc ctttcgtatc 4020
tccatgaaaa ccaaatcatt cacagagatg tcaaaggtgc caatttgcta attgacagca 4080
ctggtcagag actaagaatt gcagattttg gagctgcagc caggttggca tcaaaaggaa 4140
ctggtgcagg agagtttcag ggacaattac tggggacaat tgcatttatg gcacctgagg 4200
tactaagagg tcaacagtat ggaaggagct gtgatgtatg gagtgttggc tgtgctatta 4260
tagaaatggc ttgtgcaaaa ccaccatgga atgcagaaaa acactccaat catcttgctt 4320
tgatatttaa gattgctagt gcaactactg ctccatcgat cccttcacat ttgtctcctg 4380
gtttacgaga tgtggctctt cgttgtttag aacttcaacc tcaggacaga cctccatcaa 4440
gagagctact gaagcatcca gtctttcgta ctacatggta gccaattatg cagatcaact 4500
acagtagaaa caggatgctc aacaagagaa aaaaaacttg tggggaacca cattgatatt 4560

ctactggcca tgatgccact gaacagctat gaacgaggcc agtggggaac ccttacctaa 4620

gtatgtgatt gacaaatcat gatctgtacc taagctcagt atgcaaaagc ccaaactagt 4680

gcagaaact 4689

<210> 76
<211> 2090
<212> DNA
<213> Homo sapiens

<220>
<223> II.76 (NM_003242)

<400> 76
gttggcgagg agtttcctgt ttcccccgca gcgctgagtt gaagttgagt gagtcactcg 60

cgcgcacgga gcgacgacac ccccgcgcgt gcacccgctc gggacaggag ccggactcct 120

gtgcagcttc cctcggccgc cgggggcctc cccgcgcctc gccggcctcc aggcccctcc 180

tggctggcga gcgggcgcca catctggccc gcacatctgc gctgccggcc cggcgcgggg 240

tccggagagg gcgcggcgcg gagcgcagcc aggggtccgg gaaggcgccg tccgtgcgct 300

gggggctcgg tctatgacga gcagcggggt ctgccatggg tcgggggctg ctcaggggcc 360

tgtggccgct gcacatcgtc ctgtggacgc gtatcgccag cacgatccca ccgcacgttc 420

agaagtcggt taataacgac atgatagtca ctgacaacaa cggtgcagtc aagtttccac 480

aactgtgtaa attttgtgat gtgagatttt ccacctgtga caaccagaaa tcctgcatga 540

gcaactgcag catcacctcc atctgtgaga agccacagga agtctgtgtg gctgtatgga 600

gaaagaatga cgagaacata acactagaga cagtttgcca tgaccccaag ctcccctacc 660

atgactttat tctggaagat gctgcttctc caaagtgcat tatgaaggaa aaaaaaaagc 720

ctggtgagac tttcttcatg tgttcctgta gctctgatga gtgcaatgac aacatcatct 780

tctcagaaga atataacacc agcaatcctg acttgttgct agtcatattt caagtgacag 840

gcatcagcct cctgccacca ctgggagttg ccatatctgt catcatcatc ttctactgct 900

accgcgttaa ccggcagcag aagctgagtt caacctggga aaccggcaag acgcggaagc 960

tcatggagtt cagcgagcac tgtgccatca tcctggaaga tgaccgctct gacatcagct 1020

ccacgtgtgc caacaacatc aaccacaaca cagagctgct gcccattgag ctggacaccc 1080

tggtggggaa aggtcgcttt gctgaggtct ataaggccaa gctgaagcag aacacttcag 1140

agcagtttga gacagtggca gtcaagatct ttccctatga ggagtatgcc tcttggaaga 1200

cagagaagga catcttctca gacatcaatc tgaagcatga gaacatactc cagttcctga 1260

cggctgagga gcggaagacg gagttgggga aacaatactg gctgatcacc gccttccacg 1320

ccaagggcaa cctacaggag tacctgacgc ggcatgtcat cagctgggag gacctgcgca 1380

agctgggcag ctccctcgcc cgggggattg ctcacctcca cagtgatcac actccatgtg 1440

ggaggcccaa gatgcccatc gtgcacaggg acctcaagag ctccaatatc ctcgtgaaga 1500

acgacctaac ctgctgcctg tgtgactttg ggctttccct gcgtctggac cctactctgt 1560

ctgtggatga cctggctaac agtgggcagg tgggaactgc aagatacatg gctccagaag 1620

tcctagaatc caggatgaat ttggagaatg ctgagtcctt caagcagacc gatgtctact 1680

ccatggctct ggtgctctgg gaaatgacat ctcgctgtaa tgcagtggga gaagtaaaag 1740

attatgagcc tccatttggt tccaaggtgc gggagcaccc ctgtgtcgaa agcatgaagg 1800

acaacgtgtt gagagatcga gggcgaccag aaattcccag cttctggctc aaccaccagg 1860

gcatccagat ggtgtgtgag acgttgactg agtgctggga ccacgaccca gaggcccgtc 1920

tcacagccca gtgtgtggca gaacgcttca gtgagctgga gcatctggac aggctctcgg 1980

ggaggagctg ctcggaggag aagattcctg aagacggctc cctaaacact accaaatagc 2040

tcttatgggg caggctgggc atgtccaaag aggctgcccc tctcaccaaa 2090

<210> 77
<211> 3299
<212> DNA
<213> Homo sapiens

<220>
<223> II.77 (NM_003874)

<400> 77
ccacgcgtcc gtgcttttcc acagaaggtt agaccctgaa agagatggct cagcaccacc 60

tatggatctt gctcctttgc ctgcaaacct ggccggaagc agctggaaaa gactcagaaa 120

tcttcacagt gaatgggatt ctgggagagt cagtcacttt ccctgtaaat atccaagaac 180

cacggcaagt taaaatcatt gcttggactt ctaaaacatc tgttgcttat gtaacaccag 240

gagactcaga aacagcaccc gtagttactg tgacccacag aaattattat gaacggatac 300

atgccttagg tccgaactac aatctggtca ttagcgatct gaggatggaa gacgcaggag 360

actacaaagc agacataaat acacaggctg atccctacac caccaccaag cgctacaacc 420

tgcaaatcta tcgtcggctt gggaaaccaa aaattacaca gagtttaatg gcatctgtga 480

acagcacctg taatgtcaca ctgacatgct ctgtagagaa agaagaaaag aatgtgacat 540

acaattggag tcccctggga gaagagggta atgtccttca aatcttccag actcctgagg 600

accaagagct gacttacacg tgtacagccc agaaccctgt cagcaacaat tctgactcca 660

tctctgcccg gcagctctgt gcagacatcg caatgggctt ccgtactcac cacaccgggt 720

tgctgagcgt gctggctatg ttctttctgc ttgttctcat tctgtcttca gtgtttttgt 780

tccgtttgtt caagagaaga caagatgctg cctcaaagaa aaccatatac acatatatca 840

tggcttcaag gaacacccag ccagcagagt ccagaatcta tgatgaaatc ctgcagtcca 900

aggtgcttcc ctccaaggaa gagccagtga acacagttta ttccgaagtg cagtttgctg 960

ataagatggg gaaagccagc acacaggaca gtaaacctcc tgggacttca agctatgaaa 1020

ttgtgatcta ggctgctggg ctgaattctc cctctggaaa ctgagttaca accaccaata 1080

ctggcaggtt ccctggatcc agatcttctc tgcccaactc ttactgggag attgcaaact 1140

gccacatctc agcctgtaag caaagcagga aaccttctgc tgggcatagc ttgtgcctaa 1200
atggacaaat ggatgcatac ccttcctgaa atgactccct tctgaatgaa tgacaaagca 1260
ggttacctag tatagttttc ccaaacttct tcccatcata gcacatgtag aaaataatat 1320
ttttatggca cactgggata aacaagcaag attgctcact tctggaagct gcatatgact 1380
agaggcctct tgtgactgga ggtaacaacc ctgcccagta actgtgggag aaggggatca 1440
atattttgca cacctgtaat aggccatggc acaccagcca agatgctctg ctcacagtca 1500
gtatgtgtga agatccctgg tgcgtggcct tcaccacgca tcttgagcaa attaggaaaa 1560
tgtacccttc gcttgaggca gatgcagccc ttcccccgag tgcatggctt ggagagcaga 1620
atgtgggctg catataagca cactcatccc tttgtctggg aatctttgtg cagggcataa 1680
caggcttagt aagtccaaac acagatgaca gtgctgtgtg ggtctctgtc agagttgtgg 1740
ctctcagcca tgtagacaca ctctccaaat ggagtgttgg aaaatgttct ttctgcaggg 1800
tctagagact gctgggacac ttttcttgga gtgctacttc agaagcctta taggattttc 1860
tttctggcca agatttcctt ctgtatcact ccaagcagcc tcagcagaag aagcagccat 1920
gcccagtatt cccactctcc aaaaggaact gaccagctta tatttctcac acttctgggg 1980
aactgggtat aatccaacca tcaaaataga agaccttgca agaagcagag tcattctcca 2040
gaaggaactt gggagatgat ggtgcagatg atgaaactgg gttcatccca gttccaaaga 2100
ctcagagaac tagagtttaa gctgaggcag agtgccgcca ccctggcatg ccccacaaac 2160
agatcaccag ccagcttaca caggcattaa ctctcctcaa tgaggaagaa tcattcacaa 2220
ctgagcaaga cattcatatg atcatttaag gaagtgtttc ccttatgtgt tagcaagtat 2280
aatcggctaa ctcctaaatc ccaatgaata gtcctaggct ggacagcaat gggctgcaat 2340
taggcagata aagacatcag tcccagtaaa tgaatccata gactcatcta gcaccaacta 2400
ccattagcac tatgttagga gctgcaaggc cccaaagtag aagatgtgca taatgtctgc 2460
tcttgtgtag ctcaggagac aattccagca cagacactac agttaacgct gaactgcagc 2520
tgcaagtaat agcatgaaca gtcagaaaaa taccttatga gggggcaggg ctgaagctgg 2580
gccttgaagg atggatgaaa tttggataga gaatgaggaa gacagagggc ctccaagtga 2640
gagaagcatg aaaaatgagc aggggcctgg atcagtgggg tgtattcaga gcacctctcc 2700
agatgcacca tgcatgctca cagtcccttg cctatgtgtg gcagagtgtc ccagccagat 2760
gtgtgccccc accccatgtc catttacatg tccttcaatg cccacctcaa aaggtacctc 2820
ttctgtaaag ctttccctgg tatcaggaat caaaattaat cagggatctt ttcacactgc 2880
tgttttttcc tctttggtcc ttctatcact aaaactcatc tcattcagcc ttacagcata 2940
actaattatt tgttttcctc actacattgt acatgtggga attacagata aacggaagcc 3000
ggctggggtg gtggctcacg cctgtaaccc caacactttg ggaggccaag gcaggcggat 3060
cacctgaggt caggagttcg agattagtct ggccaacatg gtgaaacccc atctctacta 3120
aaaatacgaa attagccagg tgtggtggca cacatctgta gtcccagcta ctctggaggc 3180

tgagacagga gaatcgcttg aacccaggaa gtggaggttg cagtgagctg agatcacacc 3240

actgcactcc agcctgggag agagagagtg agtctccatc tcaaaaaaaa aaaaaaaaa 3299

<210> 78
<211> 2558
<212> DNA
<213> Homo sapiens

<220>
<223> II.78 (XM_033972)

<400> 78
aaaagtagtt tgtctttact aggccaccgt ctcgtcagcg ttacggagta ttttgtccgc 60

ctgccgccgc cgtcccagat attaatcacg gagttccagg gagaaggaac ttgtgaaatg 120

ggggagccgg ctggggttgc cggcaccatg gagtcacctt ttagcccggg actctttcac 180

aggctggatg aagattggga ttctgctctc tttgctgaac tcggttattt cacagacact 240

gatgagctgc aattggaagc agcaaatgag acgtatgaaa acaattttga taatcttgat 300

tttgatttgg atttgatgcc ttgggagtca gacatttggg acatcaacaa ccaaatctgt 360

acagttaaag atattaaggc agaacctcag ccactttctc cagcctcctc aagttattca 420

gtctcgtctc ctcggtcagt ggactcttat tcttcaactc agcatgttcc tgaggagttg 480

gatttgtctt ctagttctca gatgtctccc ctttccttat atggtgaaaa ctctaatagt 540

ctctcttcag cggagccact gaaggaagat aagcctgtca ctggtcctag gaacaagact 600

gaaaatggac tgactccaaa gaaaaaaatt caggtgaatt caaaaccttc aattcagccc 660

aagcctttat tgcttccagc agcacccaag actcaaacaa actccagtgt tccagcaaaa 720

accatcatta ttcagacagt accaacgctt atgccattgg caaagcagca accaattatc 780

agtttacaac ctgcacccac taaaggccag acggttttgc tgtctcagcc tactgtggta 840

caacttcaag cacctggagt tctgccctct gctcagccag tccttgctgt tgctggggga 900

gtcacacagc tccctaatca cgtggtgaat gtggtaccag ccccttcagc gaatagccca 960

gtgaatggaa aactttccgt gactaaacct gtcctacaaa gtaccatgag aaatgtcggt 1020

tcagatattg ctgtgctaag gagacagcaa cgtatgataa aaaatcgaga atccgcttgt 1080

cagtctcgca agaagaagaa agaatatatg ctagggttag aggcgagatt aaaggctgcc 1140

ctctcagaaa acgagcaact gaagaaagaa aatggaacac tgaagcggca gctggatgaa 1200

gttgtgtcag agaaccagag gcttaaagtc cctagtccaa agcgaagagt tgtctgtgtg 1260

atgatagtat tggcatttat aatactgaac tatggaccta tgagcatgtt ggaacaggat 1320

tccaggagaa tgaaccctag tgtgagccct gcaaatcaaa ggaggcacct tctaggattt 1380

tctgctaaag aggcacagga cacatcagat ggtattatcc agaaaaacag ctacagatat 1440

gatcattctg tttcaaatga caaagccctg atggtgctaa ctgaagaacc attgctttac 1500

attcctccac ctccttgtca gcccctaatt aacacaacag agtctctcag gttaaatcat 1560

gaacttcgag gatgggttca tagacatgaa gtagaaagga ccaagtcaag aagaatgaca 1620

aataatcaac agaaaacccg tattcttcag ggtgctctgg aacagggctc aaattctcag 1680

ctgatggctg ttcaatacac agaaaccact agtagtatca gcaggaactc agggagtgag 1740

ctacaagtgt attatgcttc acccagaagt tatcaagact tttttgaagc catccgcaga 1800

aggggagaca cattttatgt tgtgtcattt cgaagggatc acctgctgtt accagctacc 1860

acccataaca agaccacaag accaaaaatg tcaattgtgt taccagcaat aaacataaat 1920

gagaatgtga tcaatgggca ggactacgaa gtgatgatgc agattgactg tcaggtgatg 1980

gacaccagga tcctccatat caaaagttcg tcagttcctc cttacctccg agatcagcag 2040

aggaatcaaa ccaacacctt ctttggctcc cctcccgcag ccacagaggc aacccacgtt 2100

gtcagcacca tccctgagtc attacaatag caccctgcag ctatgctgga aaactgagcg 2160

tgggaccctg ccagactgaa gagcaggtga gcaaaatgct gctttctgcc ttggtggcag 2220

gcagagaact gtctcgtact agaattcaag gaggaaagaa gaagaaataa aagaagctgc 2280

tccatttttc atcatctacc catctatttg gaaagcactg gaattcagat gcaagagaac 2340

aatgtttctt cagtggcaaa tgtagccctg catcctccag tgttacctgg tgtagatttt 2400

tttttctgta cctttctaaa cctctcttcc ctctgtgatg gttttgtgtt taaacagtca 2460

tcttctttta aataatatcc acctctcctt tttgccattt cacttattga ttcataaagt 2520

gaattttatt taaagctatg ccacacatgc atgttcaa 2558

<210> 79
<211> 828
<212> DNA
<213> Homo sapiens

<220>
<223> II.79 (XM_001840)

<400> 79
caactctgga gtcctctgag agagccacca aggaggagca ggggagcgac ggccggggca 60

gaagttgaga ccacccagca gaggagctag gccagtccat ctgcatttgt cacccaagaa 120

ctcttaccat gaagaccctc ctactgttgg cagtgatcat gatctttggc ctactgcagg 180

cccatgggaa tttggtgaat ttccacagaa tgatcaagtt gacgacagga aaggaagccg 240

cactcagtta tggcttctac ggctgccact gtggcgtggg tggcagagga tcccccaagg 300

atgcaacgga tcgctgctgt gtcactcatg actgttgcta caaacgtctg gagaaacgtg 360

gatgtggcac caaatttctg agctacaagt ttagcaactc ggggagcaga atcacctgtg 420

caaaacagga ctcctgcaga agtcaactgt gtgagtgtga taaggctgct gccacctgtt 480

ttgctagaaa caagacgacc tacaataaaa agtaccagta ctattccaat aaacactgca 540

gagggagcac ccctcgttgc tgagtcccct cttccctgga aaccttccac ccagtgctga 600

atttccctct ctcataccct ccctccctac cctaaccaag ttccttggcc atgcagaaag 660

catccctcac ccatcctaga ggccaggcag gagcccttct atacccaccc agaatgagac 720

atccagcaga tttccagcct tctactgctc tcctccacct caactccgtg cttaaccaaa 780

gaagctgtac tccggggggt ctcttctgaa taaagcaatt agcaaatc 828

<210> 80
<211> 1978
<212> DNA
<213> Homo sapiens

<220>
<223> II.80 (NM_018310)

<400> 80
cgggggtgca accctttgaa gtgtcccctc ctacttcttg ctgcctcacg gttgctgttg 60

cagacccagg agtctctgaa ggcttccgcg gctttggggc cggttgtgag atgccaggca 120

gaggccgctg cccggactgc ggctccacgg agctggtgga agactcgcac tattcgcaga 180

gccagctggt gtgctccgac tgcggctgcg tggtcaccga gggggtcctt accactacct 240

tcagcgacga gggcaatctc cgagaggtaa catattcccg aagcacaggg gaaaacgaac 300

aagttagtcg cagccagcaa cgaggtctcc ggcgagtgag agacctttgt cgagttctgc 360

agttgccacc aacatttgag gataccgcgg ttgcctacta ccaacaggca tatcggcact 420

ctggcatccg agcggccagg ctgcaaaaga aggaggtgtt ggttgggtgc tgcgtcttaa 480

tcacctgccg acagcataac tggcccctaa caatgggggc catctgcacg ctgttgtatg 540

cagatttgga tgtgttttct agcacttaca tgcagatagt gaagctcctg ggactggatg 600

tgccatctct gtgcttggca gaactggtga agacctattg cagcagcttc aaactgttcc 660

aagcttcacc ttctgtgcca gccaaatacg tggaagacaa agagaagatg ctgtctcgaa 720

caatgcagtt ggtggagctg gcaaatgaga cgtggctggt gaccgggagg catcccttgc 780

ccgtcatcac tgctgcgact ttcctggctt ggcagtcgct gcagcctgca gatcggcttt 840

catgttccct tgcccgattt tgtaaattgg caaatgtgga cctgccctac ccggcgtcct 900

cccgcctgca ggagctgctg gctgtgctgc tgcggatggc tgagcagctg gcctggttac 960

gagttctgag acttgacaaa cggtctgtgg tgaagcacat cggtgacctt ctccagcacc 1020

gccagtcact ggtccgctct gcctttcggg atgggacagc agaagtggag acccgagaga 1080

aggagccacc ggggtgggga caggggcaag gagaagggga ggtgggaaat aattccttag 1140

gtttacccca ggggaagcgg ccggccagtc ctgcccttct cttgccaccc tgcatgttga 1200

agtccccgaa gcggatctgc cctgtacccc ctgtctccac tgtcactgga gatgagaaca 1260

tttctgatag tgaaatagaa cagtatttgc gtacccctca ggaagttagg gactttcaga 1320

gagcccaggc tgctagacag gctgccacga gtgtccctaa ccctccctga tggatatcca 1380

ctgggagcac ttcatcctgt tctgacagct tgataacatt cctgttataa ccaaggatgg 1440

aagtgtacac cagtccgtag gtattgcttt tcttgtttga aggaaccaag aggggctctg 1500

ccattagttg gaccctgggt cctggagtaa agtcaggagt gcagggatga ctataggtag 1560

gagagattcc catcccttgg tgtgggagag caagttgcct atgtccatgt tctgtgagat 1620

ggctttcctc atagatggat gggaaaatgt caggctcttt gctgctggtt tgaattggac 1680

acactgctgc ggctcctcct gcaggcctga gggggcttcc ctctgcttgt ggagtggttg 1740

gcattcccag cagtatcaac cctcagagga gcgggaactg gggaattctg gccctacgtg 1800

cattcacagg caatgatggg tttgtgtgta tggtgtcatg agatcctcta cctcataaca 1860

aaaggacagt gggtagacta aggcagtagc tcaaagggct ttgcaaaatt ttaatatatt 1920

aaaacaagag gcatctgcta gaaaacattc tattgtataa aacccgagtt cttaaaaa 1978

<210> 81
<211> 1816
<212> DNA
<213> Homo sapiens

<220>
<223> II.81 (AF212365)

<400> 81
gcacgagcga tgtcgctcgt gctgctaagc ctggccgcgc tgtgcaggag cgccgtaccc 60

cgagagccga ccgttcaatg tggctctgaa actgggccat ctccagagtg gatgctacaa 120

catgatctaa tccccggaga cttgagggac ctccgagtag aacctgttac aactagtgtt 180

gcaacagggg actattcaat tttgatgaat gtaagctggg tactccgggc agatgccagc 240

atccgcttgt tgaaggccac caagatttgt gtgacgggca aaagcaactt ccagtcctac 300

agctgtgtga ggtgcaatta cacagaggcc ttccagactc agaccagacc ctctggtggt 360

aaatggacat tttcctacat cggcttccct gtagagctga acacagtcta tttcattggg 420

gcccataata ttcctaatgc aaatatgaat gaagatggcc cttccatgtc tgtgaatttc 480

acctcaccag gctgcctaga ccacataatg aaatataaaa aaaagtgtgt caaggccgga 540

agcctgtggg atccgaacat cactgcttgt aagaagaatg aggagacagt agaagtgaac 600

ttcacaacca ctcccctggg aaacagatac atggctctta tccaacacag cactatcatc 660

gggttttctc aggtgtttga gccacaccag aagaaacaaa cgcgagcttc agtggtgatt 720

ccagtgactg gggatagtga aggtgctacg gtgcagctga ctccatattt tcctacttgt 780

ggcagcgact gcatccgaca taaaggaaca gttgtgctct gcccacaaac aggcgtccct 840

ttccctctgg ataacaacaa aagcaagccg ggaggctggc tgcctctcct cctgctgtct 900

ctgctggtgg ccacatgggt gctggtggca gggatctatc taatgtggag gcacgaaagg 960

atcaagaaga cttccttttc taccaccaca ctactgcccc ccattaaggt tcttgtggtt 1020

tacccatctg aaatatgttt ccatcacaca atttgttact tcactgaatt tcttcaaaac 1080

cattgcagaa gtgaggtcat ccttgaaaag tggcagaaaa agaaaatagc agagatgggt 1140

ccagtgcagt ggcttgccac tcaaaagaag gcagcagaca aagtcgtctt ccttctttcc 1200

aatgacgtca acagtgtgtg cgatggtacc tgtggcaaga gcgagggcag tcccagtgag 1260

aactctcaag actcttcccc ttgcctttaa ccttttctgc agtgatctaa gaagccagat 1320

tcatctgcac aaatacgtgg tggtctactt tagagagatt gatacaaaag acgattacaa 1380

tgctctcagt gtctgcccca agtaccacct catgaaggat gccactgctt tctgtgcaga 1440

acttctccat gtcaagtagc aggtgtcagc aggaaaaaga tcacaagcct gccacgatgg 1500

ctgctgctcc ttgtagccca cccatgagaa gcaagagacc ttaaaggctt cctatcccac 1560

caattacagg gaaaaaacgt gtgatgatcc tgaagcttac tatgcagcct acaaacagcc 1620

ttagtaatta aaacatttta taccaataaa attttcaaat attgctaact aatgtagcat 1680

taactaacga ttggaaacta catttacaac ttcaaagctg ttttatacat agaaatcaat 1740

tacagtttta attgaaaact ataaccattt tgataatgca acaataaagc atcttcagcc 1800

aaaaaaaaaa aaaaaa 1816

<210> 82
<211> 1281
<212> DNA
<213> Homo sapiens

<220>
<223> II.82 (XM_056798)

<400> 82
ccggcccgcg ccccgcaggc cgcccgccgc ccgcgccgcc atgggagtgg agggctgcac 60

caagtgcatc aagtacctgc tcttcgtctt caatttcgtc ttctggctgg ctggaggcgt 120

gatcctgggt gtggccctgt ggctccgcca tgacccgcag accaccaacc tcctgtatct 180

ggagctggga gacaagcccg cgcccaacac cttctatgta ggcatctaca tcctcatcgc 240

tgtgggcgct gtcatgatgt tcgttggctt cctgggctgc tacggggcca tccaggaatc 300

ccagtgcctg ctggggacgt tcttcacctg cctggtcatc ctgtttgcct gtgaggtggc 360

cgccggcatc tggggctttg tcaacaagga ccagatcgcc aaggatgtga agcagttcta 420

tgaccaggcc ctacagcagg ccgtggtgga tgatgacgcc aacaacgcca aggctgtggt 480

gaagaccttc cacgagacgc ttgactgctg tggctccagc acactgactg ctttgaccac 540

ctcagtgctc aagaacaatt tgtgtccctc gggcagcaac atcatcagca acctcttcaa 600

ggaggactgc caccagaaga tcgatgacct cttctccggg aagctgtacc tcatcggcat 660

tgctgccatc gtggtcgctg tgatcatgat cttcgagatg atcctgagca tggtgctgtg 720

ctgtggcatc cggaacagct ccgtgtactg aggccccgca gctctggcca cagggacctc 780

tgcagtgccc cctaagtgac ccggacactt ccgagggggc catcaccgcc tgtgtatata 840

acgtttccgg tattactctg ctacacgtag cctttttact tttggggttt tgtttttgtt 900

ctgaactttc ctgttacctt ttcagggctg acgtcacatg taggtggcgt gtatgagtgg 960

agacgggcct gggtcttggg gactggaggg caggggtcct tctgccctgg ggtcccaggg 1020

tgctctgcct gctcagccag gcctctcctg ggagccactc gcccagagac tcagcttggc 1080

caacttgggg ggctgtgtcc acccagcccg cccgtcctgt gggctgcaca gctcaccttg 1140

ttccctcctg ccccggttcg agagccgagt ctgtgggcac tctctgcctt catgcacctg 1200

tcctttctaa cacgtcgcct tcaactgtaa tcacaacatc ctgactccgt catttaataa 1260

agaaggaaca tcaggcatgc t 1281

<210> 83
<211> 2591
<212> DNA
<213> Homo sapiens

<220>
<223> II.83 (NM_000061)

<400> 83
ctcagactgt ccttcctctc tggactgtaa gaatatgtct ccagggccag tgtctgctgc 60

gatcgagtcc caccttccaa gtcctggcat ctcaatgcat ctgggaagct acctgcatta 120

agtcaggact gagcacacag gtgaactcca gaaagaagaa gctatggccg cagtgattct 180

ggagagcatc tttctgaagc gatcccaaca gaaaaagaaa acatcacctc taaacttcaa 240

gaagcgcctg tttctcttga ccgtgcacaa actctcctac tatgagtatg actttgaacg 300

tgggagaaga ggcagtaaga agggttcaat agatgttgag aagatcactt gtgttgaaac 360

agtggttcct gaaaaaaatc ctcctccaga aagacagatt ccgagaagag gtgaagagtc 420

cagtgaaatg gagcaaattt caatcattga aaggttccct tatcccttcc aggttgtata 480

tgatgaaggg cctctctacg tcttctcccc aactgaagaa ctaaggaagc ggtggattca 540

ccagctcaaa aacgtaatcc ggtacaacag tgatctggtt cagaaatatc acccttgctt 600

ctggatcgat gggcagtatc tctgctgctc tcagacagcc aaaaatgcta tgggctgcca 660

aattttggag aacaggaatg gaagcttaaa acctgggagt tctcaccgga agacaaaaaa 720

gcctcttccc ccaacgcctg aggaggacca gatcttgaaa aagccactac cgcctgagcc 780

agcagcagca ccagtctcca caagtgagct gaaaaaggtt gtggcccttt atgattacat 840

gccaatgaat gcaaatgatc tacagctgcg gaagggtgat gaatatttta tcttggagga 900

aagcaactta ccatggtgga gagcacgaga taaaaatggg caggaaggct acattcctag 960

taactatgtc actgaagcag aagactccat agaaatgtat gagtggtatt ccaaacacat 1020

gactcggagt caggctgagc aactgctaaa gcaagagggg aaagaaggag gtttcattgt 1080

cagagactcc agcaaagctg gcaaatatac agtgtctgtg tttgctaaat ccacagggga 1140

ccctcaaggg gtgatacgtc attatgttgt gtgttccaca cctcagagcc agtattacct 1200

ggctgagaag caccttttca gcaccatccc tgagctcatt aactaccatc agcacaactc 1260

tgcaggactc atatccaggc tcaaatatcc agtgtctcaa caaaacaaga atgcaccttc 1320

cactgcaggc ctgggatacg gatcatggga aattgatcca aaggacctga ccttcttgaa 1380

ggagctgggg actggacaat ttggggtagt gaagtatggg aaatggagag gccagtacga 1440

cgtggccatc aagatgatca aagaaggctc catgtctgaa gatgaattca ttgaagaagc 1500

caaagtcatg atgaatcttt cccatgagaa gctggtgcag ttgtatggcg tctgcaccaa 1560

gcagcgcccc atcttcatca tcactgagta catggccaat ggctgcctcc tgaactacct 1620

gagggagatg cgccaccgct tccagactca gcagctgcta gagatgtgca aggatgtctg 1680

tgaagccatg gaatacctgg agtcaaagca gttccttcac cgagacctgg cagctcgaaa 1740

ctgtttggta aacgatcaag gagttgttaa agtatctgat ttcggcctgt ccaggtatgt 1800

cctggatgat gaatacacaa gctcagtagg ctccaaattt ccagtccggt ggtccccacc 1860

ggaagtcctg atgtatagca agttcagcag caaatctgac atttgggctt ttggggtttt 1920

gatgtgggaa atttactccc tggggaagat gccatatgag agatttacta acagtgagac 1980

tgctgaacac attgcccaag gcctacgtct ctacaggcct catctggctt cagagaaggt 2040

atataccatc atgtacagtt gttggcatga gaaagcagat gagcgtccca ctttcaaaat 2100

tcttctgagc aatattctag atgtcatgga tgaagaatcc tgagctcgcc aataagcttc 2160

ttggttctac ttctcttctc cacaagcccc aatttcactt tctcagagga aatcccaagc 2220

ttaggagccc tggagccttt gtgctcccac tcaatacaaa aaggcccctc tctacatctg 2280

gggatgcacc tcttctttga ttccctggga tagtggcttc tgagcaaagg ccaaaaaatt 2340

attgtgcctg aaatttcccg agagaattaa gacagactga atttgcgatg aaaatatttt 2400

ttaggaggga ggatgtaaat agccgcacaa aggggtccaa cagctctttg agtaggcatt 2460

tggtagagct tgggggtgtg tgtgtggggg tggaccgaat ttggcaagaa tgaaatggtg 2520

tcataaagat gggaggggag ggtgttttga taaaataaat tctagaaagc ttaaaaaaaa 2580

aaaaaaaaaa a 2591

<210> 84
<211> 3321
<212> DNA
<213> Homo sapiens

<220>
<223> II.84 (XM_001472)

<400> 84
gacatcatgg gctattttta ggggttgact ggtagcagat aagtgttgag ctcgggctgg 60

ataagggctc agagttgcac tgagtgtggc tgaagcagcg aggcgggagt ggaggtgcgc 120

ggagtcaggc agacagacag acacagccag ccagccaggt cggcagtata gtccgaactg 180

caaatcttat tttcttttca ccttctctct aactgcccag agctagcgcc tgtggctccc 240

gggctggtgt ttcgggagtg tccagagagc ctggtctcca gccgcccccg ggaggagagc 300

cctgctgccc aggcgctgtt gacagcggcg gaaagcagcg gtacccacgc gcccgccggg 360

ggaagtcggc gagcggctgc agcagcaaag aactttcccg gctgggagga ccggagacaa 420

gtggcagagt cccggagcca acttttgcaa gcctttcctg cgtcttaggc ttctccacgg 480

cggtaaagac cagaaggcgg cggagagcca cgcaagagaa gaaggacgtg cgctcagctt 540

cgctcgcacc ggttgttgaa cttgggcgag cgcgagccgc ggctgccggg cgccccctcc 600

ccctagcagc ggaggagggg acaagtcgtc ggagtccggg cggccaagac ccgccgccgg 660

ccggccactg cagggtccgc actgatccgc tccgcgggga gagccgctgc tctgggaagt 720
gagttcgcct gcggactccg aggaaccgct gcgcacgaag agcgctcagt gagtgaccgc 780
gacttttcaa agccgggtag cgcgcgcgag tcgacaagta agagtgcggg aggcatctta 840
attaaccctg cgctccctgg agcgagctgg tgaggagggc gcagcgggga cgacagccag 900
cgggtgcgtg cgctcttaga gaaactttcc ctgtcaaagg ctccgggggg cgcgggtgtc 960
ccccgcttgc cacagccctg ttgcggcccc gaaacttgtg cgcgcagccc aaactaacct 1020
cacgtgaagt gacggactgt tctatgactg caaagatgga aacgaccttc tatgacgatg 1080
ccctcaacgc ctcgttcctc ccgtccgaga gcggacctta tggctacagt aaccccaaga 1140
tcctgaaaca gagcatgacc ctgaacctgg ccgacccagt ggggagcctg aagccgcacc 1200
tccgcgccaa gaactcggac ctcctcacct cgcccgacgt ggggctgctc aagctggcgt 1260
cgcccgagct ggagcgcctg ataatccagt ccagcaacgg gcacatcacc accacgccga 1320
cccccaccca gttcctgtgc cccaagaacg tgacagatga gcaggagggc ttcgccgagg 1380
gcttcgtgcg cgccctggcc gaactgcaca gccagaacac gctgcccagc gtcacgtcgg 1440
cggcgcagcc ggtcaacggg gcaggcatgg tggctcccgc ggtagcctcg gtggcagggg 1500
gcagcggcag cggcggcttc agcgccagcc tgcacagcga gccgccggtc tacgcaaacc 1560
tcagcaactt caacccaggc gcgctgagca gcggcggcgg ggcgccctcc tacggcgcgg 1620
ccggcctggc ctttcccgcg caaccccagc agcagcagca gccgccgcac cacctgcccc 1680
agcagatgcc cgtgcagcac ccgcggctgc aggccctgaa ggaggagcct cagacagtgc 1740
ccgagatgcc cggcgagaca ccgcccctgt cccccatcga catggagtcc caggagcgga 1800
tcaaggcgga gaggaagcgc atgaggaacc gcatcgctgc ctccaagtgc cgaaaaagga 1860
agctggagag aatcgcccgg ctggaggaaa aagtgaaaac cttgaaagct cagaactcgg 1920
agctggcgtc cacggccaac atgctcaggg aacaggtggc acagcttaaa cagaaagtca 1980
tgaaccacgt taacagtggg tgccaactca tgctaacgca gcagttgcaa acattttgaa 2040
gagagaccgt cgggggctga ggggcaacga agaaaaaaaa taacacagag agacagactt 2100
gagaacttga caagttgcga cggagagaaa aaagaagtgt ccgagaacta aagccaaggg 2160
tatccaagtt ggactgggtt gcgtcctgac ggcgccccca gtgtgcacga gtgggaagga 2220
cttggcgcgc cctcccttgg cgtggagcca gggagcggcc gcctgcgggc tgccccgctt 2280
tgcggacggg ctgtccccgc gcgaacggaa cgttggactt ttcgttaaca ttgaccaaga 2340
actgcatgga cctaacattc gatctcattc agtattaaag gggggagggg gagggggtta 2400
caaactgcaa tagagactgt agattgcttc tgtagtactc cttaagaaca caaagcgggg 2460
ggagggttgg ggaggggcgg caggagggag gtttgtgaga gcgaggctga gcctacagat 2520
gaactctttc tggcctgcct tcgttaactg tgtatgtaca tatatatatt ttttaatttg 2580
atgaaagctg attactgtca ataaacagct tcatgccttt gtaagttatt tcttgtttgt 2640
ttgtttgggt atcctgccca gtgttgtttg taaataagag atttggagca ctctgagttt 2700

accatttgta ataaagtata taattttttt atgttttgtt tctgaaaatt ccagaaagga 2760

tatttaagaa aatacaataa actattggaa agtactcccc taacctcttt tctgcatcat 2820

ctgtagatac tagctatcta ggtggagttg aaagagttaa gaatgtcgat taaaatcact 2880

ctcagtgctt cttactatta agcagtaaaa actgttctct attagacttt agaaataaat 2940

gtacctgatg tacctgatgc tatggtcagg ttatactcct cctcccccag ctatctatat 3000

ggaattgctt accaaaggat agtgcgatgt ttcaggaggc tggaggaagg ggggttgcag 3060

tggagaggga cagcccactg agaagtcaaa catttcaaag tttggattgt atcaagtggc 3120

atgtgctgtg accatttata atgttagtag aaattttaca ataggtgctt attctcaaag 3180

caggaattgg tggcagattt tacaaaagat gtatccttcc aatttggaat cttctctttg 3240

acaattccta gataaaaaga tggcctttgc ttatgaatat ttataacagc attcttgtca 3300

caataaatgt attcaaatac c 3321

<210> 85
<211> 4175
<212> DNA
<213> Homo sapiens

<220>
<223> II.85 (XM_007258)

<400> 85
ccagggtgat gctgaagatg atgaccttct tccaaggcct ctagagccat cagcctgtgc 60

caggcaccct cgacttgcct agaggccccc aaaagttgca gtccacatca gaggcagagt 120

cagaggcctc catgtcggag gcctcctctg aggacctggt gccacccctg gaggctgggg 180

cagccccata tagggaggag gaagaggcgg cgaagaagaa gaaggagaag aagaagaagt 240

ccaaaggcct ggccaatgtg ttctgcgtct tcaccaaagg gaagaagaag aagggtcagc 300

ccagctcagc ggagcccgag gacgcagccg ggtccaggca ggggctggat ggcccgcccc 360

ccacagtgga ggagctgaag gcggcgctgg agcgcgggca gctggaggcg gcgcggccgc 420

tgctggcgct ggagcgggag ctggcggcgg cggcggcggc gggcggtgtg agcgaggagg 480

agctggtgcg gcgccagagc aaggtggagg cgctgtacga gctgctgcgc gaccaggtgc 540

tgggcgtgct gcggcggccg ctggaggcgc cgcccgagcg gctgcgccag gcgctggccg 600

tggtggcgga gcaggagcgc gaggaccgcc aggcggcggc ggcggggccg gggacctcgg 660

ggctggcggc cacgcgcccg cggcgctggc tgcagctgtg gcggcgcggc gtggcggagg 720

cggccgagga gcgcatgggc cagcggccgg ccgcgggcgc cgaggtcccc gagagcgtct 780

ttctgcactt gggccgcacc atgaaggagg acctggaggc cgtggtggag cggctgaagc 840

cgctgttccc cgccgagttc ggcgtcgtgg cggcctacgc cgagagctac caccagcact 900

tcgcggccca cctggccgcc gtggcgcagt tcgagctgtg cgagcgcgac acctacatgc 960

tgctgctctg ggtgcagaac ctctacccca atgacatcat caacagcccc aagctggtgg 1020

gtgagctgca gggtatgggg ctcgggagcc tcctgccccc caggcagatc cgactgctgg 1080
aggccacatt cctgtccagt gaggcggcca atgtgaggga gttgatggac cgagctctgg 1140
agctagaggc acggcgctgg gctgaggatg tgcctcccca gaggctggac ggccactgcc 1200
acagcgagct ggccatcgac atcatccaga tcacctccca ggcccaggcc aaggccgaga 1260
gcatcacgct ggacttgggc tcacagataa agcgggtgct gctggtggag ctgcctgcgt 1320
tcctgaggag ctaccagcgc gcctttaatg aatttctgga gagaggcaag cagctgacga 1380
attacagggc caatgttatt gccaacatca acaactgcct gtccttccgg atgtccatgg 1440
agcagaattg gcaggtaccc caggacaccc tgagcctcct gctgggcccc ctgggtgagc 1500
tcaagagcca cggctttgac accctgctcc agaacctgca tgaggacctg aagccactgt 1560
tcaagaggtt cacgcacacc cgctgggcgg cccctgtgga gaccctggaa aacatcatcg 1620
ccactgtaga cacgaggctg cctgagttct cagagctgca gggctgtttc cgggaggagc 1680
tcatggaggc cttgcacctg cacctggtga aggagtacat catccaactc agcaaggggc 1740
gcctggtcct caagacggcc gagcagcagc agcagctggc tgggtacatc ctggccaatg 1800
ctgacaccat ccagcacttc tgcacccagc acggctcccc ggcgacctgg ctgcagcctg 1860
ctctccctac gctggccgag atcattcgcc tgcaggaccc cagtgccatc aagattgagg 1920
tggccactta tgccacctgc taccctgact tcagcaaagg ccacctgagc gctatcctgg 1980
ccatcaaggg gaacctatcc aacagtgagg tcaagcgcat ccggagcatc ttggacgtca 2040
gcatgggggc gcaggagccc tcccggcccc tattttccct tataaaggtt ggttagcttt 2100
tcctgtggcc tgacctgcct gtgagtgccc agcaagcctt gggcacaccc cgctgggagc 2160
tgttaagagc agcgctggtt ctcggttcct cccgggtctc ctgtgctctg atgctacttc 2220
tgcctagccc tggcggaggt gcaggccctg tcagctggaa ctggacagac cttggtttgt 2280
ttacatgtcc gatgggggca ggagctccca tcctgggcag ccaaccaggc aacaccaagg 2340
actctttgta aacgatagct gatcgtgtgc acgcaaggaa agaaccagga gggagagtgc 2400
agccaggctc agggatcccc ggacacctct gtccagagcc cctccacagt cggcctcatg 2460
actgtcctcc tcgtgggtgg ggccgagggc cctcttcagc tctctggaga caggggccga 2520
gcctcaccca tctgccctct gcagcccagg gccgccgtga gcgggattca gcaatggtgg 2580
aatggaagac agaactggaa gagaaagaag gaaaagatga gctctcgtct ggcaggggct 2640
tttagggtcc tgtggcgagc tgtgagcacc gccagcatta gacgtcacat ccaggtggcc 2700
ccacggcccc tacaggctgg ccctgcaatg gggccctgag ccctccctct tcatccccca 2760
aggcctcaac tagagggtgg tcccccgagg gcttggtgtc tactaccgaa gggcccaaga 2820
cctcctgggt cctctcaggc tcccccttcc ccaaggcagg gacaggccct gggggtgcca 2880
ccgtgggccc tgccacccag aagtctggct gaggtctggg caggggcagg gcaagcttga 2940
cctctcactg ttgacccttt ggcctctgta tttgtttcct attgccgtga caggtttcca 3000
caaacttcgt ggatcaaaac gaggtcttcc agttctgcgg gtcagaaggc tgacccgggg 3060

ctcaaatctg ggtgtcggca gtcctgcact ccttctggag gctctagggg agaattcatt 3120

tctggccttt tcatttttag aggctgaccg taattcttga cttcaggctc ctccatcttc 3180

agagccagct gtgggtagtt gaatcttttt cccgtcacct cattgaggcc tcccctctcc 3240

tgcctccctc caccactttt tttttttttt ttttgagaca gggtcttgct gtgttgccca 3300

ggctggagtg cagtggcctg gtcatggcat caaggctcac tgcagcctgg acctcctggt 3360

tcaagtgatc ctcttgtctc agtcccctga gacaatcccc cacgcccagc tacatatttt 3420

ttgtggatac agggtctcat tctgttgcct aggcttgtct ggaactcctg ggctcaaggg 3480

atcttgtagc cttagcctcc taaagtgctg ggattatagg catgagtcac tgtacccggc 3540

ctgctctacc gcttttaagg acgcttatga tcacattgcg cctacccaga gaacccaggt 3600

cgtctttcta ttttcaggtc agctgattag ccaccttagt tccatctgca actttagttc 3660

ccactggctg tgtaacctaa catagtcaca ggctctgggg actgtcacgt ggacatcttt 3720

gggaggccgt tattctgccc accgcaccct ccgttcatcc cctgccctgc cgggcacctc 3780

gctctacccc aggaaaatgt gagctcgttt tcctgctcgg catgtgctcc ccctaaggct 3840

ctgctcctcc ctgggcctga aagttccttc tcagcctgag agggggccct tcggactcag 3900

gcatgactca gcccggctga tgcctctgca gtgctgagtc aggatttggg gccggctctc 3960

ttgggtccgt ccccttttcc caggtactgc cttacaaagc tgtggccagg aagtggccgg 4020

tataaaggat gcccaaggtc tttgtacgtg tgtaggagtt agcgtgtttg atattgttaa 4080

tataataata attatttttt agagtactgc ttttgtatgt atgttgaaca ggatccaggt 4140

ttttatagct tgatataaaa cagaattcaa aagtg 4175

<210> 86
<211> 1389
<212> DNA
<213> Homo sapiens

<220>
<223> II.86 (XM_048555)

<400> 86
gcccccgcgc cggcggcggc gcggcgcccg cgcttccgta tcgctcctcg taggccgggg 60

ctcggcgcgc gcacccgcac taaagacgct tcttcccggc gggtaggaat cccgccggcg 120

agccgaacag ttccccgagc gcagcccgcg gaccaccacc cggccgcacg ggccgctttt 180

gtcccccgcc cgccgcttct gtccgagagg ccgcccgcga ggcgcatcct gaccgcgagc 240

gtcgggtccc agagccgggc gcggctgggg cccgaggcta gcatctctcg ggagccgcaa 300

ggcgagagct gcaaagatgt aaaagtcaag agaagactct aaaaatagca aagatgcttt 360

tgagccagaa tgccttcatc ttcagatcac ttaatttggt tctcatggtg tatatcagcc 420

tcgtgtttgg tatttcatat gattcgcctg attacacaga tgaatcttgc actttcaaga 480

tatcattgcg aaatttccgg tccatcttat catgggaatt aaaaaaccac tccattgtac 540

caactcacta tacattgctg tatacaatca tgagtaaacc agaagatttg aaggtggtta 600

agaactgtgc aaataccaca agatcatttt gtgacctcac agatgagtgg agaagcacac 660

acgaggccta tgtcaccgtc ctagaaggat tcagcgggaa cacaacgttg ttcagttgct 720

cacacaattt ctggctggcc atagacatgt cttttgaacc accagagttt gagattgttg 780

gttttaccaa ccacattaat gtgatggtga aatttccatc tattgttgag gaagaattac 840

agtttgattt atctctcgtc attgaagaac agtcagaggg aattgttaag aagcataaac 900

ccgaaataaa aggaaacatg agtggaaatt tcacctatat cattgacaag ttaattccaa 960

acacgaacta ctgtgtatct gtttatttag agcacagtga tgagcaagca gtaataaagt 1020

ctcccttaaa atgcaccctc cttccacctg gccaggaatc agaatcagca gaatctgcca 1080

aaataggagg aataattact gtgtttttga tagcattggt cttgacaagc accatagtga 1140

cactgaaatg gattggttat atatgcttaa gaaatagcct ccccaaagtc ttgaggcaag 1200

gtctcgctaa gggctggaat gcagtggcta ttcacaggtg cagtcataat gcactacagt 1260

ctgaaactcc tgagctcaaa cagtcgtcct gcctaagctt ccccagtagc tgggattaca 1320

agcgtgcatc cctgtgcccc agtgattaag ttttattatg tagaaaataa agagcaaaca 1380

gtacagctg 1389

<210> 87
<211> 2084
<212> DNA
<213> Homo sapiens

<220>
<223> II.87 (XM_041060)

<400> 87
aaccgcatct gcagcgagca tctgagaagc caagactgag ccggcggccg cggcgcagcg 60

aacgagcagt gaccgtgctc ctacccagct ctgctccaca gcgcccacct gtctccgccc 120

ctcggcccct cgcccggctt tgcctaaccg ccacgatgat gttctcgggc ttcaacgcag 180

actacgaggc gtcatcctcc cgctgcagca gcgcgtcccc ggccggggat agcctctctt 240

actaccactc acccgcagac tccttctcca gcatgggctc gcctgtcaac gcgcaggact 300

tctgcacgga cctggccgtc tccagtgcca acttcattcc cacggtcact gccatctcga 360

ccagtccgga cctgcagtgg ctggtgcagc ccgccctcgt ctcctccgtg gccccatcgc 420

agaccagagc ccctcaccct ttcggagtcc ccgccccctc cgctggggct tactccaggg 480

ctggcgttgt gaagaccatg acaggaggcc gagcgcagag cattggcagg aggggcaagg 540

tggaacagtt atctccagaa gaagaagaga aaaggagaat ccgaagggaa aggaataaga 600

tggctgcagc caaatgccgc aaccggagga gggagctgac tgatacactc caagcggaga 660

cagaccaact agaagatgag aagtctgctt tgcagaccga gattgccaac ctgctgaagg 720

agaaggaaaa actagagttc atcctggcag ctcaccgacc tgcctgcaag atccctgatg 780

acctgggctt cccagaagag atgtctgtgg cttcccttga tctgactggg ggcctgccag 840

aggttgccac cccggagtct gaggaggcct tcaccctgcc tctcctcaat gaccctgagc 900

ccaagccctc agtggaacct gtcaagagca tcagcagcat ggagctgaag accgagccct 960

ttgatgactt cctgttccca gcatcatcca ggcccagtgg ctctgagaca gcccgctccg 1020

tgccagacat ggacctatct gggtccttct atgcagcaga ctgggagcct ctgcacagtg 1080

gctccctggg gatggggccc atggccacag agctggagcc cctgtgcact ccggtggtca 1140

cctgtactcc cagctgcact gcttacacgt cttccttcgt cttcacctac cccgaggctg 1200

actccttccc cagctgtgca gctgcccacc gcaagggcag cagcagcaat gagccttcct 1260

ctgactcgct cagctcaccc acgctgctgg ccctgtgagg gggcagggaa ggggaggcag 1320

ccggcaccca caagtgccac tgcccgagct ggtgcattac agagaggaga aacacatctt 1380

ccctagaggg ttcctgtaga cctagggagg accttatctg tgcgtgaaac acaccaggct 1440

gtgggcctca aggacttgaa agcatccatg tgtggactca agtccttacc tcttccggag 1500

atgtagcaaa acgcatggag tgtgtattgt tcccagtgac acttcagaga gctggtagtt 1560

agtagcatgt tgagccaggc ctgggtctgt gtctcttttc tctttctcct tagtcttctc 1620

atagcattaa ctaatctatt gggttcatta ttggaattaa cctggtgctg gatattttca 1680

aattgtatct agtgcagctg attttaacaa taactactgt gttcctggca atagtgtgtt 1740

ctgattagaa atgaccaata ttatactaag aaaagatacg actttatttt ctggtagata 1800

gaaataaata gctatatcca tgtactgtag tttttcttca acatcaatgt tcattgtaat 1860

gttactgatc atgcattgtt gaggtggtct gaatgttctg acattaacag ttttccatga 1920

aaacgtttta ttgtgttttt aatttattta ttaagatgga ttctcagata tttatatttt 1980

tattttattt ttttctacct tgaggtcttt tgacatgtgg aaagtgaatt tgaatgaaaa 2040

atttaagcat tgtttgctta ttgttccaag acattgtcaa taaa 2084

<210> 88
<211> 858
<212> DNA
<213> Homo sapiens

<220>
<223> II.88 (XM_056556)

<400> 88
gccccgggag ggggctgcag tttccttcct tccttctcgg cagcgctccg cgcccccatc 60

gcccctcctg cgctagcgga ggtgatcgcc gcggcgatgc cggaggaggg ttcgggctgc 120

tcggtgcggc gcaggcccta tgggtgcgtc ctgcgggctg ctttggtccc attggtcgcg 180

ggcttggtga tctgcctcgt ggtgtgcatc cagcgcttcg cacaggctca gcagcagctg 240

ccgctcgagt cacttgggtg ggacgtagct gagctgcagc tgaatcacac aggacctcag 300

caggacccca ggctatactg gcaggggggc ccagcactgg gccgctcctt cctgcatgga 360

ccagagctgg acaaggggca gctacgtatc catcgtgatg gcatctacat ggtacacatc 420

caggtgacgc tggccatctg ctcctccacg acggcctcca ggcaccaccc caccaccctg 480

gccgtgggaa tctgctctcc cgcctcccgt agcatcagcc tgctgcgtct cagcttccac 540

caaggttgta ccattgcctc ccagcgcctg acgcccctgg cccgagggga cacactctgc 600

accaacctca ctgggacact tttgccttcc cgaaacactg atgagacctt ctttggagtg 660

cagtgggtgc gcccctgacc actgctgctg attagggttt tttaaatttt attttatttt 720

atttaagttc aagagaaaaa gtgtacacac aggggccacc cggggttggg gtgggagtgt 780

ggtggggcgt agtggtggca ggacaagaga aggcattgag ctttttcttt cattttccta 840

ttaaaaaata caaaaatc 858

<210> 89
<211> 5061
<212> DNA
<213> Homo sapiens

<220>
<223> II.89 (XM_016747)

<400> 89
gtgggctctc tcttttcccc tcttgctcct ttcttttctt tttttctgtt tttttaaacc 60

ttccaaggca agttcatgga tactaagctg atgtgtttgt tgttcttttt ctccctgcct 120

ccgctcctag tgagtaacca cactggccgc atcaaggtgg tctttactcc gagcatctgt 180

aaagtgacct gcaccaaggg cagctgtcag aacagctgtg agaaggggaa caccaccact 240

ctcattagtg agaatggtca tgctgccgac accctgacgg ccacgaactt ccgagtggta 300

atttgccatc ttccatgtat gaatggtggc cagtgcagtt caagggacaa atgtcagtgc 360

cctccaaatt tcacaggaaa actttgtcag atcccagtcc atggtgccag cgtgcctaaa 420

ctttatcagc attcccagca gccaggcaag gcgttgggga cgcatgtcat ccattcaaca 480

cataccttgc ctctgaccgt gactagccag caaggagtca aagtgaaatt tcctcctaac 540

atagtcaata tccatgtgaa acatcctcct gaagcttccg tccagataca tcaggtttca 600

agaattgatg gcccaacagg ccagaagaca aaagaagctc aaccaggcca atcccaagtc 660

tcgtaccaag ggcttcctgt ccagaagacc cagaccatac attccacata ctcccaccag 720

caggtcattc ctcacgtcta ccccgtggct gctaagacac agcttggccg gtgcttccag 780

gaaaccattg ggtcacagtg tggcaaagcg ctccctggcc tttcaaagca agaggactgc 840

tgtggaactg tgggtacctc ctggggcttt aacaaatgcc agaaatgccc caagaaacca 900

tcttatcatg gatacaacca aatgatggaa tgcctaccgg gttataagcg ggttaacaac 960

accttttgcc aagatattaa tgaatgtcag ctacaaggtg tatgccctaa tggtgagtgt 1020

ttgaatacca tgggcagcta tcgatgtacc tgcaaaatag gatttggccc ggatcctacc 1080

ttttcaagtt gtgttcctga tccccctgtg atctcggaag agaaagggcc ctgttaccga 1140

cttgtcagtt ctggaagaca gtgtatgcac cctctgtctg ttcacctcac caagcagctc 1200

tgctgttgta gtgtgggcaa ggcctggggc ccacactgtg agaaatgtcc ccttccaggc 1260

acagctgctt ttaaggaaat ctgtcctggt ggaatgggtt atacggtttc tggcgttcat 1320
agacgcaggc caatccatca ccatgtaggt aaaggacctg tatttgtcaa gccaaagaac 1380
actcaacctg ttgctaaaag tactcatcct ccacctctcc cagccaagga agagccagtg 1440
gaggccctga ccttctcccg ggaacacggg ccaggagtgg cggagccaga agtggcaact 1500
gcaccccctg aaaaggaaat accttcattg gatcaagaga aaaccaaact tgagcctggt 1560
caaccccagc tgtctccagg catttccact attcatctgc atccacagtt tccagtagtg 1620
attgaaaaaa catcacctcc tgtgcctgtt gaagtagctc ctgaagcttc tacgtctagt 1680
gccagccaag tgattgctcc tactcaagtg acagaaatca atgaatgtac tgtgaaccct 1740
gatatctgtg gagcaggaca ctgcattaac ctaccagtga gatatacctg tatatgctac 1800
gagggctaca ggttcagtga acaacagagg aaatgtgtgg atattgatga gtgtactcag 1860
gtccaacacc tctgctccca gggccgctgt gaaaacaccg agggaagttt cttgtgcatt 1920
tgcccagcag gatttatggc cagtgaggag ggtactaact gcatagatgt tgacgaatgc 1980
ctgaggccgg acgtctgtgg ggaggggcac tgtgtcaata ctgtgggggc cttccggtgt 2040
gaatactgtg acagcgggta ccgcatgact cagagaggcc gttgtgagga tattgatgaa 2100
tgtttgaatc caagcacttg tccagatgag cagtgtgtga attctcctgg atcttaccag 2160
tgcgttccct gcacagaagg attccgaggc tggaatggac agtgccttga tgtggacgag 2220
tgcctggaac caaacgtctg cgcaaatggt gattgttcca accttgaagg ctcctacatg 2280
tgttcatgcc acaaaggcta tacccggact ccggaccaca agcactgtag agatattgat 2340
gaatgtcagc aagggaatct atgtgtaaac gggcagtgca aaaataccga gggctccttc 2400
aggtgcacct gtggacaggg gtaccagctg tcggcagcta aagaccagtg tgaagacatt 2460
gatgaatgcc agcaccgtca tctctgtgct catgggcagt gcaggaacac tgagggctct 2520
tttcaatgtg tgtgtgacca gggttacaga gcatctgggc ttggagacca ctgtgaagat 2580
atcaatgaat gcttggagga caagagtgtt tgccagagag gagactgcat taatactgca 2640
gggtcctatg attgtacttg tccggatgga tttcagctag atgacaataa aacatgtcaa 2700
gatattaatg aatgtgaaca tccagggctc tgtggtccgc aaggggagtg cctaaacaca 2760
gagggttctt tccattgtgt ctgccagcag ggtttctcaa tctctgcaga tggccgtacg 2820
tgtgaagata ttgatgaatg tgtaaacaac actgtttgtg acagtcacgg gttttgtgac 2880
aatacagctg gctccttccg ctgcctctgt tatcagggct ttcaagcccc acaggatggg 2940
caagggtgtg tggatgtgaa tgaatgtgaa ctgctcagtg gggtgtgtgg tgaagccttc 3000
tgtgaaaacg tggaagggtc cttcctgtgc gtgtgtgctg atgaaaacca agagtacagc 3060
cccatgactg ggcagtgccg ctcccggacc tccacagatt tagatgtaga tgtagatcaa 3120
cccaaagaag aaaagaaaga atgctactat aatctcaatg acgccagtct ctgtgataat 3180
gtgttggccc ccaatgtcac gaaacaagaa tgctgctgta catcaggcgt gggatgggga 3240
gataactgcg aaatcttccc ctgcccggtc ttgggaactg ctgagttcac tgaaatgtgt 3300

cccaaaggga aaggttttgt gcctgctgga gaatcatctt ctgaagctgg tggtgagaac 3360
tataaagatg cagatgaatg cctacttttt ggacaagaaa tctgcaaaaa tggtttctgt 3420
ttgaacactc ggcctgggta tgaatgctac tgtaagcaag ggacgtacta tgatcctgtg 3480
aaactgcagt gctttgatat ggatgaatgt caagacccca gtagttgtat tgatggccag 3540
tgtgttaata cagagggctc ttacaactgc ttctgtactc accccatggt cctggatgcg 3600
tcagaaaaaa gatgtatacg accggctgag tcaaacgaac aaatagaaga aactgatgtc 3660
taccaagatt tgtgctggga acatctgagt gatgaatacg tgtgtagccg gcctcttgtg 3720
ggcaagcaga caacgtacac tgagtgctgc tgtctgtatg gagaggcctg gggcatgcag 3780
tgtgccctct gccccctgaa ggattcagat gactatgctc agctgtgtaa catccccgtg 3840
acgggacgcc ggcagccata tggacgggac gccttggttg acttcagtga acagtatact 3900
ccagaagccg atccctactt catccaagac cgttttctaa atagctttga ggagttacag 3960
gctgaggaat gcggcatcct caatggatgt gaaaatggtc gctgtgtgag ggtccaggaa 4020
ggttacacct gcgattgctt tgatgggtat cacttggata cggccaagat gacctgtgtc 4080
gatgtaaatg aatgcgatga gttgaacaac cggatgtctc tctgcaagaa tgccaagtgc 4140
attaacaccg atggttccta caagtgtttg tgtctgccag gctacgtgcc ttctgacaag 4200
ccaaactact gcactccgtt gaataccgcc ttgaatttag agaaagacag tgacctggag 4260
tgaaacagaa tctacataac ctaagcccat atactctgca ctgtgtaaag gaaaagggag 4320
aaatgtatta tacttgagac attgcaccta ccccggaagg ctggaaatac agaaacagca 4380
tggaattgca agtcctctga agacaatgag aggatttagg atgagcccga taggtgtggc 4440
agaccaaatg gacatttctc taaaaaacca gtatatatag tctgttcata tgtaaaattc 4500
aatggaagag aggtggaaca gtgctgttat tttaaacaga aggttgtatt attatgttgt 4560
tttgtttttt tactattgct tgattaaatt tggcatttaa atagtggtgg aaatatttta 4620
tataattttc attttttggt tgtgcagttc cttggctact gtttttcttt tacttcagtt 4680
ttttaaaaat ctcaaatgaa aaagtcttcg atacaatatt gttaagctgt attataagta 4740
ttgttacaca gggttatgca attcccggcc tggagcattt ttgaaattca aattgtctgt 4800
cctgtggagc aggcagtgat tttgttccaa aactttgtat acacatttgg agaaaagtac 4860
tttatatttt cagtgttttg tctgatttta atgtccgttc ttagccaagc tgctagcagg 4920
tgttaattgg atccctttcc ttcactgaaa tggaagagtt tataagctta cgttagtatt 4980
gtaatatgta aagtaagccc aacaaaaatt tttaaaaatt tgatgatccc caatatatct 5040
accattgtat gttaaataaa t 5061

<210> 90
<211> 1204
<212> DNA
<213> Homo sapiens

<220>

<223>  II.90 (XM_006953)

<400>  90
ggggtgcaaa gaagagacag cagcgcccag cttggaggtg ctaactccag aggccagcat      60

cagcaactgg gcacagaaag gagccgcctg ggcagggacc atggcacggc cacatccctg     120

gtggctgtgc gttctgggga ccctggtggg gctctcagct actccagccc ccaagagctg     180

cccagagagg cactactggg ctcagggaaa gctgtgctgc cagatgtgtg agccaggaac     240

attcctcgtg aaggactgtg accagcatag aaaggctgct cagtgtgatc cttgcatacc     300

gggggtctcc ttctctcctg accaccacac ccggccccac tgtgagagct gtcggcactg     360

taactctggt cttctcgttc gcaactgcac catcactgcc aatgctgagt gtgcctgtcg     420

caatggctgg cagtgcaggg acaaggagtg caccgagtgt gatcctcttc caaacccttc     480

gctgaccgct cggtcgtctc aggccctgag cccacaccct cagcccaccc acttacctta     540

tgtcagtgag atgctggagg ccaggacagc tgggcacatg cagactctgg ctgacttcag     600

gcagctgcct gcccggactc tctctaccca ctggccaccc caaagatccc tgtgcagctc     660

cgattttatt cgcatccttg tgatcttctc tggaatgttc cttgttttca ccctggccgg     720

ggccctgttc ctccatcaac gaaggaaata tagatcaaac aaaggagaaa gtcctgtgga     780

gcctgcagag ccttgtcatt acagctgccc cagggaggag gagggcagca ccatccccat     840

ccaggaggat taccgaaaac cggagcctgc ctgctccccc tgagccagca cctgcgggag     900

ctgcactaca gccctggcct ccacccccac cccgccgacc atccaaggga gagtgagacc     960

tggcagccac aactgcagtc ccatcctctt gtcagggccc tttcctgtgt acacgtgaca    1020

gagtgccttt tcgagactgg cagggacgag gacaaatatg gatgaggtgg agagtgggaa    1080

gcaggagccc agccagctgc gcctgcgctg caggagggcg ggggctctgg ttgtaaaaca    1140

cacttcctgc tgcgaaagac ccacatgcta caagacgggc aaaataaagt gacagatgac    1200

cacc                                                                 1204

<210>  91
<211>  1833
<212>  DNA
<213>  Homo sapiens

<220>
<223>  II.91 (NM_015927)

<400>  91
ggcacgaggg gagaataaaa taatcaggag gctgggtttg tgcttggctc caagcatgcc      60

ctgctctctg acctggagac taccacctcg cacatgccaa ggtcaggggc tcccaaagag     120

cgccctgcgg agcctctcac ccctccccca tcctatggcc accagccaca gacagggtct     180

ggggagtctt caggagcctc gggggacaag gaccacctgt acagcacggt atgcaagcct     240

cggtccccaa agcctgcagc ccggcggccc cctccattct cctcttccag cggtgtcttg     300

ggtaccgggc tctgtgagct agatcggttg cttcaggaac ttaatgccac tcagttcaac     360

atcacagatg aaatcatgtc tcagttccca tctagcaagg tggcttcagg agagcagaag 420

gaggaccagt ctgaagataa gaaaagaccc agcctccctt ccagcccgtc tcctggcctc 480

ccaaaggctt ctgccacctc agccactctg gagctggata gactgatggc ctcactctct 540

gacttccgcg ttcaaaacca tcttccagcc tctgggccaa ctcagccacc ggtggtgagc 600

tccacaaatg agggctcccc atccccacca gagccgactg gcaagggcag cctagacacc 660

atgctggggc tgctgcagtc cgacctcagc cgccggggtg ttcccaccca ggccaaaggc 720

ctctgtggct cctgcaataa acctattgct gggcaagtgg tgacggctct gggccgcgcc 780

tggcaccccg agcacttcgt ttgcggaggc tgttccaccg ccctgggagg cagcagcttc 840

ttcgagaagg atggagcccc cttctgcccc gagtgctact ttgagcgctt ctcgccaaga 900

tgtggcttct gcaaccagcc catccgacac aagatggtga ccgccttggg cactcactgg 960

cacccagagc atttctgctg cgtcagttgc ggggagccct tcggagatga gggtttccac 1020

gagcgcgagg gccgccccta ctgccgccgg gacttcctgc agctgttcgc cccgcgctgc 1080

cagggctgcc agggccccat cctggataac tacatctcgg cgctcagcgc gctctggcac 1140

ccggactgtt tcgtctgcag ggaatgcttc gcgcccttct cgggaggcag ctttttcgag 1200

cacgagggcc gcccgttgtg cgagaaccac ttccacgcac gacgcggctc gctgtgcgcc 1260

acgtgtggcc tccctgtgac cggccgctgc gtgtcggccc tgggtcgccg cttccacccg 1320

gaccacttca catgcacctt ctgcctgcgc ccgctcacca aggggtcctt ccaggagcgc 1380

gccggcaagc cctactgcca gccctgcttc ctgaagctct tcggctgaca gcccgctcgg 1440

ctcgccctct cccccggagg ccgcgccctc ccggaaaagc cgggtcctcc agaccccgag 1500

gccttgctct cagagcggga ggccccaccc actggagagc cccgccccta aggtactatg 1560

agttctcagg ggtcaagttc agaaacggcc cagccagacc taaacccaca cgcccacaaa 1620

gtggattgca cacagacaag aactcccgtg cgggcctcca ctctattccc acccttgagg 1680

gagccccctt actgggggag ggtccttgca attccagcga atcggaggcc aggccaggac 1740

gtccttgctc cctgcaccct cactgttctg tgcacttttt ctacctacat aaacacacgc 1800

attccacctc taaaaaaaaa aaaaaaaaaa aaa 1833

<210> 92
<211> 2687
<212> DNA
<213> Homo sapiens

<220>
<223> II.92 (XM_010807)

<400> 92
   ggctggggcc tgaggcctgg ggctcaccca cgcccccgcc gacgcctgcc gcgccgccgc 60
   cacccccgcc acccggatcc ccgggtggct cgcaggacac ctgtacgtcg tgcggcggct 120
   tccggcggcc agaggagctc ggccgagtgg acggcgactt cctggaggcg gtgaagcggc 180
   acatcttgag ccgcctgcag atgcggggcc ggcccaacat cacgcacgcc gtgcctaagg 240
   ccgccatggt cacggccctg cgcaagctgc acgcgggcaa ggtgcgcgag gacggccgcg 300
   tggagatccc gcacctcgac ggccacgcca gcccgggcgc cgacggccag gagcgcgttt 360
   ccgaaatcat cagcttcgcc gagacagatg gcctcgcctc ctcccgggtc cgcctatact 420
   tcttcatctc caacgaaggc aaccagaacc tgtttgtggt ccaggccagc ctgtggcttt 480
   acctgaaact cctgccctac gtcctggaga agggcagccg gcggaaggtg cgggtcaaag 540
   tgtacttcca ggagcagggc cacggtgaca ggtggaacat ggtggagaag agggtggacc 600
   tcaagcgcag cggctggcat accttcccac tcacggaggc catccaggcc ttgtttgagc 660
   ggggcgagcg gcgactcaac ctagacgtgc agtgtgacag ctgccaggag ctggccgtgg 720
   tgccggtgtt cgtggaccca ggcgaagagt cgcaccggcc ctttgtggtg gtgcaggctc 780
   ggctgggcga cagcaggcac cgcattcgca agcgaggcct ggagtgcgat ggccggacca 840
   acctctgttg caggcaacag ttcttcattg acttccgcct catcggctgg aacgactgga 900
   tcatagcacc caccggctac tacgggaact actgtgaggg cagctgccca gcctacctgg 960
   caggggtccc cggctctgcc tcctccttcc acacggctgt ggtgaaccag taccgcatgc 1020
   ggggtctgaa ccccggcacg gtgaactcct gctgcattcc caccaagctg agcaccatgt 1080
   ccatgctgta cttcgatgat gagtacaaca tcgtcaagcg ggacgtgccc aacatgattg 1140
   tggaggagtg cggctgcgcc tgacagtgca aggcaggggc acggtggtgg ggcacggagg 1200
   gcagtcccgg gtgggcttct tccagccccc gcgggaacgg gggtacacgg tgggctgagt 1260
   acagtcattc tgttgggctg tggagatagt gccagggtgc ggcctgagat atttttctac 1320
   agcttcatag agcaaccagt caaaaccaga gcgagaaccc tcaactgaca tgaaatactt 1380
   taaaatgcac acgtagccac gcacagccag acgcatcctg ccacccacac agcagcctcc 1440
   aggataccag caaatggatg cggtgacaaa tggcagctta gctacaaatg cctgtcagtc 1500
   ggagagaatg gggtgagcag ccaccattcc caccagctgg cccggccact ctgaattgcg 1560
   ccttccgagc acacataaaa gcacaaagac agagacgcag agagagagag agagccacgg 1620
   agaggaaaag cagatgcagg ggtggggagc gcagctcggc ggaggctgcg tgtgccccgt 1680
   ggcttttacc aggcctgctc tgcctggctc gatgtctgct tcttccccag cctgggatcc 1740
   ttcgtgcttc aaggcctggg gagcctgtcc ttccatgccc ttgtcgaggg aaagagaccc 1800
   agaaaggaca caacccgtca gagacctggg agcaggggca atgaccgttt gactgtttgt 1860
   ggcttgggcc tctgacatga cttatgtgtg tgtgtgtttt tggggtgggg agggagggag 1920
   agaagagggg gctaaatttg atgctttaac tgatctccaa cagttgacag gtcatccttg 1980
   ccagttgtat aactgaaaaa ggacttttct accaggtatg accttttaag tgaaaatctg 2040
   aattgttcta aatggaaaga aaaaaagttg caatctgtgc ccttcattgg ggacattcct 2100
   ctaggactgg tttggggacg ggtgggaatg acccctaggc aaggggatga gaccgcagga 2160
   ggaaatggcg gggaggaggc attcttgaac tgctgaggat ggggggtgtc ccctcagcgg 2220
   aggccaaggg aggggagcag cctagttggt cttggagaga tggggaaggc tttcagctga 2280
   tttgcagaag ttgcccatgt gggccccagc catcagggct ggccgtggac gtggcccctg 2340
   cccactcacc tgcccgcctg cccgcccgcc cgcatagcac ttgcagacct gcctgaacgc 2400
   acatgacata gcacttgccg atctgcgtgt gtccagaagt ggcccttggc cgagcgccga 2460
   actcgctcgc cctctagatg tccaagtgcc acgtgaacta tgcaatttaa agggttgacc 2520
   cacactagac gaaactggac tcgtacgact ctttttatat tttttatact tgaaatgaaa 2580
   tcctttgctt cttttttaag cgaatgattg cttttaatgt ttgcactgat ttagttgcat 2640
   gattagtcag aaactgccat ttgaaaaaaa gttattttta tagcagc 2687

<210> 93
<211> 3242
<212> DNA
<213> Homo sapiens

<220>
<223> II.93 (NM_002184)

<400> 93
gagcagccaa aaggcccgcg gagtcgcgct gggccgcccc ggcgcagctg aaccgggggc 60

cgcgcctgcc aggccgacgg gtctggccca gcctggcgcc aaggggttcg tgcgctgtgg 120

agacgcggag ggtcgaggcg gcgcggcctg agtgaaaccc aatggaaaaa gcatgacatt 180

tagaagtaga agacttagct tcaaatccct actccttcac ttactaattt tgtgatttgg 240

aaatatccgc gcaagatgtt gacgttgcag acttggctag tgcaagcctt gtttattttc 300

ctcaccactg aatctacagg tgaacttcta gatccatgtg gttatatcag tcctgaatct 360

ccagttgtac aacttcattc taatttcact gcagtttgtg tgctaaagga aaaatgtatg 420

gattattttc atgtaaatgc taattacatt gtctggaaaa caaaccattt tactattcct 480

aaggagcaat atactatcat aaacagaaca gcatccagtg tcacctttac agatatagct 540

tcattaaata ttcagctcac ttgcaacatt cttacattcg gacagcttga acagaatgtt 600

tatggaatca caataatttc aggcttgcct ccagaaaaac ctaaaaattt gagttgcatt 660

gtgaacgagg ggaagaaaat gaggtgtgag tgggatggtg gaagggaaac acacttggag 720

acaaacttca ctttaaaatc tgaatgggca acacacaagt ttgctgattg caaagcaaaa 780

cgtgacaccc ccacctcatg cactgttgat tattctactg tgtattttgt caacattgaa 840

gtctgggtag aagcagagaa tgcccttggg aaggttacat cagatcatat caattttgat 900

cctgtatata aagtgaagcc caatccgcca cataatttat cagtgatcaa ctcagaggaa 960

ctgtctagta tcttaaaatt gacatggacc aacccaagta ttaagagtgt tataatacta 1020

aaatataaca ttcaatatag gaccaaagat gcctcaactt ggagccagat tcctcctgaa 1080

gacacagcat ccacccgatc ttcattcact gtccaagacc ttaaaccttt tacagaatat 1140

gtgtttagga ttcgctgtat gaaggaagat ggtaagggat actggagtga ctggagtgaa 1200

gaagcaagtg ggatcaccta tgaagataga ccatctaaag caccaagttt ctggtataaa 1260

atagatccat cccatactca aggctacaga actgtacaac tcgtgtggaa gacattgcct 1320
ccttttgaag ccaatggaaa aatcttggat tatgaagtga ctctcacaag atggaaatca 1380
catttacaaa attacacagt taatgccaca aaactgacag taaatctcac aaatgatcgc 1440
tatctagcaa ccctaacagt aagaaatctt gttggcaaat cagatgcagc tgttttaact 1500
atccctgcct gtgactttca agctactcac cctgtaatgg atcttaaagc attccccaaa 1560
gataacatgc tttgggtgga atggactact ccaaggaaat ctgtaaagaa atatatactt 1620
gagtggtgtg tgttatcaga taaagcaccc tgtatcacag actggcaaca agaagatggt 1680
accgtgcatc gcacctattt aagagggaac ttagcagaga gcaaatgcta tttgataaca 1740
gttactccag tatatgctga tggaccagga agccctgaat ccataaaggc ataccttaaa 1800
caagctccac cttccaaagg acctactgtt cggacaaaaa aagtagggaa aaacgaagct 1860
gtcttagagt gggaccaact tcctgttgat gttcagaatg gatttatcag aaattatact 1920
atattttata gaaccatcat tggaaatgaa actgctgtga atgtggattc ttcccacaca 1980
gaatatacat tgtcctcttt gactagtgac acattgtaca tggtacgaat ggcagcatac 2040
acagatgaag gtgggaagga tggtccagaa ttcactttta ctaccccaaa gtttgctcaa 2100
ggagaaattg aagccatagt cgtgcctgtt tgcttagcat tcctattgac aactcttctg 2160
ggagtgctgt tctgctttaa taagcgagac ctaattaaaa aacacatctg gcctaatgtt 2220
ccagatcctt caaagagtca tattgcccag tggtcacctc acactcctcc aaggcacaat 2280
tttaattcaa aagatcaaat gtattcagat ggcaatttca ctgatgtaag tgttgtggaa 2340
atagaagcaa atgacaaaaa gccttttcca gaagatctga aatcattgga cctgttcaaa 2400
aaggaaaaaa ttaatactga aggacacagc agtggtattg gggggtcttc atgcatgtca 2460
tcttctaggc caagcatttc tagcagtgat gaaaatgaat cttcacaaaa cacttcgagc 2520
actgtccagt attctaccgt ggtacacagt ggctacagac accaagttcc gtcagtccaa 2580
gtcttctcaa gatccgagtc tacccagccc ttgttagatt cagaggagcg gccagaagat 2640
ctacaattag tagatcatgt agatggcggt gatggtattt tgcccaggca acagtacttc 2700
aaacagaact gcagtcagca tgaatccagt ccagatattt cacattttga aaggtcaaag 2760
caagtttcat cagtcaatga ggaagatttt gttagactta aacagcagat ttcagatcat 2820
atttcacaat cctgtggatc tgggcaaatg aaaatgtttc aggaagtttc tgcagcagat 2880
gcttttggtc caggtactga gggacaagta gaaagatttg aaacagttgg catggaggct 2940
gcgactgatg aaggcatgcc taaaagttac ttaccacaga ctgtacggca aggcggctac 3000
atgcctcagt gaaggactag tagttcctgc tacaacttca gcagtaccta taaagtaaag 3060
ctaaaatgat tttatctgtg aattcagatt ttaaaaagtc ttcactctct gaagatgatc 3120
atttgccctt aaggacaaaa atgaactgaa gtttcacatg agctatttcc attccagaat 3180
atctgggatt ctactttaag cactacataa actgacttta tcctcagaaa aaaaaaaaaa 3240
aa 3242

<210>  94
<211>  2155
<212>  DNA
<213>  Homo sapiens

<220>
<223>  II.94 (XM_008570)

<400>  94
tctttccgtc ccgggcagcc agcgccagtc ggagccagcg cgagccgccg ccgccatcac      60

tgccgctgcc aagtcctcca cccgctgccc ccgccatgtc tgctaccgct gccacggccc     120

cccctgctgc cccggctggg gagggtggtc cccctgcacc ccctccaaac ctcaccagta     180

acaggagact gcagcagacc caggcccagg tggatgaggt ggtggacatc atgagggtga     240

acgtggacaa ggtcctggag cgagaccaga agctgtcgga gctggacgac cgtgcagatg     300

cactccaggc gggggcctcc cagtttgaaa caagcgcagc caagctcaag cgcaaatact     360

ggtggaaaaa cctcaagatg atgatcatct tgggagtgat ttgcgccatc atcctcatca     420

tcatcatagt ttacttcagc acttaaatcc ccgaggagtc tgccctgcct agagaagggc     480

ctctccccca accctcagcc gttcctccac ctctcagcca tatctttcag cccccactcc     540

cctggatccg tgtgtgtgtg tgtccgtgtg tgtgtccccc tgtaaatagc cagctgttat     600

ttatacatat ataatattat atatatttgg tctgtttgta gttttattac tagatgattt     660

ttccggttgt ccttaacacc ccttcctgag gttcccttca cccctctctc ttgccttcct     720

tccctttccc tttcttcctg actagcccca agtcccttca tttgcatctg ctatgcaata     780

gtccctctcc tttccttctt cttccctcag atttagctga tccttcctcc caccctggcc     840

ttcctttcct ctttcctcct cactctcccc gtcatgctcc ctctgccccg ccctcaaaaa     900

aaaaaaaaaa aaaaaaaaaa caacagcacc tgtccaggct tccttaggta catcttcttt     960

gtatccattg ggaggctctg agactggccc cacttggtcc taagaatccc aaggtctttg    1020

ggagcgtcca gcatgttaat tagcgtatca ttacatactg ctatcccttt ccatttcttt    1080

ttgttccatc actcttctct caacctgtgt ttcttttttt actgaggagt tagtccccat    1140

tagttcttgt atcacatttt catttgcacg acattactcg caggtggtgg ggagcctggg    1200

cttttgggga accaggctgc tctggtcccc agcattgcct cctcctagcc cctctagtcc    1260

agtttgcctc ccttaccctc attttccaaa cctcttgtac cctcctctcc ctcccccagc    1320

tggtatgtaa gtgtcttgaa gttcagtatg ttatgatgga ccaataattc tgccacttcg    1380

ggtttctccc tacattcctg ctccccagtt ttcatgtggg gtactcaact gacattccca    1440

tggggtttcc ctcccatctg cctgatccac ctcctcctcc caccaggaga gttgggggtt    1500

ggccacaatt gatctcttgt gagaggggtg gctaccagtg tgtgtgtggg ggtcatcact    1560

gccttgggga ggagtggggc agggcagaga atccccccaa ttcctgcctg aaatctctgg    1620

cctcacccct gctgggggtt ggactgaaaa ccctcctccc caatttgggg ggtgttgccc    1680

catcactgcc cagctcctct gactgccccc cctgaattta gggtgggggt actagtcact    1740

gccaatgtgt gtatgggact tgctggaaaa cggggatgct tgcccctctc caggactatt 1800

gagcccagag agagctgtcc tctcattggg tgaactgatt gaggaagggt ctattgtctt 1860

tttaaatggc acaattttaa gggtttgagg gtacagtccc ttaacctgcc acgggagggg 1920

gcccccaaac tttcttcccc ccacacttct ggttttctgt gtggaggggg agcagggata 1980

tctaagctgt ggtgtgaaag ggtaggagag atgctggagg tgggggtgct gtgttttaga 2040

ccccccatat tatcccagtg tcccctgccc ccctcttccc ccaccccatg cccccaattc 2100

tgtggcgcat ccagattgtg aaaatgtaca ataaatgtgt aatgagtaac caggt 2155

<210> 95
<211> 2758
<212> DNA
<213> Homo sapiens

<220>
<223> II.95 (NM_006856)

<400> 95
gaattcaggg gggaaaggag gagctggaga cagattgtag gaccgagcgc gggcaggcgg 60

gaggcaacgg agctaccagc cgctcctctc tgctatatga aatatgggag acgacagacc 120

gtttgtgtgc aatgccccgg gctgtggaca gagatttaca aacgaggacc acctggcagt 180

tcataaacac aagcatgaga tgacattgaa atttggccca gcccgaactg actcagtcat 240

cattgcagat caaacgccta ctccaactag attcctgaag aactgtgagg aggtgggact 300

cttcaatgaa ctagctagct cctttgaaca tgaattcaag aaagctgcag atgaggatga 360

gaaaaaggct gctgctgggc cccttgacat gtctctgcct tccacaccag acatcaaaat 420

caaagaagaa gagccagtgg aggtagactc atccccacct gatagccctg cctctagtcc 480

ctgttcccca ccactgaagg agaaggaggt taccccaaag cctgttctga tctctacccc 540

cacacccacc attgtacgtc ctggctccct gcctctccac ttgggctatg atccacttca 600

tccaaccctt ccctccccaa cctctgtcat cacacaggct ccaccatcca acaggcaaat 660

ggggtctccc actggctccc tccctcttgt catgcatctt gctaatggac agaccatgcc 720

tgtgttgcca gggcctccag tacagatgcc gtctgttata tcgctggcca gacctgtgtc 780

catggtgccc aacattcctg gtatccctgg cccaccagtt aacagtagtg gctccatttc 840

tccctctggc caccctatac catcagaagc caagatgaga ctgaaagcca ccctaactca 900

ccaagtctcc tcaatcaatg gtggttgtgg aatggtggtg ggtactgcca gcaccatggt 960

gacagcccgc ccagagcaga gccagattct catccagcac cctgatgccc catcccctgc 1020

ccagccacag gtctcaccag ctcagcccac ccctagtact ggggggcgac ggcggcgcac 1080

agtagatgaa gatccagatg agcgacggca gcgctttctg gagcgcaacc gggctgcagc 1140

ctcccgctgc cgccaaaagc gaaagctgtg ggtgtcctcc ctagagaaga aggccgaaga 1200

actcacttct cagaacattc agctgagtaa tgaagtcaca ttactacgca atgaggtggc 1260

ccagttgaaa cagctactgt tagctcataa agactgccca gtcactgcac tacagaaaaa 1320

gactcaaggc tatttagaaa gccccaagga aagctcagag ccaacgggtt ctccagcccc 1380

tgtgattcag cacagctcag caacagcccc tagcaatggc ctcagtgttc gctctgcagc 1440

tgaagctgtg gccacctcgg tcctcactca gatggccagc caaaggacag aactgagcat 1500

gccgatacaa tcgcatgtaa tcatgacccc acagtcccag tctgcgggca gatgatgcct 1560

cctctggtgg agaggtcctc agccagagct cgcccgccca agagccaaga gagatgtcat 1620

cttatcccct cccatctgcc ttggacatgg caatatgggt ggggggaatt gtgtgttgtg 1680

agtaatggac agatatgggg ctttttagcc atgtggtcat gtacgtttga cacctgtgct 1740

gggggcctcc cagccccgga gcctcataga tcatccccca gggtggggtt tctgatgcat 1800

ccacagccaa aggcctttcc agatggtccg aacaatcacc cctgccccat gcacgtgtat 1860

ctgtctcttt taacactaac ccttggcact tctaggtttg gggtttctca gttccctctc 1920

ttcccactaa gccgtggctg ttaccttttt gttccttacc agcatgccac ttcctgccag 1980

atagagggag gctagatttg atgacatatt acccgctgac ccagccccag ccaccccatg 2040

gactcaggac tcaggtctcc catagctgct tatttacccg tttccttttt cctcttcctt 2100

aattctagtt aaaacattca cttacggaga atgtaaggtt acctttgatg tatgaagtag 2160

cagtattttc cagtttttcc ataagcttta ttccttctct ctacctgcca ccaaaaagaa 2220

agaaaccctc aagagttcta attctttttc atctatttgc acagctattt gggtacctca 2280

ttttgtccct tgactttagt gtagtggtgg ggtctttccc accaccccac taagccttgg 2340

caacctcatt ttgtggcctt attattaaag attgcttggt agtgaaaagg gaagggccca 2400

gaatctgact ggctgattct gtctcctccc ctccctcctt ctttagttta accatcaggg 2460

cagacaggaa caaagtattt caaagcttgt tccatcaagt tagggattaa tcgattcctt 2520

tatgttttat aagagtttac cggccaaagc atattgtcat ctttggtcat ttttctgctc 2580

ctgcttctct ctcttcattg gggtatcaac attaccgccc ctgtctctct tcattcccct 2640

atcaacatta ccgcccctgt ctcttctgta actacttcag actcagccct cctggaggct 2700

gtcatgtagg ggactctcga ggttcctttg ttctacccca tctctccccc ccgaattc 2758

<210> 96
<211> 2900
<212> DNA
<213> Homo sapiens

<220>
<223> II.96 (NM_000674)

<400> 96
atgagtgtca gaagtgtgaa gggtgcctgt tctgaatccc agagcctcct ctccctctgt 60

gaggctggca ggtgaggaag ggtttaacct cactggaagg aatccctgga gctagcggct 120

gctgaaggcg tcgaggtgtg ggggcacttg gacagaacag tcaggcagcc gggagctctg 180

ccagctttgg tgaccttggg ccgggctggg agcgctgcgg cgggagccgg aggactatga 240

gctgccgcgc gttgtccaga gcccagccca gccctacgcg cgcggcccgg agctctgttc 300
cctggaactt tgggcactgc ctctgggacc cctgccggcc agcaggcagg atggtgcttg 360
cctcgtgccc cttggtgccc gtctgctgat gtgcccagcc tgtgcccgcc atgccgccct 420
ccatctcagc tttccaggcc gcctacatcg gcatcgaggt gctcatcgcc ctggtctctg 480
tgcccgggaa cgtgctggtg atctgggcgg tgaaggtgaa ccaggcgctg cgggatgcca 540
ccttctgctt catcgtgtcg ctggcggtgg ctgatgtggc cgtgggtgcc ctggtcatcc 600
ccctcgccat cctcatcaac attgggccac agacctactt ccacacctgc ctcatggttg 660
cctgtccggt cctcatcctc acccagagct ccatcctggc cctgctggca attgctgtgg 720
accgctacct ccgggtcaag atccctctcc ggtacaagat ggtggtgacc ccccggaggg 780
cggcggtggc catagccggc tgctggatcc tctccttcgt ggtgggactg acccctatgt 840
ttggctggaa caatctgagt gcggtggagc gggcctgggc agccaacggc agcatggggg 900
agcccgtgat caagtgcgag ttcgagaagg tcatcagcat ggagtacatg gtctacttca 960
acttctttgt gtgggtgctg cccccgcttc tcctcatggt cctcatctac ctggaggtct 1020
tctacctaat ccgcaagcag ctcaacaaga aggtgtcggc ctcctccggc gacccgcaga 1080
agtactatgg gaaggagctg aagatcgcca agtcgctggc cctcatcctc ttcctctttg 1140
ccctcagctg gctgcctttg cacatcctca actgcatcac cctcttctgc ccgtcctgcc 1200
acaagcccag catccttacc tacattgcca tcttcctcac gcacggcaac tcggccatga 1260
accccattgt ctatgccttc cgcatccaga agttccgcgt caccttcctt aagatttgga 1320
atgaccattt ccgctgccag cctgcacctc ccattgacga ggatctccca gaagagaggc 1380
ctgatgacta gaccccgcct tccgctccca ccagcccaca tccagtgggg tctcagtcca 1440
gtcctcacat gcccgctgtc ccaggggtct ccctgagcct gccccagctg ggctgttggc 1500
tgggggcatg ggggaggctc tgaagagata cccacagagt gtggtccctc cactaggagt 1560
taactaccct acacctctgg gccctgcagg aggcctggga gggcaagggt cctacggagg 1620
gaccaggtgt ctagaggcaa cagtgttctg agcccccacc tgcctgacca tcccatgagc 1680
agtccagcgc ttcagggctg ggcaggtcct ggggaggctg agactgcaga ggagccacct 1740
gggctgggag aaggtgcttg ggcttctgcg gtgaggcagg ggagtctgct tgtcttagat 1800
gttggtggtg cagccccagg accaagctta aggagaggag agcatctgct ctgagacgga 1860
tggaaggaga gaggttgagg atgcactggc ctgttctgta ggagagactg gccagaggca 1920
gctaaggggc aggaatcaag gagcctccgt tcccacctct gaggactctg gaccccaggc 1980
cataccaggt gctagggtgc ctgctctcct tgccctgggc cagcccagga ttgtacgtgg 2040
gagaggcaga aagggtaggt tcagtaatca tttctgatga tttgctggag tgctggctcc 2100
acgccctggg gagtgagctt ggtgcggtag gtgctggcct caaacagcca cgaggtggta 2160
gctctgagcc ctccttcttg ccctgagctt tccggggagg agcctggagt gtaattacct 2220
gtcatctggg ccaccagctc cactggcccc cgttgccggg cctggactgt cctaggtgac 2280

cccatctctg ctgcttctgg gcctgatgga gaggagaaca ctagacatgc caactcggga 2340

gcattctgcc tgcctgggaa cggggtggac gagggagtgt ctgtaaggac tcagtgttga 2400

ctgtaggcgc ccctggggtg ggtttagcag gctgcagcag gcagaggagg agtacccccc 2460

tgagagcatg tgggggaagg ccttgctgtc atgtgaatcc ctcaataccc ctagtatctg 2520

gctgggtttt caggggcttt ggaagctctg ttgcaggtgt ccgggggtct aggactttag 2580

ggatctggga tctggggaag gaccaaccca tgccctgcca agcctggagc ccctgtgttg 2640

gggggcaagg tgggggagcc tggagcccct gtgtgggagg gcgaggcggg ggagcctgga 2700

gcccctgtgt gggagggcga ggcgggggat cctggagccc ctgtgtcggg gggcgaggga 2760

ggggaggtgg ccgtcggttg accttctgaa catgagtgtc aactccagga cttgcttcca 2820

agcccttccc tctgttggaa attgggtgtg ccctggctcc caagggaggc ccatgtgact 2880

aataaaaaac tgtgaaccct 2900

<210> 97
<211> 2480
<212> DNA
<213> Homo sapiens

<220>
<223> II.97 (NM_000173)

<400> 97
gacgctctgt gccttcggag gtctttctgc ctgcctgtcc tcatgcctct cctcctcttg 60

ctgctcctgc tgccaagccc cttacacccc caccccatct gtgaggtctc caaagtggcc 120

agccacctag aagtgaactg tgacaagagg aatctgacag cgctgcctcc agacctgccg 180

aaagacacaa ccatcctcca cctgagtgag aacctcctgt acaccttctc cctggcaacc 240

ctgatgcctt acactcgcct cactcagctg aacctagata ggtgcgagct caccaagctc 300

caggtcgatg ggacgctgcc agtgctgggg accctggatc tatcccacaa tcagctgcaa 360

agcctgccct tgctagggca gacactgcct gctctcaccg tcctggacgt ctccttcaac 420

cggctgacct cgctgcctct tggtgccctg cgtggtcttg gcgaactcca agagctctac 480

ctgaaaggca atgagctgaa gaccctgccc ccagggctcc tgacgcccac acccaagctg 540

gagaagctca gtctggctaa caacaacttg actgagctcc ccgctgggct cctgaatggg 600

ctggagaatc tcgacaccct tctcctccaa gagaactcgc tgtatacaat accaaagggc 660

ttttttgggt cccacctcct gccttttgct tttctccacg ggaacccctg gttatgcaac 720

tgtgagatcc tctattttcg tcgctggctg caggacaatg ctgaaaatgt ctacgtatgg 780

aagcaaggtg tggacgtcaa ggccatgacc tctaacgtgg ccagtgtgca gtgtgacaat 840

tcagacaagt ttcccgtcta caaataccca ggaaaggggt gccccaccct tggtgatgaa 900

ggtgacacag acctatatga ttactaccca gaagaggaca ctgagggcga taaggtgcgt 960

gccacaagga ctgtggtcaa gttccccacc aaagcccata caaccccctg gggtctattc 1020

tactcatggt ccactgcttc tctagacagc caaatgccct cctccttgca tccaacacaa 1080

gaatccacta aggagcagac cacattccca cctagatgga ccccaaattt cacacttcac 1140

atggaatcca tcacattctc caaaactcca aaatccacta ctgaaccaac cccaagcccg 1200

accacctcag agcccgtccc ggagcccgcc ccaaacatga ccaccctgga gcccactcca 1260

agcccgacca ccccagagcc cacctcagag cccgccccca gcccgaccac cccggagccc 1320

accccaatcc cgaccatcgc cacaagcccg accatcctgg tgtctgccac aagcctgatc 1380

actccaaaaa gcacattttt aactaccaca aaacccgtat cactcttaga atccaccaaa 1440

aaaaccatcc ctgaacttga tcagccacca aagctccgtg gggtgctcca agggcatttg 1500

gagagctcca gaaatgaccc ttttctccac cccgactttt gctgcctcct ccccctgggc 1560

ttctatgtct tgggtctctt ctggctgctc tttgcctctg tggtcctcat cctgctgctg 1620

agctgggttg ggcatgtgaa accacaggcc ctggactctg gccaaggtgc tgctctgacc 1680

acagccacac aaaccacaca cctggagctg cagaggggac ggcaagtgac agtgccccgg 1740

gcctggctgc tcttccttcg aggttcgctt cccactttcc gctccagcct cttcctgtgg 1800

gtacggccta atggccgtgt ggggcctcta gtggcaggaa ggaggccctc agctctgagt 1860

cagggtcgtg gtcaggacct gctgagcaca gtgagcatta ggtactctgg ccacagcctc 1920

tgagggtggg aggtttgggg accttgagag aagagcctgt gggctctcct attggaatct 1980

agttgggggt tggaggggta aggaacacag ggtgataggg gaggggtctt agttcctttt 2040

tctgtatcag aagccctgtc ttcacaacac aggcacacaa tttcagtccc agccaaagca 2100

gaaggggtaa tgacatggac ttggcggggg gacaagacaa agctcccgat gctgcatggg 2160

gcgctgccag atctcacggt gaaccatttt ggcagaatac agcatggttc ccacatgcat 2220

ttatgcacag aagaaaatct ggaaagtgat ttatcaggat gtgagcactc gttgtgtctg 2280

gatgttacaa atatgggtgg ttttattttc tttttccctg tttagcattt tctagttttc 2340

ttatcaggat gtgagcactc gttgtgtctg gatgttacaa atatgggtgg ttttattttc 2400

tttttccctg tttagcattt tctagttttc cactattatt gtatattatc tgtataataa 2460

aaaataattt tagggttggg 2480

<210> 98
<211> 3274
<212> DNA
<213> Homo sapiens

<220>
<223> II.98 (XM_048068)

<400> 98
   gcactgagct ctgccgcctg gctctagccg cctgcctggc cccgccgggg actcttgccc 60
   accctcagcc atggctccga tatctctgtc gtggctgctc cgcttggcca ccttctgcca 120
   tctgactgtc ctgctggctg gacagcacca cggtgtgacg aaatgcaaca tcacgtgcag 180
   caagatgaca tcaaagatac ctgtagcttt gctcatccac tatcaacaga accaggcatc 240
   atgcggcaaa cgcgcaatca tcttggagac gagacagcac aggctgttct gtgccgaccc 300
   gaaggagcaa tgggtcaagg acgcgatgca gcatctggac cgccaggctg ctgccctaac 360
   tcgaaatggc ggcaccttcg agaagcagat cggcgaggtg aagcccagga ccacccctgc 420
   cgccggggga atggacgagt ctgtggtcct ggagcccgaa gccacaggcg aaagcagtag 480
   cctggagccg actccttctt cccaggaagc acagagggcc ctggggacct ccccagagct 540
   gccgacgggc gtgactggtt cctcagggac caggctcccc ccgacgccaa aggctcagga 600
   tggagggcct gtgggcacgg agcttttccg agtgcctccc gtctccactg ccgccacgtg 660
   gcagagttct gctccccacc aacctgggcc cagcctctgg gctgaggcaa agacctctga 720
   ggccccgtcc acccaggacc cctccaccca ggcctccact gcgtcctccc cagccccaga 780
   ggagaatgct ccgtctgaag gccagcgtgt gtggggtcag ggacagagcc ccaggccaga 840
   gaactctctg gagcgggagg agatgggtcc cgtgccagcg cacacggatg ccttccagga 900
   ctgggggcct ggcagcatgg cccacgtctc tgtggtccct gtctcctcag aagggacccc 960
   cagcagggag ccagtggctt caggcagctg gacccctaag gctgaggaac ccatccatgc 1020
   caccatggac ccccagaggc tgggcgtcct tatcactcct gtccctgacg cccaggctgc 1080
   cacccggagg caggcggtgg ggctgctggc cttccttggc ctcctcttct gcctgggggt 1140
   ggccatgttc acctaccaga gcctccaggg ctgccctcga aagatggcag gagagatggc 1200
   ggagggcctt cgctacatcc cccggagctg tggtagtaat tcatatgtcc tggtgcccgt 1260
   gtgaactcct ctggcctgtg tctagttgtt tgattcagac agctgcctgg gatccctcat 1320
   cctcataccc acccccaccc aagggcctgg cctgagctgg gatgattgga ggggggaggt 1380
   gggatcctcc aggtgcacaa gctccaagct cccaggcatt ccccaggagg ccagccttga 1440
   ccattctcca ccttccaggg acagaggggg tggcctccca actcacccca gccccaaaac 1500
   tctcctctgc tgctggctgg ttagaggttc cctttgacgc catcccagcc ccaatgaaca 1560
   attatttatt aaatgcccag ccccttctga cccatgctgc cctgtgagta ctacagtcct 1620
   cccatctcac acatgagcat caggccaggc cctctgccca ctccctgcaa cctgattgtg 1680
   tctcttggtc ctgctgcagt tgccagtcac cccggccacc tgcggtgcta tctcccccag 1740
   ccccatcctc tgtacagagc ccacgccccc actggtgaca tgtcttttct tgcatgaggc 1800
   tagtgtggtg tttcctggca ctgcttccag tgaggctctg cccttggtta ggcattgtgg 1860
   gaaggggaga taagggtatc tggtgacttt cctctttggt ctacactgtg ctgagtctga 1920
   aggctgggtt ctgatcctag ttccaccatc aagccaccaa catactccca tctgtgaaag 1980
   gaaagaggga ggtaaggaat acctgtcccc ctgacaacac tcattgacct gaggcccttc 2040
   tctccagccc ctggatgcag cctcacagtc cttaccagca gagcacctta gacagtccct 2100
   gccaatggac taacttgtct ttggaccctg aggcccagag ggcctgcaag ggagtgagtt 2160
   gatagcacag accctgccct gtgggccccc aaatggaaat gggcagagca gagaccatcc 2220
   ctgaaggccc cgcccaggct tagtcactga gacagcccgg gctctgcctc ccatcacccg 2280
   ctaagaggga gggagggctc cagacacatg tccaagaagc ccaggaaagg ctccaggagc 2340
   agccacattc ctgatgcttc ttcagagact cctgcaggca gccaggccac aagacccttg 2400
   tggtcccacc ccacacacgc cagattcttt cctgaggctg ggctcccttc ccacctctct 2460
   cactccttga aaacactgtt ctctgccctc caagaccttc tccttcacct ttgtccccac 2520
   cgcagacagg accagggatt tccatgatgt tttccatgag tcccctgttt gtttctgaaa 2580
   gggacgctac ccgggaaggg ggctgggaca tgggaaaggg gaagttgtag gcataaagtc 2640
   aggggttccc ttttttggct gctgaaggct cgagcatgcc tggatggggc tgcaccggct 2700
   ggcctggccc ctcagggtcc ctggtggcag ctcacctctc ccttggattg tccccgaccc 2760
   ttgccgtcta cctgaggggc ctcttatggg ctgggttcta cccaggtgct aggaacactc 2820
   cttcacagat gggtgcttgg aggaaggaaa cccagctctg gtccatagag agcaagacgc 2880
   tgtgctgccc tgcccacctg gcctctgcac tcccctgctg ggtgtggcgc agcatattca 2940
   ggaagctcag ggcctggctc aggtggggtc actctggcag ctcagagagg gtgggagtgg 3000
   gtccaatgca ctttgttctg gctcttccag gctgggagag cctttcaggg gtgggacacc 3060
   ctgtgatggg gccctgcctc ctttgtgagg aagccgctgg ggccagttgg tcccccttcc 3120
   atggactttg ttagtttctc caagcaggac atggacaagg atgatctagg aagactttgg 3180
   aaagagtagg aagactttgg aaagactttt ccaaccctca tcaccaacgt ctgtgccatt 3240
   ttgtatttta ctaataaaat ttaaaagtct tgtg 3274

<210> 99
<211> 4485
<212> DNA
<213> Homo sapiens

<220>
<223> II.99 (NM_022162)

<400> 99
gtagacagat ccaggctcac cagtcctgtg ccactgggct tttggcgttc tgcacaaggc 60

ctacccgcag atgccatgcc tgctccccca gcctaatggg ctttgatggg ggaagagggt 120

ggttcagcct ctcacgatga ggaggaaaga gcaagtgtcc tcctcggaca ttctccgggt 180

tgtgaaatgt gctcgcagga ggcttttcag gcacagagga gccagctggt cgagctgctg 240

gtctcagggt ccctggaagg cttcgagagt gtcctggact ggctgctgtc ctgggaggtc 300

ctctcctggg aggactacga gggcttccac ctcctgggcc agcctctctc ccacttggcc 360

aggcgccttc tggacaccgt ctggaataag ggtacttggg cctgtcagaa gctcatcgcg 420

gctgcccaag aagcccaggc cgacagccag tcccccaagc tgcatggctg ctgggacccc 480

cactcgctcc acccagcccg agacctgcag agtcaccggc cagccattgt caggaggctc 540

cacagccatg tggagaacat gctggacctg gcatgggagc ggggtttcgt cagccagtat 600

gaatgtgatg aaatcaggtt gccgatcttc acaccgtccc agagggcaag aaggctgctt 660

gatcttgcca cggtgaaagc gaatggattg gctgccttcc ttctacaaca tgttcaggaa 720
ttaccagtcc cattggccct gcctttggaa gctgccacat gcaagaagta tatggccaag 780
ctgaggacca cggtgtctgc tcagtctcgc ttcctcagta cctatgatgg agcagagacg 840
ctctgcctgg aggacatata cacagagaat gtcctggagg tctgggcaga tgtgggcatg 900
gctggacccc cgcagaagag cccagccacc ctgggcctgg aggagctctt cagcacccct 960
ggccacctca atgacgatgc ggacactgtg ctggtggtgg gtgaggcggg cagtggcaag 1020
agcacgctcc tgcagcggct gcacttgctg tgggctgcag ggcaagactt ccaggaattt 1080
ctctttgtct tcccattcag ctgccggcag ctgcagtgca tggccaaacc actctctgtg 1140
cggactctac tctttgagca ctgctgttgg cctgatgttg gtcaagaaga catcttccag 1200
ttactccttg accaccctga ccgtgtcctg ttaacctttg atggctttga cgagttcaag 1260
ttcaggttca cggatcgtga acgccactgc tccccgaccg accccacctc tgtccagacc 1320
ctgctcttca accttctgca gggcaacctg ctgaagaatg cccgcaaggt ggtgaccagc 1380
cgtccggccg ctgtgtcggc gttcctcagg aagtacatcc gcaccgagtt caacctcaag 1440
ggcttctctg aacagggcat cgagctgtac ctgaggaagc gccatcatga gcccggggtg 1500
gcggaccgcc tcatccgcct gctccaagag acctcagccc tgcacggttt gtgccacctg 1560
cctgtcttct catggatggt gtccaaatgc caccaggaac tgttgctgca ggaggggggg 1620
tccccaaaga ccactacaga tatgtacctg ctgattctgc agcattttct gctgcatgcc 1680
acccccccag actcagcttc ccaaggtctg ggacccagtc ttcttcgggg ccgcctcccc 1740
accctcctgc acctgggcag actggctctg tggggcctgg gcatgtgctg ctacgtgttc 1800
tcagcccagc agctccaggc agcacaggtc agccctgatg acatttctct tggcttcctg 1860
gtgcgtgcca aaggtgtcgt gccagggagt acggcgcccc tggaattcct tcacatcact 1920
ttccagtgct tctttgccgc gttctacctg gcactcagtg ctgatgtgcc accagctttg 1980
ctcagacacc tcttcaattg tggcaggcca ggcaactcac caatggccag gctcctgccc 2040
acgatgtgca tccaggcctc ggagggaaag gacagcagcg tggcagcttt gctgcagaag 2100
gccgagccgc acaaccttca gatcacagca gccttcctgg cagggctgtt gtcccgggag 2160
cactggggcc tgctggctga gtgccagaca tctgagaagg ccctgctccg gcgccaggcc 2220
tgtgcccgct ggtgtctggc ccgcagcctc cgcaagcact tccactccat cccgccagct 2280
gcaccgggtg aggccaagag cgtgcatgcc atgcccgggt tcatctggct catccggagc 2340
ctgtacgaga tgcaggagga gcggctggct cggaaggctg cacgtggcct gaatgttggg 2400
cacctcaagt tgacattttg cagtgtgggc cccactgagt gtgctgccct ggcctttgtg 2460
ctgcagcacc tccggcggcc cgtggccctg cagctggact acaactctgt gggtgacatt 2520
ggcgtggagc agctgctgcc ttgccttggt gtctgcaagg ctctgtattt gcgcgataac 2580
aatatctcag accgaggcat ctgcaagctc attgaatgtg ctcttcactg cgagcaattg 2640
cagaagttag ctctattcaa caacaaattg actgacggct gtgcacactc catggctaag 2700

ctccttgcat gcaggcagaa cttcttggca ttgaggctgg ggaataacta catcactgcc 2760
gcgggagccc aagtgctggc cgaggggctc cgaggcaaca cctccttgca gttcctggga 2820
ttctggggca acagagtggg tgacgagggg gcccaggccc tggctgaagc cttgggtgat 2880
caccagagct tgaggtggct cagcctggtg gggaacaaca ttggcagtgt gggtgcccaa 2940
gccttggcac tgatgctggc aaagaacgtc atgctagaag aactctgcct ggaggagaac 3000
catctccagg atgaaggtgt atgttctctc gcagaaggac tgaagaaaaa ttcaagtttg 3060
aaaatcctga agttgtccaa taactgcatc acctacctag gggcagaagc cctcctgcag 3120
gcccttgaaa ggaatgacac catcctggaa gtctggctcc gagggaacac tttctctcta 3180
gaggaggttg acaagctcgg ctgcagggac accagactct tgctttgaag tctccgggag 3240
gatgttcgtc tcagtttgtt tgtgagcagg ctgtgagttt gggccccaga ggctgggtga 3300
catgtgttgg cagcctcttc aaaatgagcc ctgtcctgcc taaggctgaa cttgttttct 3360
gggaacacca taggtcacct ttattctggc agaggaggga gcatcagtgc cctccaggat 3420
agacttttcc caagcctact tttgccattg acttcttccc aagattcaat cccaggatgt 3480
acaaggacag cccctcctcc atagtatggg actggcctct gctgatcctc ccaggcttcc 3540
gtgtgggtca gtggggccca tggatgtgct tgttaactga gtgccttttg gtggagaggc 3600
ccggcctctc acaaaagacc ccttaccact gctctgatga agaggagtac acagaacaca 3660
taattcagga agcagctttc cccatgtctc gactcatcca tccaggccat tccccgtctc 3720
tggttcctcc cctcctcctg gactcctgca cacgctcctt cctctgaggc tgaaattcag 3780
aatattagtg acctcagctt tgatatttca cttacagcac ccccaaccct ggcacccagg 3840
gtgggaaggg ctacacctta gcctgccctc ctttccggtg tttaagacat ttttggaagg 3900
ggacacgtga cagccgtttg ttccccaaga cattctaggt ttgcaagaaa aatatgacca 3960
cactccagct gggatcacat gtggactttt atttccagtg aaatcagtta ctcttcagtt 4020
aagcctttgg aaacagctcg actttaaaaa gctccaaatg cagctttaaa aaattaatct 4080
gggccagaat ttcaaacggc ctcactaggc ttctggttga tgcctgtgaa ctgaactctg 4140
acaacagact tctgaaatag acccacaaga ggcagttcca tttcatttgt gccagaatgc 4200
tttaggatgt acagttatgg attgaaagtt tacaggaaaa aaaattaggc cgttccttca 4260
aagcaaatgt cttcctggat tattcaaaat gatgtatgtt gaagcctttg taaattgtca 4320
gatgctgtgc aaatgttatt attttaaaca ttatgatgtg tgaaaactgg ttaatattta 4380
taggtcactt tgttttactg tcttaagttt atactcttat agacaacatg gccgtgaact 4440
ttatgctgta aataatcaga ggggaataaa ctgttgagtc aaaac 4485

<210> 100
<211> 2487
<212> DNA
<213> Homo sapiens

<220>

<223>  II.100 (NM_001199)

<400>  100
   gccgcttccc tcgccgccgc cccgccagca tgcccggcgt ggcccgcctg ccgctgctgc      60
   tcgggctgct gctgctcccg cgtcccggcc ggccgctgga cttggccgac tacacctatg     120
   acctggcgga ggaggacgac tcggagcccc tcaactacaa agacccctgc aaggcggctg     180
   cctttcttgg ggacattgcc ctggacgaag aggacctgag ggccttccag gtacagcagg     240
   ctgtggatct cagacggcac acagctcgta agtcctccat caaagctgca gttccaggaa     300
   acacttctac ccccagctgc cagagcacca acgggcagcc tcagagggga gcctgtggga     360
   gatggagagg tagatcccgt agccggcggg cggcgacgtc ccgaccagag cgtgtgtggc     420
   ccgatggggt catccccttt gtcattgggg gaaacttcac tggtagccag agggcagtct     480
   tccggcaggc catgaggcac tgggagaagc acacctgtgt caccttcctg gagcgcactg     540
   acgaggacag ctatattgtg ttcacctatc gaccttgcgg gtgctgctcc tacgtgggtc     600
   gccgcggcgg gggcccccag gccatctcca tcggcaagaa ctgtgacaag ttcggcattg     660
   tggtccacga gctgggccac gtcgtcggct tctggcacga acacactcgg ccagaccggg     720
   accgccacgt ttccatcgtt cgtgagaaca tccagccagg gcaggagtat aacttcctga     780
   agatggagcc tcaggaggtg gagtccctgg gggagaccta tgacttcgac agcatcatgc     840
   attacgctcg gaacacattc tccaggggca tcttcctgga taccattgtc cccaagtatg     900
   aggtgaacgg ggtgaaacct cccattggcc aaaggacacg gctcagcaag ggggacattg     960
   cccaagcccg caagctttac aagtgcccag cctgtggaga gaccctgcaa gacagcacag    1020
   gcaacttctc ctcccctgaa taccccaatg gctactctgc tcacatgcac tgcgtgtggc    1080
   gcatctctgt cacacccggg gagaagatca tcctgaactt cacgtccctg gacctgtacc    1140
   gcagccgcct gtgctggtac gactatgtgg aggtccgaga tggcttctgg aggaaggcgc    1200
   cctccgaggg ccgcttctgc gggtccaaac tccctgagcc tatcgtctcc actgacagcc    1260
   gcctctgggt tgaattccgc agcagcagca attgggttgg aaagggcttc tttgcagtct    1320
   acgaagccat ctgcgggggt gatgtgaaaa aggactatgg ccacattcaa tcgcccaact    1380
   acccagacga ttaccggccc agcaaagtct gcatctggcg gatccaggtg tctgagggct    1440
   tccacgtggg cctcacattc cagtcctttg agattgagcg ccacgacagc tgtgcctacg    1500
   actatctgga ggtgcgcgac gggcacagtg agagcagcac cctcatcggg cgctactgtg    1560
   gctatgagaa gcctgatgac atcaagagca cgtccagccg cctctggctc aagttcgtct    1620
   ctgacgggtc cattaacaaa gcgggctttg ccgtcaactt tttcaaagag gtggacgagt    1680
   gctctcggcc caaccgcggg ggctgtgagc agcggtgcct caacaccctg ggcagctaca    1740
   agtgcagctg tgaccccggg tacgagctgg ccccagacaa gcgccgctgt gaggctgctt    1800
   gtggcggatt cctcaccaag ctcaacggct ccatcaccag cccgggctgg cccaaggagt    1860
   acccccccaa caagaactgc atctggcagc tggtggcccc cacccagtac cgcatctccc    1920
   tgcagtttga cttctttgag acagagggca atgatgtgtg caagtacgac ttcgtggagg    1980
   tgcgcagtgg actcacagct gactccaagc tgcatggcaa gttctgtggt tctgagaagc 2040
   ccgaggtcat cacctcccag tacaacaaca tgcgcgtgga gttcaagtcc gacaacaccg 2100
   tgtccaaaaa gggcttcaag gcccacttct tctcagaaaa gaggccagct ctgcagcccc 2160
   ctcggggacg cccccaccag ctcaaattcc gagtgcagaa aagaaaccgg accccccagt 2220
   gaggcctgcc aggcctcccg gaccccttgt tactcaggaa cctcaccttg gacggaatgg 2280
   gatgggggct tcggtgccca ccaacccccc acctccactc tgccattccg gcccacctcc 2340
   ctctggccgg acagaactgg tgctctcttc tccccactgt gcccgtccgc ggaccgggga 2400
   cccttccccg tgccctaccc cctcccattt tgatggtgtc tgtgacattt cctgttgtga 2460
   agtaaaagag ggacccctgc gtcctgc 2487

<210> 101
<211> 1862
<212> DNA
<213> Homo sapiens

<220>
<223> II.101 (NM_000960)

<400> 101
agcaagtgaa ggcacagacg cacgggacag gagagcctgg gcaagactgg agagcccaga 60

cctgggatgg cggattcgtg caggaacctc acctacgtgc ggggctcggt ggggccggcc 120

accagcaccc tgatgttcgt ggccggtgtg gtgggcaacg ggctggccct gggcatcctg 180

agcgcacggc gaccggcgcg cccctcggcc ttcgcggtgc tggtcaccgg actggcggcc 240

accgacctgc tgggcaccag cttcctgagc ccggccgtgt tcgtggccta tgcgcgcaac 300

agctccctgc tgggcctggc ccgaggcggc cccgccctgt gcgatgcctt cgccttcgcc 360

atgaccttct tcggcctggc gtccatgctc atcctctttg ccatggccgt ggagcgctgc 420

ctggcgctga gccaccccta cctctacgcg cagctggacg ggccccgctg cgcccgcctg 480

gcgctgccag ccatctacgc cttctgcgtc ctcttctgcg cgctgcccct gctgggcctg 540

ggccaacacc agcagtactg ccccggcagc tggtgcttcc tccgcatgcg ctgggcccag 600

ccgggcggcg ccgccttctc gctggcctac gccggcctgg tggccctgct ggtggctgcc 660

atcttcctct gcaacggctc ggtcaccctc agcctctgcc gcatgtaccg ccagcagaag 720

cgccaccagg gctctctggg tccacggccg cgcaccggag aggacgaggt ggaccacctg 780

atcctgctgg ccctcatgac agtggtcatg gccgtgtgct ccctgcctct cacgatccgc 840

tgcttcaccc aggctgtcgc ccctgacagc agcagtgaga tgggggacct ccttgccttc 900

cgcttctacg ccttcaaccc catcctggac ccctgggtct tcatcctttt ccgcaaggct 960

gtcttccagc gactcaagct ctgggtctgc tgcctgtgcc tcgggcctgc ccacggagac 1020

tcgcagacac ccctttccca gctcgcctcc gggaggaggg acccaagggc cccctctgct 1080

cctgtgggaa aggaggggag ctgcgtgcct ttgtcggctt ggggcgaggg gcaggtggag 1140

cccttgcctc ccacacagca gtccagcggc agcgccgtgg gaacgtcgtc caaagcagaa 1200
gccagcgtcg cctgctccct ctgctgacat ttcaagctga ccctgtgatc tctgccctgt 1260
cttcgggcga caggagccag aaaatcaggg acatggctga tggctgcgga tgctggaacc 1320
ttggccccca aactctgggg ccgatcagct gctgtttctc ctgcggcagg gcagtcgctg 1380
ctggctctgg gaagagagtg agggacagag gaaacgttta tcctggagtg cagaaagaat 1440
ggttctctca aaataaccag tggcctggcc gacctgctct ggccctggat tccccatcca 1500
tctcattgtc taaatattta gaaggcggag aagttcccag aggcttctgt acagtcaggt 1560
ctgctctggt ctgggtgctg gctccaatct gcgtccactt aggaggccca actgcccacc 1620
ccaagtcccc aggggatggc cctccccctc taccaagcca ctccaagagc cagccccctt 1680
tctgctccac aaaaaccaca gttattggaa aagctccctg ccttcccttg ccgctggtcc 1740
cccaccaggc ttgggagccc tggcatccca aaggggcaac gggaggaagg ggaggctgct 1800
gcattgtggg tgatgacgta ggacatgtgc ttggtacaaa aagggcctga gacattccac 1860
ct 1862

<210> 102
<211> 2857
<212> DNA
<213> Homo sapiens

<220>
<223> II.102 (XM_012039)

<400> 102
ctgctcctgc gcggcagctg ctttagaagg tctcgagcct cctgtacctt cccagggatg 60
aaccgggcct tccctctgga aggcgagggt tcgggccaca gtgagcgagg gccagggcgg 120
tgggcgcgcg cagagggaaa ccggatcagt tgagagagaa tcaagagtag cggatgaggc 180
gcttgtgggg cgcggcccgg aagccctcgg gcgcgggctg ggagaaggag tgggcggagg 240
cgccgcagga ggctcccggg gcctggtcgg gccggctggg ccccgggcgc agtggaagaa 300
agggacgggc ggtgcccggt tgggcgtcct ggccagctca ccttgccctg gcggctcgcc 360
ccgcccggca cttgggagga gcagggcagg gcccgcggcc tttgcattct gggaccgccc 420
ccttccattc ccgggccagc ggcgagcggc agcgacggct ggagccgcag ctacagcatg 480
agagccggtg ccgctcctcc acgcctgcgg acgcgtggcg agcggaggca gcgctgcctg 540
ttcgcgccat gggggcaccg tggggctcgc cgacggcggc ggcgggcggg cggcgcgggt 600
ggcgccgagg ccgggggctg ccatggaccg tctgtgtgct ggcggccgcc ggcttgacgt 660
gtacggcgct gatcacctac gcttgctggg ggcagctgcc gccgctgccc tgggcgtcgc 720
caaccccgtc gcgaccggtg ggcgtgctgc tgtggtggga gcccttcggg gggcgcgata 780
gcgccccgag gccgccccct gactgccggc tgcgcttcaa catcagcggc tgccgcctgc 840
tcaccgaccg cgcgtcctac ggagaggctc aggccgtgct tttccaccac cgcgacctcg 900
tgaaggggcc ccccgactgg cccccgccct ggggcatcca ggcgcacact gccgaggagg 960

tggatctgcg cgtgttggac tacgaggagg cagcggcggc ggcagaagcc ctggcgacct 1020
ccagccccag gcccccgggc cagcgctggg tttggatgaa cttcgagtcg ccctcgcact 1080
ccccggggct gcgaagcctg gcaagtaacc tcttcaactg gacgctctcc taccgggcgg 1140
actcggacgt ctttgtgcct tatggctacc tctaccccag aagccacccc ggcgacccgc 1200
cctcaggcct ggccccgcca ctgtccagga aacaggggct ggtggcatgg gtggtgagcc 1260
actgggacga gcgccaggcc cgggtccgct actaccacca actgagccaa catgtgaccg 1320
tggacgtgtt cggccggggc gggccggggc agccggtgcc cgaaattggg ctcctgcaca 1380
cagtggcccg ctacaagttc tacctggctt tcgagaactc gcagcacctg gattatatca 1440
ccgagaagct ctggcgcaac gcgttgctcg ctggggcggt gccggtggtg ctgggcccag 1500
accgtgccaa ctacgagcgc tttgtgcccc gcggcgcctt catccacgtg gacgacttcc 1560
caagtgcctc ctccctggcc tcgtacctgc ttttcctcga ccgcaacccc gcggtctatc 1620
gccgctactt ccactggcgc cggagctacg ctgtccacat cacctccttc tgggacgagc 1680
cttggtgccg ggtgtgccag gctgtacaga gggctgggga ccggcccaag agcatacgga 1740
acttggccag ctggttcgag cggtgaagcc gcgctcccct ggaagcgacc caggggaggc 1800
caagttgtca gctttttgat cctctactgt gcatctcctt gactgccgca tcatgggagt 1860
aagttcttca aacacccatt tttgctctat gggaaaaaaa cgatttacca attaatatta 1920
ctcagcacag agatgggggc ccggtttcca tattttttgc acagctagca attgggctcc 1980
ctttgctgct gatgggcatc attgtttagg ggtgaaggag ggggttcttc ctcaccttgt 2040
aaccagtgca gaaatgaaat agcttagcgg caagaagccg ttgaggcggt ttcctgaatt 2100
tccccatctg ccacaggcca tatttgtggc ccgtgcagct tccaaatctc atacacaact 2160
gttcccgatt cacgtttttc tggaccaagg tgaagcaaat ttgtggttgt agaaggagcc 2220
ttgttggtgg agagtggaag gactgtggct gcaggtggga ctttgttgtt tggattcctc 2280
acagccttgg ctcctgagaa aggtgaggag ggcagtccaa gaggggccgc tgacttcttt 2340
cacaagtact atctgttccc ctgtcctgtg aatggaagca aagtgctgga ttgtccttgg 2400
aggaaactta agatgaatac atgcgtgtac ctcactttac ataagaaatg tattcctgaa 2460
aagctgcatt taaatcaagt cccaaattca ttgacttagg ggagttcagt atttaatgaa 2520
accctatgga gaatttatcc ctttacaatg tgaatagtca tctcctaatt tgtttcttct 2580
gtctttatgt ttttctataa cctggatttt ttaaatcata ttaaaattac agatgtgaaa 2640
ataaagcaga agcaaccttt ttccctcttc ccagaaaacc agtctgtgtt tacagacaga 2700
agagaaggaa gccatagtgt cacttccaca caattattta tttcatgtct ttactggacc 2760
tgaaatttaa actgcaatgc cagtcctgca ggagtgctgg cattaccctc tgcagaacag 2820
tgaaaggtat tgcactacat tatggaatca tgcaaaa 2857

<210> 103
<211> 3855

<212>  DNA
<213>  Homo sapiens

<220>
<223>  II.103 (XM_034166)

<400>  103
ctctcggcca cctttgatga ggggactggg cagttctaga cagtcccgaa gttctcaagg      60

cacaggtctc ttcctggttt gactgtcctt accccgggga ggcagtgcag ccagctgcaa     120

gccccacagt gaagaacatc tgagctcaaa tccagataag tgacataagt gacctgcttt     180

gtaaagccat agagatggcc tgtccttgga aatttctgtt caagaccaaa ttccaccagt     240

atgcaatgaa tggggaaaaa gacatcaaca acaatgtgga gaaagccccc tgtgccacct     300

ccagtccagt gacacaggat gaccttcagt atcacaacct cagcaagcag cagaatgagt     360

ccccgcagcc cctcgtggag acgggaaaga agtctccaga atctctggtc aagctggatg     420

caaccccatt gtcctcccca cggcatgtga ggatcaaaaa ctggggcagc gggatgactt     480

tccaagacac acttcaccat aaggccaaag ggattttaac ttgcaggtcc aaatcttgcc     540

tggggtccat tatgactccc aaaagtttga ccagaggacc cagggacaag cctacccctc     600

cagatgagct tctacctcaa gctatcgaat ttgtcaacca atattacggc tccttcaaag     660

aggcaaaaat agaggaacat ctggccaggg tggaagcggt aacaaaggag atagaaacaa     720

caggaaccta ccaactgacg ggagatgagc tcatcttcgc caccaagcag gcctggcgca     780

atgccccacg ctgcattggg aggatccagt ggtccaacct gcaggtcttc gatgcccgca     840

gctgttccac tgcccgggaa atgtttgaac acatctgcag acacgtgcgt tactccacca     900

acaatggcaa catcaggtcg gccatcaccg tgttccccca gcggagtgat ggcaagcacg     960

acttccgggt gtggaatgct cagctcatcc gctatgctgg ctaccagatg ccagatggca    1020

gcatcagagg ggaccctgcc aacgtggaat tcactcagct gtgcatcgac ctgggctgga    1080

agcccaagta cggccgcttc gatgtggtcc ccctggtcct gcaggccaat ggccgtgacc    1140

ctgagctctt cgaaatccca cctgaccttg tgcttgaggt ggccatggaa catcccaaat    1200

acgagtggtt tcgggaactg gagctaaagt ggtacgccct gcctgcagtg gccaacatgc    1260

tgcttgaggt gggcggcctg gagttcccag ggtgcccctt caatggctgg tacatgggca    1320

cagagatcgg agtccgggac ttctgtgacg tccagcgcta caacatcctg gaggaagtgg    1380

gcaggagaat gggcctggaa acgcacaagc tggcctcgct ctggaaagac caggctgtcg    1440

ttgagatcaa cattgctgtg ctccatagtt tccagaagca gaatgtgacc atcatggacc    1500

accactcggc tgcagaatcc ttcatgaagt acatgcagaa tgaataccgg tcccgtgggg    1560

gctgcccggc agactggatt tggctggtcc ctcccatgtc tgggagcatc acccccgtgt    1620

ttcaccagga gatgctgaac tacgtcctgt cccctttcta ctactatcag gtagaggcct    1680

ggaaaaccca tgtctggcag gacgagaagc ggagacccaa gagaagagag attccattga    1740

aagtcttggt caaagctgtg ctctttgcct gtatgctgat gcgcaagaca atggcgtccc    1800

gagtcagagt caccatcctc tttgcgacag agacaggaaa atcagaggcg ctggcctggg 1860
acctgggggc cttattcagc tgtgccttca accccaaggt tgtctgcatg gataagtaca 1920
ggctgagctg cctggaggag gaacggctgc tgttggtggt gaccagtacg tttggcaatg 1980
gagactgccc tggcaatgga gagaaactga agaaatcgct cttcatgctg aaagagctca 2040
acaacaaatt caggtacgct gtgtttggcc tcggctccag catgtaccct cggttctgcg 2100
cctttgctca tgacattgat cagaagctgt cccacctggg ggcctctcag ctcaccccga 2160
tgggagaagg ggatgagctc agtgggcagg aggacgcctt ccgcagctgg gccgtgcaaa 2220
ccttcaaggc agcctgtgag acgtttgatg tccgaggcaa acagcacatt cagatcccca 2280
agctctacac ctccaatgtg acctgggacc cgcaccacta caggctcgtg caggactcac 2340
agcctttgga cctcagcaaa gccctcagca gcatgcatgc caagaacgtg ttcaccatga 2400
ggctcaaatc tcggcagaat ctacaaagtc cgacatccag ccgtgccacc atcctggtgg 2460
aactctcctg tgaggatggc caaggcctga actacctgcc gggggagcac cttggggttt 2520
gcccaggcaa ccagccggcc ctggtccaag gtatcctgga gcgagtggtg gatggcccca 2580
caccccacca gacagtgcgc ctggaggccc tggatgagag tggcagctac tgggtcagtg 2640
acaagaggct gcccccctgc tcactcagcc aggccctcac ctacttcctg gacatcacca 2700
cacccccaac ccagctgctg ctccaaaagc tggcccaggt ggccacagaa gagcctgaga 2760
gacagaggct ggaggccctg tgccagccct cagagtacag caagtggaag ttcaccaaca 2820
gccccacatt cctggaggtg ctagaggagt tcccgtccct gcgggtgtct gctggcttcc 2880
tgctttccca gctccccatt ctgaagccca ggttctactc catcagctcc tcccgggatc 2940
acacgcccac agagatccac ctgactgtgg ccgtggtcac ctaccacacc cgagatggcc 3000
agggtcccct gcaccacggc gtctgcagca catggctcaa cagcctgaag ccccaagacc 3060
cagtgccctg ctttgtgcgg aatgccagcg gcttccacct ccccgaggat ccctcccatc 3120
cttgcatcct catcgggcct ggcacaggca tcgcgccctt ccgcagtttc tggcagcaac 3180
ggctccatga ctcccagcac aagggagtgc ggggaggccg catgaccttg gtgtttgggt 3240
gccgccgccc agatgaggac cacatctacc aggaggagat gctggagatg gcccagaagg 3300
gggtgctgca tgcggtgcac acagcctatt cccgcctgcc tggcaagccc aaggtctatg 3360
ttcaggacat cctgcggcag cagctggcca gcgaggtgct ccgtgtgctc cacaaggagc 3420
caggccacct ctatgtttgc ggggatgtgc gcatggcccg ggacgtggcc cacaccctga 3480
agcagctggt ggctgccaag ctgaaattga atgaggagca ggtcgaggac tatttctttc 3540
agctcaagag ccagaagcgc tatcacgaag atatctttgg tgctgtattt ccttacgagg 3600
cgaagaagga cagggtggcg gtgcagccca gcagcctgga gatgtcagcg ctctgagggc 3660
ctacaggagg ggttaaagct gccggcacag aacttaagga tggagccagc tctgcattat 3720
ctgaggtcac agggcctggg gagatggagg aaagtgatat cccccagcct caagtcttat 3780
ttcctcaacg ttgctcccca tcaagccctt tacttgacct cctaacaagt agcaccctgg 3840

attgatcgga gcctc                                                    3855

<210>  104
<211>  2912
<212>  DNA
<213>  Homo sapiens

<220>
<223>  II.104 (NM_003188)

<400>  104
tccctccttt tctttccaag tcacaaaatt ctcccctccc ctaccccgga gtttacggcc      60

ctcctcctgt ttccgatttc agcccggaac cggaagtgta gtgggcgggg cccgtcggcg     120

gaaaacgcag cggagccaga gccggacacg gctgtggccg ctgcctctac ccccgccacg     180

gatcgccggg tagtaggact gcgcggctcc aggctgaggg tcggtccgga ggcgggtggg     240

cgcgggtctc acccggattg tccgggtggc accgttcccg gccccaccgg gcgccgcgag     300

ggatcatgtc tacagcctct gccgcctcct cctcctcctc gtcttcggcc ggtgagatga     360

tcgaagcccc ttcccaggtc ctcaactttg aagagatcga ctacaaggag atcgaggtgg     420

aagaggttgt tggaagagga gcctttggag ttgtttgcaa agctaagtgg agagcaaaag     480

atgttgctat taaacaaata gaaagtgaat ctgagaggaa agcgtttatt gtagagcttc     540

ggcagttatc ccgtgtgaac catcctaata ttgtaaagct ttatggagcc tgcttgaatc     600

cagtgtgtct tgtgatggaa tatgctgaag ggggctcttt atataatgtg ctgcatggtg     660

ctgaaccatt gccatattat actgctgccc acgcaatgag ttggtgttta cagtgttccc     720

aaggagtggc ttatcttcac agcatgcaac ccaaagcgct aattcacagg gacctgaaac     780

caccaaactt actgctggtt gcagggggga cagttctaaa aatttgtgat tttggtacag     840

cctgtgacat tcagacacac atgaccaata acaaggggag tgctgcttgg atggcacctg     900

aagtttttga aggtagtaat tacagtgaaa aatgtgacgt cttcagctgg ggtattattc     960

tttgggaagt gataacgcgt cggaaaccct ttgatgagat tggtggccca gctttccgaa    1020

tcatgtgggc tgttcataat ggtactcgac caccactgat aaaaaattta cctaagccca    1080

ttgagagcct gatgactcgt tgttggtcta aagatccttc ccagcgccct tcaatggagg    1140

aaattgtgaa aataatgact cacttgatgc ggtactttcc aggagcagat gagccattac    1200

agtatccttg tcagtattca gatgaaggac agagcaactc tgccaccagt acaggctcat    1260

tcatggacat tgcttctaca aatacgagta acaaaagtga cactaatatg gagcaagttc    1320

ctgccacaaa tgatactatt aagcgcttag aatcaaaatt gttgaaaaat caggcaaagc    1380

aacagagtga atctggacgt ttaagcttgg gagcctcccg tgggagcagt gtggagagct    1440

tgcccccaac ctctgagggc aagaggatga gtgctgacat gtctgaaata gaagctagga    1500

tcgccgcaac cacaggcaac ggacagccaa gacgtagatc catccaagac ttgactgtaa    1560

ctggaacaga acctggtcag gtgagcagta ggtcatccag tcccagtgtc agaatgatta    1620

ctacctcagg accaacctca gaaaagccaa ctcgaagtca tccatggacc cctgatgatt    1680

ccacagatac caatggatca gataactcca tcccaatggc ttatcttaca ctggatcacc 1740

aactacagcc tctagcaccg tgcccaaact ccaaagaatc tatggcagtg tttgaacagc 1800

attgtaaaat ggcacaagaa tatatgaaag ttcaaacaga aattgcattg ttattacaga 1860

gaaagcaaga actagttgca gaactggacc aggatgaaaa ggaccagcaa aatacatctc 1920

gcctggtaca ggaacataaa aagcttttag atgaaaacaa aagcctttct acttactacc 1980

agcaatgcaa aaaacaacta gaggtcatca gaagtcagca gcagaaacga caaggcactt 2040

catgattctc tgggaccgtt acattttgaa atatgcaaag aaagactttt tttttaagga 2100

aaggaaaacc ttataatgac gattcatgag tgttagcttt ttggcgtgtt ctgaatgcca 2160

actgcctata tttgctgcat ttttttcatt gtttattttc cttttctcat ggtggacata 2220

caattttact gtttcattgc ataacatggt agcatctgtg acttgaatga gcagcacttt 2280

gcaacttcaa aacagatgca gtgaactgtg gctgtatatg catgctcatt gtgtgaaggc 2340

tagcctaaca gaacaggagg tatcaaacta gctgctatgt gcaaacagcg tccatttttt 2400

catattagag gtggaacctc aagaatgact ttattcttgt atctcatctc aaaatattaa 2460

taattttttt cccaaaagat ggtatatacc aagttaaaga cagggtatta taaatttaga 2520

gtgattggtg gtatattacg gaaatacgga acctttaggg atagttccgt gtaagggctt 2580

tgatgccagc atccttggat cagtactgaa ctcagttcca tccgtaaaat atgtaaaggt 2640

aagtggcagc tgctctattt aatgaaagca gttttaccgg attttgttag actaaaattt 2700

gattgtgata cattgaacaa aatggaactc attttttttt aaggagtaaa gatttttaat 2760

tctgtgattg tgtgtatgtg tgttgaaact gtaaagcttt tatgactcta atattaatct 2820

cttaaatgaa attaaaaggc aaaagaacat gattgagctt aaatgatcat ttcttcctgc 2880

agtgattctt ggattgtttt ctcatgtatt tg 2912

<210> 105
<211> 3336
<212> DNA
<213> Homo sapiens


<220>
<223> II.105 (NM_006609)

<400> 105
ttttattaat atgctgatct gttttatctt ctcacggcca taaagaatgc tgatgggaga 60

accattttcc taattttcaa attgttgagc tgtttgccat aatggatgat cagcaagctt 120

tgaactcaat catgcaagat ttggctgtcc ttcataaggc cagtcgacca gcattatcct 180

tgcaggaaac cagaaaagca aaatcttcat caccaaaaaa acagaatgat gtccgagtca 240

aatttgaaca tagaggagaa aaaagaatcc ttcagttccc cagaccagtt aaactggaag 300

atctgagatc taaagctaaa attgcctttg gacagtctat ggatctacat tataccaata 360

acgagttggt aattccatta actactcaag atgacttgga caaagctctg gaactgctgg 420

atcgtagtat tcatatgaag agcctcaaga tattacttgt aataaatgga agtacacagg 480
ctactaattt agaaccattg ccatcactag aagatttgga taatacagta tttggagcag 540
agaggaaaaa acggctatct ataataggtc ctactagtag agatagaagt tctcctcccc 600
cgggttacat tccagatgaa ttacaccagg ttgcccggaa tgggtcattc actagtatca 660
acagtgaagg agagttcatt ccagagagca tggaacaaat gctggatcca ttatctttaa 720
gcagccctga aaattctggc tcaggaagtt gtccatcact tgatagtcct ttggatggag 780
agagctatcc aaaatcacga atgcctaggg ctcagagcta cccagataat catcaggaat 840
tttcagacta tgataaccct atctttgaga aatttggaaa aggaggaaca tatccaagaa 900
ggtatcatgt ttcatatcat catcaagagg taataatgat ggtcgtaaaa cttttccaag 960
agctagaagg acccagggga accagcttac ggtctcctgt gagtttcagt cctactgatc 1020
attccttaag cactagtagt ggaagcagta tctttacccc agagtatgat gatagtcgaa 1080
taagaagaag gggaagtgac atagacaatc ctactttgac agtaatggac atcagcccac 1140
ccagccgttc acctcgagct ccgaccaact ggagattggg caaactgctt ggccaaggag 1200
cctttggaag ggtctacctc tgttatgatg ttgatacagg aagagaattg gctgttaagc 1260
aagttcaatt tgaccccgat agtcctgaga ccagcaagga agtaaatgca cttgagtgtg 1320
aaattcagtt gctgaaaaac ttgctacatg agcgaattgt tcagtattat ggctgtttga 1380
gggatcccca ggaaaaaaca ctttccatat ttatggaata tatgccaggg ggttcaatta 1440
aggaccaatt aaaagcatat ggcgctctta ctgagaatgt gactaggaaa tacacccgtc 1500
agattctgga gggtgtccat tatttgcaca gtaatatgat tgtcctacga gatatcaaag 1560
gcgcaaatat cctgcgagat tcaacaggca acgtcaaact aggagatttt ggggccagca 1620
aacggcttca gaccatctgt ctctcaggga caggaatgaa gtctgtcacg ggcacaccat 1680
actggatgag ccctgaagtc atcagtggag aaggctatgg aagaaaagca gacatctgga 1740
gtgttgcatg tactgtggta gaaatgctaa ctgaaaagcc gccttgggct gaatttgaag 1800
caatggctgc catctttaaa atcgccactc agccaacaaa cccaaagctg ccacctcatg 1860
tctcagacta tactcgagat ttcctcaaac ggatttttgt agaggccaaa ctgagacctt 1920
cagctgatga actcttaagg cacatgtttg tgcattatca ctagcagcca gtaacctctc 1980
ctgtgcctct acctagctcc catctattca ttcaccttct ctctgactgc acttttcttt 2040
tttataaaaa aagagagatg ggggagaaaa aagacaagag ggaaagtatt tctcttgatt 2100
cttggttaaa tttgtttaat aataataata tcctaaattt tttatattta atcttttttt 2160
cccttacaag aacttgaagt ttttttttta atttttataa tgtactgatg tggttcagag 2220
agataaagca ctttagtaca tagtcactct ttttagtaca aacaaatcat ttggaatacc 2280
taaagattgt agagtcattc cctctatcac tgacacatca gtgacgatgg gaagacatgg 2340
aaaacaagga gaagaaaatg atgtataatt tgtagttttt agtgatagta tttaaaatat 2400
atcctcattt gtggggttga gccctaaact ttagtttagg gtaggtactc aacttaaaga 2460

atataggttt cttcttatat ctgtattctt tagatcctaa cctctgtcta ccaacctttt 2520

tgctcagtag gagtcttgat agaagatatg aatctctgag aggtatgttt atttgttaat 2580

cctaaccagt ataataagca aatacactat aatagatcca cgttactgga atctgtaaac 2640

cttgagggat agctttctgc ttaaaaacac acacacacac acacacacac ggaaaacctt 2700

tattttaaag tcaagttgtg agcaaataga aataaaagac aaaaggacat cactcttatc 2760

aaatgtgtga gcagtagaag agaccacatt tacagtcaat agaaataatg aaaaaaaatt 2820

aggtgtttag tgtattttaa acagttttgt tttgttttac ttgaggggga cgtcccaaaa 2880

ttaaaggaat ggagaaataa tcaaaatcat gtataccatc ttctatttcc agctcctgat 2940

tccccatagg taacatccct taggagcgaa gagttcaatt agtaatgttt atgtgttatg 3000

tcaggagatg aaacccttgt tcttaggatc acagaatact aaagcacctc aaaaaaacag 3060

gtatcatgtg aaacagtggt tgccaaaagt ggagcgagga tgatttcact aggcatttgg 3120

caattcttag agacatttcc ggttgtcaca attggaggga tactagtagc atgaattggg 3180

tagaggccag ggatgttgct aaatagatta taatacacaa ggaaagcagc ctcaaagaat 3240

tacccctccc aaaatgtcag aagtgctgag gctgagaaac cctgatgtaa agatcagtcc 3300

cagttataaa ctgaaaacag ctatttacaa agcagt 3336

<210> 106
<211> 1552
<212> DNA
<213> Homo sapiens

<220>
<223> II.106 (XM_027358)

<400> 106
   actcttggga aaactgctgg gcaccgtcgt cgcgctgaag gtggttctgt acctgctccg 60
   agtgtgctta gcgatggcct ggaaatccgg cggcgccagc cactcggagc taatccacaa 120
   tctccgcaaa aatggaatca tcaagacaga taaagtattt gaagtgatgc tggctacaga 180
   ccgctcccac tatgcaaaat gtaacccata catggattct ccacaatcaa taggtttcca 240
   agcaacaatc agtgctccac acatgcatgc atatgcgcta gaacttctat ttgatcagtt 300
   gcatgaagga gctaaagctc ttgatgtagg atctggaagt ggaatcctta ctgcatgttt 360
   tgcacgtatg gttggatgta ctggaaaagt cataggaatt gatcacatta aagagctagt 420
   agatgactca gtaaataatg tcaggaagga cgatccaaca cttctgtctt cagggagagt 480
   acagcttgtt gtgggggatg gaagaatggg atatgctgaa gaagcccctt atgatgccat 540
   tcatgtggga gctgcagccc ctgttgtacc ccaggcgcta atagatcagt taaagcccgg 600
   aggaagattg atattgcctg ttggtcctgc aggcggaaac caaatgttgg agcagtatga 660
   caagctacaa gatggcagca tcaaaatgaa gcctctgatg ggggtgatat acgtgccttt 720
   aacagataaa gaaaagcagt ggtccaggga tgaattgtaa aagcaacatc agcttgacca 780
   gtataaaatt acagtggatt gctcatctca gtcctcaaag ctttttgaaa accaacacca 840
   tcacagcttg ttttggactt tgttacactg ttattttcag catgaaaatg tgtgtttttt 900
   tagggtttct gattcttcaa agaggcacag agccaaattg gtagaggaag gatgcaaagt 960
   ataaatttgt gtaatattac tttaacatgc ccatatttta cttggaaata ttaaaagaaa 1020
   gggttctgta aaatggaaaa cttagtttgt gaattgattt tgaggagtgg tttttctttt 1080
   cttggacact taattctgtt ctgatattaa tttatcagat tgcttttgtg cattggataa 1140
   caccaccatt cacaagttaa gattcttggt atttggatat ctgttagatg ctactaagaa 1200
   aatagagatg agctttcttt ttaaagcttt tgatgtggtg tcatagaata gcatgttgta 1260
   gatacaatca gctgctttgt taccttaaaa ctaggcattt gtaaatatta aaccataaga 1320
   tggcaggtga tgtcctgtaa acactcagct gttcagattg gacataactg acttagttct 1380
   tcccttctct ctctctcaaa attataggag acttgtagtt tagtgtggtt ttctgtttct 1440
   aatttgctgc tgataatgta tatgaactta acatgctgtg taagctgtgt cctagttctt 1500
   gaacagttta tgcagtgctg ctttgccaaa taaagttaaa agtaaaagag gc 1552

<210> 107
<211> 5722
<212> DNA
<213> Homo sapiens

<220>
<223> II.107 (XM_007189)

<400> 107
gcagccgcca cattcaacag gcagcagcgc agcgggcgcg ccgctgggga gagcaagcgg 60

cccgcggcgt ccgtccgtcc ttccgtccgc ggccctgtca gctggagcgc ggcgcaggct 120

ctgccccggc ccggcggctc tggccggccg tccagtccgt gcggcggacc ccgaggagcc 180

tcgatgtgga tggccccgcg aagttaagtt ctgggctcgc gcttccactc cgccgcgcct 240

tcctcccagt ttccgtccgc tcgccgcacc ggcttcgttc ccccaaatct cggaccgtcc 300

cttcgcgccc cctccccgtc cgcccccagt gctgcgttct ccccctcttg gctctcctgc 360

ggctggggga ggggcggggg tcaccatggc cgaggcgcct caggtggtgg agatcgaccc 420

ggacttcgag ccgctgcccc ggccgcgctc gtgcacctgg ccgctgccca ggccggagtt 480

tagccagtcc aactcggcca cctccagccc ggcgccgtcg ggcagcgcgg ctgccaaccc 540

cgacgccgcg gcgggcctgc cctcggcctc ggctgccgct gtcagcgccg acttcatgag 600

caacctgagc ttgctggagg agagcgagga cttcccgcag gcgcccggct ccgtggcggc 660

ggcggtggcg gcggcggccg ccgcggccgc caccgggggg ctgtgcgggg acttccaggg 720

cccggaggcg ggctgcctgc acccagcgcc accgcagccc ccgccgcccg ggccgctgtc 780

gcagcacccg ccggtgcccc ccgccgccgc tgggccgctc gcggggcagc cgcgcaagag 840

cagctcgtcc cgccgcaacg cgtggggcaa cctgtcctac gccgacctca tcaccaaggc 900

catcgagagc tcggcggaga agcggctcac gctgtcgcag atctacgagt ggatggtcaa 960

gagcgtgccc tacttcaagg ataagggtga cagcaacagc tcggcgggct ggaagaattc 1020
aattcgtcat aatctgtccc tacacagcaa gttcattcgt gtgcagaatg aaggaactgg 1080
aaaaagttct tggtggatgc tcaatccaga gggtggcaag agcgggaaat ctcctaggag 1140
aagagctgca tccatggaca acaacagtaa atttgctaag agccgaagcc gagctgccaa 1200
gaagaaagca tctctccagt ctggccagga gggtgctggg gacagccctg gatcacagtt 1260
ttccaaatgg cctgcaagcc ctggctctca cagcaatgat gactttgata actggagtac 1320
atttcgccct cgaactagct caaatgctag tactattagt gggagactct cacccattat 1380
gaccgaacag gatgatcttg gagaagggga tgtgcattct atggtgtacc cgccatctgc 1440
cgcaaagatg gcctctactt tacccagtct gtctgagata agcaatcccg aaaacatgga 1500
aaatcttttg gataatctca accttctctc atcaccaaca tcattaactg tttcgaccca 1560
gtcctcacct ggcaccatga tgcagcagac gccgtgctac tcgtttgcgc caccaaacac 1620
cagtttgaat tcacccagcc caaactacca aaaatataca tatggccaat ccagcatgag 1680
ccctttgccc cagatgccta tacaaacact tcaggacaat aagtcgagtt atggaggtat 1740
gagtcagtat aactgtgcgc ctggactctt gaaggagttg ctgacttctg actctcctcc 1800
ccataatgac attatgacac cagttgatcc tggggtagcc cagcccaaca gccgggttct 1860
gggccagaac gtcatgatgg gccctaattc ggtcatgtca acctatggca gccaggcatc 1920
tcataacaaa atgatgaatc ccagctccca tacccaccct ggacatgctc agcagacatc 1980
tgcagttaac gggcgtcccc tgccccacac ggtaagcacc atgccccaca cctcgggtat 2040
gaaccgcctg acccaagtga agacacctgt acaagtgcct ctgccccacc ccatgcagat 2100
gagtgccctg gggggctact cctccgtgag cagctgcaat ggctatggca gaatgggcct 2160
tctccaccag gagaagctcc caagtgactt ggatggcatg ttcattgagc gcttagactg 2220
tgacatggaa tccatcattc ggaatgacct catggatgga gatacattgg attttaactt 2280
tgacaatgtg ttgcccaacc aaagcttccc acacagtgtc aagacaacga cacatagctg 2340
ggtgtcaggc tgagggttag tgagcaggtt acacttaaaa gtacttcaga ttgtctgaca 2400
gcaggaactg agagaagcag tccaaagatg tctttcacca actccctttt agttttcttg 2460
gttaaaaaaa aaaacaaaaa aaaaaaccct ccttttttcc tttcgtcaga cttggcagca 2520
aagacatttt tcctgtacag gatgtttgcc caatgtgtgc aggttatgtg ctgctgtaga 2580
taaggactgt gccattggaa atttcattac aatgaagtgc caaactcact acaccatata 2640
attgcagaaa agattttcag atcctggtgt gctttcaagt tttgtatata agcagtagat 2700
acagattgta tttgtgtgtg tttttggttt ttctaaatat ccaattggtc caaggaaagt 2760
ttatactctt tttgtaatac tgtgatgggc ctcatgtctt gataagttaa acttttgttt 2820
gtactacctg ttttctgcgg aactgacgga tcacaaagaa ctgaatctcc attctgcatc 2880
tccattgaac agccttggac ctgttcacgt tgccacagaa ttcacatgag aaccaagtag 2940
cctgttatca atctgctaaa ttaatggact tgttaaactt ttggaaaaaa aaagattaaa 3000

tgccagcttt gtacaggtct tttctatttt tttttgttta ttttgttatt tgcaaatttg 3060
tacaaacatt taaatggttc taatttccag ataaatgatt tttgatgtta ttgttgggac 3120
ttaagaacat ttttggaata gatattgaac tgtaataatg ttttcttaaa actagagtct 3180
actttgttac atagtcagct tgtaaatttt gtggaaccac aggtatttgg ggcagcattc 3240
ataattttca ttttgtattc taactggatt agtactaatt ttatacatgc ttaactggtt 3300
tgtacacttt gggatgctac ttagtgatgt ttctgactaa tcttaaatca ttgtaattag 3360
tacttgcata ttcaacgttt caggccctgg ttgggcagga aagtgatgta tagttatgga 3420
cactttgcgt ttcttattta ggataactta atatgttttt atgtatgtat tttaaagaaa 3480
tttcatctgc ttctactgaa ctatgcgtac tgcatagcat caagtcttct ctagagacct 3540
ctgtagtcct gggaggcctc ataatgtttg tagatcagaa aagggagatc tgcatctaaa 3600
gcaatggtcc tttgtcaaac gagggatttt gatccacttc accattttga gttgagcttt 3660
agcaaaagtt tcccctcata attctttgct cttgtttcag tccaggtgga ggttggtttt 3720
gtagttctgc cttgaggaat tatgtcaaca ctcatacttc atctcattct cccttctgcc 3780
ctgcagatta gattacttag cacactgtgg aagtttaagt ggaaggaggg aatttaaaaa 3840
tgggacttga gtggtttgta gaatttgtgt tcataagttc agatgggtag caaatggaat 3900
agaacttact taaaaattgg ggagatttat ttgaaaacca gctgtaagtt gtgcattgag 3960
attatgttaa aagccttggc ttaagaattt gaaaatttct ttagcctgta gcaacctaaa 4020
ctgtaattcc tatcattatg ttttattact ttccaattac ctgtaactga cagaccaaat 4080
taattggctt tgtgtcctat ttagtccatc agtattttca agtcatgtgg aaagcccaaa 4140
gtcatcacaa tgaagagaac aggtgcacag cactgttcct cttgtgttct tgagaaggat 4200
ctaatttttc tgtatatagc ccacatcaca cttgctttgt cttgtatgtt aattgcatct 4260
tcattggctt ggtatttcct aaatgtttaa caagaacaca agtgttcctg ataagatttc 4320
ctacagtaag ccagctctat tgtaagcttc ccactgtgat gatcattttt ttgaagattc 4380
attgaacagc caccactcta tcatcctcat tttggggcag tccaagacat agctggtttt 4440
agaaacccaa gttcctctaa gcacagcctc ccgggtatgt aactgaactt ggtgccaaag 4500
tacttgtgta ctaatttcta ttactacgta ctgtcacttt cctcccgtgc cattactgca 4560
tcataataca aggaacctca gagcccccat ttgttcatta aagaggcaac tacagccaaa 4620
atcactgtta aaatcttact acttcatgga gtagctctta ggaaaatata tcttcctcct 4680
gagtctgggt aattatacct ctcccaagcc cccattgtgt gttgaaatcc tgtcatgaat 4740
ccttggtagc tctctgagaa cagtgaagtc cagggaaagg catctggtct gtctggaaag 4800
caaacattat gtggcctctg gtagtttttt tcctgtaaga atactgactt tctggagtaa 4860
tgagtatata tcagttattg tacatgattg ctttgtgaaa tgtgcaaatg atatcaccta 4920
tgcagccttg tttgatttat tttctctggt ttgtactgtt attaaaagca tattgtatta 4980
tagagctatt cagatatttt aaatataaag atgtattgtt tccgtaatat agacgtatgg 5040

aatatattta ggtaatagat gtattacttg gaaagttctg ctttgacaaa ctgacaaagt 5100

ctaaatgagc acatgtatcc cagtgagcag taaatcaatg gaacatccca agaagaggat 5160

aaggatgctt aaaatggaaa tcattctcca acgatataca aattggactt gttcaactgc 5220

tggatatatg ctaccaataa ccccagcccc aacttaaaat tcttacattc aagctcctaa 5280

gagttcttaa tttataacta attttaaaag agaagtttct tttctggttt tagtttggga 5340

ataatcattc attaaaaaaa atgtattgtg gtttatgcga acagaccaac ctggcattac 5400

agttggcctc tccttgaggt gggcacagcc tggcagtgtg gccaggggtg gccatgtaag 5460

tcccatcagg acgtagtcat gcctcctgca tttcgctacc cgagtttagt aacagtgcag 5520

attccacgtt cttgttccga tactctgaga agtgcctgat gttgatgtac ttacagacac 5580

aagaacaatc tttgctataa ttgtataaag ccataaatgt acataaatta tgtttaaatg 5640

gcttggtgtc tttcttttct aattatgcag aataagctct ttattaggaa ttttttgtga 5700

agctattaaa tacttgagtt aa 5722

<210> 108
<211> 2769
<212> DNA
<213> Homo sapiens

<220>
<223> II.108 (XM_030523)

<400> 108
ggatcccagt ggcccggcgt gctcggctcc cacaggcctg cagccagcat cgcaccgaac 60

cttcgggggg ccgcggctgg agcgctcggc cggcgtggga gcgccaaggc cgcagatgca 120

atcttcttac cgcgaagaag ccaggggaat aggtagccac atcttgtttg cagataagaa 180

aggaagctaa cgcagtatct gcaaagccag gagtctgact cagtactttt ctcactcatg 240

catacaaagc agctaaaaat gacacagctt atttaccatg cccctgacac tgcactgagc 300

actttatgag cttgaactct gttaatcctc acgaccacct catgagactc tccagaaaga 360

gcaacagtaa tggagtacat gagcactgga agtgacaata aagaagagat tgatttatta 420

attaaacatt taaatgtgtc tgatgtaata gacattatgg aaaatcttta tgcaagtgaa 480

gagccagcag tttatgaacc cagtctaatg accatgtgtc aagacagtaa tcaaaacgat 540

gagcgttcta agtctctgct gcttagtggc caagaggtac catggttgtc atcagtcaga 600

tatggaactg tggaggattt gcttgctttt gcaaaccata tatccaacac tgcaaagcat 660

ttttatggac aacgaccaca ggaatctgga attttattaa acatggtcat cactccccaa 720

aatggacgtt accaaataga ttccgatgtt ctcctgatcc cctggaagct gacttacagg 780

aatattggtt ctgattttat tcctcggggc gcctttggaa aggtatactt ggcacaagat 840

ataaagacga agaaaagaat ggcgtgtaaa ctgatcccag tagatcaatt taagccatct 900

gatgtggaaa tccaggcttg cttccggcac gagaacatcg cagagctgta tggcgcagtc 960

ctgtggggtg aaactgtcca tctctttatg gaagcaggcg agggagggtc tgttctggag 1020

aaactggaga gctgtggacc aatgagagaa tttgaaatta tttgggtgac aaagcatgtt 1080
ctcaagggac ttgattttct acactcaaag aaagtgatcc atcatgatat taaacctagc 1140
aacattgttt tcatgtccac aaaagctgtt ttggtggatt ttggcctaag tgttcaaatg 1200
accgaagatg tctattttcc taaggacctc cgaggaacag agatttacat gagcccagag 1260
gtcatcctgt gcaggggcca ttcaaccaaa gcagacatct acagcctggg ggccacgctc 1320
atccacatgc agacgggcac cccaccctgg gtgaagcgct accctcgctc agcctatccc 1380
tcctacctgt acataatcca caagcaagca cctccactgg aagacattgc agatgactgc 1440
agtccaggga tgagagagct gatagaagct tccctggaga gaaaccccaa tcaccgccca 1500
agagccgcag acctactaaa acatgaggcc ctgaacccgc ccagagagga tcagccacgc 1560
tgtcagagtc tggactctgc cctcttggag cgcaagaggc tgctgagtag gaaggagctg 1620
gaacttcctg agaacattgc tgattcttcg tgcacaggaa gcaccgagga atctgagatg 1680
ctcaagaggc aacgctctct ctacatcgac ctcggcgctc tggctggcta cttcaatctt 1740
gttcggggac caccaacgct tgaatatggc tgaaggatgc catgtttgct ctaaattaag 1800
acagcattga tctcctggag gctggttctg ctgcctctac acaggggccc tgtacagtga 1860
atggtgccat tttcgaagga gcagtgtgac ctcctgtgac ccgtgaatgt gcctccaagc 1920
ggccctgtgt gtttgacatg tgaagctatt tgatatgcac caggtctcaa ggttctcatt 1980
tctcaggtga cgtgattcta aggcaggaat ttgagagttc acagaaggat cgtgtctgct 2040
gactgtttca ttcactgtgc actttgctca aaattttaaa aataccaatc acaaggataa 2100
tagagtagcc taaaattact attcttggtt cttatttaag tatggaatat tcattttact 2160
cagaatagct gttttgtgta tattggtgta tattatataa ctctttgagc ctttattggt 2220
aaattctggt atacattgaa ttcattataa tttgggtgac tagaacaact tgaagattgt 2280
agcaataagc tggactagtg tcctaaaaat ggctaactga tgaattagaa gccatctgac 2340
agcaggccac tagtgacagt ttcttttgtg ttcctatgga aacattttat actgtacatg 2400
ctatgctgaa gacattcaaa acgtgatgtt ttgaatgtgg ataaaactgt gtaaaccaca 2460
taatttttgt acatcccaaa ggatgagaat gtgaccttta agaaaaatga aaacttttgt 2520
aaattattga tgattttgta attcttatga ctaaattttc ttttaagcat ttgtatatta 2580
aaatagcata ctgtgtatgt tttatatcaa atgccttcat gaatctttca tacatatata 2640
tatttgtaac attgtaaagt atgtgagtag tcttatgtaa agtatgtttt tacattatgc 2700
aaataaaacc caatactttt gtccaatgtg gttggtcaaa tcaactgaat aaattcagta 2760
ttttgcctt 2769

<210> 109
<211> 2273
<212> DNA
<213> Homo sapiens

<220>
<223>  II.109 (XM_002923)

<400>  109
caggactgcc tgagacaagc cacaagctga acagagaaag tggattgaac aaggacgcat      60
ttccccagta catccacaac atgctgtcca catctcgttc tcggtttatc agaaatacca     120
acgagagcgg tgaagaagtc accacctttt ttgattatga ttacggtgct ccctgtcata     180
aatttgacgt gaagcaaatt ggggcccaac tcctgcctcc gctctactcg ctggtgttca     240
tctttggttt tgtgggcaac atgctggtcg tcctcatctt aataaactgc aaaaagctga     300
agtgcttgac tgacatttac ctgctcaacc tggccatctc tgatctgctt tttcttatta     360
ctctcccatt gtgggctcac tctgctgcaa atgagtgggt ctttgggaat gcaatgtgca     420
aattattcac agggctgtat cacatcggtt attttggcgg aatcttcttc atcatcctcc     480
tgacaatcga tagatacctg gctattgtcc atgctgtgtt tgctttaaaa gccaggacgg     540
tcacctttgg ggtggtgaca agtgtgatca cctggttggt ggctgtgttt gcttctgtcc     600
caggaatcat ctttactaaa tgccagaaag aagattctgt ttatgtctgt ggcccttatt     660
ttccacgagg atggaataat ttccacacaa taatgaggaa cattttgggg ctggtcctgc     720
cgctgctcat catggtcatc tgctactcgg gaatcctgaa aaccctgctt cggtgtcgaa     780
acgagaagaa gaggcatagg gcagtgagag tcatcttcac catcatgatt gtttactttc     840
tcttctggac tccctataac attgtcattc tcctgaacac cttccaggaa ttcttcggcc     900
tgagtaactg tgaaagcacc agtcaactgg accaagccac gcaggtgaca gagactcttg     960
ggatgactca ctgctgcatc aatcccatca tctatgcctt cgttggggag aagttcagaa    1020
gcctttttca catagctctt ggctgtagga ttgccccact ccaaaaacca gtgtgtggag    1080
gtccaggagt gagaccagga aagaatgtga aagtgactac acaaggactc ctcgatggtc    1140
gtggaaaagg aaagtcaatt ggcagagccc ctgaagccag tcttcaggac aaagaaggag    1200
cctagagaca gaaatgacag atctctgctt tggaaatcac acgtctggct tcacagatgt    1260
gtgattcaca gtgtgaatct tggtgtctac gttaccaggc aggaaggctg agaggagaga    1320
gactccagct gggttggaaa acagtatttt ccaaactacc ttccagttcc tcatttttga    1380
atacaggcat agagttcaga ctttttttaa atagtaaaaa taaaattaaa gctgaaaact    1440
gcaacttgta aatgtggtaa agagttagtt tgagttacta tcatgtcaaa cgtgaaaatg    1500
ctgtattagt cacagagata attctagctt tgagcttaag aattttgagc aggtggtatg    1560
tttgggagac tgctgagtca acccaatagt tgttgattgg caggagttgg aagtgtgtga    1620
tctgtgggca cattagccta tgtgcatgca gcatctaagt aatgatgtcg tttgaatcac    1680
agtatacgct ccatcgctgt catctcagct ggatctccat tctctcaggc ttgctgccaa    1740
aagccttttg tgttttgttt tgtatcatta tgaagtcatg cgtttaatca cattcgagtg    1800
tttcagtgct tcgcagatgt ccttgatgct catattgttc cctattttgc cagtgggaac    1860
tcctaaatca aattggcttc taatcaaagc ttttaaaccc tattggtaaa gaatggaagg    1920

tggagaagct ccctgaagta agcaaagact ttcctcttag tcgagccaag ttaagaatgt 1980

tcttatgttg cccagtgtgt ttctgatctg atgcaagcaa gaaacactgg gcttctagaa 2040

ccaggcaact tgggaactag actcccaagc tggactatgg ctctactttc aggccacatg 2100

gctaaagaag gtttcagaaa gaagtgggga cagagcagaa ctttcacctt catatatttg 2160

tatgatccta atgaatgcat aaaatgttaa gttgatggtg atgaaatgta aatactgttt 2220

ttaacaacta tgatttggaa aataaatcaa tgctataact atgttgataa aag 2273

<210> 110
<211> 3674
<212> DNA
<213> Homo sapiens

<220>
<223> II.110 (XM_054837)

<400> 110
gcgagcgcag cggagcctgg agagaaggcg ctgggctgcg agggcgcgag ggcgcgaggg 60

cagggggcaa ccggaccccg cccgcaccca tggcgcccgt cgccgtctgg gccgcgctgg 120

ccgtcggact ggagctctgg gctgcggcgc acgccttgcc cgcccaggtg gcatttacac 180

cctacgcccc ggagcccggg agcacatgcc ggctcagaga atactatgac cagacagctc 240

agatgtgctg cagcaaatgc tcgccgggcc aacatgcaaa agtcttctgt accaagacct 300

cggacaccgt gtgtgactcc tgtgaggaca gcacatacac ccagctctgg aactgggttc 360

ccgagtgctt gagctgtggc tcccgctgta gctctgacca ggtggaaact caagcctgca 420

ctcgggaaca gaaccgcatc tgcacctgca ggcccggctg gtactgcgcg ctgagcaagc 480

aggaggggtg ccggctgtgc gcgccgctgc gcaagtgccg cccgggcttc ggcgtggcca 540

gaccaggaac tgaaacatca gacgtggtgt gcaagccctg tgccccgggg acgttctcca 600

acacgacttc atccacggat atttgcaggc cccaccagat ctgtaacgtg gtggccatcc 660

ctgggaatgc aagcatggat gcagtctgca cgtccacgtc ccccacccgg agtatggccc 720

caggggcagt acacttaccc cagccagtgt ccacacgatc ccaacacacg cagccaactc 780

cagaacccag cactgctcca agcacctcct tcctgctccc aatgggcccc agccccccag 840

ctgaagggag cactggcgac ttcgctcttc cagttggact gattgtgggt gtgacagcct 900

tgggtctact aataatagga gtggtgaact gtgtcatcat gacccaggtg aaaaagaagc 960

ccttgtgcct gcagagagaa gccaaggtgc ctcacttgcc tgccgataag gcccggggta 1020

cacagggccc cgagcagcag cacctgctga tcacagcgcc gagctccagc agcagctccc 1080

tggagagctc ggccagtgcg ttggacagaa gggcgcccac tcggaaccag ccacaggcac 1140

caggcgtgga ggccagtggg gccggggagg cccgggccag caccgggagc tcagattctt 1200

cccctggtgg ccatgggacc caggtcaatg tcacctgcat cgtgaacgtc tgtagcagct 1260

ctgaccacag ctcacagtgc tcctcccaag ccagctccac aatgggagac acagattcca 1320

gcccctcgga gtccccgaag gacgagcagg tccccttctc caaggaggaa tgtgcctttc 1380

ggtcacagct ggagacgcca gagaccctgc tggggagcac cgaagagaag cccctgcccc 1440
ttggagtgcc tgatgctggg atgaagccca gttaaccagg ccggtgtggg ctgtgtcgta 1500
gccaaggtgg gctgagccct ggcaggatga ccctgcgaag gggccctggt ccttccaggc 1560
ccccaccact aggactctga ggctctttct gggccaagtt cctctagtgc cctccacagc 1620
cgcagcctcc ctctgacctg caggccaaga gcagaggcag cgagttgtgg aaagcctctg 1680
ctgccatggc gtgtccctct cggaaggctg gctgggcatg gacgttcggg gcatgctggg 1740
gcaagtccct gactctctgt gacctgcccc gcccagctgc acctgccagc ctggcttctg 1800
gagcccttgg gttttttgtt tgtttgtttg tttgtttgtt tgtttctccc cctgggctct 1860
gccccagctc tggcttccag aaaaccccag catccttttc tgcagagggg ctttctggag 1920
aggagggatg ctgcctgagt cacccatgaa gacaggacag tgcttcagcc tgaggctgag 1980
actgcgggat ggtcctgggg ctctgtgcag ggaggaggtg gcagccctgt agggaacggg 2040
gtccttcaag ttagctcagg aggcttggaa agcatcacct caggccaggt gcagtggctc 2100
acgcctatga tcccagcact ttgggaggct gaggcgggtg gatcacctga ggttaggagt 2160
tcgagaccag cctggccaac atggtaaaac cccatctcta ctaaaaatac agaaattagc 2220
cgggcgtggt ggcgggcacc tatagtccca gctactcaga agcctgaggc tgggaaatcg 2280
tttgaacccg ggaagcggag gttgcaggga gccgagatca cgccactgca ctccagcctg 2340
ggcgacagag cgagagtctg tctcaaaaga aaaaaaaaag caccgcctcc aaatgccaac 2400
ttgtcctttt gtaccatggt gtgaaagtca gatgcccaga gggcccaggc aggccaccat 2460
attcagtgct gtggcctggg caagataacg cacttctaac tagaaatctg ccaatttttt 2520
aaaaaagtaa gtaccactca ggccaacaag ccaacgacaa agccaaactc tgccagccac 2580
atccaacccc ccacctgcca tttgcaccct ccgccttcac tccggtgtgc ctgcagcccc 2640
gcgcctcctt ccttgctgtc ctaggccaca ccatctcctt tcagggaatt tcaggaacta 2700
gagatgactg agtcctcgta gccatctctc tactcctacc tcagcctaga ccctcctcct 2760
cccccagagg ggtgggttcc tcttccccac tccccacctt caattcctgg gccccaaacg 2820
ggctgccctg ccactttggt acatggccag tgtgatccca agtgccagtc ttgtgtctgc 2880
gtctgtgttg cgtgtcgtgg gtgtgtgtag ccaaggtcgg taagttgaat ggcctgcctt 2940
gaagccactg aagctgggat tcctccccat tagagtcagc cttccccctc ccagggccag 3000
ggccctgcag aggggaaacc agtgtagcct tgcccggatt ctgggaggaa gcaggttgag 3060
gggctcctgg aaaggctcag tctcaggagc atggggataa aggagaaggc atgaaattgt 3120
ctagcagagc aggggcaggg tgataaattg ttgataaatt ccactggact tgagcttggc 3180
agctgaacta ttggagggtg ggagagccca gccattacca tggagacaag aagggttttc 3240
caccctggaa tcaagatgtc agactggctg gctgcagtga cgtgcacctg tactcaggag 3300
gctgagggga ggatcactgg agcccaggag tttgaggctg cagcgagcta tgatcgcgcc 3360
actacactcc agcctgagca acagagtgag accctgtctc ttaaagaaaa aaaaagtcag 3420

actgctggga ctggccaggt ttctgcccac attggaccca catgaggaca tgatggagcg 3480

cacctgcccc ctggtggaca gtcctgggag aacctcaggc ttccttggca tcacagggca 3540

gagccgggaa gcgatgaatt tggagactct gtggggcctt ggttcccttg tgtgtgtgtg 3600

ttgatcccaa gacaatgaaa gtttgcactg tatgctggac ggcattcctg cttatcaata 3660

aacctgtttg tttt 3674

<210> 111
<211> 2502
<212> DNA
<213> Homo sapiens

<220>
<223> II.111 (NM_000634)

<400> 111
tattcatcaa gtgccctcta gctgttaagt cactctgatc tctgactgca gctcctactg 60

ttggacacac ctggccggtg cttcagttag atcaaaccat tgctgaaact gaagaggaca 120

tgtcaaatat tacagatcca cagatgtggg attttgatga tctaaatttc actggcatgc 180

cacctgcaga tgaagattac agcccctgta tgctagaaac tgagacactc aacaagtatg 240

ttgtgatcat cgcctatgcc ctagtgttcc tgctgagcct gctgggaaac tccctggtga 300

tgctggtcat cttatacagc agggtcggcc gctccgtcac tgatgtctac ctgctgaacc 360

tggccttggc cgacctactc tttgccctga ccttgcccat ctgggccgcc tccaaggtga 420

atggctggat ttttggcaca ttcctgtgca aggtggtctc actcctgaag gaagtcaact 480

tctacagtgg catcctgctg ttggcctgca tcagtgtgga ccgttacctg gccattgtcc 540

atgccacacg cacactgacc cagaagcgtc acttggtcaa gtttgtttgt cttggctgct 600

ggggactgtc tatgaatctg tccctgccct tcttcctttt ccgccaggct taccatccaa 660

acaattccag tccagtttgc tatgaggtcc tgggaaatga cacagcaaaa tggcggatgg 720

tgttgcggat cctgcctcac acctttggct tcatcgtgcc gctgtttgtc atgctgttct 780

gctatggatt caccctgcgt acactgttta aggcccacat ggggcagaag caccgagcca 840

tgagggtcat ctttgctgtc gtcctcatct tcctgctttg ctggctgccc tacaacctgg 900

tcctgctggc agacaccctc atgaggaccc aggtgatcca ggagagctgt gagcgccgca 960

acaacatcgg ccgggccctg gatgccactg agattctggg atttctccat agctgcctca 1020

accccatcat ctacgccttc atcggccaaa attttcgcca tggattcctc aagatcctgg 1080

ctatgcatgg cctggtcagc aaggagttct tggcacgtca tcgtgttacc tcctacactt 1140

cttcgtctgt caatgtctct tccaacctct gaaaaccatc gatgaaggaa tatctcttct 1200

cagaaggaaa gaataaccaa caccctgagg ttgtgtgtgg aaggtgatct ggctctggac 1260

aggcactatc tgggttttgg ggggacgcta taggatgtgg ggaagttagg aactggtgtc 1320

ttcaggggcc acaccaacct tctgaggagc tgttgaggta cctccaagga ccggcctttg 1380

cacctccatg gaaacgaagc accatcattc ccgttgaacg tcacatcttt aacccactaa 1440
ctggctaatt agcatggcca catctgagcc ccgaatctga cattagatga gagaacaggg 1500
ctgaagctgt gtcctcatga gggctggatg ctctcgttga ccctcacagg agcatctcct 1560
caactctgag tgttaagcgt tgagccacca agctggtggc tctgtgtgct ctgatccgag 1620
ctcagggggg tggttttccc atctcaggtg tgttgcagtg tctgctggag acattgaggc 1680
aggcactgcc aaaacatcaa cctgccagct ggccttgtga ggagctggaa acacatgttc 1740
cccttggggg tggtggatga acaaagagaa agagggtttg gaagccagat ctatgccaca 1800
agaaccccct ttacccccat gaccaacatc gcagacacat gtgctggcca cctgctgagc 1860
cccaagtgga acgagacaag cagcccttag cccttcccct ctgcagcttc caggctggcg 1920
tgcagcatca gcatccctag aaagccatgt gcagccacca gtccattggg caggcagatg 1980
ttcctaataa agcttctgtt ccgtgcttgt ccctgtggaa gtatcttggt tgtgacagag 2040
tcaagggtgt gtgcagcatt gttggctgtt cctgcagtag aatgggggca gcacctccta 2100
agaaggcacc tctctgggtt gaagggcagt gttccctggg gctttaactc ctgctagaac 2160
agtctcttga ggcacagaaa ctcctgttca tgcccatacc cctggccaag gaagatccct 2220
ttgtccacaa gtaaaaggaa atgctcctcc agggagtctc agcttcaccc tgaggtgagc 2280
atcatcttct gggttaggcc ttgcctaggc atagccctgc ctcaagctat gtgagctcac 2340
cagtccctcc ccaaatgctt tccatgagtt gcagtttttt cctagtctgt tttccctcct 2400
tggagacagg gccctgtcgg tttattcact gtatgtcctt ggtgcctgga gcctactaaa 2460
tgctcaataa ataatgatca caggaaaaaa aaaaaaaaaa aa 2502

<210> 112
<211> 1497
<212> DNA
<213> Homo sapiens

<220>
<223> II.112 (NM_004590)

<400> 112
cggaccacca gcaacagaca acatcttcat tcggctctcc ctgaagctgt actgcctcgc 60
tgagaggatg aaggtctccg aggctgccct gtctctcctt gtcctcatcc ttatcattac 120
ttcggcttct cgcagccagc caaaagttcc tgagtgggtg aacaccccat ccacctgctg 180
cctgaagtat tatgagaaag tgttgccaag gagactagtg gtgggataca gaaaggccct 240
caactgtcac ctgccagcaa tcatcttcgt caccaagagg aaccgagaag tctgcaccaa 300
ccccaatgac gactgggtcc aagagtacat caaggatccc aacctacctt tgctgcctac 360
caggaacttg tccacggtta aaattattac agcaaagaat ggtcaacccc agctcctcaa 420
ctcccagtga tgaccaggct ttagtggaag cccttgttta cagaagagag gggtaaacct 480
atgaaaacag gggaagcctt attaggctga aactagccag tcacattgag agaagcagaa 540
caatgatcaa aataaaggag aagtatttcg aatattttct caatcttagg aggaaatacc 600

aaagttaagg gacgtgggca gaggtacgct cttttatttt tatatttata tttttatttt 660

tttgagatag ggtcttactc tgtcacccag gctggagtgc agtggtgtga tcttggctca 720

cttgatcttg gctcactgta acctccacct cccaggctca agtgatcctc ccaccccagc 780

ctcctgagta gctgggacta caggcttgcg ccaccacacc tggctaattt ttgtattttt 840

ggtagagacg gggttctacc atgttgccca ggctggtctc aaactcgtgt gcccaagcaa 900

tccacctgcc tcagccttcc aaaagtgctg ggattacagg cgtgagccac cacatccggc 960

cagtgcactc ttaatacaca gaaaaatata tttcacatcc ttctcctgct ctctttcaat 1020

tcctcacttc acaccagtac acaagccatt ctaaatactt agccagtttc cagccttcca 1080

gatgatcttt gccctctggg tcttgaccca ttaagagccc catagaactc ttgatttttc 1140

ctgtccatct ttatggattt ttctggatct atattttctt caattattct ttcattttat 1200

aatgcaactt tttcatagga agtccggatg ggaatattca cattaatcat ttttgcagag 1260

actttgctag atcctctcat attttgtctt cctcagggtg gcaggggtac agagagtgcc 1320

tgattggaaa aaaaaaaaaa agagagagag agagaagaag aagaagaaga gacacaaatc 1380

tctacctccc atgttaagct ttgcaggaca gggaaagaaa gggtatgaga cacggctagg 1440

ggtaaactct tagtccaaaa cccaagcatg caataaataa aactccctta tttgaca 1497

<210> 113
<211> 1973
<212> DNA
<213> Homo sapiens

<220>
<223> II.113 (XM_012717)

<400> 113
ggcggctcct ttaggcagct gaaaggggat ttaggcccgg aagatccgag tccatccgcg 60

gcggggagag ggcaagcggg accggtaggg catgcagcgg cggcgccgat cggacatgtc 120

ccatccgccc cgtattgagg cgctgggagc gcggtgcgcg acaggaaagc gatggtgaaa 180

gcggggccgt gaggtgggcg gcaggaacgg ccggacccgc agtagcgcag cagcggcgcc 240

gcctcccaga gttcagaccc aggaagcggc cgggaggcag gagcgaatcg ggccgccgcc 300

gccatggagc tgagagtcgg gaacaggtac cggctgggcc ggaagcatcg gcagcggctc 360

cttcggagac atctatctcg gtacggacat tgctgcagga gaagaggttg ccatcaagct 420

tgaatgtgtc aaaaccaaac accctcagct ccacattgag agcaaaatct acaagatgat 480

gcagggagga gtgggcatcc ccaccatcag atggtgcggg gcagaggggg actacaacgt 540

catggtgatg gagctgctgg ggccaagcct ggaggacctc ttcaacttct gctccaggaa 600

attcagcctc aaaaccgtcc tgctgcttgc tgaccaaatg atcagtcgca tcgaatacat 660

tcattcaaag aacttcatcc accgggatgt gaagccagac aacttcctca tgggcctggg 720

gaagaagggc aacctggtgt acatcatcga cttcgggctg gccaagaagt accgggatgc 780

acgcacccac cagcacatcc cctatcgtga gaacaagaac ctcacgggga cggcgcggta 840

cgcctccatc aacacgcacc ttggaattga acaatcccga agagatgact tggagtctct 900

gggctacgtg ctaatgtact tcaacctggg ctctctcccc tggcaggggc tgaaggctgc 960

caccaagaga cagaaatacg aaaggattag cgagaagaaa atgtccaccc ccatcgaagt 1020

gttgtgtaaa ggctaccctt ccgaatttgc cacatacctg aatttctgcc gttccttgcg 1080

ttttgacgac aagcctgact actcgtacct gcggcagctt ttccggaatc tgttccatcg 1140

ccagggcttc tcctatgact acgtgttcga ctggaacatg ctcaaatttg gtgccagccg 1200

ggccgccgat gacgccgagc gggagcgcag ggaccgagag gagcggctga gacactcgcg 1260

gaacccggct acccgcggcc tcccttccac agcctccagg ccgtctgcgg gggacgcagg 1320

gaatgtggct ccccccacac ccctcacccc tacctcacac acggctaaca cctccccccg 1380

gcccgtctcc ggcatggaga gagagcggaa agtgagtatg cggctgcacc cggggccccc 1440

gtcaacatct cctcgtccga cctcacaggc cgacaagata cctctcgcat gtccacctca 1500

cagattcctg gtcgggtggc ttccagtggt cttcagtctg tcgtgcaccg atgagaactc 1560

tccttattgc tgtgaagggc agacaatgca tggctgatct actctgttac caatggcttt 1620

actagtgaca cgtcccccgg tctaggatcg aaatgttaac accgggagct ctccaggcca 1680

ctcacccagc gacgctcgtg ggggaaacat actaaacgga cagactccaa gagctgccac 1740

cgctggggct gcactgcggc cccccacgtg aactcggttg taacggggct gggaagaaaa 1800

gcagagagag aattgcagag aatcagactc cttttccagg gcctcagctc cctccagtgg 1860

tggccgccct gtactccctg acgattccac tgtaactacc aatcttctac ttggttaaga 1920

cagttttgta tcattttgct aaaaattatt ggcttaaatc tgtgtaaaga aaa 1973

<210> 114
<211> 805
<212> DNA
<213> Homo sapiens

<220>
<223> II.114 (XM_012649)

<400> 114
agcagagggg ctgagaccaa accagaaacc tccaattctc atgtggaagc ccatgccctc 60

accctccaac atgaaagcct ctgcagcact tctgtgtctg ctgctcacag cagctgcttt 120

cagcccccag gggcttgctc agccagttgg gattaatact tcaactacct gctgctacag 180

atttatcaat aagaaaatcc ctaagcagag gctggagagc tacagaagga ccaccagtag 240

ccactgtccc cgggaagctg taatcttcaa gaccaaactg gacaaggaga tctgtgctga 300

ccccacacag aagtgggtcc aggactttat gaagcacctg gacaagaaaa cccaaactcc 360

aaagctttga acattcatga ctgaactgaa aacaagccat gacttgagaa acaaataatt 420

tgtataccct gtcctttctc agagtggttc tgagattatt ttaatctaat tctaaggaat 480

atgagcttta tgtaataatg tgaatcatgg tttttcttag tagattttaa aagttattaa 540

tattttaatt taatcttcca tggattttgg tgggttttga acataaagcc ttggatgtat 600

atgtcatctc agtgctgtaa aaactgtggg atgctcctcc cttctctacc tcatgggggt 660

attgtataag tccttgcaag aatcagtgca aagatttgct ttaattgtta agatatgatg 720

tccctatgga agcatattgt tattatataa ttacatattt gcatatgtat gactcccaaa 780

ttttcacata aaatagattt ttgta 805

<210> 115
<211> 2621
<212> DNA
<213> Homo sapiens

<220>
<223> II.115 (XM_008679)

<400> 115
acttgtcatg gcgactgtcc agctttgtgc caggagcctc gcaggggttg atgggattgg 60

ggttttcccc tcccatgtgc tcaagactgg cgctaaaagt tttgagcttc tcaaaagtct 120

agagccaccg tccagggagc aggtagctgc tgggctccgg ggacactttg cgttcgggct 180

gggagcgtgc tttccacgac ggtgacacgc ttccctggat tggcagccag actgccttcc 240

gggtcactgc catggaggag ccgcagtcag atcctagcgt cgagccccct ctgagtcagg 300

aaacattttc agacctatgg aaactacttc ctgaaaacaa cgttctgtcc cccttgccgt 360

cccaagcaat ggatgatttg atgctgtccc cggacgatat tgaacaatgg ttcactgaag 420

acccaggtcc agatgaagct cccagaatgc cagaggctgc tccccgcgtg gcccctgcac 480

cagcagctcc tacaccggcg gcccctgcac cagccccctc ctggcccctg tcatcttctg 540

tcccttccca gaaaacctac cagggcagct acggtttccg tctgggcttc ttgcattctg 600

ggacagccaa gtctgtgact tgcacgtact cccctgccct caacaagatg ttttgccaac 660

tggccaagac ctgccctgtg cagctgtggg ttgattccac acccccgccc ggcacccgcg 720

tccgcgccat ggccatctac aagcagtcac agcacatgac ggaggttgtg aggcgctgcc 780

cccaccatga gcgctgctca gatagcgatg gtctggcccc tcctcagcat cttatccgag 840

tggaaggaaa tttgcgtgtg gagtatttgg atgacagaaa cacttttcga catagtgtgg 900

tggtgcccta tgagccgcct gaggttggct ctgactgtac caccatccac tacaactaca 960

tgtgtaacag ttcctgcatg ggcggcatga accggaggcc catcctcacc atcatcacac 1020

tggaagactc cagtggtaat ctactgggac ggaacagctt tgaggtgcgt gtttgtgcct 1080

gtcctgggag agaccggcgc acagaggaag agaatctccg caagaaaggg gagcctcacc 1140

acgagctgcc cccagggagc actaagcgag cactgcccaa caacaccagc tcctctcccc 1200

agccaaagaa gaaaccactg gatggagaat atttcaccct tcagatccgt gggcgtgagc 1260

gcttcgagat gttccgagag ctgaatgagg ccttggaact caaggatgcc caggctggga 1320

aggagccagg ggggagcagg gctcactcca gccacctgaa gtccaaaaag ggtcagtcta 1380

cctcccgcca taaaaaactc atgttcaaga cagaagggcc tgactcagac tgacattctc 1440

cacttcttgt tccccactga cagcctccca cccccatctc tccctcccct gccattttgg 1500

gttttgggtc tttgaaccct tgcttgcaat aggtgtgcgt cagaagcacc caggacttcc 1560

atttgctttg tcccggggct ccactgaaca agttggcctg cactggtgtt ttgttgtggg 1620

gaggaggatg gggagtagga cataccagct tagattttaa ggtttttact gtgagggatg 1680

tttgggagat gtaagaaatg ttcttgcagt taagggttag tttacaatca gccacattct 1740

aggtaggggc ccacttcacc gtactaacca gggaagctgt ccctcactgt tgaattttct 1800

ctaacttcaa ggcccatatc tgtgaaatgc tggcatttgc acctacctca cagagtgcat 1860

tgtgagggtt aatgaaataa tgtacatctg gccttgaaac caccttttat tacatggggt 1920

ctagaacttg acccccttga gggtgcttgt tccctctccc tgttggtcgg tgggttggta 1980

gtttctacag ttgggcagct ggttaggtag agggagttgt caagtctctg ctggcccagc 2040

caaaccctgt ctgacaacct cttggtgaac cttagtacct aaaaggaaat ctcaccccat 2100

cccacaccct ggaggatttc atctcttgta tatgatgatc tggatccacc aagacttgtt 2160

ttatgctcag ggtcaatttc ttttttcttt tttttttttt tttttctttt tctttgagac 2220

tgggtctcgc tttgttgccc aggctggagt ggagtggcgt gatcttggct tactgcagcc 2280

tttgcctccc cggctcgagc agtcctgcct cagcctccgg agtagctggg accacaggtt 2340

catgccacca tggccagcca acttttgcat gttttgtaga gatggggtct cacagtgttg 2400

cccaggctgg tctcaaactc ctgggctcag gcgatccacc tgtctcagcc tcccagagtg 2460

ctgggattac aattgtgagc caccacgtcc agctggaagg gtcaacatct tttacattct 2520

gcaagcacat ctgcattttc accccaccct tcccctcctt ctcccttttt atatcccatt 2580

tttatatcga tctcttattt tacaataaaa ctttgctgcc a 2621

<210> 116
<211> 5600
<212> DNA
<213> Homo sapiens

<220>
<223> II.116 (XM_030509)

<400> 116
ggagtgggcc aggccgccag ccccgccagc cccgccagcc ccgccagccc cgcgatggct 60

tgggccgcgc tcctcggcct cctggccgca ctgttgctgc tgctgctact gagccgccgc 120

cgcacgcggc gacctggtga gcctcccctg gacctgggca gcatcccctg gttggggtat 180

gccttggact ttggaaaaga tgctgccagc ttcctcacga ggatgaagga gaagcacggt 240

gacatcttta ctatactggt tgggggcagg tatgtcaccg ttctcctgga cccacactcc 300

tacgacgcgg tggtgtggga gcctcgcacc aggctcgact tccatgccta tgccatcttc 360

ctcatggaga ggatttttga tgtgcagctt ccacattaca gccccagtga tgaaaaggcc 420

aggatgaaac tgactcttct ccacagagag ctccaggcac tcacagaagc catgtatacc 480

aacctccatg cagtgctgtt gggcgatgct acagaagcag gcagtggctg gcacgagatg 540
ggtctcctcg acttctccta cagcttcctg ctcagagccg gctacctgac tctttacgga 600
attgaggcgc tgccacgcac ccatgaaagc caggcccagg accgcgtcca ctcagctgat 660
gtcttccaca cctttcgcca gctcgaccgg ctgctcccca aactggcccg tggctccctg 720
tcagtggggg acaaggacca catgtgcagt gtcaaaagtc gcctgtggaa gctgctatcc 780
ccagccaggc tggccaggcg ggcccaccgg agcaaatggc tggagagtta cctgctgcac 840
ctggaggaga tgggtgtgtc agaggagatg caggcacggg ccctggtgct gcagctgtgg 900
gccacacagg ggaatatggg tcccgctgcc ttctggctcc tgctcttcct tctcaagaat 960
cctgaagccc tggctgctgt ccgcggagag ctcgagagta tcctttggca agcggagcag 1020
cctgtctcgc agacgaccac tctcccacag aaggttctag acagcacacc tgtgcttgat 1080
agcgtgctga gtgagagcct caggcttaca gctgccccct tcatcacccg cgaggttgtg 1140
gtggacctgg ccatgcccat ggcagacggg cgagaattca acctgcgacg tggtgaccgc 1200
ctcctcctct tccccttcct gagcccccag agagacccag aaatctacac agacccagag 1260
gtatttaaat acaaccgatt cctgaaccct gacggatcag agaagaaaga cttttacaag 1320
gatgggaaac ggctgaagaa ttacaacatg ccctgggggg cggggcacaa tcactgcctg 1380
gggaggagtt atgcggtcaa cagcatcaaa caatttgtgt tccttgtgct ggtgcacttg 1440
gacttggagc tgatcaacgc agatgtggag atccctgagt ttgacctcag caggtacggc 1500
ttcggtctga tgcagccgga acacgacgtg cccgtccgct accgcatccg cccatgacac 1560
agggagcaga tggatccacg tgctcgcctc tgcccagcct gccccagcct gccccagcct 1620
cccagctttc tgtgtgcaca gttggcccgg gtgcaggtgc tagcattacc acttccctgc 1680
ttttctccca gaaggctggg tccaggggag ggaaaagcta agagggtgaa caaagaaaag 1740
acattgaaag ctctatggat tatccactgc aaagttttct ttccaaaatc aggctttgtc 1800
tgctcccaat tcacctcgtt actctcacct cgtgatatcc acaaatgcta ttcagataag 1860
gcagaactag gagtcttcac tgctctgccc ccaactcccg gaggtgtcac cttcctagtt 1920
cttatgagct agcatggccc gggccttatc cagtcaaagc ggatgctggc cacagaaagg 1980
ccactcagga tgtcctttgt gtccattgat gtcattcagc agtcagtccc ccaataatcc 2040
ttaaactagc taaaaccaaa ggagtccctt agaagatctg cttccctggg gccccatttg 2100
ccagattgcc ccattgctca cactacttga gaaaatgcag gagagcttcc cccaaggctg 2160
atgcattccc ggtgcagaac aggggcaccc tccaaacact gggctctgag gagtggagtt 2220
ctctgttcta gagtgacagg caccagatgg gatgggcttt ctcagtgtca gcactcaggt 2280
agggagctaa ggaagacaca gcccagacaa gatggctgga aggagccagc caggactcct 2340
tagactgatc aagccaaaaa agaaggtgcc gatttcatgc attctagtgc agaagcccca 2400
actgtgatca cgatccagtc tgcagacgtg ttttgtttgg acttcactta aaaaaatgcc 2460
ttagttgtta tcatctttgg gagagttcat tcaaaatgtc cagcttctct tgaaaacttg 2520

gtatatctgg ccacactggg ctcacattcc caagggtaac tcttggccag agctgagtgg 2580
cagccgcctc ccttatgcag gacatgtgct ctcggcttca ccagggttct gaccgggtct 2640
gcttctgcat tcacagcgcc tcctggacct gaaggcatct gagtgtgaga ccctgttcta 2700
actcttagaa gtgacattgt aagaggtggt ggggaccagc taattggtcc aacccagcct 2760
gagtgcacca ccctttgaac aaatgtatca gtgatgaaaa tttgcctttg ccccggcttg 2820
cctgtaatcc cagcactttg ggaggccgag gtgggcggat cacttgaggt cgggagttca 2880
agaccagcct ggccaacatg gcgaaacccc gtctctacta aacataaaaa aattagtcag 2940
gtgtggcggt gcgtgcctgt aatcccagct attcaggagg ctgaggcacc agaattgctt 3000
gaacccagga ggtggaggtt gcagtgaact gagactgcgc cacggcactc cagcctgggc 3060
gacagagcaa gactctgtct caataaataa ataattaatt aaaataaaaa acagcttaaa 3120
gagaaaaatg gcctgcaaac cttttttatg atgctatttt tattaatata aagtcctgtt 3180
tattgagacc ctttaaatgc ctgcgagaga ccctacagac agtatgtcta ctcctcacag 3240
catctctatg aagagaagga gggttgtgcc cacttcatag atgagaaaac tgagaggtga 3300
ggtgacttgc ctggggccac atagctcata agctgtagaa ctctgcaggc agatttactg 3360
tcccaggagc aaatgctgga tgagcaactc ctgttctttg ggctcaaggg gactggtgat 3420
gggacaattc ttctcgactt caggagctga cagagccaga ggcacctaaa cttgggtaca 3480
tcttgtaaga cacataatga ggtccctcta gctttagctg gagggagata aaagaacccc 3540
agacctctga atgtcccaga ggctagtctc ttctcagagc agcactgggg tttgggggct 3600
tccctgggcc tcagcctcca ggcaccccca ggattcccag agagacgctg tgattggcag 3660
gaggcaggat atccccaggg aaacccatct tcacgcctgg gtgaccccca ctgccgcctc 3720
cttcttaact ctgcaggaaa tcagagctgg cagcctccag tgggaggaca gagccggttt 3780
ccgtggcaac cctcagctgc ctcatcgtgg ctgggaaggg aaggaagcaa gccggcagaa 3840
atagctatgg aaggtcttgc gcaggctgca accctggtgt gctgggcgaa aacccttatg 3900
actcccccct tccaaatcag gctgggttgt cactgagact agattctcac ctgccttcaa 3960
agaaggccca attcccttta aaggtcgcac ctccttggaa ccacagtcat tagtgaatta 4020
cactcaagga aaagatgtgc tcccaccagg cagctccagc tgttacctga gatactgaag 4080
tgcagctcag gagacgatta tttaaacctg cccttgtttt aatcgttatt tttctcttta 4140
aaaaaaatag aagctataaa gaaaagaggg agagatgagt gggttagcta cctgctatgc 4200
gctagttagg aagttacctg gatgccattg tatttcttca tcccttgctt aagaatcaaa 4260
attactggac atatgttagg aatactcttt ctttctttct ttctttttaa gctcagtggc 4320
aagaatgaaa tactcttttt tttttttttt ttttgagatg gagtctcgct ctgctgccca 4380
ggctagagtg cagtggcgtg atctcggctc actgcaagct ctgcctcccg tgttcacacc 4440
attctcctgc ctcagcctcc tgagtagctg ggactacagg cacccgccac cacacccggc 4500
taatttttgg atttttagta gagatgggat ttcaccgtat tagccaggat ggtcttgatc 4560

tcctgagctc gtgatctgcc cgcctcggcc tcccaaagtg ctgggattac aggtgtgagc 4620

caccgcgccc agccaagaat aaaatactct taagttgatc taatgaagtg tttccttacc 4680

attgtgatta ttgttactat tatttgctat attttaatat tgttgtttac caaatattct 4740

cctttaaaca gactcgcttt ttaaactttt tttttttttt ttgagacgga gtttcacgct 4800

tattgcccag gctggagtgc aatggcacga tctcagctca ccacaacctc cacctcctgg 4860

gtttaagcaa tgcttctgcc tcagcttccc aagtagctga gattacaggc gcacaccacc 4920

acgcccaact gatttttgta tttttagtag agacggggtt tccccatgtt ggtcaggctg 4980

gtctcgaact cctgacctcg tgatttgcct gcctgggcct cccaaagtgc taggattaca 5040

ggcatgagcc accatgcccg gcctaaactt tgtttttaaa atgaactttt tttcccccca 5100

attgctgcca atagtggata acatgtatca ctcactgcca aaaatagaaa gtgaccatga 5160

aaaataaatt cgctggggaa gggggctcca tgctggtgtg gccaaggctg agagctctct 5220

cttctctgtt acaaaacgag ataagcaagt gttagaattg ccttaaggcc acactggcat 5280

ctccctgacc ttctccaggg acagaagcag gagtaagttt ctcatcccat gggcgaccag 5340

ggccatctcc tcccaccagt ggcccccact cacagggagc tggcaatgcc ctacctgcct 5400

gttctccaga tggagaaaca ggctctgaga tttcacaggt cttgcccaaa gtcattgatt 5460

ttgatgatta aaaagaataa acacagtgtt tcctgagtag cagtgattgt tatgccttgc 5520

tattttaata aagattctat tttcgtataa cattgtcaag tggaaacatg ctgaaatcta 5580

ttaaaccatc tttgtttgtg 5600

<210> 117
<211> 430
<212> DNA
<213> Homo sapiens

<220>
<223> II.117 (XM_039086)

<400> 117
gacagccacg aagatcctac caaaatgaag cgcttcctct tcctcctact caccatcagc 60

ctcctggtta tggtacagat acaaactgga ctctcaggac aaaacgacac cagccaaacc 120

agcagcccct cagcatccag caacataagc ggaggcattt tccttttctt cgtggccaat 180

gccataatcc acctcttctg cttcagttga ggtgacacgt ctcagcctta gccctgtgcc 240

ccctgaaaca gctgccacca tcactcgcaa gagaatcccc tccatctttg ggaggggttg 300

atgccagaca tcaccaggtt gtagaagttg acaggcagtg ccatgggggc aacagccaaa 360

ataggggggt aatgatgtag gggccaagca gtgcccagct gggggtcaat aaagttaccc 420

ttgtacttgc 430

<210> 118
<211> 2056
<212> DNA

<213>  Homo sapiens

<220>
<223>  II.118 (XM_027978)

<400>  118
cgagtctcaa ctaaaaggga ctcccggagc taggggtggg gactcggcct cacacagtga      60

gtgccggcta ttggactttt gtccagtgac agctgagaca acaaggacca cgggaggagg     120

tgtaggagag aagcgccgcg aacagcgatc gcccagcacc aagtccgctt ccaggctttc     180

ggtttctttg cctccatctt gggtgcgcct tcccggcgtc taggggagcg aaggctgagg     240

tggcagcggc aggagagtcc ggccgcgaca ggacgaactc ccccactgga aaggattctg     300

aaagaaatga agtcagccct cagaaatgaa gttgactgcc tgctggcttt ctgttgactg     360

gcccggagct gtactgcaag acccttgtga gcttccctag tctaagagta ggatgtctgc     420

tgaagtcatc catcaggttg aagaagcact tgatacagat gagaaggaga tgctgctctt     480

tttgtgccgg gatgttgcta tagatgtggt tccacctaat gtcagggacc ttctggatat     540

tttacgggaa agaggtaagc tgtctgtcgg ggacttggct gaactgctct acagagtgag     600

gcgatttgac ctgctcaaac gtatcttgaa gatggacaga aaagctgtgg agacccacct     660

gctcaggaac cctcaccttg tttcggacta tagagtgctg atggcagaga ttggtgagga     720

tttggataaa tctgatgtgt cctcattaat tttcctcatg aaggattaca tgggccgagg     780

caagataagc aaggagaaga gtttcttgga ccttgtggtt gagttggaga aactaaatct     840

ggttgcccca gatcaactgg atttattaga aaaatgccta aagaacatcc acagaataga     900

cctgaagaca aaaatccaga agtacaagca gtctgttcaa ggagcaggga caagttacag     960

gaatgttctc caagcagcaa tccaaaagag tctcaaggat ccttcaaata acttcaggag    1020

catacctgaa gagagataca agatgaagag caagccccta ggaatctgcc tgataatcga    1080

ttgcattggc aatgagacag agcttcttcg agacaccttc acttccctgg gctatgaagt    1140

ccagaaattc ttgcatctca gtatgcatgg tatatcccag attcttggcc aatttgcctg    1200

tatgcccgag caccgagact acgacagctt tgtgtgtgtc ctggtgagcc gaggaggctc    1260

ccagagtgtg tatggtgtgg atcagactca ctcagggctc cccctgcatc acatcaggag    1320

gatgttcatg ggagattcat gcccttatct agcagggaag ccaaagatgt tttttattca    1380

gaactatgtg gtgtcagagg gccagctgga ggacagcagc ctcttggagg tggatgggcc    1440

agcgatgaag aatgtggaat tcaaggctca gaagcgaggg ctgtgcacag ttcaccgaga    1500

agctgacttc ttctggagcc tgtgtactgc ggacatgtcc ctgctggagc agtctcacag    1560

ctcaccatcc ctgtacctgc agtgcctctc ccagaaactg agacaagaaa gaaaacgccc    1620

actcctggat cttcacattg aactcaatgg ctacatgtat gattggaaca gcagagtttc    1680

tgccaaggag aaatattatg tctggctgca gcacactctg agaaagaaac ttatcctctc    1740

ctacacataa gaaaccaaaa ggctgggcgt agtggctcac acctgtaatc ccagcacttt    1800

gggaggccaa ggagggcaga tcacttcagg tcaggagttc gagaccagcc tggccaacat    1860

ggtaaacgct gtccctagta aaaatacaaa aattagctgg gtgtgggtgt gggtacctgt 1920

attcccagtt acttgggagg ctgaggtggg aggatctttt gaacccagga gttcagggtc 1980

atagcatgct gtgattgtgc ctacgaatag ccactgcata ccaacctggg caatatagca 2040

agatcccatc tcttta 2056

<210> 119
<211> 570
<212> DNA
<213> Homo sapiens

<220>
<223> II.119 (NM_005343)

<400> 119
atgacggaat ataagctggt ggtggtgggc gccggcggtg tgggcaagag tgcgctgacc 60

atccagctga tccagaacca ttttgtggac gaatacgacc ccactataga ggattcctac 120

cggaagcagg tggtcattga tggggagacg tgcctgttgg acatcctgga taccgccggc 180

caggaggagt acagcgccat gcgggaccag tacatgcgca ccggggaggg cttcctgtgt 240

gtgtttgcca tcaacaacac caagtctttt gaggacatcc accagtacag ggagcagatc 300

aaacgggtga aggactcgga tgacgtgccc atggtgctgg tggggaacaa gtgtgacctg 360

gctgcacgca ctgtggaatc tcggcaggct caggacctcg cccgaagcta cggcatcccc 420

tacatcgaga cctcggccaa gacccggcag ggagtggagg atgccttcta cacgttggtg 480

cgtgagatcc ggcagcacaa gctgcggaag ctgaaccctc ctgatgagag tggccccggc 540

tgcatgagct gcaagtgtgt gctctcctga 570

<210> 120
<211> 1489
<212> DNA
<213> Homo sapiens

<220>
<223> II.120 (XM_043574)

<400> 120
gtgcaaggat gatgctgaaa ggaataacaa ggcttatctc taggatccat aagttggacc 60

ctgggcgttt tttacacatg ggacccaggg ctcgccaaag cattgctgct cacctagata 120

accaggttcc agttgagagt ccgagagcta tttcccgcac caatgagaat gacccggcca 180

agcatgggga tcagcacgag ggtcagcact acaacatctc cccccaggat ttggagactg 240

tatttcccca tggccttcct cctcgctttg tgatgcaggt gaagacattc agtgaagctt 300

gcctgatggt aaggaaacca gccctagaac ttctgcatta cctgaaaaac accagttttg 360

cttatccagc tatacgatat cttctgtatg gagagaaggg aacaggaaaa accctaagtc 420

tttgccatgt tattcatttc tgtgcaaaac aggactggct gatactacat attccagatg 480

ctcatctttg ggtgaaaaat tgtcgggatc ttctgcagtc cagctacaac aaacagcgct 540

ttgatcaacc tttagaggct tcaacctggc tgaagaattt caaaactaca aatgagcgct 600

tcctgaacca gataaaagtt caagagaagt atgtctggaa taagagagaa agcactgaga 660

aagggagtcc tctgggagaa gtggttgaac agggcataac acgggtgagg aacgccacag 720

atgcagttgg aattgtgctg aaagagctaa agaggcaaag ttctttgggt atgtttcacc 780

tcctagtggc cgtggatgga atcaatgctc tttggggaag aaccactctg aaaagagaag 840

ataaaagccc gattgccccc gaggaattag cacttgttca caacttgagg aaaatgatga 900

aaaatgattg gcatggaggc gccattgtgt cggctttgag ccagactggg tctctcttta 960

agccccggaa agcctatctg ccccaggagt tgctgggaaa ggaaggattt gatgccctgg 1020

atccctttat tcccatcctg gtttccaact ataacccaaa ggaatttgaa agttgtattc 1080

agtattattt ggaaaacaat tggcttcaac atgagaaagc tcctacagaa gaagggaaaa 1140

aagagctgct gttcctaagt aacgcgaacc cctcgctgct ggagcggcac tgtgcctacc 1200

tctaagccaa gatcacagca tgtgaggaag acagtggaca tctgctttat gctggaccca 1260

gtaagatgag gaagtcgggc agtacacagg aagaggagcc aggcccttgt acctatggga 1320

ttggacagga ctgcagttgg ctctggacct gcattaaaat gggtttcact gtgaatgcgt 1380

gacaataaga tattcccttg ttcctaaaac tttatatcag tttattggat gtggtttttc 1440

acatttaaga taattatggc tcttttccta aaaaataaaa tatctttct 1489

<210> 121
<211> 1283
<212> DNA
<213> Homo sapiens

<220>
<223> II.121 (NM_002188)

<400> 121
aagccaccca gcctatgcat ccgctcctca atcctctcct gttggcactg ggcctcatgg 60

cgcttttgtt gaccacggtc attgctctca cttgccttgg cggctttgcc tccccaggcc 120

ctgtgcctcc ctctacagcc ctcagggagc tcattgagga gctggtcaac atcacccaga 180

accagaaggc tccgctctgc aatggcagca tggtatggag catcaacctg acagctggca 240

tgtactgtgc agccctggaa tccctgatca acgtgtcagg ctgcagtgcc atcgagaaga 300

cccagaggat gctgagcgga ttctgcccgc acaaggtctc agctgggcag ttttccagct 360

tgcatgtccg agacaccaaa atcgaggtgg cccagtttgt aaaggacctg ctcttacatt 420

taaagaaact ttttcgcgag ggacggttca actgaaactt cgaaagcatc attatttgca 480

gagacaggac ctgactattg aagttgcaga ttcatttttc tttctgatgt caaaaatgtc 540

ttgggtaggc gggaaggagg gttagggagg ggtaaaattc cttagcttag acctcagcct 600

gtgctgcccg tcttcagcct agccgacctc agccttcccc ttgcccaggg ctcagcctgg 660

tgggcctcct ctgtccaggg ccctgagctc ggtggaccca gggatgacat gtccctacac 720

ccctcccctg ccctagagca cactgtagca ttacagtggg tgcccccctt gccagacatg 780

tggtgggaca gggacccact tcacacacag gcaactgagg cagacagcag ctcaggcaca 840

cttcttcttg gtcttattta ttattgtgtg ttatttaaat gagtgtgttt gtcaccgttg 900

gggattgggg aagactgtgg ctgctggcac ttggagccaa gggttcagag actcagggcc 960

ccagcactaa agcagtggac cccaggagtc cctggtaata agtactgtgt acagaattct 1020

gctacctcac tggggtcctg gggcctcgga gcctcatccg aggcagggtc aggagagggg 1080

cagaacagcc gctcctgtct gccagccagc agccagctct cagccaacga gtaatttatt 1140

gtttttcctc gtatttaaat attaaatatg ttagcaaaga gttaatatat agaagggtac 1200

cttgaacact gggggagggg acattgaaca agttgtttca ttgactatca aactgaagcc 1260

agaaataaag ttggtgacag ata 1283

<210> 122
<211> 1842
<212> DNA
<213> Homo sapiens

<220>
<223> II.122 (XM_055699)

<400> 122
gagtctaacg tgaagacatt ttgctccaag aatatcctag ccatccttgg cttctcctct 60

atcatagctg tgatagcttt gcttgctgtg gggttgaccc agaacaaagc attgccagaa 120

aacgttaagt atgggattgt gctggatgcg ggttcttctc acacaagttt atacatctat 180

aagtggccag cagaaaagga gaatgacaca ggcgtggtgc atcaagtaga agaatgcagg 240

gttaaaggtc ctggaatctc aaaatttgtt cagaaagtaa atgaaatagg catttacctg 300

actgattgca tggaaagagc tagggaagtg attccaaggt cccagcacca agagacaccc 360

gtttacctgg gagccacggc aggcatgcgg ttgctcagga tggaaagtga agagttggca 420

gacagggttc tggatgtggt ggagaggagc ctcagcaact acccctttga cttccagggt 480

gccaggatca ttactggcca agaggaaggt gcctatggct ggattactat caactatctg 540

ctgggcaaat tcagtcagaa aacaaggtgg ttcagcatag tcccatatga aaccaataat 600

caggaaacct ttggagcttt ggaccttggg ggagcctcta cacaagtcac ttttgtaccc 660

caaaaccaga ctatcgagtc cccagataat gctctgcaat ttcgcctcta tggcaaggac 720

tacaatgtct acacacatag cttcttgtgc tatgggaagg atcaggcact ctggcagaaa 780

ctggccaagg acattcaggt tgcaagtaat gaaattctca gggacccatg ctttcatcct 840

ggatataaga aggtagtgaa cgtaagtgac ctttacaaga ccccctgcac caagagattt 900

gagatgactc ttccattcca gcagtttgaa atccagggta ttggaaacta tcaacaatgc 960

catcaaagca tcctggagct cttcaacacc agttactgcc cttactccca gtgtgccttc 1020

aatgggattt tcttgccacc actccagggg gattttgggg cattttcagc tttttacttt 1080

gtgatgaagt ttttaaactt gacatcagag aaagtctctc aggaaaaggt gactgagatg 1140

atgaaaaagt tctgtgctca gccttgggag gagataaaaa catcttacgc tggagtaaag 1200

gagaagtacc tgagtgaata ctgcttttct ggtacctaca ttctctccct ccttctgcaa 1260

ggctatcatt tcacagctga ttcctgggag cacatccatt tcattggcaa gatccagggc 1320

agcgacgccg gctggacttt gggctacatg ctgaacctga ccaacatgat cccagctgag 1380

caaccattgt ccacacctct ctcccactcc acctatgtct tcctcatggt tctattctcc 1440

ctggtccttt tcacagtggc catcataggc ttgcttatct ttcacaagcc ttcatatttc 1500

tggaaagata tggtatagca aaagcagctg aaatatgctg gctggagtga ggaaaaaaat 1560

cgtccaggga gcattttcct ccatcgcagt gttcaaggcc atccttccct gtctgccagg 1620

gccagtcttg acgagtgtga agcttccttg gcttttactg aagcctttct tttggaggta 1680

ttcaatatcc tttgcctcaa ggacttcggc agatactgtc tctttcatga gtttttccca 1740

gctacacctt tctcctttgt actttgtgct tgtataggtt ttaaagacct gacacctttc 1800

ataatctttg ctttataaaa gaacaatatt gactttgtct ag 1842

<210> 123
<211> 4176
<212> DNA
<213> Homo sapiens

<220>
<223> II.123 (NM_000565)

<400> 123
ggcggtcccc tgttctcccc gctcaggtgc ggcgctgtgg caggaagcca ccccctcggt 60

cggccggtgc gcggggctgt tgcgccatcc gctccggctt tcgtaaccgc accctgggac 120

ggcccagaga cgctccagcg cgagttcctc aaatgttttc ctgcgttgcc aggaccgtcc 180

gccgctctga gtcatgtgcg agtgggaagt cgcactgaca ctgagccggg ccagagggag 240

aggagccgag cgcggcgcgg ggccgaggga ctcgcagtgt gtgtagagag ccgggctcct 300

gcggatgggg gctgcccccg gggcctgagc ccgcctgccc gcccaccgcc ccgccccgcc 360

cctgccaccc ctgccgcccg gttcccatta gcctgtccgc ctctgcggga ccatggagtg 420

gtagccgagg aggaagcatg ctggccgtcg gctgcgcgct gctggctgcc ctgctggccg 480

cgccgggagc ggcgctggcc ccaaggcgct gccctgcgca ggaggtggcg agaggcgtgc 540

tgaccagtct gccaggagac agcgtgactc tgacctgccc gggggtagag ccggaagaca 600

atgccactgt tcactgggtg ctcaggaagc cggctgcagg ctcccacccc agcagatggg 660

ctggcatggg aaggaggctg ctgctgaggt cggtgcagct ccacgactct ggaaactatt 720

catgctaccg ggccggccgc ccagctggga ctgtgcactt gctggtggat gttccccccg 780

aggagcccca gctctcctgc ttccggaaga gccccctcag caatgttgtt tgtgagtggg 840

gtcctcggag caccccatcc ctgacgacaa aggctgtgct cttggtgagg aagtttcaga 900

acagtccggc cgaagacttc caggagccgt gccagtattc ccaggagtcc cagaagttct 960

cctgccagtt agcagtcccg gagggagaca gctctttcta catagtgtcc atgtgcgtcg 1020
ccagtagtgt cgggagcaag ttcagcaaaa ctcaaacctt tcagggttgt ggaatcttgc 1080
agcctgatcc gcctgccaac atcacagtca ctgccgtggc cagaaacccc cgctggctca 1140
gtgtcacctg gcaagacccc cactcctgga actcatcttt ctacagacta cggtttgagc 1200
tcagatatcg ggctgaacgg tcaaagacat tcacaacatg gatggtcaag gacctccagc 1260
atcactgtgt catccacgac gcctggagcg gcctgaggca cgtggtgcag cttcgtgccc 1320
aggaggagtt cgggcaaggc gagtggagcg agtggagccc ggaggccatg ggcacgcctt 1380
ggacagaatc caggagtcct ccagctgaga acgaggtgtc cacccccatg caggcactta 1440
ctactaataa agacgatgat aatattctct tcagagattc tgcaaatgcg acaagcctcc 1500
cagtgcaaga ttcttcttca gtaccactgc ccacattcct ggttgctgga gggagcctgg 1560
ccttcggaac gctcctctgc attgccattg ttctgaggtt caagaagacg tggaagctgc 1620
gggctctgaa ggaaggcaag acaagcatgc atccgccgta ctctttgggg cagctggtcc 1680
cggagaggcc tcgacccacc ccagtgcttg ttcctctcat ctccccaccg gtgtccccca 1740
gcagcctggg gtctgacaat acctcgagcc acaaccgacc agatgccagg gacccacgga 1800
gcccttatga catcagcaat acagactact tcttccccag atagctggct gggtggcacc 1860
agcagcctgg accctgtgga tgataaaaca caaacgggct cagcaaaaga tgcttctcac 1920
tgccatgcca gcttatctca ggggtgtgcg gcctttggct tcacggaaga gccttgcgga 1980
aggttctacg ccaggggaaa atcagcctgc tccagctgtt cagctggttg aggtttcaaa 2040
cctccctttc caaatgccca gcttaaaggg gctagagtga acttgggcca ctgtgaagag 2100
aaccatatca agactctttg gacactcaca cggacactca aaagctgggc aggttggtgg 2160
gggcctcggt gtggagaagc ggctggcagc ccacccctca acacctctgc acaagctgca 2220
ccctcaggca ggtgggatgg atttccagcc aaagcctcct ccagccgcca tgctcctggc 2280
ccactgcatc gtttcatctt ccaactcaaa ctcttaaaac ccaagtgcct tagcaaattc 2340
tgtttttcta ggcctgggga cggcttttac ttaaaccgcc aaggctgggg gaagaagctc 2400
tctcctccct ttcttcccta cagttgaaaa acagctgagg gtgagtgggt gaataataca 2460
gtatctcagg gcctggtcgt tttcaacaga attataatta gttcctcatt agcattttgc 2520
taaatgtgaa tgatgatcct aggcatttgc tgaatacaga ggcaactgca ttggctttgg 2580
gttgcaggac ctcaggtgag aagcagagga aggagaggag aggggcacag ggtctctacc 2640
atcccctgta gagtgggagc tgagtggggg atcacagcct ctgaaaacca atgttctctc 2700
ttctccacct cccacaaagg agagctagca gcagggaggg cttctgccat ttctgagatc 2760
aaaacggttt tactgcagct ttgtttgttg tcagctgaac ctgggtaact agggaagata 2820
atattaagga agacaatgtg aaaagaaaaa tgagcctggc aagaatgtgt ttaaacttgg 2880
tttttaaaaa actgctgact gttttctctt gagagggtgg aatatccaat attcgctgtg 2940
tcagcataga agtaacttac ttaggtgtgg gggaagcacc ataactttgt ttagcccaaa 3000

accaagtcaa gtgaaaaagg aggaagagaa aaaatatttt cctgccaggc atggtggccc 3060

acgcacttcg ggaggtcgag gcaggaggat cacttgagtc cagaagtttg agatcagcct 3120

gggcaatgtg ataaaacccc atctctacaa aaagcataaa aattagccaa gtgtggtaga 3180

gtgtgcctga agtcccagat acttgggggg ctgaggtggg aggatctctt gagcctggga 3240

ggtcaaggct gcagtgagcc gagattgcac cactgcactc cagcctgggt gacagagcaa 3300

gtgagaccct gtctcaaaaa aagaaaaaga aaaagaaaaa atattttccc tattagagaa 3360

gagattgtgg tttcattctg tattttgttt ttgtcttaaa aagtggaaaa atagcctgcc 3420

tcttctctac tctagggaaa aaccagcgtg tgactactcc cccaggtggt tatggagagg 3480

gtgtccggtc cctgtcccag tgccgagaag gaagcctccc acgactgccc ggcagggtcc 3540

tagaaattcc ccaccctgaa agccctgagc tttctgctat caaagaggtt ttaaaaaaat 3600

cccatttaaa aaaaatccct tacctcggtg ccttcctctt tttatttagt tccttgagtt 3660

gattcagctc tgcaagaatt gaagcaggac taaatgtcta gttgtaacac catgattaac 3720

cacttcagct gacttttctg tccgagcttt gaaaattcag tggtgttagt ggttacccag 3780

ttagctctca agttatcagg gtattccaga gtggggatat gatttaaatc agccgtgtaa 3840

ccatggaccc aatatttacc agaccacaaa acttttctaa tactctaccc tcttagaaaa 3900

accaccacca tcaccagaca ggtgcgaaag gatgaaagtg accatgtttt gtttacggtt 3960

ttccaggttt aagctgttac tgtcttcagt aagccgtgat tttcattgct gggcttgtct 4020

gtagatttta gaccctattg ctgcttgagg caactcatct taggttggca aaaaggcagg 4080

atggccgggc gcggtggctc acgcctgtaa tcctagcact ttgggaggcc aaggtgggag 4140

gattgcttga gctcaggagt ttgagaccaa cctggg 4176

<210> 124
<211> 3700
<212> DNA
<213> Homo sapiens

<220>
<223> II.124 (NM_002211)

<400> 124
agccgccgcc acccgccgcg cccgacaccc gggaggcccc gccagcccgc gggagaggcc 60

cagcgggagt cgcggaacag caggcccgag cccaccgcgc cgggccccgg acgccgcgcg 120

gaaaagatga atttacaacc aattttctgg attggactga tcagttcagt ttgctgtgtg 180

tttgctcaaa cagatgaaaa tagatgttta aaagcaaatg ccaaatcatg tggagaatgt 240

atacaagcag ggccaaattg tgggtggtgc acaaattcaa catttttaca ggaaggaatg 300

cctacttctg cacgatgtga tgatttagaa gccttaaaaa agaagggttg ccctccagat 360

gacatagaaa atcccagagg ctccaaagat ataaagaaaa ataaaaatgt aaccaaccgt 420

agcaaaggaa cagcagagaa gctcaagcca gaggatatta ctcagatcca accacagcag 480

ttggttttgc gattaagatc aggggagcca cagacattta cattaaaatt caagagagct 540

gaagactatc ccattgacct ctactacctt atggacctgt cttactcaat gaaagacgat 600
ttggagaatg taaaaagtct tggaacagat ctgatgaatg aaatgaggag gattacttcg 660
gacttcagaa ttggatttgg ctcatttgtg gaaaagactg tgatgcctta cattagcaca 720
acaccagcta agctcaggaa cccttgcaca agtgaacaga actgcaccag cccatttagc 780
tacaaaaatg tgctcagtct tactaataaa ggagaagtat ttaatgaact tgttggaaaa 840
cagcgcatat ctggaaattt ggattctcca gaaggtggtt tcgatgccat catgcaagtt 900
gcagtttgtg gatcactgat tggctggagg aatgttacac ggctgctggt gttttccaca 960
gatgccgggt ttcactttgc tggagatggg aaacttggtg gcattgtttt accaaatgat 1020
ggacaatgtc acctggaaaa taatatgtac acaatgagcc attattatga ttatccttct 1080
attgctcacc ttgtccagaa actgagtgaa aataatattc agacaatttt tgcagttact 1140
gaagaatttc agcctgttta caaggagctg aaaaacttga tccctaagtc agcagtagga 1200
acattatctg caaattctag caatgtaatt cagttgatca ttgatgcata caattccctt 1260
tcctcagaag tcattttgga aaacggcaaa ttgtcagaag gagtaacaat aagttacaaa 1320
tcttactgca agaacggggt gaatggaaca ggggaaaatg gaagaaaatg ttccaatatt 1380
tccattggag atgaggttca atttgaaatt agcataactt caaataagtg tccaaaaaag 1440
gattctgaca gctttaaaat taggcctctg ggctttacgg aggaagtaga ggttattctt 1500
cagtacatct gtgaatgtga atgccaaagc gaaggcatcc ctgaaagtcc caagtgtcat 1560
gaaggaaatg ggacatttga gtgtggcgcg tgcaggtgca atgaagggcg tgttggtaga 1620
cattgtgaat gcagcacaga tgaagttaac agtgaagaca tggatgctta ctgcaggaaa 1680
gaaaacagtt cagaaatctg cagtaacaat ggagagtgcg tctgcggaca gtgtgtttgt 1740
aggaagaggg ataatacaaa tgaaatttat tctggcaaat tctgcgagtg tgataatttc 1800
aactgtgata gatccaatgg cttaatttgt ggaggaaatg gtgtttgcaa gtgtcgtgtg 1860
tgtgagtgca accccaacta cactggcagt gcatgtgact gttctttgga tactagtact 1920
tgtgaagcca gcaacggaca gatctgcaat ggccggggca tctgcgagtg tggtgtctgt 1980
aagtgtacag atccgaagtt tcaagggcaa acgtgtgaga tgtgtcagac ctgccttggt 2040
gtctgtgctg agcataaaga atgtgttcag tgcagagcct tcaataaagg agaaaagaaa 2100
gacacatgca cacaggaatg ttcctatttt aacattacca aggtagaaag tcgggacaaa 2160
ttaccccagc cggtccaacc tgatcctgtg tcccattgta aggagaagga tgttgacgac 2220
tgttggttct attttacgta ttcagtgaat gggaacaacg aggtcatggt tcatgttgtg 2280
gagaatccag agtgtcccac tggtccagac atcattccaa ttgtagctgg tgtggttgct 2340
ggaattgttc ttattggcct tgcattactg ctgatatgga agcttttaat gataattcat 2400
gacagaaggg agtttgctaa atttgaaaag gagaaaatga atgccaaatg ggacacgggt 2460
gaaaatccta tttataagag tgccgtaaca actgtggtca atccgaagta tgagggaaaa 2520
tgagtactgc ccgtgcaaat cccacaacac tgaatgcaaa gtagcaattt ccatagtcac 2580

agttaggtag ctttagggca atattgccat ggttttactc atgtgcaggt tttgaaaatg 2640

tacaatatgt ataattttta aaatgtttta ttattttgaa aataatgttg taattcatgc 2700

cagggactga caaaagactt gagacaggat ggttattctt gtcagctaag gtcacattgt 2760

gcctttttga ccttttcttc ctggactatt gaaatcaagc ttattggatt aagtgatatt 2820

tctatagcga ttgaaagggc aatagttaaa gtaatgagca tgatgagagt ttctgttaat 2880

catgtattaa aactgatttt tagctttaca aatatgtcag tttgcagtta tgcagaatcc 2940

aaagtaaatg tcctgctagc tagttaagga ttgttttaaa tctgttattt tgctatttgc 3000

ctgttagaca tgactgatga catatctgaa agacaagtat gttgagagtt gctggtgtaa 3060

aatacgtttg aaatagttga tctacaaagg ccatgggaaa aattcagaga gttaggaagg 3120

aaaaaccaat agctttaaaa cctgtgtgcc attttaagag ttacttaatg tttggtaact 3180

tttatgcctt cactttacaa attcaagcct tagataaaag aaccgagcaa ttttctgcta 3240

aaaagtcctt gatttagcac tatttacata caggccatac tttacaaagt atttgctgaa 3300

tggggacctt ttgagttgaa tttattttat tatttttatt ttgtttaatg tctggtgctt 3360

tctatcacct cttctaatct tttaatgtat ttgtttgcaa ttttggggta agactttttt 3420

atgagtactt tttctttgaa gttttagcgg tcaatttgcc tttttaatga acatgtgaag 3480

ttatactgtg gctatgcaac agctctcacc tacgcgagtc ttactttgag ttagtgccat 3540

aacagaccac tgtatgttta cttctcacca tttgagttgc ccatcttgtt tcacactagt 3600

cacattcttg ttttaagtgc ctttagtttt aacagttcac tttttacagt gctatttact 3660

gaagttattt attaaatatg cctaaaatac ttaaatcgga 3700

<210> 125
<211> 1512
<212> DNA
<213> Homo sapiens

<220>
<223> II.125 (XM_049864)

<400> 125
agcccggagc ctgcagccca gccccaccca gacccatggc tggacctgcc acccagagcc 60

ccatgaagct gatggccctg cagctgctgc tgtggcacag tgcactctgg acagtgcagg 120

aagccacccc cctgggccct gccagctccc tgccccagag cttcctgctc aagtgcttag 180

agcaagtgag gaagatccag ggcgatggcg cagcgctcca ggagaagctg gtgagtgagt 240

gtgccaccta caagctgtgc caccccgagg agctggtgct gctcggacac tctctgggca 300

tcccctgggc tcccctgagc agctgcccca gccaggccct gcagctggca ggctgcttga 360

gccaactcca tagcggcctt ttcctctacc aggggctcct gcaggccctg gaagggatct 420

cccccgagtt gggtcccacc ttggacacac tgcagctgga cgtcgccgac tttgccacca 480

ccatctggca gcagatggaa gaactgggaa tggcccctgc cctgcagccc acccagggtg 540

ccatgccggc cttcgcctct gctttccagc gccgggcagg aggggtcctg gttgcctccc 600

atctgcagag cttcctggag gtgtcgtacc gcgttctacg ccaccttgcc cagccctgag 660

ccaagccctc cccatcccat gtatttatct ctatttaata tttatgtcta tttaagcctc 720

atatttaaag acagggaaga gcagaacgga gccccaggcc tctgtgtcct tccctgcatt 780

tctgagtttc attctcctgc ctgtagcagt gagaaaaagc tcctgtcctc ccatcccctg 840

gactgggagg tagataggta aataccaagt atttattact atgactgctc cccagccctg 900

gctctgcaat gggcactggg atgagccgct gtgagcccct ggtcctgagg gtccccacct 960

gggacccttg agagtatcag gtctcccacg tgggagacaa gaaatccctg tttaatattt 1020

aaacagcagt gttccccatc tgggtccttg cacccctcac tctggcctca gccgactgca 1080

cagcggcccc tgcatcccct tggctgtgag gcccctggac aagcagaggt ggccagagct 1140

gggaggcatg gccctggggt cccacgaatt tgctggggaa tctcgttttt cttcttaaga 1200

cttttgggac atggtttgac tcccgaacat caccgacgcg tctcctgttt ttctgggtgg 1260

cctcgggaca cctgccctgc ccccacgagg gtcaggactg tgactctttt tagggccagg 1320

caggtgcctg gacatttgcc ttgctggacg gggactgggg atgtgggagg gagcagacag 1380

gaggaatcat gtcaggcctg tgtgtgaaag gaagctccac tgtcaccctc cacctcttca 1440

ccccccactc accagtgtcc cctccactgt cacattgtaa ctgaacttca ggataataaa 1500

gtgtttgcct cc 1512

<210> 126
<211> 1126
<212> DNA
<213> Homo sapiens

<220>
<223> II.126 (XM_045933)

<400> 126
aagggcagtg acgcgctcct ctccaacacc gtgggcacgc ccgccttcat ggcacccgag 60

tcgctctctg agacccgcaa gatcttctct gggaaggcct tggatgtttg ggccatgggt 120

gtgacactat actgctttgt ctttggccag tgcccattca tggacgagcg gatcatgtgt 180

ttacacagta agatcaagag tcaggccctg gaatttccag accagcccga catagctgag 240

gacttgaagg acctgatcac ccgtatgctg gacaagaacc ccgagtcgag gatcgtggtg 300

ccggaaatca agctgcaccc ctgggtcacg aggcatgggg cggagccgtt gccgtcggag 360

gatgagaact gcacgctggt cgaagtgact gaagaggagg tcgagaactc agtcaaacac 420

attcccagct tggcaaccgt gatcctggtg aagaccatga tacgtaaacg ctcctttggg 480

aacccattcg agggcagccg gcgggaggaa cgctcactgt cagcgcctgg aaacttgctc 540

accaaaaaac caaccaggga atgtgagtcc ctgtctgagc tcaagaccta gaaaataagt 600

ccccttcctg cctgttgcaa agtaacgtaa gagttccctc acccgagtgg atgcagacct 660

tcttgctgtc agccaccctt ccttcataca catagccagc ccaggtgacc agaacctccc 720

aggacagatg aggctttgtg tccttatgag actgggagaa cctgctgggc acccctgctg 780

caggtgctgt ggtgggtggg gaccccactg cccttcccac tgagcacatc atggctacct 840

gacttggtgg gagctccagg cagtcacttc tgtttcttaa acatagcttt actgaggtac 900

aattcacata ccatgtaatt cacccacggg aagtgtatga ttcagtggtt tctaatacag 960

acttctgcag ccattaccac cgtcaacttt acgacatttt catcagccca agaagacacc 1020

ctacactcct tagctgtccc catccaactc ccccacccca gtaaccactc agaataggta 1080

tggatttgcc tattctggac gtttcgtata aatggcgtca tacact 1126

<210> 127
<211> 3023
<212> DNA
<213> Homo sapiens

<220>
<223> II.127 (NM_033357)

<400> 127
gattctgcct ttctgctgga gggaagtgtt ttcacaggtt ctcctccttt tatcttttgt 60

gttttttttc aagccctgct gaatttgcta gtcaactcaa caggaagtga ggccatggag 120

ggaggcagaa gagccagggt ggttattgaa agtagaagaa acttcttcct gggagccttt 180

cccaccccct tccctgctga gcacgtggag ttaggcaggt taggggactc ggagactgcg 240

atggtgccag gaaagggtgg agcggattat attctcctgc cttttaaaaa gatggacttc 300

agcagaaatc tttatgatat tggggaacaa ctggacagtg aagatctggc ctccctcaag 360

ttcctgagcc tggactacat tccgcaaagg aagcaagaac ccatcaagga tgccttgatg 420

ttattccaga gactccagga aaagagaatg ttggaggaaa gcaatctgtc cttcctgaag 480

gagctgctct tccgaattaa tagactggat ttgctgatta cctacctaaa cactagaaag 540

gaggagatgg aaagggaact tcagacacca ggcagggctc aaatttctgc ctacagggtc 600

atgctctatc agatttcaga agaagtgagc agatcagaat tgaggtcttt taagtttctt 660

ttgcaagagg aaatctccaa atgcaaactg gatgatgaca tgaacctgct ggatattttc 720

atagagatgg agaagagggt catcctggga gaaggaaagt tggacatcct gaaaagagtc 780

tgtgcccaaa tcaacaagag cctgctgaag ataatcaacg actatgaaga attcagcaaa 840

gagagaagca gcagccttga aggaagtcct gatgaatttt caaatgggga ggagttgtgt 900

ggggtaatga caatctcgga ctctccaaga gaacaggata gtgaatcaca gactttggac 960

aaagtttacc aaatgaaaag caaacctcgg ggatactgtc tgatcatcaa caatcacaat 1020

tttgcaaaag cacgggagaa agtgcccaaa cttcacagca ttagggacag gaatggaaca 1080

cacttggatg caggtacagt agaacccaaa agagaaaagt aaaatatttc ttatgcctat 1140

ttttttttaa atcaaaaggg agagaacaaa agctatacca aaagggccat gtttcaagaa 1200

aatggattta aacatatttc cctgtggagg ggctttgacc acgacctttg aagagcttca 1260

ttttgagatc aagccccacg atgactgcac agtagagcaa atctatgaga ttttgaaaat 1320
ctaccaactc atggaccaca gtaacatgga ctgcttcatc tgctgtatcc tctcccatgg 1380
agacaaaggc atcatctatg gcactgatgg acaggaggcc cccatctatg agctgacatc 1440
tcagttcact ggtttgaagt gcccttccct tgctggaaaa cccaaagtgt tttttattca 1500
ggcttgtcag ggggataact accagaaagg tatacctgtt gagactgatt cagaggagca 1560
accctattta gaaatggatt tatcatcacc tcaaacgaga tatatcccgg atgaggctga 1620
ctttctgctg gggatggcca ctgtgaataa ctgtgtttcc taccgaaacc ctgcagaggg 1680
aacctggtac atccagtcac tttgccagag cctgagagag cgatgtcctc gaggcgatga 1740
tattctcacc atcctgactg aagtgaacta tgaagtaagc aacaaggatg acaagaaaaa 1800
catggggaaa cagatgcctc agcctacttt cacactaaga aaaaaacttg tcttcccttc 1860
tgattgatgg tgctattttg tttgttttgt tttgttttgt ttttttgaga cagaatctcg 1920
ctctgtcgcc caggctggag tgcagtggcg tgatctcggc tcaccgcaag ctccgcctcc 1980
cgggttcacg ccattctcct gcctcagcct cccgagtagc tgggactaca ggggcccgcc 2040
accacacctg gctaattttt taaaaatatt tttagtagag acagggtttc actgtgttag 2100
ccagggtggt cttgatctcc tgacctcgtg atccacccac ctcggcctcc caaagtgctg 2160
ggattacagg cgtgagccac cgcgcctggc cgatggtact atttagatat aacactatgt 2220
ttatttacta attttctaga ttttctactt tattaattgt tttgcacttt tttataagag 2280
ctaaagttaa ataggatatt aacaacaata acactgtctc ctttctctta cgcttaaggc 2340
tttgggaatg tttttagctg gtggcaataa ataccagaca cgtacaaaat ccagctatga 2400
atatagaggg cttatgattc agattgttat ctatcaacta taagcccact gttaatattc 2460
tattaacttt aattctcttt caaagctaaa ttccacacta ccacattaaa aaaattagaa 2520
agtagccacg tatggtggct catgtctata atcccagcac tttgggaggt tgaggtggga 2580
ggatttgctt gaacccaaga ggtccaaggc tgcagtgagc catgttcaca ccgctgcact 2640
caagcttggg tgacagagca agaccccgtc cccaaaaaaa tttttttttt aataaaccca 2700
aatttgtttg aaaactttta aaaattcaaa tgatttttac aagttttaaa taagctctcc 2760
ccaaacttgc tttatgcctt cttattgctt ttatgatata tatatgcttg gctaactata 2820
tttgcttttt gctaacaatg ctctggggtc tttttatgca tttgcatttg ctctttcatc 2880
tctgcttgga ttattttaaa tcattaggaa ttaagttatc tttaaaattt aagtatcttt 2940
tttccaaaac attttttaat agaataaaat ataatttgat cttaaaaaaa aaaaaaaaaa 3000
aaaaaaaaaa aaaaaaaaaa aaa 3023

<210> 128
<211> 2563
<212> DNA
<213> Homo sapiens

<220>

<223>  II.128 (XM_008704)

<400>  128
cgctcccctc agctcctgca gtgctaatta agggagggag cagcggggag cttgcagtga      60

ccaagagggt gttgaggcta ggaggccacg ataaacagga tacgataaaa gtccttaacc     120

aagacgcaga tgggaagaag cgttagagcg agcagcactc acatctcaag aaccagcctt     180

tcaaacagtt tccagagatg gattatccta ctttactttt ggctcttctt catgtataca     240

gagctctatg tgaagaggtg ctttggcata catcagttcc ctttgccgag aacatgtctc     300

tagaatgtgt gtatccatca atgggcatct taacacaggt ggagtggttc aagatcggga     360

cccagcagga ttccatagcc attttcagcc ctactcatgg catggtcata aggaagccct     420

atgctgagag ggtttacttt ttgaattcaa cgatggcttc caataacatg actcttttct     480

ttcggaatgc ctctgaagat gatgttggct actattcctg ctctctttac acttacccac     540

agggaacttg gcagaaggtg atacaggtgg ttcagtcaga tagttttgag gcagctgtgc     600

catcaaatag ccacattgtt tcggaacctg gaaagaatgt cacactcact tgtcagcctc     660

agatgacgtg gcctgtgcag gcagtgaggt gggaaaagat ccagccccgt cagatcgacc     720

tcttaactta ctgcaacttg gtccatggca gaaatttcac ctccaagttc ccaagacaaa     780

tagtgagcaa ctgcagccac ggaaggtgga gcgtcatcgt catccccgat gtcacagtct     840

cagactcggg gctttaccgc tgctacttgc aggccagcgc aggagaaaac gaaaccttcg     900

tgatgagatt gactgtagcc gagggtaaaa ccgataacca atataccctc tttgtggctg     960

gagggacagt tttattgttg ttgtttgtta tctcaattac caccatcatt gtcattttcc    1020

ttaacagaag gagaaggaga gagagaagag atctatttac agagtcctgg gatacacaga    1080

aggcacccaa taactataga agtcccatct ctaccagtca acctaccaat caatccatgg    1140

atgatacaag agaggatatt tatgtcaact atccaacctt ctctcgcaga ccaaagacta    1200

gagtttaagc ttattcttga catgagtgca ttagtaatga ctcttatgta ctcatgcatg    1260

gatctttatg caattttttt ccactaccca aggtctacct tagatactag ttgtctgaat    1320

tgagttactt tgataggaaa aatacttcat tacctaaaat catttttcat agaactgttt    1380

cagaaaacct gactctaact ggtttatata caaaagaaaa cttactgtat catataacag    1440

aatgatccag gggagattaa gctttgggca agggctattt accagggctt aaatgttgtg    1500

tctagaatta agtatgggca taaactggct tctgaatccc tttccagagt gttggatcca    1560

tttccctggt cttggcctca ctctcatgca ggctttcctc ttgtgttggc aagatggctg    1620

ccaactcttg gcaattcata catccttgtt tctgtctggt agagagtttg cttctcaaat    1680

ggagcaaaca aatttgatta ttttttcatt gttaaatagg caacatgacc agaaaggatg    1740

gaatggctta agtaaactaa gggttcactt ctagagctga gaagcagggt caaagcacaa    1800

tactgggcaa ttcagagcat ggttagaaga ggaaagggga gtctcaaagc tggagagttt    1860

accaacaaat attgactgca gtgattaacc aagacatttt tgttaactaa aaagtgaaat    1920

atgggatgga ttctagaaat ggggtatctc tgtccatact tctagaatcc actctatcag    1980

catagtccag aagaatacct ggcagtagaa gaaatgaata ttcaagagga agataaatgc 2040

gagagggcaa tcctttacta ttctcatatt tatttatctc tcattctgta tagaattctt 2100

gccgccatcc caggtctagc cttaggagca aatgtagtag atagtcgaat aataaataac 2160

ttaatgtttt ggacatattt tgtctacttt tgagaattat ttttaatatg taaattctct 2220

caaaagggtc aggcacctag ttattatttt ttaatgatta tgtgaaagtt gaatataata 2280

taccactaaa agtgacagtt gaaagtggtg gcataggacg gtagggtaga aatttgggag 2340

ggaaaaaaga aattgggagg gtacaggcaa caggagaaag gaatcaaacc acagaaaaat 2400

acaaagggaa acttctgctt cactattcag acaaagacag ccctaatgac atcaccaaca 2460

gtcaaagcaa ttagagacca tacctaatat tgtttaaatt ctagatgtag gctaacaatg 2520

aaaagtattt gccaaactga ataaaactgt catggttacc ttg 2563

<210> 129
<211> 3952
<212> DNA
<213> Homo sapiens

<220>
<223> II.129 (NM_030751)

<400> 129
attttagaca caagcgagag gatcatggcg gatggcccca ggtgtaagcg cagaaagcag 60

gcgaacccgc ggcgcaataa cgttacaaat tataatactg tggtagaaac aaattcagat 120

tcagatgatg aagacaaact gcatattgtg gaagaagaaa gtgttacaga tgcagctgac 180

tgtgaaggtg taccagagga tgacctgcca acagaccaga cagtgttacc agggaggagc 240

agtgaaagag aagggaatgc taagaactgc tgggaggatg acagaaagga agggcaagaa 300

atcctggggc ctgaagctca ggcagatgaa gcaggatgta cagtaaaaga tgatgaatgc 360

gagtcagatg cagaaaatga gcaaaaccat gatcctaatg ttgaagagtt tctacaacaa 420

caagacactg ctgtcatttt tcctgaggca cctgaagagg accagaggca gggcacacca 480

gaagccagtg gtcatgatga aaatggaaca ccagatgcat tttcacaatt actcacctgt 540

ccatattgtg atagaggcta taaacgcttt acctctctga aagaacacat taaatatcgt 600

catgaaaaga atgaagataa ctttagttgc tccctgtgca gttacacctt tgcatacaga 660

acccaacttg aacgtcacat gacatcacat aaatcaggaa gagatcaaag acatgtgacg 720

cagtctgggt gtaatcgtaa attcaaatgc actgagtgtg gaaaagcttt caaatacaaa 780

catcacctaa aagagcactt aagaattcac agtggagaga agccatatga atgcccaaac 840

tgcaagaaac gcttttccca ttctggctcc tatagctcac acataagcag taagaaatgt 900

atcagcttga tacctgtgaa tgggcgacca agaacaggac tcaagacatc tcagtgttct 960

tcaccgtctc tttcagcatc accaggcagt cccacacgac cacagatacg gcaaaagata 1020

gagaataaac cccttcaaga acaactttct gttaaccaaa ttaaaactga acctgtggat 1080

tatgaattca aacccatagt ggttgcttca ggaatcaact gttcaacccc tttacaaaat 1140
ggggttttca ctggtggtgg cccattacag gcaaccagtt ctcctcaggg catggtgcaa 1200
gctgttgttc tgccaacagt tggtttggtg tctcccataa gtatcaattt aagtgatatt 1260
cagaatgtac ttaaagtggc gatagatggt aatgtaataa ggcaagtgtt ggagaataat 1320
caagccaatc ttgcatccaa agaacaagaa acaatcaatg cttcacccat acaacaaggt 1380
ggccattctg ttatttcagc catcagtctt cctttggttg atcaagatgg aacaaccaaa 1440
attatcatca actacagtct tgagcagcct agccaacttc aagttgttcc tcaaaattta 1500
aaaaaagaaa atccagtcgc tacaaacagt tgtaaaagtg aaaagttacc agaagatctt 1560
actgttaagt ctgagaagga caaaagcttt gaaggggggg tgaatgatag cacttgtctt 1620
ctgtgtgatg attgtccagg agatattaat gcacttccag aattaaagca ctatgaccta 1680
aagcagccta ctcagcctcc tccactccct gcagcagaag ctgagaagcc tgagtcctct 1740
gtttcatcag ctactggaga tggcaatttg tctcctagtc agccaccttt aaagaacctc 1800
ttgtctctcc taaaagcata ttatgctttg aatgcacaac caagtgcaga agagctctca 1860
aaaattgctg attcagtaaa cctaccactg gatgtagtaa aaaagtggtt tgaaaagatg 1920
caagctggac agatttcagt gcagtcttct gaaccatctt ctcctgaacc aggcaaagta 1980
aatacccctg ccaagaacaa tgatcagcct caatctgcaa atgcaaatga accccaggac 2040
agcacagtaa atctacaaag tcctttgaag atgactaact ccccagtttt accagtggga 2100
tcaaccacca atggttccag aagtagtaca ccatccccat cacctctaaa cctttcctca 2160
tccagaaata cacagggtta cttgtacaca gctgagggtg cacaagaaga gccacaagta 2220
gaacctcttg atctttcact accaaagcaa cagggagaat tattagaaag gtcaactatc 2280
actagtgttt accagaacag tgtttattct gtccaggaag aacccttgaa cttgtcttgc 2340
gcaaaaaagg agccacaaaa ggacagttgt gttacagact cagaaccagt tgtaaatgta 2400
atcccaccaa gtgccaaccc cataaatatc gctataccta cagtcactgc ccagttaccc 2460
acaatcgtgg ccattgctga ccagaacagt gttccatgct taagagcgct agctgccaat 2520
aagcaaacga ttctgattcc ccaggtggca tacacctact caactacggt cagccctgca 2580
gtccaagaac cacccttgaa agtgatccag ccaaatggaa atcaggatga aagacaagat 2640
actagctcag aaggagtatc aaatgtagag gatcagaatg actctgattc tacaccgccc 2700
aaaaagaaaa tgcggaagac agaaaatgga atgtatgctt gtgatttgtg tgacaagata 2760
ttccaaaaga gtagttcatt attgagacat aaatatgaac acacaggtaa aagacctcat 2820
gagtgtggaa tctgtaaaaa ggcatttaaa cacaaacatc atttgattga acacatgcga 2880
ttacattctg gagaaaagcc ctatcaatgt gacaaatgtg gaaagcgctt ctcacactct 2940
gggtcttatt ctcaacacat gaatcatcgc tactcctact gtaagagaga agcggaagaa 3000
cgtgacagca cagagcagga agaggcaggg cctgaaatcc tctcgaatga gcacgtgggt 3060
gccagggcgt ctccctcaca gggcgactcg gacgagagag agagtttgac aagggaagag 3120

gatgaagaca gtgaaaaaga ggaagaggag gaggataaag agatggaaga attgcaggaa 3180

gaaaaagaat gtgaaaaacc acaaggggat gaggaagagg aggaggagga ggaagaagtg 3240

gaagaagaag aggtagaaga ggcagagaat gagggagaag aagcaaaaac tgaaggtctg 3300

atgaaggatg acagggctga aagtcaagca agcagcttag gacaaaaagt aggcgagagt 3360

agtgagcaag tgtctgaaga aaagacaaat gaagcctaat cgtttttcta gaaggaaaat 3420

aaattctaat tgataatgaa tttcgttcaa tattatcctt gcttttcatg gaaacacagt 3480

aacctgtatg ctgtgattcc tgttcactac tgtgtaaagt aaaaactaaa aaaatacaaa 3540

atacaaaaca cacacacaca cacacacaca cacacacaca cacacacaca caaaataaat 3600

ccgggtgtgc ctgaacctca gacctagtaa tttttcatgc agttttcaaa gttaggaaca 3660

agtttgtaac atgcagcaga ttagaaaacc ttaatgactc agagagcaac aatacaagag 3720

gttaaaggaa gctgattaat tagatatgca tctggcattg ttttatctta tcagtattat 3780

cactcttatg ttggtttatt cttaagctgt acaattggga gaaattttat aattttttat 3840

tggtaaacat atgctaaatc cgcttcagta ttttattatg ttttttaaaa tgtgagaact 3900

tctgcactac aaaattccct tcacagagaa gtataatgta gttccggaat tc 3952

<210> 130
<211> 70
<212> DNA
<213> Homo sapiens

<220>
<223> II.130 (D49410)

<400> 130
aaatgaaaaa aaataatagt tcactcaaac acaacttccg ggttgaaggt tcaacgattc 60

tcctcctcac 70

## Beschreibung

Die vorliegende Erfindung betrifft ein Verfahren zur in vitro Erkennung schwerer Sepsis gemäß dem Oberbegriff des Anspruch 1.

Zur Beschreibung der vorliegenden Erfindung gehört ein Sequenzprotokoll, welches die SEQ-IDs: 1 bis 130 umfasst.

Das vollständige Sequenzprotokoll ist Bestandteil der Beschreibung und somit Bestandteil der Offenbarung der vorliegenden Erfindung.

Insbesondere betrifft die vorliegende Erfindung Genaktivitätsmarker für die Diagnose und Therapieoptimierung von schwerer Sepsis.

Leider zählen Sepsis und konsekutive Organfunktionsstörungen auch heute noch zu den Haupttodesursache auf nichtkardiologischen Intensivstationen [1-3]. Man nimmt an, daß in den U.S.A. jährlich ca. 400.000 Patienten an einer Sepsis erkranken [4]. Die Letalität beträgt ca. 40 % und steigt bei Entwicklung eines Schocks auf 70-80 % an [5,6]. Die von der Grunderkrankung der Patienten und der zugrundeliegenden Infektion unabhängige Exzessletalität beträgt bis zu 35 % [8].

Im internationalen Schrifttum haben sich zwischenzeitlich ebenfalls die Kriterien der Konsensuskonferenz des ,,American College of Chest Physicians/Society of Critical Care Medicine Consensus Conference (ACCP/SCCM)" aus dem Jahr 1992 am breitesten zur Definition des Sepsisbegriffs durchgesetzt [4]. Entsprechend dieser Kriterien [4] werden die klinisch definierten Schweregrade ,,systemic inflammatory response syndrom" (SIRS), "sepsis", ,,severe sepsis" und ,,septic shock" unterschieden. Als SIRS (in diesem Patent als ,,sepsisähnlicher akuter entzündlicher Zustand übersetzt) wird dabei die systemische Antwort des inflammatorischen Systems auf einen infektiösen oder nichtinfektiösen Reiz definiert. Dazu müssen mindestens zwei der folgenden klinischen Kriterien erfüllt sein: Fieber >38°C oder Hypothermie <36°C, eine Leukozytose >12G/l oder eine Leukopenie <4G/l bzw. eine Linksverschiebung im Differentialblutbild, eine Herzfrequenz von über 90/min, eine Tachypnoe >20 Atemzüge/min oder ein PaCO₂ <4,3 kPa. Als Sepsis werden solche klinischen Zustände definiert, bei denen die SIRS-Kriterien erfüllt sind und ursächlich eine Infektion nachgewiesen wird oder zumindest sehr wahrscheinlich ist. Eine schwere Sepsis ist vom zusätzlichen Auftreten von Organfehlfunktionen gekennzeichnet. Häufige Organfehlfunktionen sind Änderungen der Bewusstseinslage, eine Oligurie, eine Laktazidose oder eine sepsisinduzierte Hypotension mit einem systolischen Blutdruck von weniger als 90 mmHg bzw. ein Druckabfall um mehr als 40 mmHg vom Ausgangswert. Wenn eine solche Hypotension nicht durch die Verabreichung von Kristalloiden und/oder Kolloiden zu beheben ist und es zusätzlich zu einer Katecholaminpflichtigkeit des Patienten kommt, so spricht man von einem septischen Schock. Dieser wird bei etwa 20 % aller Sepsispatienten nachgewiesen.

Die klinische Anwendung von Katecholaminen bei der Behandlung von Patienten mit einer schweren Sepsis ist stark subjektiv geprägt. Während viele Ärzte auf den Abfall des Blutdruckes mit der Verabreichung großer Mengen von Infusionslösungen reagieren und so den Einsatz von Katecholaminen vermeiden, gibt es auch viele Ärzte, welche ein solches Vorgehen ablehnen und bei gleicher klinischer Schwere der Erkrankung viel frühzeitiger Katecholamine verabreichen und diese auch noch höher dosieren. Konsequenz dessen ist, dass in der täglichen Praxis Patienten mit gleicher klinischer Schwere nur auf Grund rein subjektiver Gründe in die Gruppe ,,severe sepsis" oder ,,septic shock" [4] eingeordnet werden können. Vor diesem Hintergrund hat es sich im internationalen Schrifttum durchgesetzt, Patienten mit den Schweregraden ,,severe sepsis" und ,,septic shock" [4] in einer Gruppe zusammenzufassen. Der in dieser Patentschrift benutzte Begriff "schwere Sepsis" wird deshalb entsprechend der o.g. Konsensuskonferenzdefinition auf Patienten mit Sepsis plus zusätzlichem Nachweis von Organfunktionsstörungen angewendet und umfasst damit alle Patienten der Gruppen ,,severe sepsis" und ,,septic shock" entsprechend [4]

Die Sterblichkeit bei Sepsis beträgt ca. 40 % und steigt bei Entwicklung einer schweren Sepsis auf 70-80 % an [5,6]. Der Morbiditäts- und Letalitätsbeitrag von Sepsis und schwerer Sepsis ist von fachübergreifender Bedeutung. Im Vergleich dazu stieg die Häufigkeit der Erkrankungen kontinuierlich an (z.B. zwischen 1979 und 1987 um 139% von 73,6 auf 176 Krankheitsfälle je 100.000 Krankenhauspatienten) [7]. Dadurch werden in zunehmenden Maße die Behandlungeerfolge der fortgeschrittensten oder experimentellen Therapieverfahren zahlreicher medizinischer Fachgebiete (z.B. Viszeralchirurgie, Transplantationsmedizin, Hämatologie/Onkologie) gefährdet, da diesen ohne Ausnahme eine Erhöhung des Sepsisrisikos immanent ist. Die Senkung der Morbidität und Letalität einer Vielzahl von schwer erkrankten Patienten ist daher an einen gleichzeitigen Fortschritt in der Vorbeugung, Behandlung und insbesondere der Erkennung und Verlaufsbeobachtung der Sepsis und schweren Sepsis gebunden. Von namhaften Autoren wird deshalb schon lange kritisiert, dass zu Lasten einer verbesserten Sepsisdiagnose in der vergangenen Dekade zuviel Energie und finanzielle Ressourcen für die Suche nach Sepsistherapeutika aufgewendet wurden.

Sepsis ist ein Ergebnis von komplexen und stark heterogenen molekularen Vorgängen, die gekennzeichnet sind durch eine Einbeziehung von vielen Komponenten und deren Wechselwirkungen auf jeder organisatorischen Ebene des menschlichen Körpers: Gene, Zellen, Gewebe, Organe. Die Komplexität der zugrundeliegenden biologischen und immunologischen Prozesse haben viele Arten von Forschungsstudien hervorgerufen, die einen weiten Bereich klinischer Aspekte umfassen. Eines der hieraus zu erkennenden Ergebnisse war, daß die Bewertung neuer Sepsis-Therapien durch relativ unspezifische, klinisch-basierte Einschlusskriterien, welche die molekularen Mechanismen in nicht ausreichender Weise wiedergeben, erschwert wird [9].

Daher ist ein dringender Bedarf für innovative diagnostische Mittel entstanden, welche die Fähigkeit des Fachmanns verbessern sollen, Patienten mit SIRS, Sepsis, sepsisähnlichen Zuständen und schwerer Sepsis frühzeitig zu diagnostizieren, die Schwere einer SIRS auf molekularer Ebene messbar zu machen und im klinischem Verlauf vergleichbar zu gestalten, und bezüglich der individuellen Prognose und dem Ansprechen auf spezifische Behandlungen Aussagen abzuleiten.

Der Morbiditäts- und Letalitätsbeitrag der Sepsis ist von fachübergreifender Bedeutung. In den letzten Jahrzehnten veränderte sich die Letalität der Sepsis nur unwesentlich. Im Vergleich dazu stieg die Inzidenz kontinuierlich an (z.B. zwischen 1979 und 1987 um 139 % von 73,6 auf 176 pro 100.000 Krankenhauspatienten) [7]. Dadurch werden in zunehmenden Maße die Behandlungserfolge der fortgeschrittensten oder experimentellen Therapieverfahren zahlreicher Fachgebiete (Viszeralchirurgie, Transplantationsmedizin, Hämatologie/Onkologie) gefährdet, da diesen ohne Ausnahme eine Erhöhung des Sepsisrisikos immanent ist. Die Senkung der Morbidität und Letalität einer Vielzahl von schwer erkrankten Patienten ist daher an einen gleichzeitigen Fortschritt in der Prophylaxe, Behandlung und insbesondere der Diagnose der Sepsis gebunden.

Sepsis ist ein Ergebnis von stark heterogenen molekularen Vorgängen, die gekennzeichnet sind durch eine Einbeziehung von vielen Komponenten und deren Wechselwirkungen auf jeder organisatorischen Ebene des menschlichen Körpers: Gene, Zellen, Gewebe, Organe. Die Komplexität der zugrundeliegenden biologischen und immunologischen Prozesse haben viele Arten von Forschungsstudien hervorgerufen, die einen weiten Bereich klinischer Aspekte umfassen. Eines der hieraus zu erkennenden Ergebnisse war, daß die Bewertung neuer Sepsis-Therapien durch relativ unspezifische, klinisch-basierte Einschlusskriterien, welche die molekularen Mechanismen in nicht ausreichender Weise wiedergeben, erschwert wird [9].

Technologische Fortschritte, insbesondere die Entwicklung der Microarray-Technologie, versetzen den Fachmann nun in die Lage, 10 000 oder mehr Gene und deren Genprodukte gleichzeitig zu vergleichen. Die Anwendung solcher Microarray-Technologien kann nun Hinweise auf den Status von Gesundheit, Regulationsmechanismen, biochemischer Wechselwirkungen und Signalisierungsnetzwerken geben. Das Verbessern des Verständnisses darüber, wie ein Organismus auf Infektionen reagiert, sollte die Entwicklung von verstärkten Erkennungs-, Diagnose- und Behandlungsmodalitäten für systemische Erkrankungen erleichtern.

Microarrays stammen vom ,,Southern blotting" [10] ab, was die erste Herangehensweise darstellt, DNA-Moleküle in einer räumlich ansprechbaren Art und Weise auf einer festen Matrix zu immobilisieren. Die ersten Microarrays bestanden aus DNA-Fragmenten, oft mit unbekannter Sequenz, und wurden auf eine poröse Membran (normalerweise Nylon) punktweise aufgebracht. Routinegemäß wurden cDNA, genomische DNA oder Plasmid-Bilbliotheken verwendet, und das hybridisierte Material wurde mit einer radioaktiven Gruppe markiert [11-13].

Kürzlich hat es die Verwendung von Glas als Substrat und Fluoreszenz zur Detektion zusammen mit der Entwicklung neuer Technologien für die Synthese und für das Aufbringen der Nukleinsäuren in sehr hohen Dichten erlaubt, die Nukleinsäurearrays zu miniaturisieren bei gleichzeitiger Erhöhung des experimentellen Durchsatzes und des Informationsgehaltes [14-16].

Weiterhin ist aus WO 03/002763 bekannt, dass Microarrays grundsätzlich für die Diagnose von Sepsis und sepsisähnlichen Zuständen verwendet werden können.

Eine Begründung für die Anwendbarkeit der Microarray-Technologie wurde zunächst durch klinische Untersuchungen auf dem Gebiet der Krebsforschung geliefert. Hier haben Expressionsprofile ihre Nützlichkeit bei der Identifizierung von Aktivitäten einzelner Gene oder Gengruppen gezeigt, die mit bestimmten klinischen Phänotypen korrelieren [17]. Durch die Analyse vieler Proben, die von Individuen mit oder ohne akute Leukämie oder diffuse Lymphome großer B-Zellen stammten, wurden Genexpressionsmarker (RNA) gefunden und auf die Klassifizierung dieser Krebsarten angewandt [17,18]. Golub et al. haben herausgefunden, daß verläßliche Vorhersagen nicht aufgrund von irgendeinem einzelnen Gen gemacht werden können, aber daß Vorhersagen, die auf den Expressionsspiegeln von 53 Genen (ausgewählt aus über 6000 Genen, die auf den Arrays vertreten waren) basieren, sehr genau sind [17].

Alisadeh et al. [18] untersuchten große B-Zell Lymphome (DLBCL). Die Autoren erarbeiteten Expressionsprofile mit einem "Lymphochip", einem Microarray, der 18 000 Klone komplementärer DNA trug und entwickelt worden war, um Gene zu überwachen, die in normale und abnormale Lymphozytenentwicklung involviert sind. Unter Verwendung von Cluster-Analyse waren sie in der Lage, DILBCL in zwei Kategorien einzuteilen, welche starke Unterschiede bezüglich der Überlebenschancen der Patienten aufzeigten. Die Genexpressionsprofile dieser Untergruppen entsprachen zwei bedeutsamer Stadien der B-Zelldifferenzierung.

Besonders wertvoll hat sich die Bestimmung von Genexpressionsprofilen zur differentialdiagnostischen Unterscheidung von Krankheitserscheinungen, die auf systemische Microbielle Infektionen zurückzuführen sind, von anderen Krankheitserscheinungen nichtinfektiöser Ätiologie erwiesen, die aufgrund ihres klinischen Erscheinungsbildes auf eine Sepsis hindeuten könnten, jedoch in Wirklichkeit nicht auf eine systemische Microbielle Infektion zurückzuführen sind, z.B. von Krankheitserscheinungen, die auf nicht-infektiöse Entzündungen einzelner Organe zurückzuführen sind [19-22]. Die Messung von Genexpressionprofilen zur Diagnose von SIRS aus Körperflüssigkeiten wurde noch nicht beschrieben.

Ausgangspunkt für die in der vorliegenden Patentanmeldung offenbarten Erfindung ist die Erkenntnis, daß vor der Diagnose von SIRS, Sepsis, sepsisähnlichen Zuständen und schwerer Sepsis in biologischen Proben eines Individuums sich von normalen Werten unterscheidende RNA-Spiegel, bzw. sich davon ableitbare Peptid- und Teilpeptid-Spiegel in einem Serum oder Plasma eines Patienten, bei dem ein SIRS-Risiko besteht bzw. bei dem SIRStypische Krankheitserscheinungen festgestellt werden, feststellbar sind.

Der vorliegenden Erfindung liegt somit die Aufgabe zugrunde, ein Verfahren zur Verfügung zu stellen, das die Erkennung, die Beurteilung des Schweregrads und/oder die therapiebegleitende Verlaufsbeurteilung von schwerer Sepsis ermöglicht.

Diese Aufgabe wird durch ein Verfahren mit den kennzeichnenden Merkmalen des Anspruchs 1 gelöst.

Ein in der Stammanmeldung zu der vorliegenden Teilanmeldung beanspruchtes Verfahren ist dadurch gekennzeichnet, daß man in einer Probe einer biologischen Flüssigkeit eines Individuums die Aktivität eines oder mehrerer Markergene bestimmt und aus der festgestellten Anwesenheit und/oder Menge des bestimmten Genprodukts SIRS und/oder den Erfolg einer therapeutischen Behandlung ableiten kann.

Die Erfindung ist dadurch gekennzeichnet, dass ein Verfahren zur in vitro Erkennung von schwerer Sepsis, folgende Schritte umfasst:
a) Isolieren von Proben-RNA aus einer aus einem Menschen stammenden Probe;
b) Markieren der Proben-RNA und/oder wenigstens einer DNA, die ein für schwere Sepsis spezifisches Gen oder Genfragment ist, mit einem detektierbaren Marker;
c) In-Kontakt-Bringen der Proben-RNA mit der DNA unter Hybridisierungsbedingungen;
d) In-Kontakt-Bringen von Kontroll-RNA, welche eine Kontrolle für nichtpathologische Zustände darstellt, mit wenigstens einer DNA, unter Hybridisierungsbedingungen, wobei die DNA ein für schwere Sepsis spezifisches Gen oder Genfragment ist;
e) quantitatives Erfassen der Markierungssignale der hybridisierten Proben-RNA und der Kontroll-RNA;
f) Vergleichen der quantitativen Daten der Markierungssignale, um eine Aussage zu treffen, ob für schwere Sepsis spezifische Gene oder Genfragmente in der Probe stärker oder schwächer exprimiert sind als in der Kontrolle;
g) wobei das für schwere Sepsis spezifische Gen oder Genfragment ausgewählt wird aus der Gruppe bestehend aus SEQ-ID No. 1 bis SEQ-ID No. 130, sowie Genfragmenten davon mit 20-200 Nucleotiden.
Eine Ausführungsform der Erfindung ist dadurch gekennzeichnet, daß man die Kontroll-RNA vor dem Messen der Proben-RNA mit der DNA hybridisiert und die Markierungssignale des Kontroll-RNA/DNA-Komplexes erfaßt und gegebenenfalls in Form einer Kalibrierkurve oder -tabelle ablegt.

Eine weitere Ausführungsform der Erfindung ist dadurch gekennzeichnet, daß als Proben-RNA mRNA verwendet wird.

Eine weitere Ausführungsform der Erfindung ist dadurch gekennzeichnet, daß die DNA an vorbestimmten Bereichen auf einem Träger in Form eines Microarrays angeordnet, insbesondere immobilisiert, wird.

Eine weitere Ausführungsform der Erfindung ist dadurch gekennzeichnet, daß das Verfahren zur differentialdiagnostischen Früherkennung, zur Kontrolle des therapeutischen Verlaufs, zur Risikoabschätzung für Patienten sowie zur post mortem Diagnose von schwerer Sepsis eingesetzt wird.

Eine weitere Ausführungsform der Erfindung ist dadurch gekennzeichnet, daß die Probe ausgewählt wird aus: Körperflüssigkeiten, insbesondere Blut, Liquor, Urin, Ascitesflüssigkeit, Seminalflüssigkeit, Speichel, Punktat; Zellinhalt oder eine Mischung davon.

Eine weitere Ausführungsform der Erfindung ist dadurch gekennzeichnet, daß Zellproben gegebenenfalls einer lytischen Behandlung unterzogen werden, um deren Zellinhalte freizusetzen.

Eine weitere Ausführungsform der Erfindung ist dadurch gekennzeichnet, daß das für schwere Sepsis spezifische Gen oder Genfragment ausgewählt wird aus der Gruppe bestehend aus SEQUENZ-ID No. 1 bis SEQUENZ-ID No. 130, sowie Genfragmenten davon mit 20-200, bevorzugt 20-80 Nukleotiden.

Eine weitere Ausführungsform der Erfindung ist dadurch gekennzeichnet, dass die immobilisierten Sonden markiert werden. Für diese Ausführungsform finden selbstkomplementäre Oligonukleotide, so genannte Molecular beacons, als Sonden Verwendung. Sie tragen an ihren Enden ein Fluorophor/Quencher-Paar, so daß sie in Abwesenheit einer komplementären Sequenz in einer gefalteten Haarnadelstruktur vorliegen und erst mit einer entsprechenden Probensequenz ein Fluoreszenzsignal liefern. Die Haarnadelstruktur der Molecular Beacons ist so lange stabil, bis die Probe an der spezifischen Fängersequenz hybridisiert, was zu einer Konformationsänderung und damit auch Freisetzung der Reporterfluoreszenz führt.

Eine weitere Ausführungsform der Erfindung ist dadurch gekennzeichnet, daß wenigstens 2 bis 100 unterschiedliche cDNAs verwendet werden.

Eine weitere Ausführungsform der Erfindung ist dadurch gekennzeichnet, daß als detektierbarer Marker ein radioaktiver Marker, insbesondere ³²P, ¹⁴C, ¹²⁵I, ¹⁵⁵Eu, ³³P oder ³H verwendet wird.

Eine weitere Ausführungsform der Erfindung ist dadurch gekennzeichnet, daß als detektierbarer Marker ein nichtradioaktiver Marker, insbesondere ein Farb-oder Fluoreszenzmarker, ein Enzymmarker oder Immunmarker, und/oder quantum dots oder ein elektrisch messbares Signal, insbesondere Potential-und/oder Leitfähigkeits- und/oder Kapazitätsänderung bei Hybridisierungen, verwendet wird.

Eine weitere Ausführungsform der Erfindung ist dadurch gekennzeichnet, daß die Proben-RNA und Kontroll-RNA dieselbe Markierung tragen.

Eine weitere Ausführungsform der Erfindung ist dadurch gekennzeichnet, daß die Proben-RNA und Kontroll-RNA unterschiedliche Markierungen tragen.

Eine weitere Ausführungsform der Erfindung ist dadurch gekennzeichnet, daß die cDNA-Sonden auf Glas oder Kunststoff, immobilisiert werden.

Eine weitere Ausführungsform der Erfindung ist dadurch gekennzeichnet, daß die einzelnen cDNA Moleküle über eine kovalente Bindung an das Trägermaterial immobilisiert werden.

Eine weitere Ausführungsform der Erfindung ist dadurch gekennzeichnet, daß die einzelnen cDNA Moleküle mittels Adsorption, insbesondere mittels elektrostatischer- und/oder Dipol-Dipol- und/oder hydrophober Wechselwirkungen und/oder Wasserstoffbrücken an das Trägermaterial immobilisiert werden.

Eine weitere Ausführungsform der Erfindung ist dadurch gekennzeichnet, daß das Verfahren zur differentialdiagnostischen Früherkennung, zur Kontrolle des therapeutischen Verlaufs, zur Risikoabschätzung für Patienten sowie zur post mortem Diagnose von schwerer Sepsis eingesetzt wird.

Eine weitere Ausführungsform der Erfindung ist dadurch gekennzeichnet, daß das für schwere Sepsis spezifische Peptid ein Expressionsprodukt eines Gens oder Genfragmentes ist, welches ausgewählt wird aus der Gruppe bestehend aus SEQUENZ-ID No. 1 bis SEQUENZ-ID No. 130, sowie Genfragmenten davon mit 20-200, bevorzugt 20-80 Nukleotiden.

Eine weitere Ausführungsform der Erfindung ist dadurch gekennzeichnet, daß wenigstens 2 bis 100 unterschiedliche Peptide verwendet werden.

Eine weitere Ausführungsform der Erfindung ist dadurch gekennzeichnet, daß als detektierbarer Marker ein radioaktiver Marker, insbesondere ³²P, ¹⁴C, ¹²⁵I, ¹⁵⁵Ep, ³³P oder ³H verwendet wird.

Eine weitere Ausführungsform der Erfindung ist dadurch gekennzeichnet, daß als detektierbarer Marker ein nichtradioaktiver Marker, insbesondere ein Farb-oder Fluoreszenzmarker, ein Enzymmarker oder Immunmarker und/oder quantum dots oder ein elektrische messbares Signal, insbesondere Potential-und/oder Leitfähigkeits- und/oder Kapazitätsänderung bei Hybridisierungen, verwendet wird.

Eine weitere Ausführungsform der Erfindung ist dadurch gekennzeichnet, daß die Proben-Peptide und Kontroll-Peptide dieselbe Markierung tragen.

Eine weitere Ausführungsform der Erfindung ist dadurch gekennzeichnet, daß die Proben-Peptide und Kontroll-Peptide unterschiedliche Markierungen tragen.

Eine weitere Ausführungsform der Erfindung ist dadurch gekennzeichnet, daß die Peptid-Sonden auf Glas oder Kunststoff, immobilisiert werden.

Eine weitere Ausführungsform der Erfindung ist dadurch gekennzeichnet, daß die einzelnen Peptidmoleküle über eine kovalente Bindung an das Trägermaterial immobilisiert werden.

Eine weitere Ausführungsform der Erfindung ist dadurch gekennzeichnet, daß die einzelnen Peptidmoleküle mittels Adsorption, insbesondere mittels elektrostatischer- und/oder Dipol-Dipol- und/oder hydrophober Wechselwirkungen und/oder Wasserstoffbrücken an das Trägermaterial immobilisiert werden an das Trägermaterial immobilisiert werden.

Eine weitere Ausführungsform der Erfindung ist dadurch gekennzeichnet, daß die einzelnen Peptidmoleküle mittels monoklonaler Antikörper oder deren bindenden Fragmenten erkannt werden.

Eine weitere Ausführungsform der Erfindung ist dadurch gekennzeichnet, daß das Bestimmen einzelner Peptide mittels Immunoassay, oder Präzipitationsassay unter Verwendung monoklonaler Antikörper durchgeführt wird.

Es ist dem Fachmann klar, daß die in den Ansprüchen dargelegten einzelnen Merkmale der Erfindung ohne Einschränkung beliebig miteinander kombinierbar sind.

Als Markergene im Sinne der Erfindung werden alle abgeleiteten DNA-Sequenzen, Partialsequenzen und synthetischen Analoga (beispielsweise Peptido-Nukleinsäuren, PNA) verstanden. Weiterhin werden im Sinne der Erfindung alle von den Markergenen kodierten Proteine, Peptide bzw. Partialsequenzen oder synthetische Peptidomimetica verstanden. Die auf Bestimmung der Genexpression auf RNA-Ebene bezogene Beschreibung der Erfindung stellt keine Einschränkung sondern nur eine beispielhafte Anwendung dar.

Die auf Blut bezogene Beschreibung der Erfindung stellt nur eine beispielhafte Anwendung der Erfindung dar. Als biologische Flüssigkeiten im Sinne der Erfindung werden alle Körperflüssigkeiten des Menschen verstanden.
Eine Anwendung des erfindungsgemäßen Verfahrens liegt in der Messung der differentiellen Genexpression bei schwerer Sepsis. Hierzu wird die RNA aus dem Vollblut von entsprechenden Patienten und eine Kontrollprobe eines gesunden Probanden oder eines nicht an einer der erwähnten Krankheiten erkrankten Patienten isoliert. Die RNA wird anschließend markiert, beispielsweise radioaktiv mit ³²P oder mit Farbstoffmolekülen (Fluoreszenz). Als Markierungsmoleküle können alle im Stand der Technik zu diesem Zwecke bekannten Moleküle und/oder Detektionssignale eingesetzt werden. Entsprechende Moleküle und/oder Verfahren sind dem Fachmann ebenfalls bekannt.

Die so markierte RNA wird anschließend mit auf einem Microarray immobilisierten cDNA-Molekülen hybridisiert. Die auf dem Microarray immobilisierten cDNA-Moleküle stellen eine spezifische Auswahl der Gene gemäß Anspruch 1 dieser Erfindung für die Messung von schwerer Sepsis, dar.

Die Intensitätssignale der hybridisierten Moleküle werden im Anschluss durch geeignete Messgeräte (Phosporimager, Microarray-Scanner) gemessen und durch weitere softwaregestützte Auswertungen analysiert. Aus den gemessenen Signalintensitäten werden die Expressionsverhältnisse zwischen der Patientenprobe und der Kontrolle bestimmt. Aus den Expressionsverhältnissen der unter- und/oder überregulierten Gene lassen sich, wie in den nachstehend dargestellten Experimenten, Rückschlüsse auf das Vorhandensein von schwerer Sepsis ziehen.

Eine weitere Anwendung des erfindungsgemäßen Verfahrens besteht in der Messung der differentiellen Genexpression für die therapiebegleitende Bestimmung der Wahrscheinlichkeit, daß Patienten auf die geplante Therapie ansprechen werden, und/oder für die Bestimmung des Ansprechens auf eine spezialisierte Therapie und/oder auf die Festlegung des Therapieendes im Sinne eines ,,drug monitoring" bei Patienten mit schwerer Sepsis. Hierzu wird aus den in zeitlichen Abständen gesammelten Blutproben des Patienten die RNA (Proben-RNA) isoliert. Die verschiedenen RNA-Proben werden zusammen mit der Kontrollprobe markiert und mit ausgewählten Genen, welche auf einem Microarray immobilisiert sind, hybridisiert. Aus den jeweiligen Expressionsverhältnissen läßt sich somit beurteilen, welche Wahrscheinlichkeit besteht, daß Patienten auf die geplante Therapie ansprechen werden und/oder ob die begonnene Therapie wirksam ist und/oder wie lange die Patienten noch entsprechend therapiert werden müssen und/oder ob der maximale Therapieeffekt mit der verwendeten Dosis und Dauer schon erreicht worden ist.

Eine weitere Anwendung des erfindungsgemäßen Verfahrens besteht in der Messung des Bindungsgrades von Proteinen, beispielsweise monoklonaler Antikörper, mittels der Verwendung von Immunoassays, Protein- oder Peptidarrays oder Präpitationsassays. Durch die Bestimmung der Konzentration der von den Sequenzen der in Anwendungsbeispiel 1 aufgeführten Nukleinsäuren entsprechenden Proteine oder Peptide kann auf ein erhöhtes Risiko zur Entwicklung einer SIRS geschlossen werden. Weiterhin ermöglicht diese Verfahrenweise die differentialdiagnostische Erkennung bei Patienten mit SIRS, Sepsis, sepsisähnlichen Zuständen und schwerer Sepsis.

Ebenso kann auf ein erhöhtes Risiko zur Entwicklung einer Sepsis, sepsisähnlichen Zuständen und schwerer Sepsis geschlossen werden.

Weitere Vorteile und Merkmale der vorliegenden Erfindung ergeben sich aufgrund der Beschreibung eines Ausführungsbeispieles.

### Ausführungsbeispiel - schwere Sepsis:

Untersuchungen zur differentiellen Genexpression bei Patienten mit schwerer Sepsis.

Für die Messung der differentiellen Genexpression bei schwerer Sepsis wurden Untersuchungen von Vollblutproben von Patienten, welche auf einer operativen Intensivstation behandelt worden, durchgeführt.

Als Kontrollproben dienten Vollblutproben von Patienten nach einer unkomplizierten neurochirurgischen Operation, welche auf der gleichen Intensivstation behandelt wurden. Keiner dieser Patienten entwicklete während seiner gesamten stationären Behandlung eine Infektion und/oder wies klinische Zeichen einer generalisierten Entzündungsreaktion (definiert entsprechend SIRS-Kriterien [4]) auf.

Zusätzlich wurden Vollblutproben von sechs männlichen und zwei weiblichen Patienten abgenommen (Patientenproben). Jeder dieser Patienten entwickelte im Zeitraum von 24 Stunden vor Vollblutentnahme neu eine schwere Sepsis mit Organfunktionsstörung. Ausgewählte Charakteristika der Patienten mit schwerer Sepsis sind in Tabelle 1 dargestellt. Dabei werden Angaben zum Alter, Geschlecht, der Ursache der schweren Sepsis (siehe Diagnose) sowie klinischer Schwere, gemessen anhand der im klinischen Schrifttum gut belegten APACHE-II- und SOFA-Scores (jeweils in Punkte), gemacht. Gleichfalls sind die Plasmaproteinspiegel von Procalcitonin (PCT), einem neuartigen Sepsismarker, und der individuelle Überlebensstatus angegeben.

**Tabelle1: Daten der Patientengruppe**

| **Alter** | **Geschlecht** | **Diagnose** | **Klassifikation nach [4]** | **APACHE-II Score [Punkte]** | **SOFA Score [Punkte]** | **PCT [ng/ml]** | **Überlebensstatus** |
|---|---|---|---|---|---|---|---|
| 68 | weiblich | Peritonitis | severe sepsis | 17 | 4 | 269 | gestorben |
| 39 | männlich | ARDS | septic shock | 17 | 11 | 0,39 | gestorben |
| 36 | männlich | Peritonitis | septic shock | 11 | 7 | 9,77 | überlebt |
| 80 | männlich | Peritonitis | severe sepsis | 28 | 4 | 23,61 | überlebt |
| 32 | männlich | bakterielle Pancreatitis | septic shock | 21 | 7 | 1,69 | überlebt |
| 73 | männlich | ARDS | septic shock | 16 | 14 | 9,96 | gestorben |
| 67 | männlich | ARDS | septic shock | 24 | 12 | 12,88 | überlebt |
| 76 | weiblich | Peritonitis | septic shock | 30 | 11 | 4,19 | gestorben |

Nach Abnahme des Vollblutes wurde die totale RNA unter Anwendungen des PAXGene Blood RNA Kit gemäß den Vorgaben des Herstellers (Qiagen) isoliert. Im Anschluss wurde aus der totalen RNA die cDNA mittels reverser Transkrition mittels Superscript II RT (Invitrogen) nach dem Protokoll des Herstellers synthetisiert, mit Aminoallyl-dUTP und Succinimidylester von der Fluoreszenzfarbstoffe Cy3 und Cy5 (Amersham) markiert und hydrolisiert.

Für die Hybridisierung wurden die Microarrays (Lab-Arraytor human 500-1 cDNA) der Firma SIRS-Lab GmbH verwendet. Diese Microarrays sind mit 340 humanen cDNA-Molekülen bestückt. Die 340 humanen cDNA-Moleküle sind auf jedem Microarray 3-fach in drei Subarrays immobilisiert.

Die vorbereiteten und markierten Proben wurden mit den Microarrays entsprechend den Anweisungen des Herstellers hybridisiert und im Anschluss gewaschen. Die Fluoreszenzsignale der hybridisierten Moleküle wurden mittels eines Auslesegerätes (AXON 4000B) gemessen.

### Auswertung

Die Auswertung eines Experiments erfolgte aufgrund von eingescannten Bildern der Microarrays nach der Hybridisierung. Der mittlere Intensitätswert der detektierten Spots wurde als der gemessene Expressionswert des zugehörigen Gens definiert. In einer Bildanalyse wurden Spots automatisch erkannt und ihre Homogenität überprüft. Die Analyse wurde manuell kontrolliert. Die ermittelten Signale beinhalteten neben der gewünschten Information, nämlich der Menge gebundener Nukleinsäuren aber auch Hintergrundsignale, welche durch unspezifische Bindungen an der Membranoberfläche verursacht wurden. Die Definition des Hintergrundbereichs ermöglichte eine optimale Unterscheidung zwischen Spots und der Chipoberfläche, welche ebenfalls Farbeffekte aufwies. In der Auswertung der Microarrays wurden leere Spots als Hintergrund gewählt. Der mittlere Expressionswert ausgewählter leeren Spots innerhalb eines Blocks (von 14 mal 14 Spots) wurde von Expressionswerten der Gen-Spots (im entsprechenden Block) subtrahiert.

Punktuelle Signale, die nicht durch Bindung von Nukleinsäuren sondern durch Staubpartikel oder sonstige Störungen auf dem Filter verursacht wurden, konnten von realen Spots durch ihre Unregelmäßigkeit der Form unterschieden werden und wurden von der weiteren Analyse ausgeschlossen.

Um die Werte zwischen den 3 Subarrays und zwischen verschiedenen Microarrays miteinander vergleichbar zu machen, wurde anschließend eine Normalisierung der Daten notwendig. Wegen der hohen Anzahl der Spots auf dem Microarray wurde als Normalisierungsreferenz der Mittelwert aller Expressionswerte festgelegt. Für die Berechnung der mittleren Expression pro Gen wurden die zwei (aus drei) Wiederholungen gewählt, welche am nächsten zueinander lagen.

Aus den Signalintensitäten wurden mittels der Software AIDA Array Evaluation die Expressionsverhältnisse zwischen Kontroll- und Patientenproben Probe berechnet. Die Höhe des Expressionsverhältnisses jedes Gens stellte das Kriterium für eine Sortierung der untersuchten Gene dar. Von Interesse waren die Gene, die in den Patientenproben gegenüber Kontrollproben am meisten überexprimiert bzw. unterexprimiert wurden.

Aus Tabelle 2 ist ersichtlich, dass 41 Gene der Patientenprobe gefunden wurden, die gegenüber der Kontrollprobe signifikant überexprimiert waren. Weiterhin wird aus Tabelle 3 deutlich, dass 89 Gene der Patientenprobe signifikant unterexprimiert gegenüber der Kontrollprobe waren. Aus den Ergebnissen wird deutlich, dass die in Tabelle 2 und Tabelle 3 aufgeführten Gene mit dem Auftreten einer schweren Sepsis korrelieren. Auch korrelieren diese Ergebnisse mit der klinischen Eingruppierung entsprechend [4] sowie PCT-Konzentrationen der Patienten, welche typisch sind für das Auftreten einer schweren Sepsis [23]. Somit stellen die aufgeführten Genaktivitäten der Gene Marker für eine Diagnose einer schweren Sepsis dar.

**Tabelle 2: Expressionverhältnis überexprimierter Gene zwischen Patienten- und Kontrollprobe**

| **GenBank Accession-Nr.** | **HUGO Name** | **Expressionsverhältnis überexprimierter Genegegenüber der Kontrolle** | **SEQUENZ-ID** |
|---|---|---|---|
| XM_086400 | S100A8 | 4.4 | 1 |
| XM_001682 | S100A12 | 3.03 | 2 |
| NM_002619 | PF4 | 2.21 | 3 |
| NM_002704 | PPBP | 1.66 | 4 |
| NM_001101 | ACTB | 1.65 | 5 |
| NM_001013 | RPS9 | 1.61 | 6 |
| XM_057445 | SELP | 1.61 | 7 |
| BC018761 | IGKC | 1.53 | 8 |
| XM_030906 | TGFB1 | 1.51 | 9 |
| NM_001760 | CCND3 | 1.48 | 10 |
| XM_035922 | IL11 | 1.28 | 11 |
| XM_039625 | DUSP10 | 1.17 | 12 |
| XM_002762 | TNFAIP6 | 1.17 | 13 |
| XM_015396 | ALOX5AP | 1.15 | 14 |
| NM_003823 | TNFRSF6B | 1.15 | 15 |
| XM_029300 | DPP4 | 1.15 | 16 |
| NM_001562 | IL18 | 1.14 | 17 |
| NM_005037 | PPARG | 1.11 | 18 |
| M90746 | FCGR3B | 1.07 | 19 |
| NM_001315 | MAPK14 | 0.99 | 20 |
| BC001506 | CD59 | 0.88 | 21 |
| XM_042018 | BSG | 0.88 | 22 |
| XM_010177 | DUSP9 | 0.87 | 23 |
| BC013992 | MAPK3 | 0.84 | 24 |
| NM_001560 | IL13RA1 | 0.82 | 25 |
| NM_004555 | NFATC3 | 0.74 | 26 |
| NM_001154 | ANXA5 | 0.73 | 27 |
| NM_001310 | CREBL2 | 0.7 | 28 |
| XM_036107 | ITGB2 | 0.65 | 29 |
| XM_009064 | JUNB | 0.65 | 30 |
| NM_001774 | CD37 | 0.62 | 31 |
| XM_049849 | TNFRSF14 | 0.6 | 32 |
| NM_003327 | TNFRSF4 | 0.57 | 33 |
| BC001374 | CD151 | 0.56 | 34 |
| XM_051958 | ALOX5 | 0.56 | 35 |
| NM_021805 | SIGIRR | 0.5 | 36 |
| NM_017526 | HSOBRGR | 0.48 | 37 |
| XM_011780 | DAPK1 | 0.46 | 38 |
| NM_006017 | PROML1 | 0.44 | 39 |
| D49410 | IL3RA | 0.43 | 130 |
| XM_027885 | RPL13A | 0.33 | 40 |

**Tabelle 3: Expressionverhältnis unterexprimierter Gene zwischen Patienten- und Kontrollprobe**

| **GeneBank Accession-Nr.** | **HUGO Name** | **Expressionsverhältnis unterexprimierter Genegegenüber der Kontrolle** | **SEQUENZ-ID** |
|---|---|---|---|
| NM_007289 | MME | -2.11 | 41 |
| XM_008411 | SCYA13 | -2.06 | 42 |
| XM_055188 | ENG | -2.01 | 43 |
| NM_021073 | BMP5 | -1.99 | 44 |
| XM_007417 | TGFB3 | -1.93 | 45 |
| NM 001495 | GFRA2 | -1.88 | 46 |
| XM_009475 | AHCY | -1.86 | 47 |
| XM_006738 | CD36L1 | -1.86 | 48 |
| NM_001772 | CD33 | -1.86 | 49 |
| NM 057158 | DUSP4 | -1.83 | 50 |
| XM_058179 | CD244 | -1.77 | 51 |
| NM_001770 | CD19 | -1.75 | 52 |
| NM_004931 | CD8B1 | -1.73 | 53 |
| XM_006454 | CD3G | -1.71 | 54 |
| XM_041847 | TNF | -1.65 | 55 |
| NM_45319 | MAP3K6 | -1.62 | 56 |
| XM_045985 | ITGA2B | -1.61 | 57 |
| XM_055756 | TIMP1 | -1.61 | 58 |
| NM_004740 | TIAF1 | -1.61 | 59 |
| XM_008432 | ITGA3 | -1.57 | 60 |
| XM_034770 | PAFAH1B1 | -1.56 | 61 |
| NM 014326 | DAPK2 | -1.55 | 62 |
| XM_043864 | PIK3R1 | -1.49 | 63 |
| U54994 | CCR5 | -1.49 | 64 |
| NM_004089 | DSIPI | -1.49 | 65 |
| XM_037260 | F2R | -1.45 | 66 |
| NM_172217 | IL16 | -1.45 | 67 |
| AF244129 | LY9 | -1.45 | 68 |
| NM_003775 | EDG6 | -1.43 | 69 |
| NM 001781 | CD69 | -1.41 | 70 |
| NM_019846 | CCL28 | -1.39 | 71 |
| NM_001511 | CXCL1 | -1.38 | 72 |
| NM_006505 | PVR | -1.33 | 73 |
| NM 000075 | CDK4 | -1.33 | 74 |
| XM_042066 | MAP3K1 | -1.32 | 75 |
| NM_003242 | TGFBR2 | -1.31 | 76 |
| NM_003874 | CD84 | -1.31 | 77 |
| XM_033972 | ATF6 | -1.3 | 78 |
| XM_001840 | PLA2G2A | -1.3 | 79 |
| NM_018310 | BRF2 | -1.29 | 80 |
| AF212365 | IL17BR | -1.25 | 81 |
| XM_056798 | CD81 | -1.25 | 82 |
| NM 000061 | BTK | -1.24 | 83 |
| XM_001472 | JUN | -1.23 | 84 |
| XM_007258 | TNFAIP2 | -1.23 | 85 |
| XM_048555 | IFNAR2 | -1.23 | 86 |
| | | | |
| XM_041060 | FOS | -1.23 | 87 |
| XM_056556 | TNFSF7 | -1.23 | 88 |
| XM_016747 | LTBP1 | -1.22 | 89 |
| XM_006953 | TNFRSF7 | -1.21 | 90 |
| NM_015927 | TGFB1I1 | -1.19 | 91 |
| XM_010807 | INHBB | -1.16 | 92 |
| NM_002184 | IL6ST | -1.14 | 93 |
| XM_008570 | VAMP2 | -1.13 | 94 |
| NM_006856 | ATF7 | -1.1 | 95 |
| NM_000674 | ADORA1 | -1.09 | 96 |
| NM_000173 | GP1BA | -1.08 | 97 |
| XM_048068 | SCYD1 | -1.07 | 98 |
| NM_022162 | CARD15 | -1.07 | 99 |
| NM_001199 | BMP1 | -1.02 | 100 |
| NM_000960 | PTGIR | -1.01 | 101 |
| XM_012039 | FUT4 | -0.99 | 102 |
| XM_034166 | NOS2A | -0.99 | 103 |
| NM_003188 | MAP3K7 | -0.98 | 104 |
| NM_006609 | MAP3K2 | -0.98 | 105 |
| XM_027358 | PCMT1 | -0.95 | 106 |
| XM_007189 | FOXO1A | -0.93 | 107 |
| XM_030523 | MAP3K8 | -0.92 | 108 |
| XM_002923 | CCR2 | -0.88 | 109 |
| XM_054837 | TNFRSF1B | -0.87 | 110 |
| NM_000634 | IL8RA | -0.87 | 111 |
| NM_004590 | CCL16 | -0.86 | 112 |
| XM_012717 | CSNK1D | -0.86 | 113 |
| XM_012649 | SCYA7 | -0.84 | 114 |
| XM_008679 | TP53 | -0.84 | 115 |
| XM_030509 | PTGIS | -0.83 | 116 |
| XM_039086 | CDW52 | -0.82 | 117 |
| XM_027978 | CFLAR | -0.81 | 118 |
| NM_005343 | HRAS | -0.79 | 119 |
| XM_043574 | DAP3 | -0.78 | 120 |
| NM_002188 | IL13 | -0.77 | 121 |
| XM_055699 | ENTPD1 | -0.72 | 122 |
| NM_000565 | IL6RA | -0.67 | 123 |
| NM_002211 | ITGB1 | -0.65 | 124 |
| XM_049864 | CSF3 | -0.63 | 125 |
| XM_045933 | CAM KK2 | -0.63 | 126 |
| NM_033357 | CASP8 | -0.55 | 127 |
| XM_008704 | DNAM-1 | -0.52 | 128 |
| NM_030751 | TCF8 | -0.5 | 129 |

Diese charakteristischen Veränderungen sind für das erfindungsgemäße Verfahren ausnutzbar.

Die in den Tabellen 2 und 3 aufgeführten GenBank Accession Nummern (Internet-Zugang über http://www.ncbi.nlm.nih.gov/) der einzelnen Sequenzen sind in dem dieser Anmeldung angefügten Sequenzprotokoll, das somit Teil der Erfindung ist, im Einzelnen jeweils einer SEQUENZ-ID (SEQ-ID: 1 bis zur SEQ-ID: 130) zugeordnet. Dieses Sequenzprotokoll ist Teil der vorliegenden Erfindung.

### Literatur

1. Natanson C, Hoffmann WD, Suffredini A, Eichacker PQ, and Danner RL: Selected treatment strategies for septic shock based on proposed mechanisms of pathogenesis. Ann Intern Med 1994;120:771-783
2. Danner RL, Elin RJ, Hosseini JM, Wesley RA, Reilly JM, Parillo JE: Endotoxemia in human septic shock. Chest 1991; 99: 169-175
3. Niederman MS, Fein AM: Sepsis syndrome, the adult respiratory distress syndrome, and nosocomial pneumonia. A common clinical sequence. Clin Chest Med 1990 ;11: 633-656
4. American College of Chest Physicians/Society of Critical Care Medicine Consensus Conference: definitions for sepsis and organ failure and guidelines for the use of innovative therapies in sepsis.. Crit Care Med 1992; 20: 864-874
5. Brun-Buisson C, Doyon F, Carlet J, Dellamonica P, Gouin F, Lepoutre A, Mercier JC, Offenstadt G, Regnier B: Incidence, risk factors, and outcome of severe sepsis and septic shock in adults. A multicenter prospective study in intensive care units. French ICU Group for Severe Sepsis. JAMA 1995; 274: 968-974
6. Le-Gall JR, Lemeshow S, Leleu G, Klar J, Huillard J, Rue M, Teres D, Artigas A: Customized probability models for early severe sepsis in adult intensive care patients. Intensive Care Unit Scoring Group. JAMA 1995; 273: 644-650
7. Increase in National Hospital Discharge Survey rates for septicemia--United States, 1979-1987. MMWR Morb Mortal Wkly Rep 1990 ; 39: 31-34
8. Pittet D, Tarara D, Wenzel RP: Nosocomial bloodstream infection in critically ill patients. Excess length of stay, extra costs, and attributable mortality. JAMA 1994; 271: 1598-1601
9. Vincent JL, Angus D, Annane D, et al. (2001) Clinical expert round table discussion (session 5) at the Margaux Conference on Critical Illness: outcomes of clinical trials in sepsis: lessons learned. Crit Care Med 29:S136-137.
10. Southern EM (1974) An improved method for transferring nucleotides from electrophoresis strips to thin layers of ion-exchange cellulose. Anal Biochem 62:317-318
11. Gillespie D, Spiegelman S (1965) A quantitative assay for DNA-RNA hybrids with DNA immobilized on a membrane. J Mol Biol 12:829-842
12. Lennon GG, Lehrach H (1991) Hybridization analyses of arrayed cDNA libraries. Trends Genet 7: 314-317
13. Kafatos FC, Jones CW, Efstratiadis A (1979) Determination of nucleic acid sequence homologies and relative concentrations by a dot hybridization procedure. Nucl Acid Res 7:1541-1552
14. Fodor SP, Read JL, Pirrung MC, Stryer L, Lu AT, Solas D (1991) Lightdirected, spatially addressable parallel chemical synthesis. Science 251:767-773
15. Pease AC, Solas D, Sullivan EJ, Cronin MT, Holmes CP, Fodor SP (1994) Light-generated oligonucleotide arrays for rapid DNA sequence analysis. Proc Natl Acad Sci USA 91:5022-5026
16. Schena M, Shalon D, Davis RW, Brown PO (1995) Quantitative monitoring of gene expression patterns with a complementary DNA microarray. Science 270:467-470
17. Golub TR, Slonim DK, Tamayo P, et al. (1999) Molecular classification of cancer: class discovery and class prediction by gene expression monitoring. Science 286:531-537
18. Alizadeh AA, Eisen MB, Davis RE, et al. (2000) Distinct types of diffuse large B-cell lymphoma identified by gene expression profiling. Nature 403:503-511
19. Varambally S, Dhanasekaran SM, Zhou M, et al. (2002) The polycomb group protein EZH2 is involved in progression of prostate cancer. Nature 419:624-629
20. Fillion I, Ouellet N, Simard M, et al.(2002) Role of chemokines and formyl peptides in pneumococcal pneumonia-induced monocyte/macrophage recruitment. J Immunol.;166(12):7353-61.
21. Zhao B, Bowden RA, Stavchansky SA, Bowman PD (2001) Human endothelial cell response to gram-negative lipopolysaccharide assessed with cDNA microarrays. Am J Physiol Cell Physiol. Nov;281(5):C1587-95.
22. Chinnaiyan AM, Huber-Lang M, Kumar-Sinha C et al. (2001) Molecular signatures of sepsis: multiorgan gene expression profiles of systemic inflammation. Am J Pathol. 159(4):1199-209.

## Patentansprüche

1. Verfahren zur in vitro Erkennung von schwerer Sepsis,
**dadurch gekennzeichnet, dass**
es folgende Schritte umfasst:
a) Isolieren von Proben-RNA aus einer aus einem Menschen stammenden Probe;
b) Markieren der Proben-RNA und/oder wenigstens einer DNA, die ein für schwere Sepsis spezifisches Gen oder Genfragment ist, mit einem detektierbaren Marker;
c) In-Kontakt-Bringen der Proben-RNA mit der DNA unter Hybridisierungsbedingungen;
d) In-Kontakt-Bringen von Kontroll-RNA, welche eine Kontrolle für nichtpathologische Zustände darstellt, mit wenigstens einer DNA, unter Hybridisierungsbedingungen, wobei die DNA ein für schwere Sepsis spezifisches Gen oder Genfragment ist;
e) quantitatives Erfassen der Markierungssignale der hybridisierten Proben-RNA und der Kontroll-RNA;
f) Vergleichen der quantitativen Daten der Markierungssignale, um eine Aussage zu treffen, ob für schwere Sepsis spezifische Gene oder Genfragmente in der Probe stärker oder schwächer exprimiert sind als in der Kontrolle;
g) wobei das für schwere Sepsis spezifische Gen oder Genfragment ausgewählt wird aus der Gruppe bestehend aus SEQ-ID No. 1 bis SEQ-ID No. 130, sowie Genfragmenten davon mit 20-200 Nucleotiden.

2. Verfahren nach Anspruch 1, **dadurch gekennzeichnet, dass** man die Kontroll-RNA vor dem Messen der Proben-RNA mit der DNA hybridisiert und die Markierungssignale des Kontroll-RNA/DNA-Komplexes erfasst und gegebenenfalls in Form einer Kalibrierkurve oder -tabelle ablegt.

3. Verfahren nach Anspruch 1 oder 2, **dadurch gekennzeichnet, daß** nicht veränderte Gene aus der Proben- und/oder Kontroll-RNA als Bezugsgene für die Quantifizierung genutzt werden.

4. Verfahren nach einem der Ansprüche 1 bis 3, **dadurch gekennzeichnet, daß** als Proben-RNA mRNA verwendet wird.

5. Verfahren nach einem der Ansprüche 1 bis 4, **dadurch gekennzeichnet, daß** die DNA an vorbestimmten Bereichen auf einem Träger in Form eines Microarrays angeordnet, insbesondere immobilisiert, wird.

6. Verfahren nach einem der Ansprüche 1 bis 5, **dadurch gekennzeichnet, daß** das Verfahren zur differentialdiagnostischen Früherkennung, zur Kontrolle des klinischen Verlaufs, zur individuellen Risikoabschätzung für Patienten, zur Abschätzung des wahrscheinlichen Ansprechens auf eine spezifische Behandlung sowie zur post mortem Diagnose von schwerer Sepsis, eingesetzt wird.

7. Verfahren nach einem der Ansprüche 1 bis 6, **dadurch gekennzeichnet, daß** die Probe ausgewählt wird aus: Körperflüssigkeiten, insbesondere Blut, Liquor, Urin, Ascitesflüssigkeit, Seminalflüssigkeit, Speichel, Punktat; Zellinhalt oder eine Mischung davon.

8. Verfahren nach einem der Ansprüche 1 bis 7, **dadurch gekennzeichnet, daß** Zellproben gegebenenfalls einer lytischen Behandlung unterzogen werden, um deren Zellinhalte freizusetzen.

9. Verfahren nach einem der Ansprüche 1 bis 8, **dadurch gekennzeichnet, daß** die Genfragmente 20-80 Nukleotide aufweisen.

10. Verfahren nach einem der Ansprüche 1 bis 9 **dadurch gekennzeichnet, daß** wenigstens 2 bis 100 unterschiedliche cDNAs verwendet werden.

11. Verfahren nach einem der Ansprüche 1 bis 10, **dadurch gekennzeichnet, daß** als detektierbarer Marker ein radioaktiver Marker, insbesondere ³²P, ¹⁴C, ¹²⁵I, ¹⁵⁵Eu, ³³P oder ³H verwendet wird.

12. Verfahren nach einem der Ansprüche 1 bis 11, **dadurch gekennzeichnet, daß** als detektierbarer Marker ein nicht radioaktiver Marker, insbesondere ein Farb- oder Fluoreszenzmarker, ein Enzymmarker oder Immunmarker, und/oder quantum dots oder ein elektrisch messbares Signal, insbesondere Potential- und/oder Leitfähigkeits- und/oder Kapazitätsänderung bei Hybridisierungen, verwendet wird.

13. Verfahren nach einem der Ansprüche 1 bis 12, **dadurch gekennzeichnet, daß** die Proben-RNA und Kontroll-RNA dieselbe Markierung tragen.

14. Verfahren nach einem der Ansprüche 1 bis 13, **dadurch gekennzeichnet, daß** die Proben-RNA und Kontroll-RNA unterschiedliche Markierungen tragen.

15. Verfahren nach einem der Ansprüche 1 bis 14, **dadurch gekennzeichnet, dass** die immobilisierten Sonden eine Markierung tragen.

16. Verfahren nach einem der Ansprüche 1 bis 15 **dadurch gekennzeichnet, daß** die cDNA-Sonden auf Glas oder Kunststoff immobilisiert werden, wobei die einzelnen cDNA Moleküle über eine kovalente Bindung und/oder mittels Adsorption, insbesondere mittels elektrostatischer- und/oder Dipol-Dipol- und/oder hydrophobe Wechselwirkungen und/oder Wasserstoffbrücken an das Trägermaterial immobilisiert werden.
